# EUROPEAN PATENT APPLICATION

(11) **EP 1 688 420 A1**
(43) Date of publication of application: **09.08.2006**
(21) Application number: 04799807.5
(22) Date of filing: 18.11.2004
(51) Int. Cl.: C07D 495/04, A61K 31/407, A61K 31/4188, A61K 31/454, A61K 31/496, A61K 31/5377, A61K 38/21, A61P 1/16, A61P 31/14, A61P 43/00

(54) **5-5-MEMBERED FUSED HETEROCYCLIC COMPOUND AND USE THEREOF AS HCV POLYMERASE INHIBITOR**

(30) Priority: 19.11.2003 JP 2003389976
(71) Applicant: Japan Tobacco Inc., Tokyo 105-8422 (JP)
(72) Inventor: MIZOJIRI,Ryo. Japan Tobacco Inc., Takatsuki-shi, Osaka 569-1125 (JP); HIRASHIMA, Shintaro, Japan Tobacco Inc., Takatsuki-shi, Osaka 569-1125 (JP); OKA, Takahiro, Japan Tobacco Inc., Tatkatsuki-shi, Osaka 569-1125 (JP); AOKI, Kenta, Japan Tobacco Inc., Takatsuki-shi, Osaka 569-1125 (JP); NOJI, Satoru, Japan Tobacco Inc., Takatsuki-shi, Osaka 569-1125 (JP); ANDO, Izuru, Japan Tobacco Inc., Takatsuki-shi, Osaka 569-1125 (JP); SATO, Toshihiro, Japan Tobacco Inc., Takatsuki-shi, Osaka 569-1125 (JP); NIWA, Yasushi, Japan Tobacco Inc., Takatsuki-shi, Osaka 569-1125 (JP)
(74) Representative: Vossius & Partner
(86) International application number: PCT/JP2004/017518
(87) International publication number: WO 2005/049622

(57) **Abstract**

The present invention relates to a fused ring compound represented by the following formula [I] 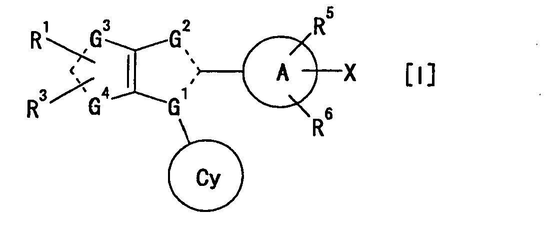 wherein each symbol is as defined in the specification, or a pharmaceutically acceptable a salt thereof, and a hepatitis C virus (HCV) polymerase inhibitor and a therapeutic agent for hepatitis C containing this compound. The compound of the present invention shows an anti-HCV activity based on the HCV polymerase inhibitory activity, and useful as an agent for the prophylaxis or treatment of hepatitis C.

## Description

### Technical Field

The present invention relates to a fused ring compound or a pharmaceutically acceptable salt thereof, which shows anti-hepatitis C virus (HCV) activity, particularly anti-HCV activity based on an RNA-dependent RNA polymerase inhibitory activity. In addition, the present invention relates to a hepatitis C virus polymerase inhibitor, an anti-hepatitis C virus agent and a therapeutic agent for hepatitis C containing said fused ring compound or a pharmaceutically acceptable salt thereof.

### Background Art

In 1989, a main causative virus of non-A non-B posttransfusion hepatitis was found and named hepatitis C virus (HCV). Since then, several types of hepatitis viruses have been found besides type A, type B and type C, wherein hepatitis caused by HCV is called hepatitis C.

The patients infected with HCV are considered to involve several percent of the world population, and the infection with HCV characteristically becomes chronic.

HCV is an envelope RNA virus, wherein the genome is a single strand plus-strand RNA, and belongs to the genus Hepacivirus of Flavivirus (from The International Committee on Taxonomy of Viruses, International Union of Microbiological Societies). Of the same hepatitis viruses, for example, hepatitis B virus (HBV), which is a DNA virus, is eliminated by the immune system and the infection with this virus ends in an acute infection except for neonates and infants having yet immature immunological competence. In contrast, HCV somehow avoids the immune system of the host due to an unknown mechanism. Once infected with this virus, even an adult having a mature immune system frequently develops persistent infection.

When chronic hepatitis is associated with the persistent infection with HCV, it advances to cirrhosis or hepatic cancer in a high rate. Enucleation of tumor by operation does not help much, because the patient often develops recurrent hepatic cancer due to the sequela inflammation in non-cancerous parts. In addition, there is a report on the involvement of HCV infection in dermatosis such as chronic urticaria, lichen planus, cryoglobulinemic purpura and the like (The Japanese Journal of Dermatology, Vol. 111, No. 7, pages 1075-1081, 2001).

Thus, an effective therapeutic method of hepatitis C is desired. Apart from the symptomatic therapy to suppress inflammation with an anti-inflammatory agent, the development of a therapeutic agent that reduces HCV to a low level free from inflammation and that eradicates HCV has been strongly demanded.

At present, a treatment with interferon is the only effective method known for the eradication of HCV. However, interferon can eradicate the virus only in about one-third of the patient population. For the rest of the patients, it has no effect or provides only a temporary effect. In recent years, polyethylene glycolated interferon has been put to practical use, and enhanced effects and reduced side effects have been achieved. However, complete response rate still remains at a low level, and therefore, an anti-HCV drug to be used in the place of or concurrently with interferon is awaited in great expectation.

In recent years, Ribavirin (1-β-D-ribofuranosyl-1H-1,2,4-triazole-3-carboxamide) has become commercially available as a therapeutic agent for hepatitis C, which is to be used concurrently with interferon. It enhances the efficacy of interferon but only to a low efficacy rate, and a different novel therapeutic agent for hepatitis C is desired.

Also, an attempt has been made to potentiate the immunocompetence of the patient with an interferon agonist, an interleukin-12 agonist and the like, thereby to eradicate the virus, but an effective pharmaceutical agent has not been found yet.

In addition, the inhibition of HCV growth, wherein HCV-specific protein is targeted, has been drawing attention these days.

The gene of HCV encodes a protein such as serine protease, RNA helicase, RNA-dependent RNA polymerase and the like. These proteins function as a specific protein essential for the growth of HCV.

One of the specific proteins, RNA-dependent RNA polymerase (hereinafter to be also briefly referred to as an HCV polymerase), is an enzyme essential for the growth of the virus. The gene replication of HCV having a plus-strand RNA gene is considered to involve synthesis of a complementary minus-strand RNA by the use of the plus-strand RNA as a template and using the obtained minus-strand RNA as a template, amplifying the plus-strand RNA. The portion called NS5B of a protein precursor, that HCV codes for, has been found to show an RNA-dependent RNA polymerase activity (EMBO J., Vol. 15, pages 12-22, 1996), and is considered to play a central role in the HCV gene replication.

Therefore, an HCV polymerase inhibitor can be a target in the development of an anti-HCV drug, and the development thereof is eagerly awaited. However, an effective HCV polymerase inhibitor has not been developed yet, like in other attempts to develop an anti-HCV drug based on other action mechanisms. As the situation stands, no pharmaceutical agent can treat hepatitis C satisfactorily.

The following describes known compounds comparatively similar to the present invention.

As the thieno[3,2-b]pyrrole derivative, the following compound a etc. are known (Chemistry of Heterocyclic Compounds, pages 1133-1136, 1976 (compound IIc (page 1134, Table 1))).

This reference includes descriptions relating to the synthetic methods of thieno[3,2-b]pyrrole derivative, but does not disclose the compound of the present invention, nor does it contain a description relating to use as a pharmaceutical product. In addition, it does not contain a description suggesting such use.

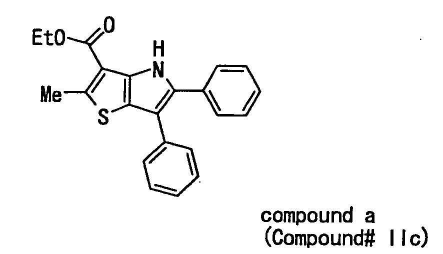

As a therapeutic agent for hepatitis C having an indole skeleton, WO03/010140 is known.

In this publication, as an anti-HCV agent having a polymerase inhibitory activity, the following indole compounds A, B, C, D etc. are described (Example Nos. 1 (page 41), 10 (page 51), 14 (page 57), compound No. 149 (page 79)). 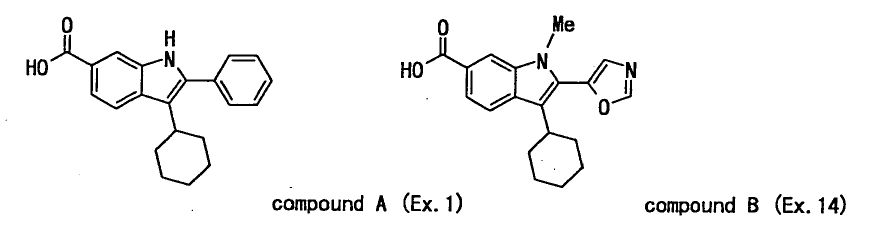 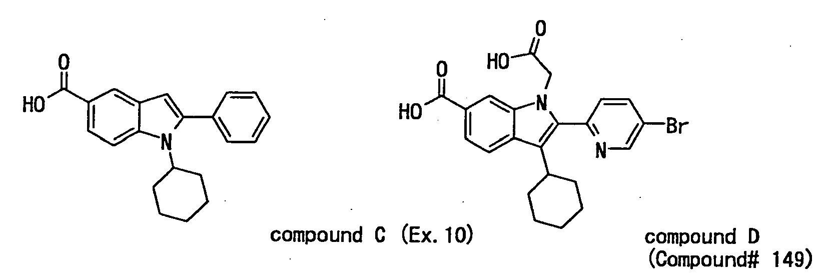 wherein Ex. means Example No. in the publication.

In this publication, as compounds having other skeleton, the following compounds E, F, G etc. are described (Example Nos. 18 (page 60), 20 (page 63), 22 (page 64)).

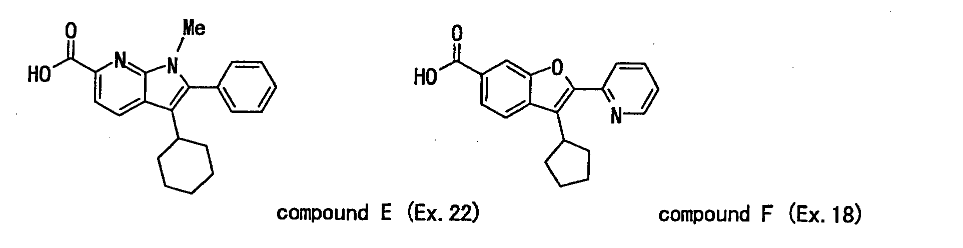

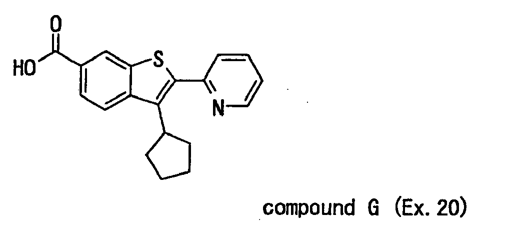

In WO03/010141, as a synthetic intermediate for an anti-HCV agent having a polymerase inhibitory activity, the above-mentioned compounds etc. are described (page 92, page 101, page 108, page 112, page 115, page 116).

Furthermore, JP-A-2001-247550 (WO01/47883, EP1162196A1, US2003/0050320) and WO03/000254 (US2003/0050320) describe, as an anti-HCV agent having a polymerase inhibitory activity, the following indole compound H etc., benzimidazole compound I etc. (WO03/000254, Example compound Nos. 502 (page 206), 1198 (page 315)).

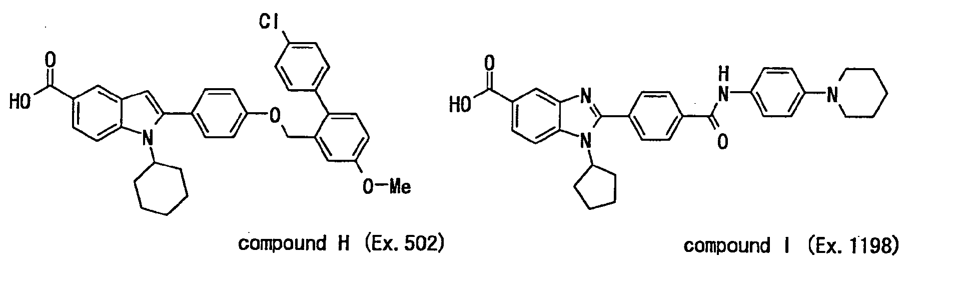

This publication also describes the following compound J etc. as compounds having other skeletons (WO03/000254, Example compound No. 701 (page 417)).

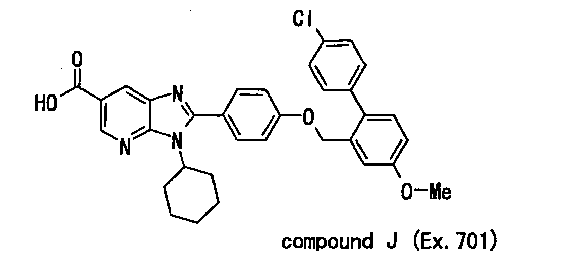

The above-mentioned WO03/000254 further describes the following benzimidazole compounds K, L, M, N, O etc. (Example compound Nos. 371 (page 468), 405 (page 479), 407 (page 480), 423, 424 (page 485)).

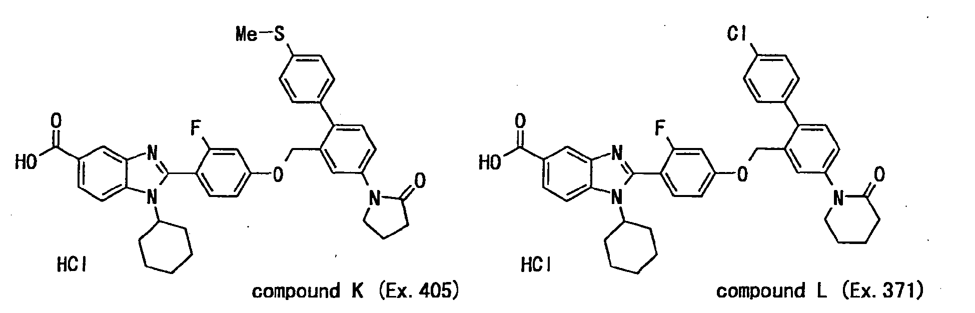

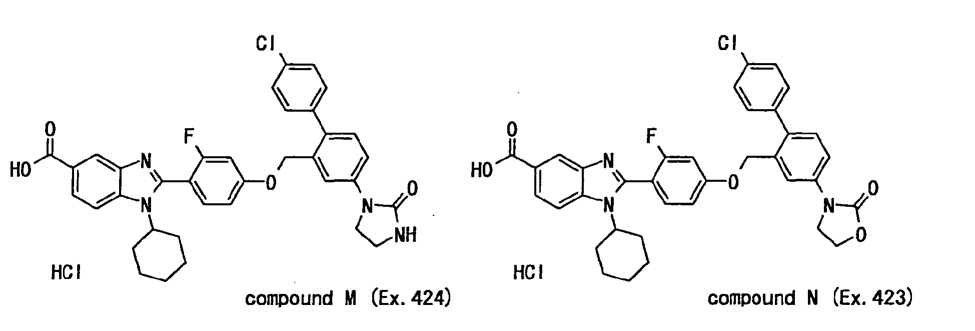

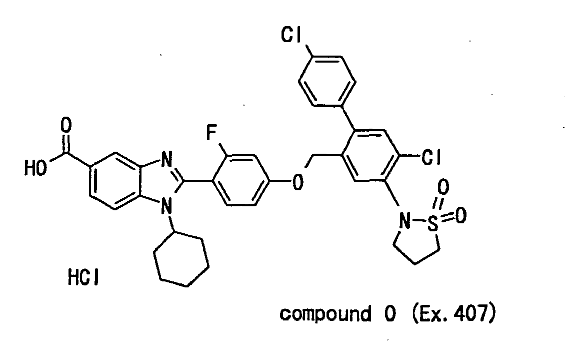

In addition, WO02/04425 describes the following benzimidazole compound P etc. as anti-HCV agents having a polymerase inhibitory activity (entry No. 7005 (page 228)).

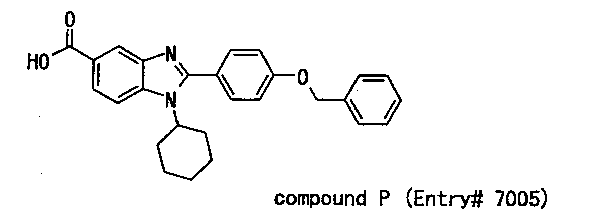

In this publication, the following compounds Q, R etc. are described as compounds having other skeletons (Example Nos. 28 (page 84), 148 (page 163)).

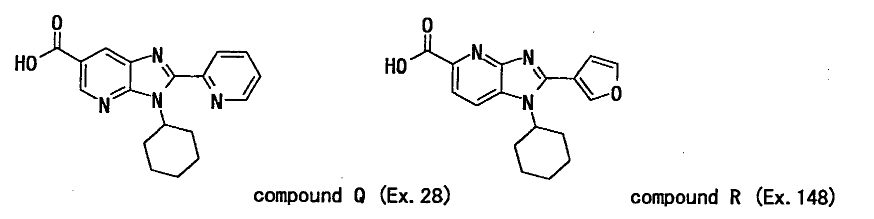

WO03/026587 also discloses the following compounds S, T etc. as anti-HCV agents having a polymerase inhibitory activity (Example Nos. 12 (page 56), 65 (page 65)).

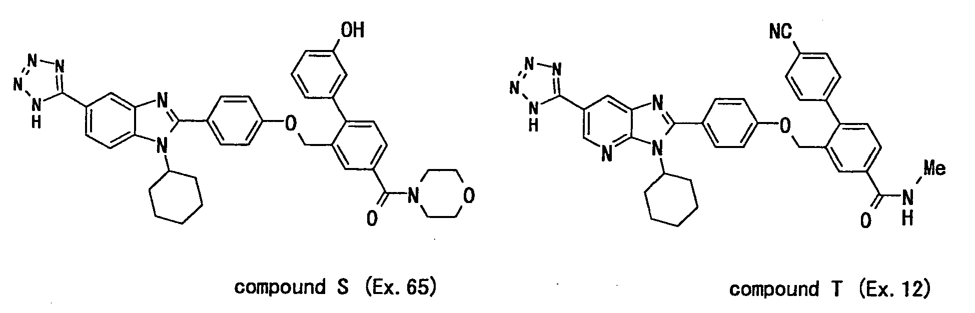

As therapeutic agents for hepatitis C having a benzimidazole skeleton, the compounds described in WO97/36866, JP-A-2000-511899 (EP906097) and WO99/51619 are also known.

WO03/007945 also describes benzimidazole compound etc. as synthetic intermediates for anti-HCV agents having a polymerase inhibitory activity.

Furthermore, WO99/09007 and US5932743 describe the following indole compound U etc. as chemical library compounds that can be used for screening of pharmaceutical products (WO99/09007, Example 12 (page 25)).

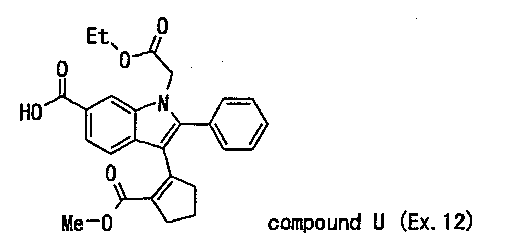

In addition, WO2004/065367 describes the following compounds V, W etc. as anti-HCV agents having a polymerase inhibitory activity (compound Nos. 1023 (page 164), Example 29 (page 140)).

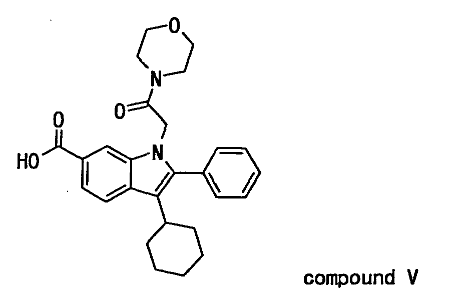

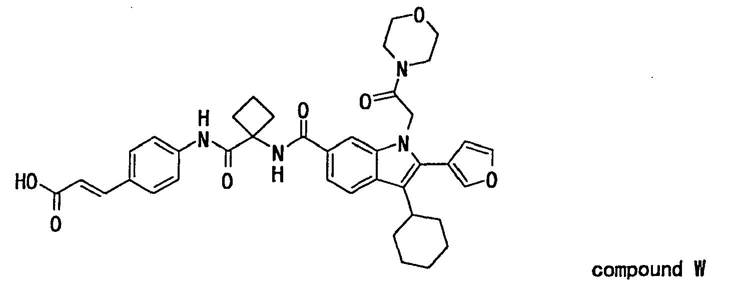

In addition, WO2004/064925 describes the following compounds X etc. as anti-HCV agents having a polymerase inhibitory activity (compound No. 101 (page 50)).

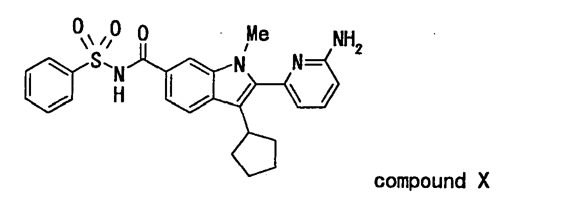

Moreover, WO2004/087714 describes the following compounds Y, Z etc. as anti-HCV agents having a polymerase inhibitory activity (compounds in Table 2 (page 92), compounds in Table 3 (page 98)).

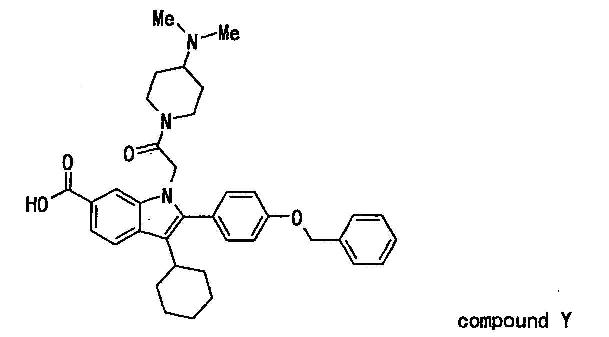

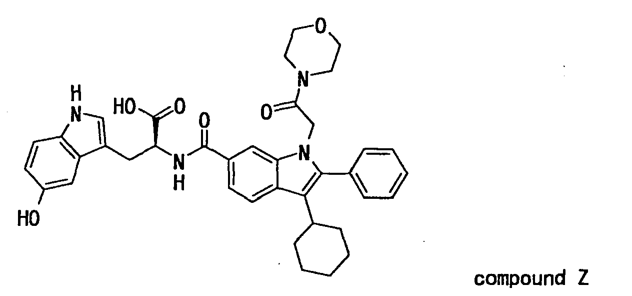

However, none of these publications do not describe the 5-5-membered fused heterocyclic compound of the present invention, nor do they include any description suggestive thereof.

### Disclosure of the Invention

Based on the findings from the preceding studies, it has been elucidated that a compound having an anti-HCV activity is effective for the prophylaxis and treatment of hepatitis C, and particularly an anti-HCV agent having an inhibitory activity on RNA-dependent RNA polymerase of HCV can be a prophylactic and therapeutic agent effective against hepatitis C and a prophylactic and therapeutic agent for the disease caused by hepatitis C.

Accordingly, the present invention provides a compound having an anti-HCV activity, particularly a compound having an RNA-dependent RNA polymerase inhibitory activity.

The present inventors have made an in-depth study of compounds having an anti-HCV activity, particularly RNA-dependent RNA polymerase inhibitory activity, and completed the present invention.

More particularly, the present invention provides the following [1] to [47].

### [1] A fused ring compound represented by the following formula

### [I] or a pharmaceutically acceptable salt thereof:

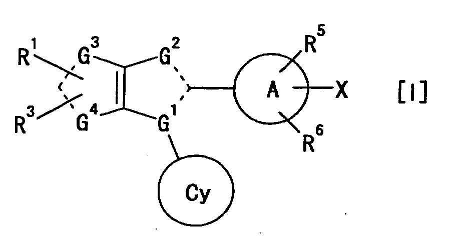

wherein 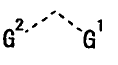 is G²'=C-G¹ or G²-C=G¹', 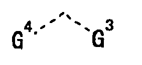 is G⁴'=C-G³ or G⁴-C=G³',
G¹ is a carbon atom or a nitrogen atom,
G¹' is a carbon atom,
G², G³ and G⁴ are each independently an oxygen atom, a sulfur atom, a nitrogen atom or a carbon atom,
G²', G³' and G⁴' are each independently a nitrogen atom or a carbon atom,
provided that when G², G³ and G⁴ are all carbon atoms, G¹ is a nitrogen atom,
when G² is a nitrogen atom or a carbon atom, G² is optionally substituted by the following R²,
R¹ and R³ optionally substitute any atom of 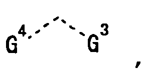 provided that when G³ is an oxygen atom or a sulfur atom, G³ is unsubstituted, and when G⁴ is an oxygen atom or a sulfur atom, G⁴ is unsubstituted,
R¹ is
(1) a carboxyl group,
(2) a carboxylic acid equivalent,
(3) -CONR¹¹R¹²
   (wherein R¹¹ and R¹² are each independently
   (1') a hydrogen atom,
   (2') a C₁₋₆ alkyl group optionally substituted by 1 to 3 substituents selected from the following group E,
   (3') a C₂₋₆ alkenyl group optionally substituted by 1 to 3 substituents selected from the following group E,
   (4') a C₆₋₁₄ aryl group optionally substituted by 1 to 5 substituents selected from the following group E,
   (5') a heterocyclic group optionally substituted by 1 to 5 substituents selected from the following group E or
   (6') a C₃₋₈ cycloalkyl group optionally substituted by 1 to 5 substituents selected from the following group E) or
(4) -COOR¹⁰³
   (wherein R¹⁰³ is a group selected from the following group C or a glucuronic acid residue),
   R² is
   (1) a hydrogen atom,
   (2) a group selected from the following group E,
   (3) a C₁₋₆ alkyl group optionally substituted by 1 to 3 substituents selected from the following group E,
   (4) a C₂₋₆ alkenyl group optionally substituted by 1 to 3 substituents selected from the following group E,
(5) 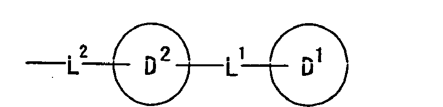
(6) 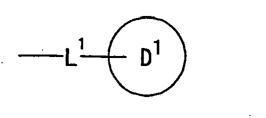
(7) 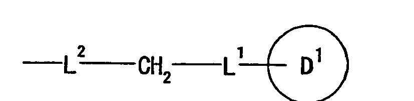 or
(8) 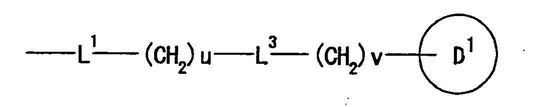 {wherein u and v are each independently 0 or an integer of 1 to 6,
L¹ and L² are each independently
(1') a bond,
(2') C₁₋₆ alkylene,
(3') C₂₋₆ alkenylene,
(4') -(CHR^{L4})ᵤ₁-O-(CHR^{L5})ᵥ₁-,
(5') -(CHR^{L4})ᵤ₁-S-(CHR^{L5})ᵥ₁-,
(6') -(CHR^{L4})ᵤ₁-NR^{L1}-(CHR^{L5})ᵥ₁-,
(7') -(CHR^{L4})ᵤ₁-CO-(CHR^{L5})ᵥ₁-,
(8') -(CHR^{L4})ᵤ₁-CONR^{L2}-(CHR^{L5})ᵥ₁-,
(9') -(CHR^{L4})ᵤ₁-NR^{L2}CO₂-(CHR^{L5})ᵥ₁-,
(10') -(CHR^{L4})ᵤ₁-NR^{L2}CONR^{L3}-(CHR^{L5})ᵥ₁-,
(11') -(CHR^{L4})ᵤ₁-NR^{L2}CO-(CHR^{L5})ᵥ₁-,
(12') -(CHR^{L4})ᵤ₁-NR^{L2}SO₂-(CHR^{L5})ᵥ₁-,
(13') -(CHR^{L4})ᵤ₁-SO₂-(CHR^{L5})ᵥ₁- or
( 14') - (CHR^{L4})ᵤ₁-SO₂NR^{L2}-(CHR^{L5})ᵥ₁-
(wherein u1 and v1 are each independently 0 or an integer of 1 to 6,
R^{L1} is
(1") a hydrogen atom,
(2") a group selected from the following group C,
(3") -COR^{L11},
(4") -CONR^{L11}R^{L12},
(5") -COOR^{L11} or
(6") -SO₂R^{L13}
(wherein R^{L11} and R^{L12} are each independently a hydrogen atom or a group selected from the following group C, and R^{L13} is a group selected from the following group C),
R^{L2} and R^{L3} are each independently
(1") a hydrogen atom,
(2") a group selected from the following group C,
(3") -COR^{L11} or
(4") -SO₂R^{L13}
(wherein R^{L11} and R^{L13} are as defined above),
R^{L4} and R^{L5} are each independently a hydrogen atom or a C₁₋₆ alkyl group),
L³ is
(1') -CHR^{L14}- or
(2') -NR^{L14}-
(wherein R^{L14} is a group selected from the following group F), ring D¹ and ring D² are each independently
(1') a C₆₋₁₄ aryl group optionally substituted by 1 to 5 substituents selected from the following group E,
(2') a C₃₋₈ cycloalkyl group optionally substituted by 1 to 5 substituents selected from the following group E or
(3') a heterocyclic group optionally substituted by 1 to 5 substituents selected from the following group E
(wherein said heterocyclic group comprises 1 to 4 heteroatoms selected from oxygen atom, nitrogen atom and sulfur atom)},
R³ is independently a group selected from the following (1) to (20) :
(1) a hydrogen atom,
(2) a halogen atom,
(3) a C₁₋₆ alkanoyl group,
(4) a carboxyl group,
(5) a cyano group,
(6) a nitro group,
(7) a C₁₋₆ alkyl group optionally substituted by 1 to 3 substituents selected from the following group A,
(8) -OR¹⁰¹
   (wherein R¹⁰¹ is a hydrogen atom or a group selected from the following group C),
(9) -NR¹⁰²R¹¹⁹
   (wherein R¹⁰² and R¹¹⁹ are each independently a hydrogen atom, a C₁₋₆ alkanoyl group or a C₁₋₆ alkylsulfonyl group),
(10) -COOR¹⁰³
   (wherein R¹⁰³ is a group selected from the following group C or a glucuronic acid residue),
(11) -CONR¹⁰⁴R¹⁰⁵
   (wherein R¹⁰⁴ and R¹⁰⁵ are each independently a hydrogen atom, a hydroxyl group, a cyano group, a C₁₋₆ alkoxy group or a C₁₋₆ alkyl group optionally substituted by 1 to 3 substituents selected from the following group A),
(12) -SO₂R¹⁰⁶
   (wherein R¹⁰⁶ is a hydroxyl group, an amino group, a C₁₋₆ alkyl group or a C₁₋₆ alkylamino group),
(13) -NHCOR¹⁰⁷
   (wherein R¹⁰⁷ is an amino group or a C₁₋₆ alkylamino group),
(14) -C (=NR¹⁰⁸)-NH₂
   (wherein R¹⁰⁸ is a hydrogen atom, a C₁₋₆ alkyl group optionally substituted by 1 to 3 substituents selected from the following group A, a hydroxyl group or a C₁₋₆ alkoxy group),
(15) -P(=O)(OR¹⁰⁹)₂
   (wherein each R¹⁰⁹ is independently a hydrogen atom or a group selected from the following group C),
(16) -P(=O)(OR¹¹⁰)NR¹¹¹R¹¹²
   (wherein R¹¹⁰, R¹¹¹ and R¹¹² are each independently a hydrogen atom or a group selected from the following group C),
(17) -CONHCO-R¹¹³
   (wherein R¹¹³ is a group selected from the following group C),
(18) -CONHSO₂-R¹¹⁴
   (wherein R¹¹⁴ is a group selected from the following group C),
(19) -SO₂NHCO-R¹¹⁵
   (wherein R¹¹⁵ is a group selected from the following group C) or (20) a heterocyclic group optionally substituted by 1 to 5 substituents selected from the following group B
(wherein said heterocyclic group comprises 1 to 4 heteroatoms selected from oxygen atom, nitrogen atom and sulfur atom), ring Cy is
(1) a C₃₋₈ cycloalkyl group optionally substituted by 1 to 5 substituents selected from the following group B,
(2) a C₃₋₈ cycloalkenyl group optionally substituted by 1 to 5 substituents selected from the following group B or
(3) a heterocyclic group optionally substituted by 1 to 5 substituents selected from the following group B
(wherein said heterocyclic group comprises 1 to 4 heteroatoms selected from oxygen atom, nitrogen atom and sulfur atom),
ring A is
(1) a C₆₋₁₄ aryl group,
(2) a C₃₋₈ cycloalkyl group,
(3) a C₃₋₈ cycloalkenyl group or
(4) a heterocyclic group comprising 1 to 4 heteroatoms selected from oxygen atom, nitrogen atom and sulfur atom,
R⁵ and R⁶ are each independently
(1) a hydrogen atom,
(2) a halogen atom,
(3) a C₁₋₆ alkyl group optionally substituted by 1 to 3 substituents selected from the following group A,
(4) -OR¹¹⁶
   (wherein R¹¹⁶ is a hydrogen atom or a group selected from the following group C) or
(5) -NR¹¹⁷R¹¹⁸
(wherein R¹¹⁷ and R¹¹⁸ are each independently a hydrogen atom, a C₁₋₆ alkanoyl group or a group selected from the following group C),
X is
(1) a hydrogen atom,
(2) a halogen atom,
(3) a cyano group,
(4) a nitro group,
(5) an amino group,
(6) a C₁₋₆ alkanoylamino group,
(7) a C₁₋₆ alkylsulfonyl group,
(8) a C₁₋₆ alkyl group optionally substituted by 1 to 3 substituents selected from the following group A,
(9) a C₂₋₆ alkenyl group optionally substituted by 1 to 3 substituents selected from the following group A,
(10) -COOR^{a9}
   (wherein R^{a9} is a hydrogen atom or a C₁₋₆ alkyl group),
(11) -CONH- (CH₂)₁-R^{a10}
   (wherein R^{a10} is a C₁₋₆ alkyl group optionally substituted by 1 to 3 substituents selected from the following group A, a C₁₋₆ alkoxycarbonyl group or a C₁₋₆ alkanoylamino group, and 1 is 0 or an integer of 1 to 6),
(12) -OR^{a11}
   (wherein R^{a11} is a hydrogen atom or a C₁₋₆ alkyl group optionally substituted by 1 to 3 substituents selected from the following group A) or
(13) 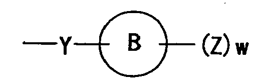
wherein ring B is
(1') a C₆₋₁₄ aryl group,
(2') a C₃₋₈ cycloalkyl group or
(3') a heterocyclic group comprising 1 to 4 heteroatoms selected from oxygen atom, nitrogen atom and sulfur atom,
each Z is independently
(1') a group selected from the following group D,
(2') a C₆₋₁₄ aryl group optionally substituted by 1 to 5 substituents selected from the following group D,
(3') a C₃₋₈ cycloalkyl group optionally substituted by 1 to 5 substituents selected from the following group D,
(4') a C₆₋₁₄ aryl C₁₋₆ alkyl group optionally substituted by 1 to 5 substituents selected from the following group D,
(5') a heterocyclic group optionally substituted by 1 to 5 substituents selected from the following group D
   (wherein said heterocyclic group comprises 1 to 4 heteroatoms selected from oxygen atom, nitrogen atom and sulfur atom) or
(6') a heterocycle C₁₋₆ alkyl group optionally substituted by 1 to 5 substituents selected from the following group D
(wherein said heterocycle C₁₋₆ alkyl group is a C₁₋₆ alkyl group substituted by "a heterocyclic group optionally substituted by 1 to 5 substituents selected from group D" as defined above),
w is an integer of 1 to 3,
Y is
(a) C₁₋₆ alkylene,
(b) C₂₋₆ alkenylene or
(c) -Y¹-(CH₂)ₘ-Y²-(CH₂)ₙ-
(wherein m and n are each independently 0 or an integer of 1 to 6,
Y¹ and Y² are each independently
(1') a bond,
(2') -O-,
(3') -NR^{y1}-,
(4') -S-,
(5') -CO-,
(6') -SO-,
(7') -SO₂-,
(8') -CO₂-,
(9') -OCO-,
(10') -CONR^{y2}-,
(11') -NR^{y2}CO-,
(12') -SO₂NR^{y2}-,
(13') -NR^{y2}SO₂-,
(14') -NR^{y2}CO₂-,
(15') -OCONR^{y2}-,
(16') -NR^{y2}CONR^{y3}-,
(17') -CR^{y4}R^{y5}- or
(18') -CH=CH-
(wherein R^{y1} is
(1") a hydrogen atom,
(2") a group selected from the following group C,
(3") -COOR^{y11},
(4") -CONR^{y11}R^{y12},
(5") -COR^{y11} or
(6") -SO₂R^{y13}
(wherein R^{y11} and R^{y12} are each independently a hydrogen atom or a group selected from the following group C, and R^{y13} is a group selected from the following group C),
R^{y2} and R^{y3} are each independently
(1") a hydrogen atom,
(2") a group selected from the following group C,
(3") -COR^{y11} or
(4") -SO₂R^{y13}
(wherein R^{y11} and R^{y13} are as defined above) ,
R^{y4} and R^{y5} are each independently
(1") a hydrogen atom,
(2") a carboxyl group,
(3") a group selected from group F,
(4") -OR^{y14} or
(5") -NHR^{y15}
(wherein R^{y14} is a group selected from the following group C, and
R^{y15} is a hydrogen atom, a C₁₋₆ alkyl group, a C₁₋₆ alkanoyl group or a C₆₋₁₄ aryl C₁₋₆ alkyloxycarbonyl group))) group A;
(1) a halogen atom,
(2) a C₁₋₆ alkoxy C₁₋₆ alkoxy group,
(3) -OR^{a1},
(4) -SR^{a1},
(5) -NR^{a1}R^{a2},
(6) -COOR^{a1},
(7) -CONR^{a1}R^{a2},
(8) -SO₃H,
(9) -SO₂NR^{a1}R^{a2},
(10) -NHCOR^{a1} and
(11) -NHSO₂R^{a3}
(wherein R^{a1} and R^{a2} are each independently a hydrogen atom or a C₁₋₆ alkyl group, and R^{a3} is a C₁₋₆ alkyl group)
group B;
(1) a halogen atom,
(2) a cyano group,
(3) a nitro group,
(4) a C₁₋₆ alkyl group,
(5) a halogenated C₁₋₆ alkyl group,
(6) -(CH₂)ᵣ-OR^{b1},
(7) -(CH₂)ᵣ-SR^{b1},
(8) -(CH₂)ᵣ-NR^{b1}R^{b2},
(9) -(CH₂)ᵣ-COOR^{b1},
(10) -(CH₂)ᵣ-CONR^{b1}R^{b2},
(11) -(CH₂)ᵣ-COR^{b1},
(12) - (CH₂)ᵣ-NR^{b1}-COR^{b2},
(13) -(CH₂)ᵣ-NR^{b1}-SO₂R^{b3},
(14) -(CH₂)ᵣ-SO₂R^{b3},
(15) -(CH₂)ᵣ-SO₂NR^{b1}R^{b2},
(16) -(CH₂)ᵣ-CONR^{b1}-SO₂R^{b3},
(17) - (CH₂)ᵣ-SO₂NR^{b1}-COR^{b2},
(18) -(CH₂)ᵣ-NR^{b1}-COOR^{b3} and
(19) - (CH₂)ᵣ-NR^{b1}-CONR^{b2}R^{b4}
(wherein R^{b1}, R^{b2} and R^{b4} are each independently a hydrogen atom or a C₁₋₆ alkyl group, R^{b3} is a C₁₋₆ alkyl group, and r is 0 or an integer of 1 to 6)
group C:
(1) a C₁₋₆ alkyl group optionally substituted by 1 to 3 substituents selected from the aforementioned group A,
(2) a C₆₋₁₄ aryl group optionally substituted by 1 to 5 substituents selected from the aforementioned group B,
(3) a C₆₋₁₄ aryl C₁₋₆ alkyl group optionally substituted by 1 to 5 substituents selected from the aforementioned group B,
(4) a heterocyclic group optionally substituted by 1 to 5 substituents selected from the aforementioned group B and
(5) a heterocycle C₁₋₆ alkyl group optionally substituted by 1 to 5 substituents selected from the aforementioned group B
group D:
(a) a hydrogen atom,
(b) a halogen atom,
(c) a cyano group,
(d) a nitro group,
(e) a C₁₋₆ alkyl group optionally substituted by 1 to 3 substituents selected from the aforementioned group A,
(f) -(CH₂)ₜ-OR^{d1},
   wherein R^{d1} is
   (1) a hydrogen atom,
   (2) a group selected from the following group F,
   (3) a C₂₋₆ alkenyl group optionally substituted by 1 to 3 substituents selected from the aforementioned group A or
   (4) a C₂₋₆ alkynyl group optionally substituted by 1 to 3 substituents selected from the aforementioned group A, hereinafter each t is independently 0 or an integer of 1 to 6,
(g) -(CH₂)ₜ-S-(O)_{q}-R^{d2},
   wherein R^{d2} is
   (1) a hydrogen atom or
   (2) a group selected from the following group F, q is 0, 1, 2 or 3,
(h) -(CH₂)ₜNR^{d3}R^{d4},
   wherein R^{d3} and R^{d4} are each independently,
   (1) a hydrogen atom or
   (2) a group selected from the following group F,
(i) -(CH₂)ₜ-COOR^{d5},
   wherein R^{d5} is
   (1) a hydrogen atom or
   (2) a group selected from the following group F,
(j) -(CH₂)ₜ-CONR^{d6}R^{d7},
   wherein R^{d6} and R^{d7} are each independently
   (1) a hydrogen atom,
   (2) a hydroxyl group,
   (3) a group selected from the following group F or
   (4) a C₁₋₆ alkoxy group,
(k) -(CH₂)ₜ-COR^{d8},
   wherein R^{d8} is
   (1) a hydrogen atom or
   (2) a group selected from the following group F,
(l) -(CH₂)ₜ-NR^{d9}CO-R^{d10},
   wherein R^{d9} is
   (1) a hydrogen atom,
   (2) a C₁₋₆ alkyl group optionally substituted by 1 to 3 substituents selected from the aforementioned group A or
   (3) a C₁₋₆ alkanoyl group,
   R^{d10} is
   (1) a hydrogen atom,
   (2) an amino group,
   (3) a C₁₋₆ alkylamino group or
   (4) a group selected from the following group F,
(m) -(CH₂)ₜ-NR^{d11}SO₂-R^{d12},
   wherein R^{d11} is
   (1) a hydrogen atom,
   (2) a C₁₋₆ alkyl group optionally substituted by 1 to 3 substituents selected from the aforementioned group A or
   (3) a C₁₋₆ alkanoyl group,
   R^{d12} is
   (1) a hydrogen atom or
   (2) a group selected from the following group F,
(n) -(CH₂)ₜ-SO₂-NR^{d13}R^{d14},
   wherein R^{d13} and R^{d14} are each independently
   (1) a hydrogen atom or
   (2) a group selected from the following group F,
(o) -(CH₂)ₜ-CONR^{d15}-SO₂R^{d16},
   wherein R^{d15} and R^{d16} are each independently
   (1) a hydrogen atom or
   (2) a group selected from the following group F,
(p) -(CH₂)ₜSO₂NR^{d17}-COR^{d18},
   wherein R^{d17} is
   (1) a hydrogen atom or
   (2) a group selected from the following group F,
      R^{d18} is a group selected from the following group F,
(q) -(CH₂)ₜ-NR^{d19}-COOR^{d20},
   wherein R^{d19} and R^{d20} are each independently
   (1) a hydrogen atom or
   (2) a group selected from the following group F,
(r) - (CH₂)ₜ-NR^{d21}-CONR^{d22}R^{d23},
   wherein R^{d21}, R^{d22} and R^{d23} are each independently
   (1) a hydrogen atom or
   (2) a group selected from the following group F,
(s) -(CH₂)ₜ-C(=NR^{d24})NH₂,
   wherein R^{d24} is
   (1) a hydrogen atom,
   (2) a hydroxyl group,
   (3) a C₁₋₆ alkyl group optionally substituted by 1 to 3 substituents selected from the aforementioned group A or
   (4) a C₁₋₆ alkoxy group,
(t) -(CH₂)ₜ-O-(CH₂)ₚ-COR^{d25},
   wherein R^{d25} is
   (1) an amino group,
   (2) a C₁₋₆ alkylamino group or
   (3) a heterocyclic group optionally substituted by 1 to 5 substituents selected from the aforementioned group B,
   p is 0 or an integer of 1 to 6, and
(u) a heterocyclic group optionally substituted by 1 to 5 substituents selected from the aforementioned group B (wherein said heterocyclic group comprises 1 to 4 heteroatoms selected from oxygen atom, nitrogen atom and sulfur atom)
group E:
(a) a halogen atom,
(b) a cyano group,
(c) a nitro group,
(d) -OR^{e1},
   wherein R^{e1} is
   (1) a hydrogen atom,
   (2) a group selected from the following group F,
   (3) a C₂₋₆ alkenyl group optionally substituted by 1 to 3 substituents selected from the aforementioned group A or
   (4) a C₂₋₆ alkynyl group optionally substituted by 1 to 3 substituents selected from the aforementioned group A,
(e) -S(O)_{q}-R^{e2}
   wherein R^{e2} is
   (1) a hydrogen atom or
   (2) a group selected from the following group F,
      q is 0, 1, 2 or 3,
(f) -NR^{e3}R^{e4},
   wherein R^{e3} and R^{e4} are each independently
   (1) a hydrogen atom or
   (2) a group selected from the following group F,
(g) -COOR^{e5},
   wherein R^{e5} is
   (1) a hydrogen atom or
   (2) a group selected from the following group F,
(h) -CONR^{e6}R^{e7},
   wherein R^{e6} and R^{e7} are each independently
   (1) a hydrogen atom,
   (2) a hydroxyl group,
   (3) a group selected from the following group F or
   (4) a C₁₋₆ alkoxy group,
(i) -COR^{e8},
   wherein R^{e8} is
   (1) a hydrogen atom or
   (2) a group selected from the following group F,
(j) -NR^{e9}CO-R^{e10},
   whrein R^{e9} is
   (1) a hydrogen atom,
   (2) a C₁₋₆ alkyl group or
   (3) a C₁₋₆ alkanoyl group,
   R^{e10} is
   (1) a hydrogen atom,
   (2) an amino group,
   (3) a C₁₋₆ alkylamino group or
   (4) a group selected from the following group F,
(k) -NR^{e11}SO₂-R^{e12},
   wherein R^{e11} is
   (1) a hydrogen atom,
   (2) a C₁₋₆ alkyl group or
   (3) a C₁₋₆ alkanoyl group,
   R^{e12} is
   (1) a hydrogen atom or
   (2) a group selected from the following group F,
(1) -SO₂-NR^{e13}R^{e14},
   wherein R^{e13} and R^{e14} are each independently
   (1) a hydrogen atom or
   (2) a group selected from the following group F,
(m) -CONR^{e15}-SO₂R^{e16},
   wherein R^{e15} and R^{e16} are each independently
   (1) a hydrogen atom or
   (2) a group selected from the following group F,
(n) -SO₂NR^{e17}-COR^{e18},
   wherein R^{e17} is
   (1) a hydrogen atom or
   (2) a group selected from the following group F,
      R^{e18} is a group selected from the following group F,
(o) -NR^{e19}-COOR^{e20},
   whrein R^{e19} and R^{e20} are each independently
   (1) a hydrogen atom or
   (2) a group selected from the following group F,
(p) -NR^{e21}-CONR^{e22}R^{e23}
   wherein R^{e21}, R^{e22} and R^{e23} are each independently
   (1) a hydrogen atom or
   (2) a group selected from the following group F,
(q) a C₆₋₁₄ aryl group optionally substituted by 1 to 5 substituents selected from the aforementioned group B and
(r) a heterocyclic group optionally substituted by 1 to 5 substituents selected from the aforementioned group B
   (wherein said heterocyclic group comprises 1 to 4 heteroatoms selected from oxygen atom, nitrogen atom and sulfur atom)
group F:
(1) a C₁₋₆ alkyl group optionally substituted by 1 to 3 substituents selected from the aforementioned group A,
(2) a C₆₋₁₄ aryl group optionally substituted by 1 to 5 substituents selected from the aforementioned group B,
(3) a heterocyclic group optionally substituted by 1 to 5 substituents selected from the aforementioned group B
   (wherein said heterocyclic group comprises 1 to 4 heteroatoms selected from oxygen atom, nitrogen atom and sulfur atom),
(4) a C₃₋₈ cycloalkyl group optionally substituted by 1 to 5 substituents selected from the aforementioned group B,
(5) a C₆₋₁₄ aryl C₁₋₆ alkyl group optionally substituted by 1 to 5 substituents selected from the aforementioned group B,
(6) a heterocycle C₁₋₆ alkyl group optionally substituted by 1 to 5 substituents selected from the aforementioned group B
   (wherein said heterocycle C₁₋₆ alkyl group is a C₁₋₆ alkyl group substituted by "a heterocyclic group optionally substituted by 1 to 5 substituents selected from group B" as defined above), and,
(7) a C₃₋₈ cycloalkyl C₁₋₆ alkyl group optionally substituted by 1 to 5 substituents selected from the aforementioned group B.
[2] The fused ring compound of [1], wherein 1 to 4 of G¹, G², G³ and G⁴ is a nitrogen atom, or a pharmaceutically acceptable salt thereof.
[3] The fused ring compound of [2], wherein at least one of G¹ and G² is a nitrogen atom, or a pharmaceutically acceptable salt thereof.
[4] The fused ring compound of [1], wherein at least one of G¹ and G² is a heteroatom, and at least one of G³ and G⁴ is a heteroatom, or a pharmaceutically acceptable salt thereof.
[5] The fused ring compound of [1], wherein, in the formula [I], the moiety 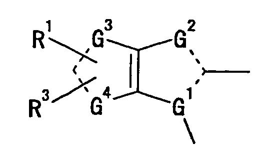 is a fused ring selected from the group consisting of 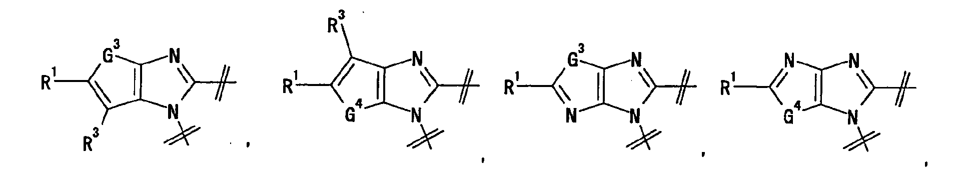 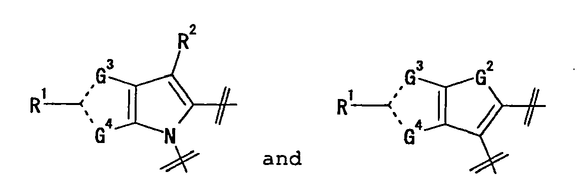 or a pharmaceutically acceptable salt thereof.
[6] The fused ring compound of [5], wherein, in the formula [I], the moiety 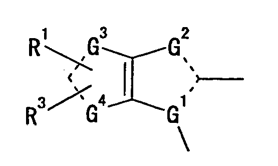 is a fused ring selected from the group consisting of 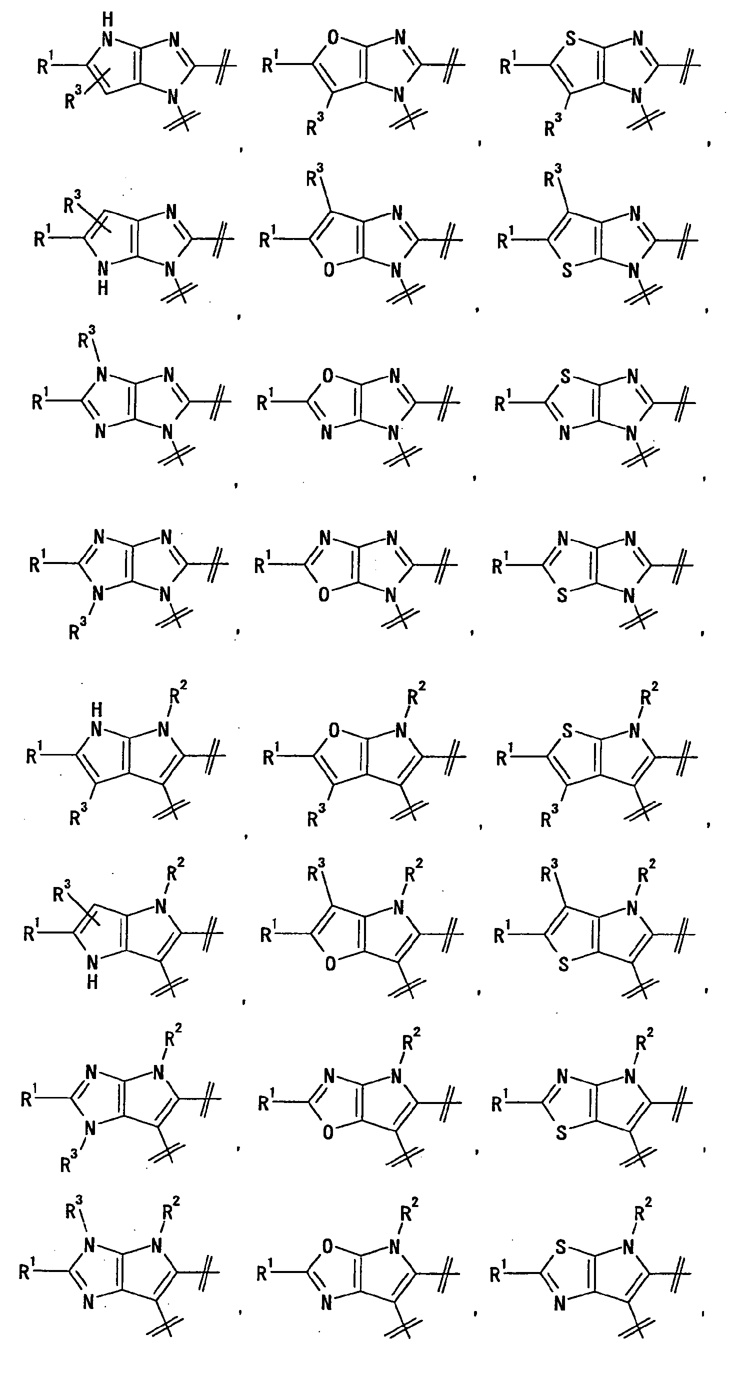 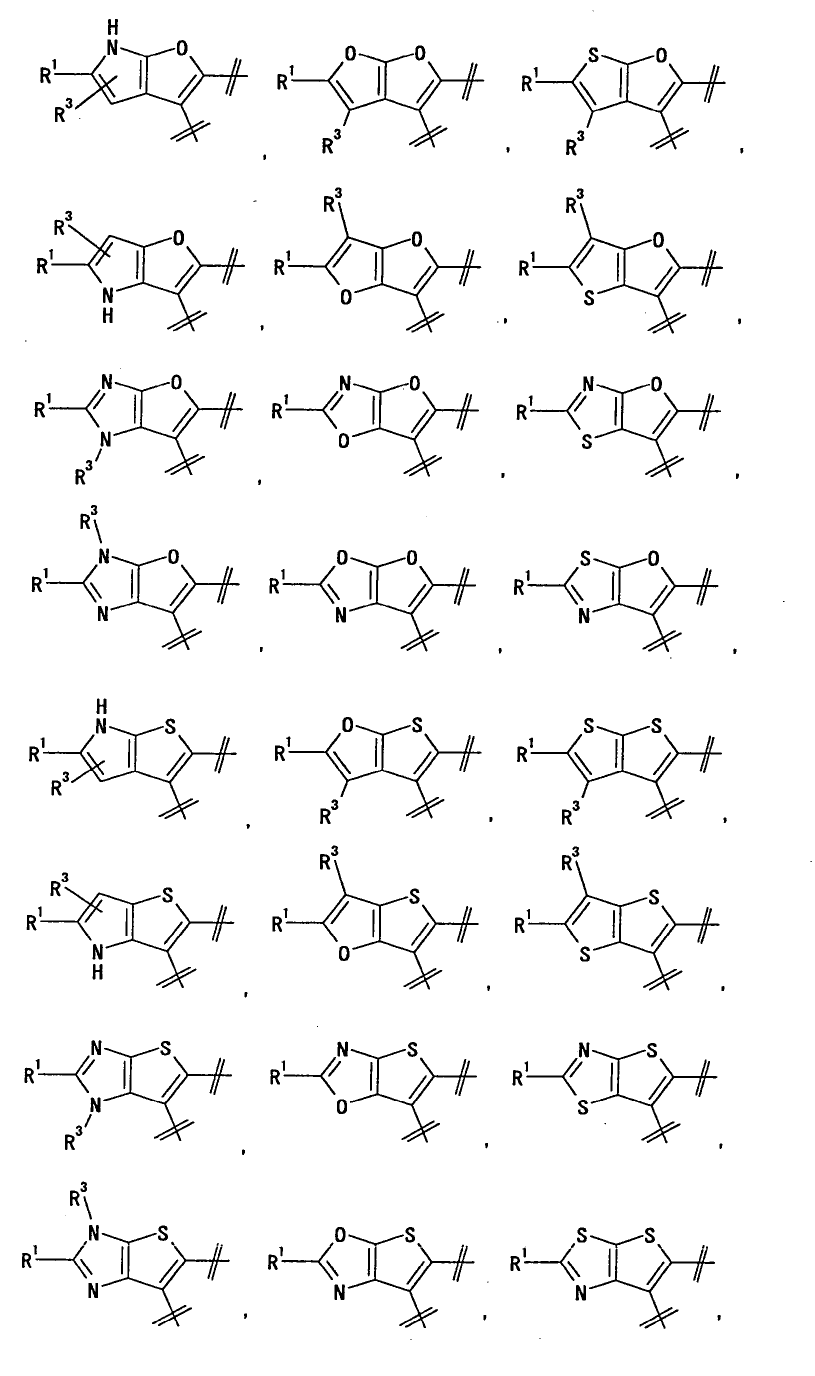 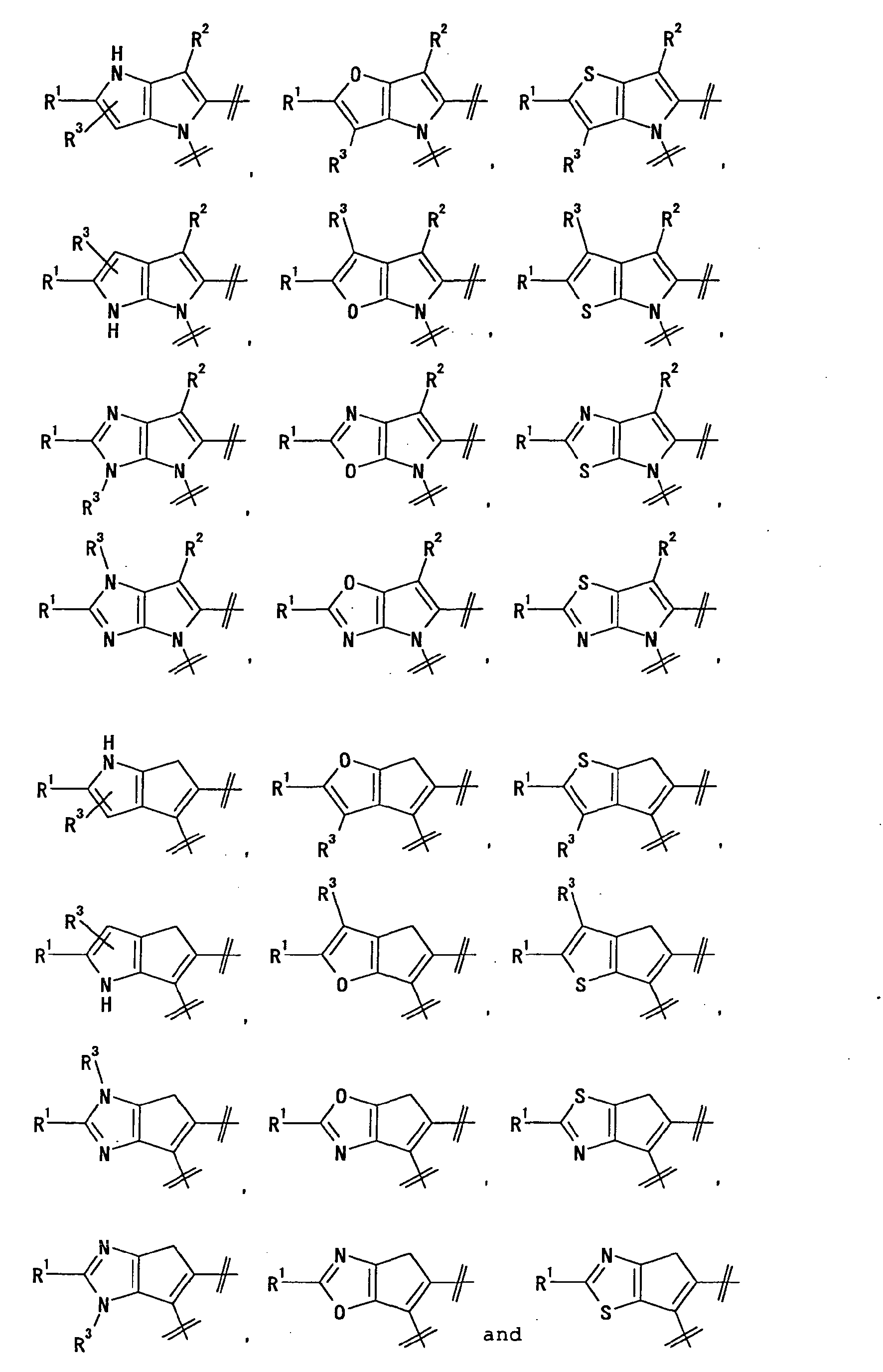 or a pharmaceutically acceptable salt thereof.
[7] The fused ring compound of [6], wherein, in the formula [I], the moiety 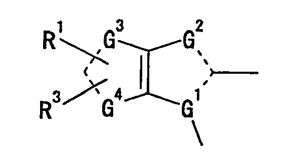 is a fused ring selected from the group consisting of 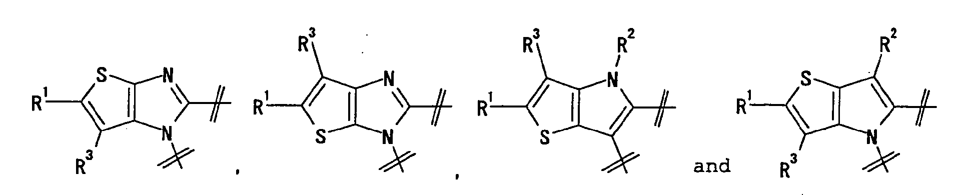 or a pharmaceutically acceptable salt thereof.
[8] The fused ring compound of [7], which is represented by the following formula [I-1]: 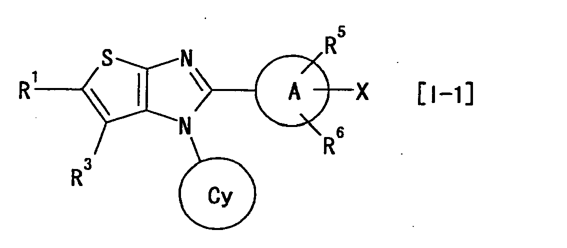 wherein each symbol is as defined in [1],
   or a pharmaceutically acceptable salt thereof.
[9] The fused ring compound of [7], which is represented by the following formula [I-2]: 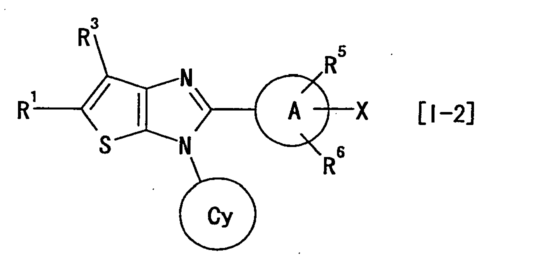 wherein each symbol is as defined in [1],
   or a pharmaceutically acceptable salt thereof.
[10] The fused ring compound of [7], which is represented by the following formula [I-3]: 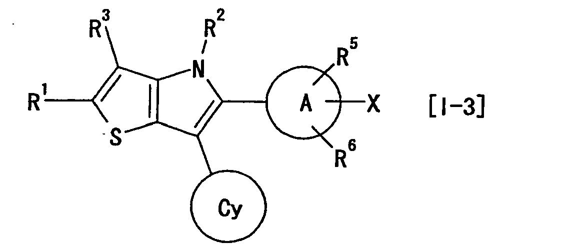 wherein each symbol is as defined in [1],
   or a pharmaceutically acceptable salt thereof.
[11] The fused ring compound of [7], which is represented by the following formula [I-4]: 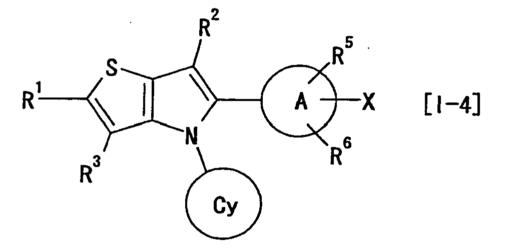 wherein each symbol is as defined in [1],
   or a pharmaceutically acceptable salt thereof.
[12] The fused ring compound of [1], wherein R¹ is a carboxyl group, or a pharmaceutically acceptable salt thereof.
[13] The fused ring compound of [1], wherein R² is
   (1) a hydrogen atom,
   (2) a C₁₋₆ alkyl group optionally substituted by 1 to 3 substituents selected from group E or
   (3) 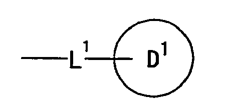
   wherein L¹ and ring D¹ are as defined in [1], or a pharmaceutically acceptable salt thereof.
[14] The fused ring compound of [13], wherein R² is a C₁₋₆ alkyl group optionally substituted by 1 to 3 substituents selected from group E, or a pharmaceutically acceptable salt thereof.
[15] The fused ring compound of [13], wherein R² is 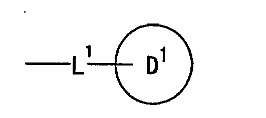 wherein L¹ and ring D¹ are as defined in [1], or a pharmaceutically acceptable salt thereof.
[16] The fused ring compound of [1], wherein R³ is a hydrogen atom, or a pharmaceutically acceptable salt thereof.
[17] The fused ring compound of [1], wherein ring Cy is a C₃₋₈ cycloalkyl group or a C₃₋₈ cycloalkenyl group, or a pharmaceutically acceptable salt thereof.
[18] The fused ring compound of [17], wherein ring Cy is a C₃₋₈ cycloalkyl group, or a pharmaceutically acceptable salt thereof.
[19] The fused ring compound of [1], wherein ring A is a C₆₋₁₄ aryl group, or a pharmaceutically acceptable salt thereof.
[20] The fused ring compound of [1], wherein R⁵ and R⁶ are each independently a hydrogen atom or a halogen atom, or a pharmaceutically acceptable salt thereof.
[21] The fused ring compound of [20], wherein R⁵ and R⁶ are each a hydrogen atom, or a pharmaceutically acceptable salt thereof.
[22] The fused ring compound of [1], wherein X is a hydrogen atom, a halogen atom, a C₁₋₆ alkyl group optionally substituted by 1 to 3 substituents selected from group A or -OR^{a11} (wherein R^{a11} is a hydrogen atom or a C₁₋₆ alkyl group optionally substituted by 1 to 3 substituents selected from group A), or a pharmaceutically acceptable salt thereof.
[23] The fused ring compound of [22], wherein X is -OR^{a11}, or a pharmaceutically acceptable salt thereof.
[24] The fused ring compound of [1], wherein X is 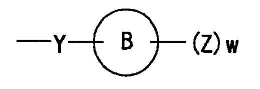 wherein each symbol is as defined in [1], or a pharmaceutically acceptable salt thereof.
[25] The fused ring compound of [24], wherein Y is -(CH₂)ₘ-O-(CH₂)ₙ- wherein each symbol is as defined in [1], or a pharmaceutically acceptable salt thereof.
[26] The fused ring compound of [24], wherein ring B is a C₆₋₁₄ aryl group, or a pharmaceutically acceptable salt thereof.
[27] The fused ring compound of [24], wherein Z shows 1 to 3 substituents selected from
   (1) a hydrogen atom,
   (2) a C₆₋₁₄ aryl group optionally substituted by 1 to 5 substituents selected from group D,
   (3) a heterocyclic group optionally substituted by 1 to 5 substituents selected from group D,
   (4) -(CH₂)ₜ-S(O)_{q}-R^{d2},
   (5) -(CH₂)ₜ-NR^{d3}R^{d4} and
   (6) -(CH₂)ₜ-NR^{d9}CO-R^{d10}
   wherein each symbol is as defined in [1], or a pharmaceutically acceptable salt thereof.
[28] The fused ring compound of [27], wherein at least one of Z is a substituent selected from
   a C₆₋₁₄ aryl group optionally substituted by 1 to 5 substituents selected from group D and
   a heterocyclic group optionally substituted by 1 to 5 substituents selected from group D, or a pharmaceutically acceptable salt thereof.
[29] The fused ring compound of [1] or a pharmaceutically acceptable salt thereof, which is selected from the group consisting of
   methyl 3-cyclohexyl-2-(4-hydroxyphenyl)-3H-thieno[2,3-d]imidazole-5-carboxylate,
   methyl 2-{4-[4'-chloro-4-(2-oxopyrrolidin-1-yl)-biphenyl-2-ylmethoxy]phenyl}-3-cyclohexyl-3H-thieno[2,3-d]imidazole-5-carboxylate,
   2-{4-[4'-chloro-4-(2-oxopyrrolidin-1-yl)biphenyl-2-ylmethoxy]phenyl}-3-cyclohexyl-3H-thieno[2,3-d]imidazole-5-carboxylic acid hydrochloride,
   2-(4-benzyloxyphenyl)-3-cyclohexyl-3H-thieno[2,3-d]imidazole-5-carboxylic acid hydrochloride,
   3-cyclohexyl-2-{4-[2-morpholino-5-(2-oxopyrrolidin-1-yl)benzyloxy]phenyl}-3H-thieno[2,3-d]imidazole-5-carboxylic acid,
   methyl 2-(4-benzyloxy-2-fluorophenyl)-1-cyclohex-2-enyl-1H-thieno[2,3-d]imidazole-5-carboxylate,
   2-(4-benzyloxy-2-fluorophenyl)-1-cyclohex-2-enyl-1H-thieno[2,3-d]imidazole-5-carboxylic acid hydrochloride,
   5-(4-benzyloxyphenyl)-4,6-dicyclohexyl-4H-thieno[3,2-b]pyrrole-2-carboxylic acid,
   ethyl 5-(4-benzyloxyphenyl)-6-cyclohex-2-enyl-4H-thieno[3,2-b]pyrrole-2-carboxylate,
   ethyl 5-(4-benzyloxyphenyl)-6-cyclohex-2-enyl-4-methyl-4H-thieno[3,2-b]pyrrole-2-carboxylate,
   ethyl 5-(4-benzyloxyphenyl)-6-cyclohexyl-4-methyl-4H-thieno[3,2-b]pyrrole-2-carboxylate,
   5-(4-benzyloxyphenyl)-6-cyclohexyl-4-methyl-4H-thieno[3,2-b]pyrrole-2-carboxylic acid,
   ethyl 6-cyclohexyl-5-(4-hydroxyphenyl)-4-methyl-4H-thieno[3,2-b]pyrrole-2-carboxylate,
   5-(4-benzyloxyphenyl)-6-cyclohexyl-4-dimethylcarbamoylmethyl-4H-thieno[3,2-b]pyrrole-2-carboxylic acid,
   6-cyclohexyl-4-methyl-5-{4-[2-morpholino-5-(2-oxopyrrolidin-1-yl)benzyloxy]phenyl}-4H-thieno[3,2-b]pyrrole-2-carboxylic acid hydrochloride,
   ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate,
   5-{4-[5-(N-acetyl-N-methylamino)-2-morpholinobenzyloxy]phenyl}-6-cyclohexyl-4-methyl-4H-thieno[3,2-b]pyrrole-2-carboxylic acid hydrochloride,
   6-cyclohexyl-5-{4-[2-(4-methanesulfonylpiperazin-1-yl)-5-(2-oxopyrrolidin-1-yl)benzyloxy]phenyl}-4-methyl-4H-thieno[3,2-b]pyrrole-2-carboxylic acid hydrochloride,
   5-{4-[5-amino-2-(4-methanesulfonylpiperazin-1-yl)benzyloxy]phenyl}-6-cyclohexyl-4-methyl-4H-thieno[3,2-b]pyrrole-2-carboxylic acid hydrochloride,
   5-{4-[5-(N-acetyl-N-methylamino)-2-(4-methanesulfonylpiperazin-1-yl)benzyloxy]phenyl}-6-cyclohexyl-4-methyl-4H-thieno[3,2-b]pyrrole-2-carboxylic acid hydrochloride,
   6-cyclohexyl-4-dimethylcarbamoylmethyl-5-(4-methoxyphenyl)-4H-thieno[3,2-b]pyrrole-2-carboxylic acid,
   6-cyclohexyl-5-[4-(5-methanesulfonyl-2-morpholinobenzyloxy)phenyl]-4-methyl-4H-thieno[3,2-b]pyrrole-2-carboxylic acid hydrochloride,
   ethyl 6-cyclohexyl-4-methyl-5-{4-[2-morpholino-5-(2-oxopyrrolidin-1-yl)benzyloxy]phenyl}-4H-thieno[3,2-b]pyrrole-2-carboxylate hydrochloride,
   6-cyclohexyl-5-(4-methoxyphenyl)-4-morpholinocarbonylmethyl-4H-thieno[3,2-b]pyrrole-2-carboxylic acid,
   6-cyclohexyl-4-(4-ethylpiperazin-1-yl)carbonylmethyl-5-(4-methoxyphenyl)-4H-thieno[3,2-b]pyrrole-2-carboxylic acid,
   6-cyclohexyl-4-(4-dimethylaminopiperidino)carbonylmethyl-5-(4-methoxyphenyl)-4H-thieno[3,2-b]pyrrole-2-carboxylic acid,
   6-cyclohexyl-5-[4-(5-isobutyrylamino-2-morpholinobenzyloxy)phenyl]-4-methyl-4H-thieno[3,2-b]pyrrole-2-carboxylic acid hydrochloride,
   6-cyclohexyl-5-{4-[5-(N-isobutyryl-N-methylamino)-2-morpholinobenzyloxy]phenyl}-4-methyl-4H-thieno[3,2-b]pyrrole-2-carboxylic acid hydrochloride,
   6-cyclohexyl-4-methyl-5-[4-(2-morpholinobenzyloxy)phenyl]-4H-thieno[3,2-b]pyrrole-2-carboxylic acid hydrochloride,
   4-(benzylcarbamoylmethyl)-6-cyclohexyl-5-(4-methoxyphenyl)-4H-thieno[3,2-b]pyrrole-2-carboxylic acid,
   4-(tert-butylcarbamoylmethyl)-6-cyclohexyl-5-(4-methoxyphenyl)-4H-thieno[3,2-b]pyrrole-2-carboxylic acid,
   6-cyclohexyl-4-methyl-5-{4-[2-morpholino-4-(2-oxopyrrolidin-1-yl)benzyloxy]phenyl}-4H-thieno[3,2-b]pyrrole-2-carboxylic acid hydrochloride,
   5-(2-benzyloxyphenyl)-6-cyclohexyl-4-methyl-4H-thieno[3,2-b]pyrrole-2-carboxylic acid,
   5-(2-benzyloxyphenyl)-6-cyclohexyl-4-dimethylcarbamoylmethyl-4H-thieno[3,2-b]pyrrole-2-carboxylic acid,
   5-[4-(1-tert-butoxycarbonylpiperidin-3-yloxy)phenyl]-6-cyclohexyl-4-dimethylcarbamoylmethyl-4H-thieno[3,2-b]pyrrole-2-carboxylic acid,
   6-cyclohexyl-4-dimethylcarbamoylmethyl-5-[4-(2-morpholinobenzyloxy)phenyl]-4H-thieno[3,2-b]pyrrole-2-carboxylic acid,
   5-[4-(1-tert-butoxycarbonylpiperidin-4-yloxy)phenyl]-6-cyclohexyl-4-dimethylcarbamoylmethyl-4H-thieno[3,2-b]pyrrole-2-carboxylic acid,
   6-cyclohexyl-5-{4-[5-(2-dimethylaminoacetylamino)-2-morpholinobenzyloxy]phenyl}-4-methyl-4H-thieno[3,2-b]pyrrole-2-carboxylic acid dihydrochloride,
   6-cyclohexyl-4-methyl-5-{4-[2-morpholino-5-(2-morpholinoacetylamino)benzyloxy]phenyl}-4H-thieno[3,2-b]pyrrole-2-carboxylic acid dihydrochloride,
   6-cyclohexyl-4-dimethylcarbamoylmethyl-5-(4-phenoxyphenyl)-4H-thieno[3,2-b]pyrrole-2-carboxylic acid,
   6-cyclohexyl-5-(4-{5-[N-(2-dimethylaminoacetyl)-N-methylamino]-2-morpholinobenzyloxy}phenyl)-4-methyl-4H-thieno[3,2-b]pyrrole-2-carboxylic acid dihydrochloride,
   5-(4-benzyloxyphenyl)-4-(tert-butylcarbamoylmethyl)-6-cyclohexyl-4H-thieno[3,2-b]pyrrole-2-carboxylic acid,
   4-benzyl-5-(4-benzyloxyphenyl)-6-cyclohexyl-4H-thieno[3,2-b]pyrrole-2-carboxylic acid,
   5-(4-benzyloxyphenyl)-6-cyclohexyl-4-(2-dimethylaminoethyl)-4H-thieno[3,2-b]pyrrole-2-carboxylic acid,
   5-(4-benzyloxyphenyl)-6-cyclohexyl-4-(2-morpholinoethyl)-4H-thieno[3,2-b]pyrrole-2-carboxylic acid,
   5-(4-benzyloxyphenyl)-6-cyclohexyl-4-cyclohexylmethyl-4H-thieno[3,2-b]pyrrole-2-carboxylic acid,
   5-(4-benzyloxyphenyl)-6-cyclohexyl-4-(2-methoxyethyl)-4H-thieno[3,2-b]pyrrole-2-carboxylic acid,
   5-(4-benzyloxyphenyl)-6-cyclohexyl-4-{[N-methyl-N-(2-oxo-2-piperidinoethyl)carbamoyl]methyl}-4H-thieno[3,2-b]pyrrole-2-carboxylic acid,
   5-(4-benzyloxyphenyl)-6-cyclohexyl-4-[(4-morpholinophenylcarbamoyl)methyl]-4H-thieno[3,2-b]pyrrole-2-carboxylic acid hydrochloride,
   5-(4-benzyloxyphenyl)-6-cyclohexyl-4-[2-(4-cyclohexylpiperazin-1-yl)-2-oxoethyl]-4H-thieno[3,2-b]pyrrole-2-carboxylic acid hydrochloride,
   5-(4-benzyloxyphenyl)-6-cyclohexyl-4-[2-(4-cyclohexylpiperazin-1-yl)ethyl]-4H-thieno[3,2-b]pyrrole-2-carboxylic acid dihydrochloride,
   5-(4-benzyloxyphenyl)-4-[2-(1,4'-bipiperidinyl-1'-yl)-2-oxoethyl]-6-cyclohexyl-4H-thieno[3,2-b]pyrrole-2-carboxylic acid hydrochloride,
   5-(4-benzyloxyphenyl)-4-[2-(1,4'-bipiperidinyl-1'-yl)ethyl]-6-cyclohexyl-4H-thieno[3,2-b]pyrrole-2-carboxylic acid dihydrochloride,
   6-cyclohexyl-4-(2-dimethylaminoethyl)-5-[4-(5-methanesulfonyl-2-morpholinobenzyloxy)phenyl]-4H-thieno[3,2-b]pyrrole-2-carboxylic acid dihydrochloride,
   6-cyclohexyl-4-methyl-5-(4-{5-[N-methyl-N-(2-morpholinoacetyl)amino]-2-morpholinobenzyloxy}phenyl)-4H-thieno[3,2-b]pyrrole-2-carboxylic acid dihydrochloride,
   5-(4-benzyloxyphenyl)-6-cyclohexyl-4-{2-[N-methyl-N-(2-morpholinoethyl)amino]ethyl}-4H-thieno[3,2-b]pyrrole-2-carboxylic acid dihydrochloride,
   5-(4-benzyloxyphenyl)-6-cyclohexyl-4-{2-[N-methyl-N-(2-piperidinoethyl)amino]ethyl}-4H-thieno[3,2-b]pyrrole-2-carboxylic acid dihydrochloride, and
   5-(4-benzyloxyphenyl)-6-cyclohexyl-4-{1-[N-methyl-N-(4-morpholinophenyl)carbamoyl]ethyl}-4H-thieno[3,2-b]pyrrole-2-carboxylic acid hydrochloride.
[30] A pharmaceutical composition comprising a fused ring compound of any of [1] to [29], or a pharmaceutically acceptable salt thereof, and a pharmaceutically acceptable carrier.
[31] A hepatitis C virus polymerase inhibitor comprising a fused ring compound of any of [1] to [29] or a pharmaceutically acceptable salt thereof as an active ingredient.
[32] An anti-hepatitis C virus agent comprising a fused ring compound of any of [1] to [29] or a pharmaceutically acceptable salt thereof as an active ingredient.
[33] A therapeutic agent for hepatitis C comprising a fused ring compound of any of [1] to [29] or a pharmaceutically acceptable salt thereof as an active ingredient.
[34] A therapeutic agent for hepatitis C comprising (a) the hepatitis C virus polymerase inhibitor of [31], and (b) at least one pharmaceutical agent selected from the group consisting of a different antiviral agent, an antiinflammatory agent and an immunostimulant.
[35] A therapeutic agent for hepatitis C comprising (a) the hepatitis C virus polymerase inhibitor of [31], and (b) interferon.
[36] An anti-hepatitis C virus agent comprising (a) the anti-hepatitis C virus agent of [32], and (b) at least one agent selected from the group consisting of a different antiviral agent, an antiinflammatory agent and an immunostimulant.
[37] An anti-hepatitis C virus agent comprising (a) the anti-hepatitis C virus agent of [32], and (b) interferon.
[38] A pharmaceutical composition comprising (a) a fused ring compound of any of [1] to [29] or a pharmaceutically acceptable salt thereof, and (b) at least one pharmaceutical agent selected from the group consisting of a different antiviral agent, an antiinflammatory agent and an immunostimulant.
[39] A pharmaceutical composition comprising (a) a fused ring compound of any of [1] to [29] or a pharmaceutically acceptable salt thereof, and (b) interferon.
[40] Use of a fused ring compound of any of [1] to [29] or a pharmaceutically acceptable salt thereof for the production of a pharmaceutical agent for treating hepatitis C.
[41] Use of a fused ring compound of any of [1] to [29] or a pharmaceutically acceptable salt thereof for the production of a hepatitis C virus polymerase inhibitor.
[42] A method for treating hepatitis C, which comprises administering an effective amount of a fused ring compound of any of [1] to [29] or a pharmaceutically acceptable salt thereof to a mammal.
[43] The method of [42], further comprising administering an effective amount of at least one pharmaceutical agent selected from the group consisting of a different antiviral agent, an antiinflammatory agent and an immunostimulant to the mammal.
[44] The method of [42], further comprising administering an effective amount of interferon to the mammal.
[45] A method for inhibiting hepatitis C virus polymerase, which comprises administering an effective amount of a fused ring compound of any of [1] to [29] or a pharmaceutically acceptable salt thereof to a mammal.
[46] The method of [45], further comprising administering an effective amount of at least one pharmaceutical agent selected from the group consisting of a different antiviral agent, an antiinflammatory agent and an immunostimulant to the mammal.
[47] The method of [45], further comprising administering an effective amount of interferon to the mammal.

### Best Mode for Embodying the Invention

The definitions of respective substituents and moieties used in the present specification are as follows.

The "halogen atom" is a fluorine atom, a chlorine atom, a bromine atom or an iodine atom, preferably a fluorine atom, a chlorine atom or a bromine atom.

The "C₁₋₆ alkyl group" is a linear or branched chain alkyl group having 1 to 6 carbon atoms, preferably a linear or branched chain alkyl group having 1 to 4 carbon atoms. Specifically, methyl group, ethyl group, propyl group, isopropyl group, butyl group, isobutyl group, sec-butyl group, tert-butyl group, pentyl group, isopentyl group, tert-pentyl group, hexyl group and the like can be mentioned.

The "C₂₋₆ alkenyl group" is a linear or branched chain alkenyl group having 2 to 6 carbon atoms. Specifically, vinyl group, allyl group, 1-propenyl group, isopropenyl group, 1-butenyl group, 2-butenyl group, 1,3-butadienyl group, 2-isopentenyl group, 3-isohexenyl group, 4-methyl-3-pentenyl group can be mentioned.

The "C₂₋₆ alkynyl group" is a linear or branched chain alkynyl group having 2 to 6 carbon atoms. Specifically, ethynyl group, 1-propynyl group, 2-propynyl group, 3-butynyl group and the like can be mentioned.

The "halogenated C₁₋₆ alkyl group" is the above-defined "C₁₋₆ alkyl group" substituted by the above-defined "halogen atom", which is preferably a halogenated alkyl group wherein the alkyl moiety is a linear or branched chain alkyl group having 1 to 4 carbon atoms. Specifically, fluoromethyl group, difluoromethyl group, trifluoromethyl group, bromomethyl group, chloromethyl group, 1,2-dichloroethyl group, 2,2-dichloroethyl group, 2,2,2-trifluoroethyl group and the like can be mentioned.

The "C₁₋₆ alkylene" is a straight chain alkylene having 1 to 6 carbon atoms, and methylene, ethylene, trimethylene, tetramethylene, pentamethylene, hexamethylene can be mentioned.

The "C₂₋₆ alkenylene" is a straight chain alkenylene having 2 to 6 carbon atoms, and vinylene, propenylene, 1-butenylene, 1,3-butadienylene and the like can be mentioned.

The "C₁₋₆ alkoxy group" is an alkyl-oxy group wherein the alkyl moiety is the above-defined "C₁₋₆ alkyl group", preferably an alkoxy group wherein the alkyl moiety is a linear or branched chain alkyl group having 1 to 4 carbon atoms. Specifically, methoxy group, ethoxy group, propoxy group, isopropyloxy group, butoxy group, isobutyloxy group, tert-butyloxy group, pentyloxy group, hexyloxy group and the like can be mentioned.

The "C₁₋₆ alkoxy C₁₋₆ alkoxy group" is an alkyl-oxy-alkyl-oxy group wherein the above-defined "C₁₋₆ alkoxy group" is substituted by the above-defined "C₁₋₆ alkoxy group", preferably that wherein the alkyl moiety is a linear or branched chain alkyl group having 1 to 4 carbon atoms. Specifically, methoxymethoxy group, ethoxymethoxy group, 1-(methoxy)ethoxy group, 2-(methoxy)ethoxy group, methoxypropoxy group, isopropyloxyethoxy group and the like can be mentioned.

The "C₁₋₆ alkanoyl group" is an alkyl-carbonyl group wherein the alkyl moiety is the above-defined "C₁₋₆ alkyl group", preferably an alkyl-carbonyl group wherein the alkyl moiety is a linear or branched chain alkyl group having 1 to 4 carbon atoms. Specifically, acetyl group, propionyl group, butyryl group, isobutyryl group, pivaloyl group and the like can be mentioned.

The "C₁₋₆ alkoxycarbonyl group" is an alkyl-oxy-carbonyl group wherein the alkoxy moiety is the above-defined "C₁₋₆ alkoxy group", preferably an alkyl-oxy-carbonyl group wherein the alkyl moiety is a linear or branched chain alkyl group having 1 to 4 carbon atoms. Specifically, methoxycarbonyl group, ethoxycarbonyl group, propoxycarbonyl group, isopropyloxycarbonyl group, butoxycarbonyl group, isobutyloxycarbonyl group, tert-butyloxycarbonyl group, pentyloxycarbonyl group, hexyloxycarbonyl group and the like can be mentioned.

The "C₁₋₆ alkylamino group" is an alkyl-amino group or a dialkyl-amino group wherein the alkyl moiety is the above-defined "C₁₋₆ alkyl group", preferably an alkyl-amino group or a dialkyl-amino group wherein the alkyl moiety is a linear or branched chain alkyl group having 1 to 4 carbon atoms. Specifically, methylamino group, ethylamino group, propylamino group, isopropylamino group, butylamino group, isobutylamino group, tert-butylamino group, pentylamino group, hexylamino group, dimethylamino group, diethylamino group, N-ethyl-N-methylamino group, N-isobutyl-N-isopropylamino group and the like can be mentioned.

The "C₁₋₆ alkanoylamino group" is an alkyl-carbonyl-amino group wherein the alkanoyl moiety is the above-defined "C₁₋₆ alkanoyl group", preferably an alkyl-carbonyl-amino group wherein the alkyl moiety is a linear or branched chain alkyl group having 1 to 4 carbon atoms. Specifically, acetylamino group, propionylamino group, butyrylamino group, isobutyrylamino group, pivaloylamino group and the like can be mentioned.

The "C₁₋₆ alkylsulfonyl group" is an alkyl-sulfonyl group
wherein the alkyl moiety is the above-defined "C₁₋₆ alkyl group", preferably an alkyl-sulfonyl group wherein the alkyl moiety is a linear or branched chain alkyl group having 1 to 4 carbon atoms. Specifically, methanesulfonyl group, ethylsulfonyl group, propylsulfonyl group, isopropylsulfonyl group, butylsulfonyl group, isobutylsulfonyl group, tert-butylsulfonyl group, pentylsulfonyl group, hexylsulfonyl group and the like can be mentioned.

The "C₆₋₁₄ aryl group" is an aromatic hydrocarbon group having 6 to 14 carbon atoms. Specifically, phenyl group, naphthyl group, anthryl group, indenyl group, azulenyl group, fluorenyl group, phenanthryl group and the like can be mentioned, with preference given to phenyl group.

The "C₃₋₈ cycloalkyl group" is a saturated cycloalkyl group having 3 to 8, more preferably 5 to 7, carbon atoms. Specifically, cyclopropyl group, cyclobutyl group, cyclopentyl group, cyclohexyl group, cycloheptyl group and cyclooctyl group can be mentioned.

The "C₃₋₈ cycloalkenyl group" is a cycloalkenyl group having 3 to 8, more preferably 5 to 7, carbon atoms, and includes at least one, preferably 1 or 2, double bonds. Specifically, cyclopropenyl group, cyclobutenyl group, cyclopentenyl group, cyclopentadienyl group, cyclohexenyl group, 2,4-cyclohexadien-1-yl group, 2,5-cyclohexadien-1-yl group, cycloheptenyl group, cyclooctenyl group and the like can be mentioned. It does not include aryl group such as phenyl group and completely saturated cycloalkyl group.

The "C₆₋₁₄ aryl C₁₋₆ alkyloxycarbonyl group" is an arylalkyl-oxy-carbonyl group wherein the alkyl moiety is the above-defined "C₁₋₆ alkyl group", and the aryl moiety is the above-defined "C₆₋₁₄ aryl group". Preferred is an aryl-alkyl-oxy-carbonyl group wherein the alkyl moiety is a linear or branched chain alkyl group having 1 to 4 carbon atoms and the aryl moiety is a phenyl group. Specifically, benzyloxycarbonyl group, phenethyloxycarbonyl group, 3-phenylpropyloxycarbonyl group, 2-phenylpropyloxycarbonyl group, 4-phenylbutyloxycarbonyl group and the like can be mentioned.

The "bond" means a direct connection. For example, when L¹ is a "bond" in -O-L¹-Ph, it means -O-Ph.

The "glucuronic acid residue" is a group remaining after removing any hydroxyl group from glucuronic acid, and preferably substitutes at the 1-posotion of β-D-glucuronic acid.

The "heterocyclic group" and "heterocyclic group comprising 1 to 4 heteroatoms selected from oxygen atom, nitrogen atom and sulfur atom" has, as a ring-constituting atom, 1 to 4 heteroatoms selected from oxygen atom, nitrogen atom and sulfur atom besides carbon atom, wherein the number of atom constituting the ring is 3 to 14, includes saturated ring and unsaturated ring, monocycle and fused ring.

As the monocyclic heterocyclic group, specifically, pyridyl group, pyrazinyl group, pyrimidinyl group, pyridazinyl group, 1,3,5-triazinyl group, pyrrolyl group, pyrazolyl group, imidazolyl group, triazolyl group (1,2,3-triazolyl group, 1,2,4-triazolyl group), tetrazolyl group, thienyl group, furyl group, oxazolyl group, isoxazolyl group, thiazolyl group, isothiazolyl group, oxadiazolyl group (1,2,4-oxadiazolyl group, 1,3,4-oxadiazolyl group, 1,2,5-oxadiazolyl group), thiadiazolyl group (1,2,4-thiadiazolyl group, 1,3,4-thiadiazolyl group, 1,2,5-thiadiazolyl group), pyrrolinyl group (1-pyrrolinyl group, 2-pyrrolinyl group, 3-pyrrolinyl group), pyrrolidinyl group, 4,5-dihydro-1H-imidazolyl group, 4,5-dihydro-1H-oxazolyl group, 4,5-dihydro-1H-thiazolyl group, imidazolidinyl group, piperidyl group, piperazinyl group, 1,2,3,6-tetrahydropyridyl group, morpholinyl group, thiomorpholinyl group, 3,6-dihydro-2H-pyranyl group, tetrahydropyranyl group and the like can be mentioned.

This heterocyclic group includes the groups represented by the following formulas. 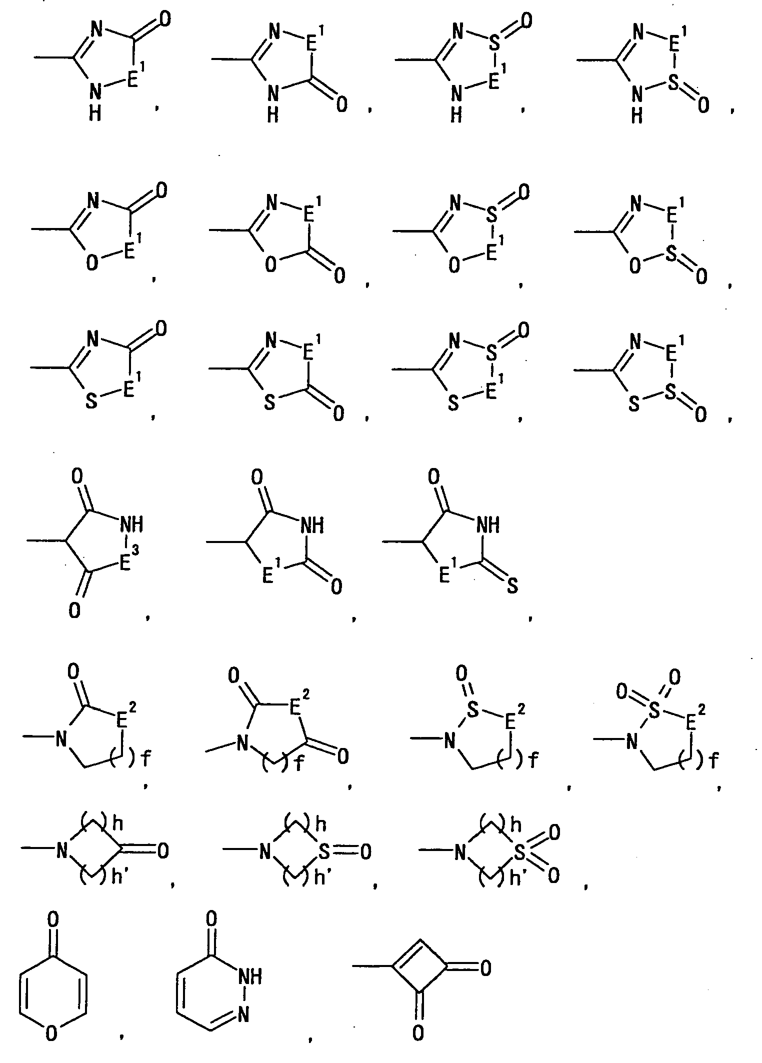 or 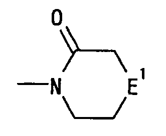 wherein E¹ is an oxygen atom, a sulfur atom or NH, E² is an oxygen atom, CH₂ or NH, E³ is an oxygen atom or a sulfur atom,
wherein f is an integer of 1 to 3, h and h' are the same or different and each is an integer of 1 to 3.

Specifically, 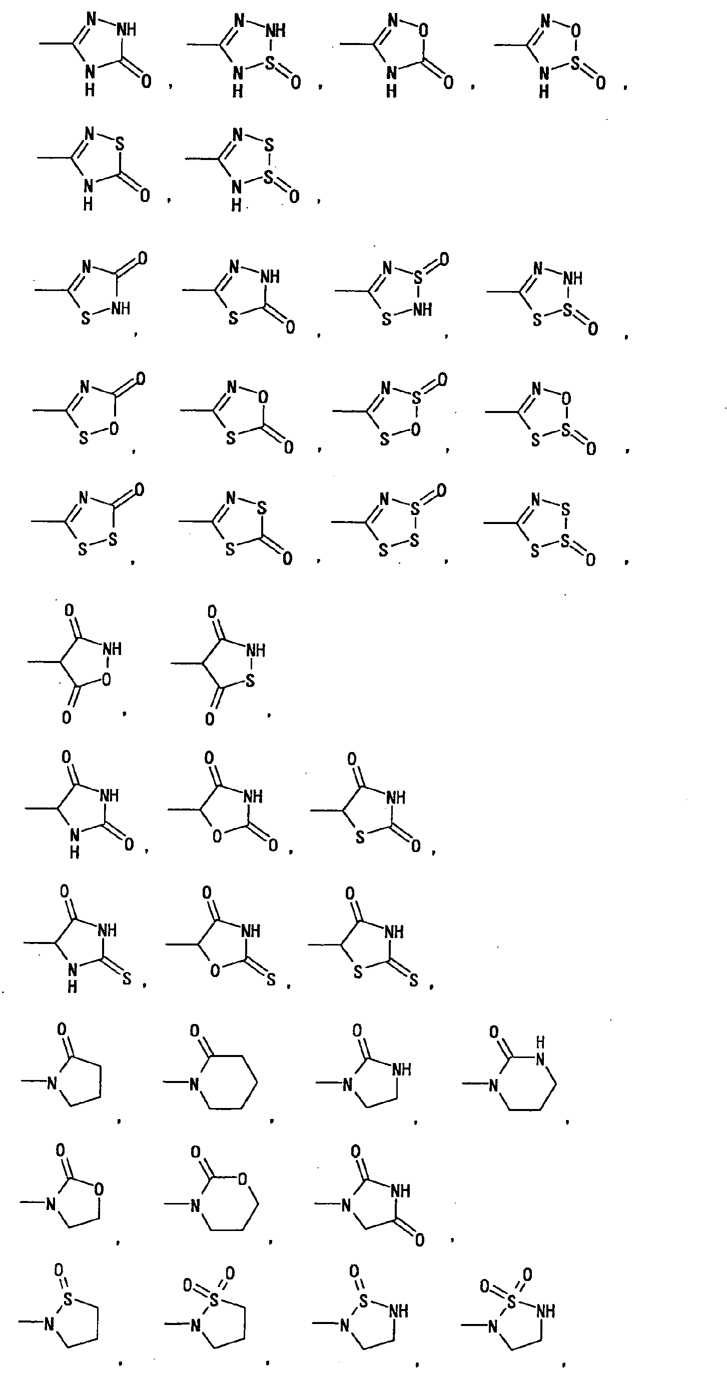 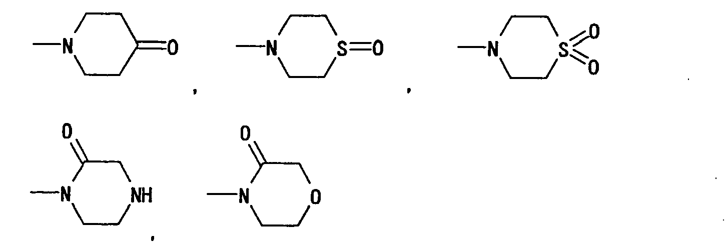 and the like can be mentioned.

As a fused heterocyclic group, specifically, quinolyl group, isoquinolyl group, quinazolinyl group, quinoxalinyl group, phthalazinyl group, cinnolinyl group, naphthyridinyl group, 5,6,7,8-tetrahydroquinolyl group, indolyl group, benzimidazolyl group, 2,3-dihydrobenzimidazolyl group, 2,3-dihydro-2-oxobenzimidazolyl group, indolinyl group, benzofuranyl group, benzothienyl group, benzoxazolyl group, benzothiazolyl group, 3,4-dihydro-2H-benzo[1,4]oxazinyl group, 3-oxo-3,4-dihydro-2H-benzo[1,4]oxazinyl group and the like can be mentioned.

The "group A" means the substituent groups of the following (1) to (11).
(R^{a1} and R^{a2} are each independently a hydrogen atom or the above-defined "C₁₋₆ alkyl group", and R^{a3} is the above-defined "C₁₋₆ alkyl group")
(1) the above-defined "halogen atom",
(2) the above-defined "C₁₋₆ alkoxy C₁₋₆ alkoxy group",
(3) -OR^{a1} (e.g., hydroxyl group, methoxy group, ethoxy group, isopropyloxy group, tert-butyloxy group etc.),
(4) -SR^{a1} (e.g., mercapto group, methylsulfanyl group etc.),
(5) -NR^{a1}R^{a2} (e.g., amino group, methylamino group, ethylamino group, isopropylamino group, dimethylamino group, diethylamino group, diisopropylamino group, di-tert-butylamino group, N-ethyl-N-methylamino group etc.),
(6) -COOR^{a1} (e.g., carboxyl group, methoxycarbonyl group, ethoxycarbonyl group, isopropyloxycarbonyl group, tert-butoxycarbonyl group etc.),
(7) -CONR^{a1}R^{a2} (e.g., carbamoyl group, methylcarbamoyl group, ethylcarbamoyl group, isopropylcarbamoyl group, dimethylcarbamoyl group, diethylcarbamoyl group, diisopropylcarbamoyl group, di-tert-butylcarbamoyl group, N-ethyl-N-methylcarbamoyl group etc.),
(8) -SO₃H,
(9) -SO₂NR^{a1}R^{a2} (e.g., sulfamoyl group, methylsulfamoyl group, ethylsulfamoyl group, isopropylsulfamoyl group, dimethylsulfamoyl group, diethylsulfamoyl group, diisopropylsulfamoyl group, di-tert-butylsulfamoyl group, N-ethyl-N-methylsulfamoyl group etc.),
(10) -NHCOR^{a1} (e.g., formylamino group, acetylamino group, propionylamino group, isobutyrylamino group, pivaloylamino group etc.) and
(11) -NHSO₂R^{a3} (e.g., methanesulfonylamino group, ethylsulfonylamino group, isopropylsulfonylamino group, tert-butylsulfonylamino group etc.).

The "group B" means the substituent groups of the following (1) to (19).
(the following R^{b1}, R^{b2} and R^{b4} are each independently a hydrogen atom or the above-defined "C₁₋₆ alkyl group", R^{b3} is the above-defined "C₁₋₆ alkyl group", and r is 0 or an integer of 1 to 6)
(1) the above-defined "halogen atom",
(2) a cyano group,
(3) a nitro group,
(4) the above-defined "C₁₋₆ alkyl group",
(5) the above-defined "halogenated C₁₋₆ alkyl group",
(6) -(CH₂)ᵣ-OR^{b1} (e.g., hydroxyl group, methoxy group, ethoxy group, isopropyloxy group, tert-butyloxy group, hydroxymethyl group, methoxymethyl group, 2-(methoxy)ethyl group etc.),
(7) -(CH₂)ᵣ-SR^{b1} (e.g., mercapto group, methylsulfanyl group, mercaptomethyl group, 2-(methylsulfanyl)ethyl group etc.),
(8) -(CH₂)ᵣ-NR^{b1}R^{b2} (e.g., amino group, methylamino group, ethylamino group, isopropylamino group, dimethylamino group, diethylamino group, diisopropylamino group, di-tert-butylamino group, N-ethyl-N-methylamino group, aminomethyl group, 2-(methylamino)ethyl group etc.),
(9) -(CH₂)ᵣ-COOR^{b1} (e.g., carboxyl group, methoxycarbonyl group, ethoxycarbonyl group, isopropyloxycarbonyl group, tert-butoxycarbonyl group, carboxymethyl group, 2-(carboxy)ethyl group etc.),
(10) -(CH₂)ᵣ-CONR^{b1}R^{b2} (e.g., carbamoyl group, methylcarbamoyl group, ethylcarbamoyl group, isopropylcarbamoyl group, dimethylcarbamoyl group, diethylcarbamoyl group, diisopropylcarbamoyl group, di-tert-butylcarbamoyl group, N-ethyl-N-methylcarbamoyl group, carbamoylmethyl group, dimethylcarbamoylmethyl group, 2-(methylcarbamoyl)ethyl group etc.),
(11) -(CH₂)ᵣ-COR^{b1} (e.g., formyl group, acetyl group, propionyl group, isobutyryl group, pivaloyl group, acetylmethyl group, 2-pivaloylethyl group etc.),
(12) -(CH₂)ᵣ-NR^{b1}-COR^{b2} (e.g., formylamino group, acetylamino group, propionylamino group, isobutyrylamino group, pivaloylamino group, N-acetyl-N-methylamino group, acetylaminomethyl group, 2-(isobutyrylamino)ethyl group etc.),
(13) -(CH₂)ᵣ-NR^{b1}-SO₂R^{b3} (e.g., methanesulfonylamino group, ethylsulfonylamino group, isopropylsulfonylamino group, tert-butylsulfonylamino group, N-methyl-N-(methanesulfonyl)amino group, methanesulfonylaminomethyl group, 2-(tert-butylsulfonylamino)ethyl group etc.),
(14) -(CH₂)ᵣ-SO₂R^{b3} (e. g., methanesulfonyl group, ethylsulfonyl group, isopropylsulfonyl group, tert-butylsulfonyl group, methanesulfonylmethyl group, 2-(ethylsulfonyl)ethyl group etc.), (15) -(CH₂)ᵣ-SO₂NR^{b1}R^{b2} (e. g., sulfamoyl group, methylsulfamoyl group, ethylsulfamoyl group, isopropylsulfamoyl group, dimethylsulfamoyl group, diethylsulfamoyl group, diisopropylsulfamoyl group, di-tert-butylsulfamoyl group, N-ethyl-N-methylsulfamoyl group, sulfamoylmethyl group, 2-(methylsulfamoyl)ethyl group etc.),
(16) -(CH₂)ᵣ-CONR^{b1}-SO₂R^{b3} (e.g., methanesulfonylcarbamoyl group, ethylsulfonylcarbamoyl group, isopropylsulfonylcarbamoyl group, tert-butylsulfonylcarbamoyl group, N-methyl-N-(methanesulfonyl)carbamoyl group, methanesulfonylcarbamoylmethyl group, 2-(ethylsulfonylcarbamoyl)ethyl group etc.),
(17) -(CH₂)ᵣ-SO₂NR^{b1}-COR^{b2} (e.g., acetylsulfamoyl group, propionylsulfamoyl group, isobutyrylsulfamoyl group, pivaloylsulfamoyl group, N-acetyl-N-methylsulfamoyl group, acetylsulfamoylmethyl group, 2-(pivaloylsulfamoyl)ethyl group etc.),
(18) -(CH₂)ᵣ-NR^{b1}-COOR^{b3} (e.g., methoxycarbonylamino group, ethoxycarbonylamino group, isopropyloxycarbonylamino group, tert-butoxycarbonylamino group, methoxycarbonylaminomethyl group, 2-(tert-butoxycarbonylamino)ethyl group etc.) and
(19) -(CH₂)ᵣ-NR^{b1}-CONR^{b2}R^{b4} (e.g., ureido group, 3-methylureido group, 3-ethylureido group, 3-isopropylureido group, 3,3-dimethylureido group, 3,3-diethylureido group, 3,3-diisopropylureido group, 3,3-di-tert-butylureido group, 3-ethyl-3-methylureido group, 1,3-dimethylureido group, trimethylureido group, ureidomethyl group, 2-(3,3-dimethylureido)ethyl group etc.).

The "C₁₋₆ alkyl group optionally substituted by 1 to 3 substituents selected from group A" is a group wherein the above-defined "C₁₋₆ alkyl group" is optionally substituted by 1 to 3 substituents selected from the above-defined "group A", which includes non-substituted alkyl group.

Specifically, methyl group, ethyl group, propyl group, isopropyl group, butyl group, isobutyl group, sec-butyl group, tert-butyl group, pentyl group, isopentyl group, tert-pentyl group, neopentyl group, 1-ethylpropyl group, hexyl group, trifluoromethyl group, hydroxymethyl group, 2-hydroxyethyl group, 3-hydroxypropyl group, 4-hydroxybutyl group, 1-hydroxy-1-methylethyl group, 1-hydroxypropan-2-yl group, 1,3-dihydroxypropan-2-yl group, 1-hydroxy-2-methylpropan-2-yl group, carboxymethyl group, ethoxycarbonylmethyl group, 2-carboxyethyl group, methoxymethyl group, methoxyethyl group, methoxyethoxyethyl group, ethoxycarbonylmethyl group, 2-ethoxycarbonylethyl group, 2-dimethylaminoethyl group, carbamoylmethyl group, methylcarbamoylmethyl group, sulfomethyl group, sulfamoylmethyl group, 2-sulfamoylethyl group, methylsulfamoylmethyl group and the like can be mentioned.

The "C₂₋₆ alkenyl group optionally substituted by 1 to 3 substituents selected from group A" is the above-defined "C₂₋₆ alkenyl group" optionally substituted by 1 to 3 substituents selected from the above-defined "group A", which includes non-substituted alkenyl group.

Specifically, vinyl group, allyl group, 1-propenyl group, isopropenyl group, 1-butenyl group, 2-butenyl group, 1,3-butadienyl group, 2-isopentenyl group, 3-isohexenyl group, 4-methyl-3-pentenyl group, 2-carboxyethenyl group and the like can be mentioned.

The "C₂₋₆ alkynyl group optionally substituted by 1 to 3 substituents selected from group A" is the above-defined "C₂₋₆ alkynyl group" optionally substituted by 1 to 3 substituents selected from the above-defined "group A", which includes non-substituted alkynyl group.

Specifically, ethynyl group, 1-propynyl group, 2-propynyl group, 3-butynyl group and the like can be mentioned.

The "C₆₋₁₄ aryl group optionally substituted by 1 to 5 substituents selected from group B" is the above-defined "C₆₋₁₄ aryl group" optionally substituted by 1 to 5 substituents selected from the above-defined "group B", which includes non-substituted aryl group.

Specifically, phenyl group, naphthyl group, anthryl group, indenyl group, azulenyl group, fluorenyl group, phenanthryl group, 3-fluorophenyl group, 4-fluorophenyl group, 3-chlorophenyl group, 4-chlorophenyl group, 2,4-dichlorophenyl group, 3,5-dichlorophenyl group, pentafluorophenyl group, 4-tolyl group, 4-tert-butylphenyl group, 2-trifluoromethylphenyl group, 4-trifluoromethylphenyl group, 4-nitrophenyl group, 4-cyanophenyl group, 4-acetylphenyl group, 4-carboxyphenyl group, 4-carbamoylphenyl group, 4-aminophenyl group, 4-dimethylaminophenyl group, 4-acetylaminophenyl group, 4-(methylsulfonylamino)phenyl group, 4-methoxyphenyl group, 3,4,5-trimethoxyphenyl group, 4-methylthiophenyl group, 4-methylsulfonylphenyl group, 4-aminosulfonylphenyl group, 3-nitro-4-methoxyphenyl group and 4-nitro-3-methoxyphenyl group can be mentioned.

The "C₃₋₈ cycloalkyl group optionally substituted by 1 to 5 substituents selected from group B" is the above-defined "C₃₋₈ cycloalkyl group" optionally substituted by 1 to 5 substituents selected from the above-defined "group B", which includes non-substituted cycloalkyl group.

Specifically, cyclopropyl group, cyclobutyl group, cyclopentyl group, cyclohexyl group, cycloheptyl group, 4-fluorocyclohexyl group, 2-methylcyclopentyl group, 3-methylcyclohexyl group, 4-methylcyclohexyl group, 4,4-dimethylcyclohexyl group, 3,5-dimethylcyclohexyl group, 4-tert-butylcyclohexyl group, 4-hydroxycyclohexyl group, 4-methoxycyclohexyl group, 2,3,4,5,6-pentafluorocyclohexyl group can be mentioned.

The "C₃₋₈ cycloalkenyl group optionally substituted by 1 to 5 substituents selected from group B" is the above-defined "C₃₋₈ cycloalkenyl group" optionally substituted by 1 to 5 substituents selected from the above-defined "group B", which includes non-substituted cycloalkenyl group.

Specifically, cyclopropenyl group, cyclobutenyl group, cyclopentenyl group, cyclopentadienyl group, cyclohexenyl group (cyclohex-1-enyl group, cyclohex-2-enyl group, cyclohex-3-enyl group), 5-methylcyclohex-3-enyl group, 5-methoxycyclohex-3-enyl group, 5-acetylcyclohex-3-enyl group, 2,4-cyclohexadien-1-yl group, 2,5-cyclohexadien-1-yl group, cycloheptenyl group and cyclooctenyl group and the like can be mentioned.

The "heterocyclic group optionally substituted by 1 to 5 substituents selected from group B" is the above-defined "heterocyclic group" optionally substituted by 1 to 5 substituents selected from the above-defined "group B", which includes non-substituted heterocyclic group.

Specifically, 2-pyridyl group, 3-pyridyl group, 4-pyridyl group, 3-fluoropyridin-4-yl group, 3-chloropyridin-4-yl group, 4-chloropyridin-3-yl group, pyrazinyl group, pyrimidinyl group, pyridazinyl group, 1,3,5-triazinyl group, pyrrolyl group, pyrazolyl group, imidazolyl group, 1,2,4-triazolyl group, tetrazolyl group, 2-thienyl group, 3-thienyl group, furyl group, oxazolyl group, 2-methyloxazol-4-yl group, isoxazolyl group, thiazolyl group, 2-methylthiazol-4-yl group, 2,5-dimethylthiazol-4-yl group, 2,4-dimethylthiazol-5-yl group, isothiazolyl group, thiadiazolyl group, pyrrolinyl group, pyrrolidinyl group, 3-hydroxypyrrolidinyl group, imidazolidinyl group, azetidinyl group, piperidyl group, 3-hydroxypiperidino group, 4-hydroxypiperidino group, 3,4-dihydroxypiperidino group, 4-methoxypiperidino group, 4-carboxypiperidino group, 4-(hydroxymethyl)piperidino group, 2,2,6,6-tetramethylpiperidino group, 2,2,6,6-tetramethyl-4-hydroxypiperidino group, N-methylpiperidin-4-yl group, N-(tert-butoxycarbonyl)piperidin-4-yl group, N-acetylpiperidin-4-yl group, N-methylsulfonylpiperidin-4-yl group, piperazinyl group, 4-methylpiperazinyl group, 4-methylsulfonylpiperazinyl group, morpholinyl group, thiomorpholinyl group, 1-oxothiomorpholin-4-yl group, 1,1-dioxothiomorpholin-4-yl group, tetrahydropyranyl group, quinolyl group, isoquinolyl group, quinazolinyl group, quinoxalinyl group, phthalazinyl group, cinnolinyl group, naphthyridinyl group, 5,6,7,8-tetrahydroquinolyl group, indolyl group, benzimidazolyl group, indolinyl group, benzofuranyl group, benzothienyl group, benzoxazolyl group, benzothiazolyl group, 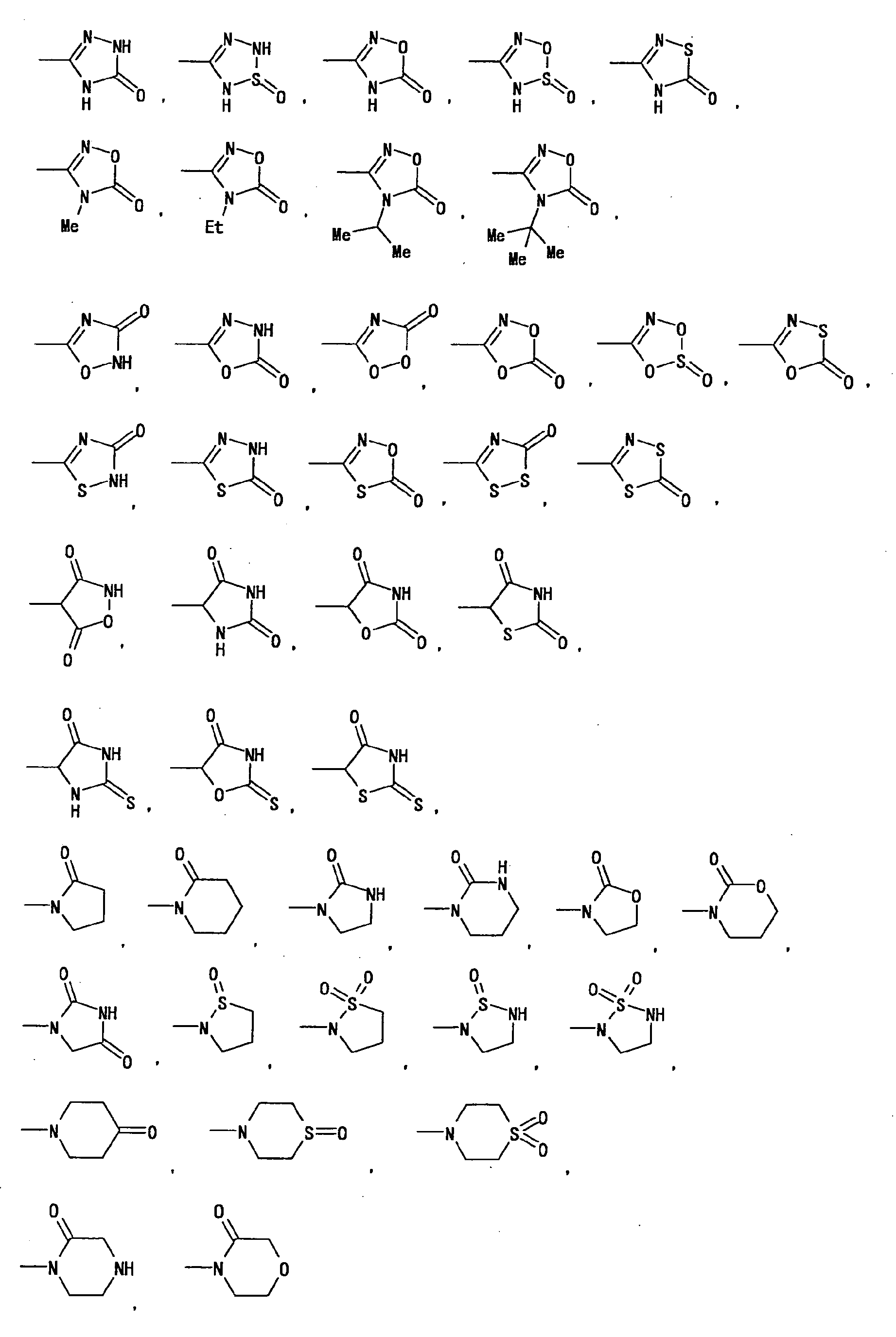 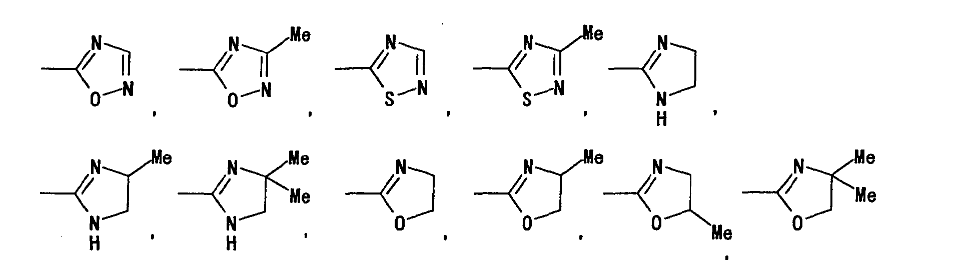 and the like can be mentioned.

For ring Cy, preferable "heterocyclic group optionally substituted by 1 to 5 substituents selected from group B" is 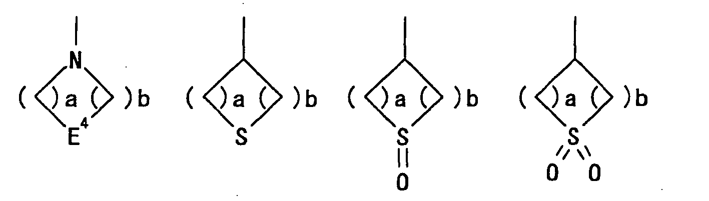 wherein E⁴ is an oxygen atom, a sulfur atom, CH₂ or N(-R^{Cy1}),
wherein R^{Cy1} is a hydrogen atom or a C₁₋₆ alkyl group, and a and b are each independently an integer of 1 to 3.

Specifically, pyrrolidinyl group, imidazolidinyl group, piperidyl group, piperazinyl group, morpholinyl group, thiomorpholinyl group, tetrahydropyranyl group, tetrahydrothiopyranyl group, 1-oxotetrahydrothiopyranyl group, 1,1-dioxotetrahydrothiopyranyl group and the like can be mentioned.

The "C₆₋₁₄ aryl C₁₋₆ alkyl group optionally substituted by 1 to 5 substituents selected from group B" is the above-defined "C₁₋₆ alkyl group" substituted by the above-defined "C₆₋₁₄ aryl group optionally substituted by 1 to 5 substituents selected from group B".

Specifically, benzyl group, 1-naphthylmethyl group, 2-naphthylmethyl group, phenethyl group, 3-phenylpropyl group, 2-phenylpropyl group, 3-fluorobenzyl group, 4-fluorobenzyl group, 3-chlorobenzyl group, 4-chlorobenzyl group, 2,4-dichlorobenzyl group, 3,5-dichlorobenzyl group, pentafluorobenzyl group, 4-methylbenzyl group, 4-tert-butylbenzyl group, 2-trifluoromethylbenzyl group, 4-trifluoromethylbenzyl group, 4-nitrobenzyl group, 4-cyanobenzyl group, 4-acetylbenzyl group, 4-carboxybenzyl group, 4-carbamoylbenzyl group, 4-aminobenzyl group, 4-dimethylaminobenzyl group, 4-acetylaminobenzyl group, 4-(methylsulfonylamino)benzyl group, 4-methoxybenzyl group, 3,4,5-trimethoxybenzyl group, 4-methylthiobenzyl group, 4-methylsulfonylbenzyl group, 4-aminosulfonylbenzyl group, 3-nitro-4-methoxybenzyl group, 4-nitro-3-methoxybenzyl group can be mentioned.

The "heterocycle C₁₋₆ alkyl group optionally substituted by 1 to 5 substituents selected from group B" is the above-defined "C₁₋₆ alkyl group" substituted by the above-defined "heterocyclic group optionally substituted by 1 to 5 substituents selected from group B".

Specifically, 2-pyridylmethyl group, 3-pyridylmethyl group, 2-chloropyridin-4-ylmethyl group, 4-pyridylmethyl group, pyrrolylmethyl group, imidazolylmethyl group, 2-thienylmethyl group, 3-thienylmethyl group, 2-furylmethyl group, 2-oxazolylmethyl group, 5-isothiazolylmethyl group, 2-methyloxazol-4-ylmethyl group, 2-thiazolylmethyl group, 4-thiazolylmethyl group, 5-thiazolylmethyl group, 2-methylthiazol-4-ylmethyl group, 2-methylthiazol-5-ylmethyl group, 2,5-dimethylthiazol-4-ylmethyl group, 4-methylthiazol-2-ylmethyl group, 2,4-dimethylthiazol-5-ylmethyl group, 2-isothiazolylmethyl group, 2-pyrrolinylmethyl group, pyrrolidinylmethyl group, piperidylmethyl group, 4-piperidylmethyl group, 1-methylpiperidin-4-ylmethyl group, 4-hydroxypiperidinomethyl group, 3-hydroxypyrrolidinylmethyl group, 2-(4-hydroxypiperidino)ethyl group, 1-(tert-butoxycarbonyl)piperidin-4-ylmethyl group, 1-acetylpiperidin-4-ylmethyl group, 1-methylsulfonylpiperidin-4-ylmethyl group, piperazinylmethyl group, morpholinomethyl group, thiomorpholinylmethyl group, 1-tetrahydropyranylmethyl group, 2-quinolylmethyl group, 1-isoquinolylmethyl group and the like can be mentioned.

The "C₃₋₈ cycloalkyl C₁₋₆ alkyl group optionally substituted by 1 to 5 substituents selected from group B" is the above-defined "C₁₋₆ alkyl group" substituted by the above-defined "C₃₋₈ cycloalkyl group optionally substituted by 1 to 5 substituents selected from group B".

Specifically, cyclopropylmethyl group, cyclobutylmethyl group, cyclopentylmethyl group, cyclohexylmethyl group, 2-(cyclopentyl)ethyl group, 2-(cyclohexyl)ethyl group, cycloheptylmethyl group, 4-fluorocyclohexylmethyl group, 2-methylcyclopentylmethyl group, 3-methylcyclohexylmethyl group, 4-methylcyclohexylmethyl group, 4,4-dimethylcyclohexylmethyl group, 3,5-dimethylcyclohexylmethyl group, 4-tert-butylcyclohexylmethyl group, 4-hydroxycyclohexylmethyl group, 4-methoxycyclohexylmethyl group, 2,3,4,5,6-pentafluorocyclohexylmethyl group can be mentioned.

The "group C" means the substituent groups of the following (1) to (5).
(1) the above-defined "C₁₋₆ alkyl group optionally substituted by 1 to 3 substituents selected from group A",
(2) the above-defined "C₆₋₁₄ aryl group optionally substituted by 1 to 5 substituents selected from group B",
(3) the above-defined "C₆₋₁₄ aryl C₁₋₆ alkyl group optionally substituted by 1 to 5 substituents selected from group B",
(4) the above-defined "heterocyclic group optionally substituted by 1 to 5 substituents selected from group B" and
(5) the above-defined "heterocycle C₁₋₆ alkyl group optionally substituted by 1 to 5 substituents selected from group B".

The "group F" means the substituent groups of the following (1) to (7).
(1) the above-defined "C₁₋₆ alkyl group optionally substituted by 1 to 3 substituents selected from group A",
(2) the above-defined "C₆₋₁₄ aryl group optionally substituted by 1 to 5 substituents selected from group B",
(3) the above-defined "heterocyclic group optionally substituted by 1 to 5 substituents selected from group B",
(4) the above-defined "C₃₋₈ cycloalkyl group optionally substituted by 1 to 5 substituents selected from group B",
(5) the above-defined "C₆₋₁₄ aryl C₁₋₆ alkyl group optionally substituted by 1 to 5 substituents selected from group B",
(6) the above-defined "heterocycle C₁₋₆ alkyl group optionally substituted by 1 to 5 substituents selected from group B" and
(7) the above-defined "C₃₋₈ cycloalkyl C₁₋₆ alkyl group optionally substituted by 1 to 5 substituents selected from group B".

The "group D" means the substituent groups of the following (a) to (u).
(in the following, each t independently means 0 or an integer of 1 to 6)
(a) a hydrogen atom,
(b) the above-defined "halogen atom",
(c) a cyano group,
(d) a nitro group,
(e) the above-defined "C₁₋₆ alkyl group optionally substituted by 1 to 3 substituents selected from group A",
(f) -(CH₂)ₜ-OR^{d1},
   wherein R^{d1} is
   (1) a hydrogen atom,
   (2) the above-defined "group selected from group F",
   (3) the above-defined "C₂₋₆ alkenyl group optionally substituted by 1 to 3 substituents selected from group A" or
   (4) the above-defined "C₂₋₆ alkynyl group optionally substituted by 1 to 3 substituents selected from group A",
      (e.g., substituent exemplified for "-(CH₂)ᵣ-OR^{b1}" in group B, trifluoromethyloxy group, methoxymethoxy group, phenoxy group, benzyloxy group, 4-pyridylmethoxy group, 4-carboxybenzyloxy group, vinyloxy group, ethynyloxy group etc.)
(g) -(CH₂)ₜ-S(O)_{q}-R^{d2},
   wherein R^{d2} is
   (1) a hydrogen atom or
   (2) the above-defined "group selected from group F",
      q is 0, 1, 2 or 3
      (e.g., substituent exemplified for "-(CH₂)ᵣ-SR^{b1}" and
      "-(CH₂)ᵣ-SO₂R^{b3}" in group B, methylsulfinyl group, sulfo group, trifluoromethanesulfonyl group, 2-(methylamino)ethylsulfonyl group, 2-(dimethylamino)ethylsulfonyl group, 3-(dimethylamino)propylsulfonyl group, phenylsulfonyl group, 4-tolylsulfonyl group, benzylsulfonyl group etc.)
(h) -(CH₂)ₜ-NR^{d3}R^{d4},
   wherein R^{d3} and R^{d4} are each independently
   (1) a hydrogen atom or
   (2) the above-defined "group selected from group F", (e.g., substituent exemplified for "-(CH₂)ᵣ-NR^{b1}R^{b2}" in group B, phenylamino group, benzyloxyamino group, methoxymethylamino group, N-ethyl-N-(carbamoylmethyl)amino group, N-ethyl-N-[2-(acetylamino)ethyl]amino group, N-[2-amino-2-(dimethylcarbamoyl)ethyl]-N-ethylamino group, N,N-bis(aminomethyl)amino group etc.)
(i) -(CH₂)ₜ-COOR^{d5},
   wherein R^{d5} is
   (1) a hydrogen atom or
   (2) the above-defined "group selected from group F",
      (e.g., substituent exemplified for "-(CH₂)ᵣ-COOR^{b1}" in group B, trifluoromethyloxycarbonyl group, phenoxycarbonyl group, benzyloxycarbonyl group, 2-morpholinoethoxycarbonyl group, 2-(dimethylamino)ethoxycarbonyl group etc.)
(j) -(CH₂)ₜ-CONR^{d6}R^{d7},
   wherein R^{d6} and R^{d7} are each independently
   (1) a hydrogen atom,
   (2) a hydroxyl group,
   (3) the above-defined "group selected from group F" or
   (4) the above-defined "C₁₋₆ alkoxy group",
      (e.g., substituent exemplified for "-(CH₂)ᵣ-CONR^{b1}R^{b2}" in group B, hydroxycarbamoyl group, methoxycarbamoyl group, phenylcarbamoyl group, benzylcarbamoyl group, 2-morpholinoethylcarbamoyl group, 2-(dimethylamino)ethylcarbamoyl group, methoxymethylcarbamoyl group etc.)
(k) -(CH₂)ₜ-COR^{d8},
   wherein R^{d8} is
   (1) a hydrogen atom or
   (2) the above-defined "group selected from group F",
      (e.g., substituent exemplified for "-(CH₂)ᵣ-COR^{b1}" in group B, trifluoroacetyl group, methoxyacetyl group, carboxyacetyl group, benzoyl group, phenylacetyl group, 3-(dimethylamino)propionyl group, 3-morpholinopropionyl group etc.)
(1) -(CH₂)ₜ-NR^{d9}CO-R^{d10},
   wherein R^{d9} is
   (1) a hydrogen atom,
   (2) the above-defined "C₁₋₆ alkyl group optionally substituted by 1 to 3 substituents selected from group A" or
   (3) the above-defined "C₁₋₆ alkanoyl group",
   R^{d10} is
   (1) a hydrogen atom,
   (2) an amino group,
   (3) the above-defined "C₁₋₆ alkylamino group" or
   (4) the above-defined "group selected from group F",
      (e.g., substituent exemplified for "-(CH₂)ᵣ-NR^{b1}-COR^{b2}" in group B, ureido group, 3-methylureido group, 3-ethylureido group, 3-isopropylureido group, 3,3-dimethylureido group, 3,3-diethylureido group, 3,3-diisopropylureido group, 3,3-di-tert-butylureido group, 3-ethyl-3-methylureido group, 1,3-dimethylureido group, trimethylureido group, ureidomethyl group, 2-(3,3-dimethylureido)ethyl group, benzoylamino group, phenylacetylamino group, trifluoroacetylamino group, methylaminoacetylamino group, N-acetyl-N-methylamino group, N-isopropyl-N-pivaloylamino group, dimethylaminoacetylamino group, N-(dimethylaminoacetyl)-N-methylamino group, morpholinoacetylamino group, N-methyl-N-(morpholinoacetyl)amino group, N-isobutyryl-N-methylamino group etc.)
(m) - (CH₂)ₜ-NR^{d11}SO₂-R^{d12},
   wherein R^{d11} is
   (1) a hydrogen atom,
   (2) the above-defined "C₁₋₆ alkyl group optionally substituted by 1 to 3 substituents selected from group A" or
   (3) the above-defined "C₁₋₆ alkanoyl group",
   R^{d12} is
   (1) a hydrogen atom or
   (2) the above-defined "group selected from group F",
      (e.g., substituent exemplified for "-(CH₂)ᵣ-NR^{b1}-SO₂R^{b3}" in group B, trifluoromethylsulfonylamino group, phenylsulfonylamino group, benzylsulfonylamino group, 2-(dimethylamino)ethylsulfonylamino group, 2-morpholinoethylsulfonylamino group, N-acetyl-N-methanesulfonylamino group, N-benzyl-N-methanesulfonylamino
      group etc.)
(n) -(CH₂)ₜ-SO₂-NR^{d13}R^{d14},
   wherein R^{d13} and R^{d14} are each independently
   (1) a hydrogen atom or
   (2) the above-defined "group selected from group F",
      (e. g. , substituent exemplified for "-(CH₂)ᵣ-SO₂NR^{b1}R^{b2}" in group B, trifluoromethylsulfamoyl group, 2-(dimethylamino)ethylsulfamoyl group, phenylsulfamoyl group, benzylsulfamoyl group, 2-morpholinoethylsulfamoyl group etc.)
(o) -(CH₂)ₜ-CONR^{d15}-SO₂R^{d16},
   wherein R^{d15} and R^{d16} are each independently
   (1) a hydrogen atom or
   (2) the above-defined "group selected from group F",
      (e. g. , substituent exemplified for "- (CH₂)ᵣ-CONR^{b1}-SO₂R^{b3}" in group B, trifluoromethylsulfonylcarbamoyl group, 2-(dimethylamino)ethylsulfonylcarbamoyl group, phenylsulfonylcarbamoyl group, benzylsulfonylcarbamoyl group, 2-morpholinoethylsulfonylcarbamoyl group, N-benzyl-N-(methanesulfonyl)carbamoyl group etc.)
(p) -(CH₂)ₜSO₂NR^{d17}-COR^{d18},
   wherein R^{d17} is
   (1) a hydrogen atom or
   (2) the above-defined "group selected from group F",
      R^{d18} is the above-defined "group selected from group F", (e.g., substituent exemplified for "-(CH₂)ᵣ-SO₂NR^{b1}-COR^{b2}" in group B, trifluoroacetylsulfamoyl group, 2-(dimethylamino)ethylcarbonylsulfamoyl group, benzoylsulfamoyl group, phenylacetylsulfamoyl group, 3-morpholinopropionylsulfamoyl group, N-acetyl-N-benzylsulfamoyl group etc.)
(q) -(CH₂)ₜ-NR^{d19}-COOR^{d20},
   wherein R^{d19} and R^{d20} are each independently
   (1) a hydrogen atom or
   (2) the above-defined "group selected from group F",
      (e.g., substituent exemplified for "-(CH₂)ᵣ-NR^{b1}-COOR^{b3}" in group B, trifluoromethyloxycarbonylamino group, 2-(dimethylamino)ethoxycarbonylamino group, phenoxycarbonylamino group, benzyloxycarbonylamino group, 2-morpholinoethoxycarbonylamino group, N-ethoxycarbonyl-N-benzylamino group etc.)
(r) -(CH₂)ₜ-NR^{d21}-CONR^{d22}R^{d23},
   wherein R^{d21}, R^{d22} and R^{d23} are each independently
   (1) a hydrogen atom or
   (2) the above-defined "group selected from group F",
      (e.g., substituent exemplified for "-(CH₂)ᵣ-NR^{b1}-CONR^{b2}R^{b4}" in group B etc.)
(s)-(CH₂)ₜ-C(=NR^{d24})NH₂,
   wherein R^{d24} is
   (1) a hydrogen atom,
   (2) a hydroxyl group,
   (3) the above-defined "C₁₋₆ alkyl group optionally substituted by 1 to 3 substituents selected from group A" or
   (4) the above-defined "C₁₋₆ alkoxy group",
      (e.g., carbamimidoyl group, N-hydroxycarbamimidoyl group, N-methylcarbamimidoyl group, N-methoxycarbamimidoyl group, N-(2-methoxyethyl)carbamimidoyl group etc.)
(t) -(CH₂)ₜ-O-(CH₂)ₚ-COR^{d25},
   wherein R^{d25} is
   (1) an amino group,
   (2) the above-defined "C₁₋₆ alkylamino group" or
   (3) the above-defined "heterocyclic group optionally substituted by 1 to 5 substituents selected from group B", p is 0 or an integer of 1 to 6
      (e.g., carbamoylmethoxy group, methylcarbamoylmethoxy group, 2-(dimethylcarbamoyl)ethoxy group, 2-oxo-2-(pyridin-2-yl)ethoxy group, 2-oxo-2-(piperidin-1-yl)ethoxy group, 2-oxo-2-(piperazin-1-yl)ethoxy group, 2-oxo-2-(pyrrolidin-1-yl)ethoxy group, 2-(morpholin-4-yl)-2-oxoethoxy group etc.)
      and
(u) the above-defined "heterocyclic group optionally substituted by 1 to 5 substituents selected from group B".

The "group E" means the substituent groups of the following (a) to (r).
(a) the above-defined "halogen atom",
(b) a cyano group,
(c) a nitro group,
(d) -OR^{e1},
   wherein R^{e1} is
   (1) a hydrogen atom,
   (2) the above-defined "group selected from group F",
   (3) the above-defined "C₂₋₆ alkenyl group optionally substituted by 1 to 3 substituents selected from group A" or
   (4) the above-defined "C₂₋₆ alkynyl group optionally substituted by 1 to 3 substituents selected from group A",
      (e.g., hydroxyl group, methoxy group, ethoxy group, isopropyloxy group, tert-butyloxy group, trifluoromethyloxy group, methoxymethoxy group, phenoxy group, benzyloxy group, 4-pyridylmethoxy group, 4-carboxybenzyloxy group, vinyloxy group, ethynyloxy group etc.)
(e) -S(O)_{q}-R^{e2},
   wherein R^{e2} is
   (1) a hydrogen atom or
   (2) the above-defined "group selected from group F", q is 0, 1, 2 or 3
      (e.g., mercapto group, methylsulfanyl group, methanesulfonyl group, ethylsulfonyl group, isopropylsulfonyl group, tert-butylsulfonyl group, methylsulfinyl group, sulfo group, trifluoromethanesulfonyl group, 2-(methylamino)ethylsulfonyl group, 2-(dimethylamino)ethylsulfonyl group, 3-(dimethylamino)propylsulfonyl group, phenylsulfonyl group, 4-tolylsulfonyl group, benzylsulfonyl group etc.)
(f) -NR^{e3}R^{e4},
   wherein R^{e3} and R^{e4} are each independently
   (1) a hydrogen atom or
   (2) the above-defined "group selected from group F",
      (e.g., amino group, methylamino group, ethylamino group, isopropylamino group, dimethylamino group, diethylamino group, diisopropylamino group, di-tert-butylamino group, N-ethyl-N-methylamino group, phenylamino group, benzyloxyamino group, methoxymethylamino group, N-ethyl-N-(carbamoylmethyl)amino group, N-ethyl-N-[2-(acetylamino)ethyl]amino group, N-[2-amino-2-(dimethylcarbamoyl)ethyl]-N-ethylamino group, N,N-bis(aminomethyl)amino group etc.)
(g) -COOR^{e5},
   wherein R^{e5} is
   (1) a hydrogen atom or
   (2) the above-defined "group selected from group F",
      (e.g., carboxyl group, methoxycarbonyl group, ethoxycarbonyl group, isopropyloxycarbonyl group, tert-butoxycarbonyl group, trifluoromethyloxycarbonyl group, phenoxycarbonyl group, benzyloxycarbonyl group, 2-morpholinoethoxycarbonyl group, 2-(dimethylamino)ethoxycarbonyl group etc.)
(h) -CONR^{e6}R^{e7},
   wherein R^{e6} and R^{e7} are each independently
   (1) a hydrogen atom,
   (2) a hydroxyl group,
   (3) the above-defined "group selected from group F" or
   (4) the above-defined "C₁₋₆ alkoxy group",
      (e.g., carbamoyl group, methylcarbamoyl group, ethylcarbamoyl group, isopropylcarbamoyl group, dimethylcarbamoyl group, diethylcarbamoyl group, diisopropylcarbamoyl group, di-tert-butylcarbamoyl group, N-ethyl-N-methylcarbamoyl group, hydroxycarbamoyl group, methoxycarbamoyl group, phenylcarbamoyl group, benzylcarbamoyl group, 2-morpholinoethylcarbamoyl group, 2-(dimethylamino)ethylcarbamoyl group, methoxymethylcarbamoyl group etc.)
(i) -COR^{e8},
   wherein R^{e8} is
   (1) a hydrogen atom or
   (2) the above-defined "group selected from group F",
      (e.g., formyl group, acetyl group, propionyl group, isobutyryl group, pivaloyl group, trifluoroacetyl group, methoxyacetyl group, carboxyacetyl group, benzoyl group, phenylacetyl group, 3-(dimethylamino)propionyl group, 3-morpholinopropionyl group etc.)
(j) -NR^{e9}CO-R^{e10},
   wherein R^{e9} is
   (1) a hydrogen atom,
   (2) the above-defined "C₁₋₆ alkyl group" or
   (3) the above-defined "C₁₋₆ alkanoyl group",
   R^{e10} is
   (1) a hydrogen atom,
   (2) an amino group,
   (3) the above-defined "C₁₋₆ alkylamino group" or
   (4) the above-defined "group selected from group F",
      (e.g., formylamino group, acetylamino group, propionylamino group, isobutyrylamino group, pivaloylamino group, N-acetyl-N-methylamino group, ureido group, 3-methylureido group, 3-ethylureido group, 3-isopropylureido group, 3,3-dimethylureido group, 3,3-diethylureido group, 3,3-diisopropylureido group, 3,3-di-tert-butylureido group, 3-ethyl-3-methylureido group, 1,3-dimethylureido group, trimethylureido group, benzoylamino group, phenylacetylamino group, trifluoroacetylamino group, methylaminoacetylamino group, N-acetyl-N-methylamino group, N-isopropyl-N-pivaloylamino group etc.)
(k) -NR^{e11}SO₂-R^{e12},
   wherein R^{e11} is
   (1) a hydrogen atom,
   (2) the above-defined "C₁₋₆ alkyl group" or
   (3) the above-defined "C₁₋₆ alkanoyl group",
   R^{e12} is
   (1) a hydrogen atom or
   (2) the above-defined "group selected from group F",
      (e.g., methanesulfonylamino group, ethylsulfonylamino group, isopropylsulfonylamino group, tert-butylsulfonylamino group, N-methyl-N-(methanesulfonyl)amino group, trifluoromethylsulfonylamino group, phenylsulfonylamino group, benzylsulfonylamino group, 2-(dimethylamino)ethylsulfonylamino group, 2-morpholinoethylsulfonylamino group, N-acetyl-N-methanesulfonylamino group, N-benzyl-N-methanesulfonylamino group etc.)
(l) -SO₂-NR^{e13}R^{e14},
   wherein R^{e13} and R^{e14} are each independently
   (1) a hydrogen atom or
   (2) the above-defined "group selected from group F",
      (e.g., sulfamoyl group, methylsulfamoyl group, ethylsulfamoyl group, isopropylsulfamoyl group, dimethylsulfamoyl group, diethylsulfamoyl group, diisopropylsulfamoyl group, di-tert-butylsulfamoyl group, trifluoromethylsulfamoyl group, 2-(dimethylamino)ethylsulfamoyl group, phenylsulfamoyl group, benzylsulfamoyl group, 2-morpholinoethylsulfamoyl group etc.)
(m) -CONR^{e15}-SO₂R^{e16},
   wherein R^{e15} and R^{e16} are each independently
   (1) a hydrogen atom or
   (2) the above-defined "group selected from group F",
      (e.g., methanesulfonylcarbamoyl group, ethylsulfonylcarbamoyl group, isopropylsulfonylcarbamoyl group, tert-butylsulfonylcarbamoyl group, N-methyl-N-(methanesulfonyl)carbamoyl group, trifluoromethylsulfonylcarbamoyl group, 2-(dimethylamino)ethylsulfonylcarbamoyl group, phenylsulfonylcarbamoyl group, benzylsulfonylcarbamoyl group, 2-morpholinoethylsulfonylcarbamoyl group, N-benzyl-N-(methanesulfonyl)carbamoyl group etc.)
(n) -SO₂NR^{e17}-COR^{e18},
   wherein R^{e17} is
   (1) a hydrogen atom or
   (2) the above-defined "group selected from group F",
      R^{e18} is the above-defined "group selected from group F",
      (e.g., acetylsulfamoyl group, propionylsulfamoyl group, isobutyrylsulfamoyl group, pivaloylsulfamoyl group, N-acetyl-N-methylsulfamoyl group, trifluoroacetylsulfamoyl group, 2-(dimethylamino)ethylsulfamoyl group, benzoylsulfamoyl group, phenylacetylsulfamoyl group, 3-morpholinopropionylsulfamoyl group, N-acetyl-N-benzylsulfamoyl group etc.)
(o) -NR^{e19}-COOR^{e20},
   wherein R^{e19} and R^{e20} are each independently
   (1) a hydrogen atom or
   (2) the above-defined "group selected from group F",
      (e.g., methoxycarbonylamino group, ethoxycarbonylamino group, isopropyloxycarbonylamino group, tert-butoxycarbonylamino group, trifluoromethyloxycarbonylamino group, 2-(dimethylamino)ethyloxycarbonylamino group, phenoxycarbonylamino group, benzyloxycarbonylamino group, 2-morpholinoethoxycarbonylamino group, N-ethoxycarbonyl-N-benzylamino group etc.)
(p) -NR^{e21}-CONR^{e22}R^{e23}
   wherein R^{e21}, R^{e22} and R^{e23} are each independently
   (1) a hydrogen atom or
   (2) the above-defined "group selected from group F",
      (e.g., ureido group, 3-methylureido group, 3-ethylureido group, 3-isopropylureido group, 3,3-dimethylureido group, 3,3-diethylureido group, 3,3-diisopropylureido group, 3,3-di-tert-butylureido group, 3-ethyl-3-methylureido group, 1,3-dimethylureido group, trimethylureido group etc.)
(q) the above-defined "aryl group optionally substituted by 1 to 5 substituents selected from group B" and
(r) the above-defined "heterocyclic group optionally substituted by 1 to 5 substituents selected from group B".

The "C₆₋₁₄ aryl group optionally substituted by 1 to 5 substituents selected from group D" is the above-defined "C₆₋₁₄ aryl group" optionally substituted by 1 to 5 substituents selected from the above-defined "group D", which includes non-substituted aryl group.

Specifically, phenyl group, naphthyl group, anthryl group, indenyl group, azulenyl group, fluorenyl group, phenanthryl group, 3-fluorophenyl group, 4-fluorophenyl group, 3-chlorophenyl group, 4-chlorophenyl group, 2,4-dichlorophenyl group, 3,5-dichlorophenyl group, 4-bromophenyl group, 4-nitrophenyl group, pentafluorophenyl group, 4-methylphenyl group, 4-tert-butylphenyl group, 2-trifluoromethylphenyl group, 4-trifluoromethylphenyl group, 4-(hydroxymethyl)phenyl group, 4-(methoxymethyl)phenyl group, 4-(2-carboxyethyl)phenyl group, 3-carboxyphenyl group, 4-carboxyphenyl group, 4-methoxyphenyl group, 3,4,5-trimethoxyphenyl group, 4-carbamoylphenyl group, 4-methylthiophenyl group, 4-(dimethylaminocarbonyl)phenyl group, 4-methylsulfonylphenyl group, 4-acetylaminophenyl group, 4-cyanophenyl group, 4-acetylphenyl group, 4-aminophenyl group, 4-dimethylaminophenyl group, 4-(methylsulfonylamino)phenyl group, 4-methylsulfinylphenyl group, 4-aminosulfonylphenyl group, 3-nitro-4-methoxyphenyl group, 4-nitro-3-methoxyphenyl group and 4-(tetrazol-5-yl)phenyl group can be mentioned.

The "C₃₋₈ cycloalkyl group optionally substituted by 1 to 5 substituents selected from group D" is the above-defined "C₃₋₈ cycloalkyl group" optionally substituted by 1 to 5 substituents selected from the above-defined "group D", which includes non-substituted cycloalkyl group.

Specifically, cyclopropyl group, cyclobutyl group, cyclopentyl group, cyclohexyl group, cycloheptyl group, 4-fluorocyclohexyl group, 2-methylcyclopentyl group, 3-methylcyclohexyl group, 4-methylcyclohexyl group, 4,4-dimethylcyclohexyl group, 3,5-dimethylcyclohexyl group, 4-tert-butylcyclohexyl group, 4-hydroxycyclohexyl group, 4-methoxycyclohexyl group and 2,3,4,5,6-pentafluorocyclohexyl group can be mentioned.

In addition, such group wherein cyclopentyl group or cyclohexyl group is substituted by fluorine atom, chlorine atom, bromine atom, nitro group, methyl group, tert-butyl group, carboxyl group, trifluoromethyl group, hydroxymethyl group, methoxymethyl group, 2-carboxyethyl group, methoxy group, carbamoyl group, methylthio group, dimethylaminocarbonyl group, methylsulfonyl group or acetylamino group can be mentioned.

The "heterocyclic group optionally substituted by 1 to 5 substituents selected from group D" is the above-defined "heterocyclic group" optionally substituted by 1 to 5 substituents selected from the above-defined "group D", which includes non-substituted heterocyclic group.

Specifically, 2-pyridyl group, 3-pyridyl group, 4-pyridyl group, 3-fluoropyridin-4-yl group, 3-chloropyridin-4-yl group, 4-chloropyridin-3-yl group, pyrazinyl group, pyrimidinyl group, pyridazinyl group, 1,3,5-triazinyl group, pyrrolyl group, pyrazolyl group, imidazolyl group, 1,2,4-triazolyl group, tetrazolyl group, 2-thienyl group, 3-thienyl group, furyl group, oxazolyl group, 2-methyloxazol-4-yl group, isoxazolyl group, thiazolyl group, 2-methylthiazol-4-yl group, 2,5-dimethylthiazol-4-yl group, 2,4-dimethylthiazol-5-yl group, isothiazolyl group, thiadiazolyl group, pyrrolinyl group, pyrrolidinyl group, imidazolidinyl group, piperidyl group, N-methylpiperidin-4-yl group, N-(tert-butoxycarbonyl)piperidin-4-yl group, N-acetylpiperidin-4-yl group, N-methylsulfonylpiperidin-4-yl group, piperazinyl group, morpholinyl group, thiomorpholinyl group, tetrahydropyranyl group, quinolyl group, isoquinolyl group, quinazolinyl group, quinoxalinyl group, phthalazinyl group, cinnolinyl group, naphthyridinyl group, 5,6,7,8-tetrahydroquinolyl group, indolyl group, benzimidazolyl group, indolinyl group, benzofuranyl group, benzothienyl group, benzoxazolyl group, benzothiazolyl group, 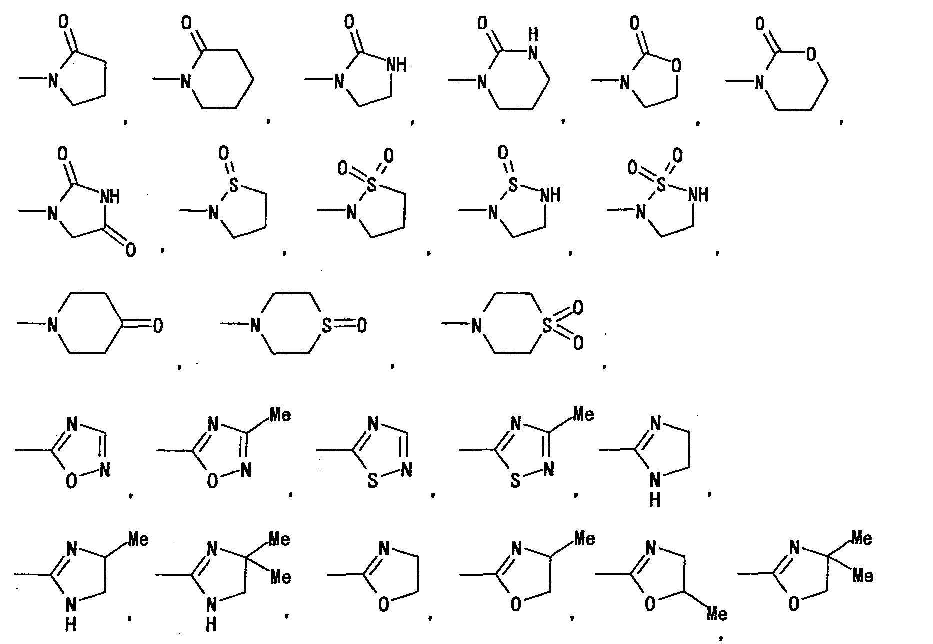 and the like can be mentioned.

In addition, such group wherein the 3, 4, 5 or 6-position of 2-pyridyl group, 2, 4, 5 or 6-position of 3-pyridyl group, 2, 3, 5 or 6-position of 4-pyridyl group, 3, 4 or 5-position of 2-thienyl group, and 2, 4 or 5-position of 3-thienyl group are substituted by fluorine atom, chlorine atom, bromine atom, nitro group, methyl group, tert-butyl group, carboxyl group, trifluoromethyl group, hydroxymethyl group, methoxymethyl group, 2-carboxyethyl group, methoxy group, carbamoyl group, methylthio group, dimethylaminocarbonyl group, methylsulfonyl group, amino group or acetylamino group can be mentioned.

The "C₆₋₁₄ aryl C₁₋₆ alkyl group optionally substituted by 1 to 5 substituents selected from group D" is the above-defined "C₁₋₆ alkyl group" substituted by the above-defined "C₆₋₁₄ aryl group optionally substituted by 1 to 5 substituents selected from group D".

Specifically, benzyl group, 1-naphthylmethyl group, 2-naphthylmethyl group, phenethyl group, 3-phenylpropyl group, 2-phenylpropyl group, 3-fluorobenzyl group, 4-fluorobenzyl group, 3-chlorobenzyl group, 4-chlorobenzyl group, 2,4-dichlorobenzyl group, 3,5-dichlorobenzyl group, 4-bromobenzyl group, 4-nitrobenzyl group, pentafluorobenzyl group, 4-methylbenzyl group, 4-tert-butylbenzyl group, 2-trifluoromethylbenzyl group, 4-trifluoromethylbenzyl group, 4-(hydroxymethyl)benzyl group, 4-(methoxymethyl)benzyl group, 4-(2-carboxyethyl)benzyl group, 3-carboxybenzyl group, 4-carboxybenzyl group, 4-methoxybenzyl group, 3,4,5-trimethoxybenzyl group, 4-carbamoylbenzyl group, 4-methylthiobenzyl group, 4-(dimethylaminocarbonyl)benzyl group, 4-methylsulfonylbenzyl group, 4-(acetylamino)benzyl group, 4-cyanobenzyl group, 4-acetylbenzyl group, 4-aminobenzyl group, 4-dimethylaminobenzyl group, 4-(methylsulfonylamino)benzyl group, 4-methylsulfinylbenzyl group, 4-aminosulfonylbenzyl group, (3-nitro-4-methoxyphenyl)methyl group or (4-nitro-3-methoxyphenyl)methyl group can be mentioned.

The "heterocycle C₁₋₆ alkyl group optionally substituted by 1 to 5 substituents selected from group D" is the above-defined "C₁₋₆ alkyl group" substituted by the above-defined "heterocyclic group optionally substituted by 1 to 5 substituents selected from group D".

Specifically, 2-pyridylmethyl group, 3-pyridylmethyl group, 2-chloropyridin-4-ylmethyl group, 4-pyridylmethyl group, pyrrolylmethyl group, imidazolylmethyl group, 2-thienylmethyl group, 3-thienylmethyl group, 2-furylmethyl group, 2-oxazolylmethyl group, 5-isothiazolylmethyl group, 2-methyloxazol-4-ylmethyl group, 2-thiazolylmethyl group, 4-thiazolylmethyl group, 5-thiazolylmethyl group, 2-methylthiazol-4-ylmethyl group, 2-methylthiazol-5-ylmethyl group, 2,5-dimethylthiazol-4-ylmethyl group, 4-methylthiazol-2-ylmethyl group, 2,4-dimethylthiazol-5-ylmethyl group, 2-isothiazolylmethyl group, 2-pyrrolinylmethyl group, pyrrolidinylmethyl group, piperidylmethyl group, 4-piperidylmethyl group, 1-methylpiperidin-4-ylmethyl group, 4-hydroxypiperidinomethyl group, 2-(4-hydroxypiperidino)ethyl group, 1-(tert-butoxycarbonyl)piperidin-4-ylmethyl group, 1-acetylpiperidin-4-ylmethyl group, 1-methylsulfonylpiperidin-4-ylmethyl group, piperazinylmethyl group, morpholinomethyl group, thiomorpholinylmethyl group, 1-tetrahydropyranylmethyl group, 2-quinolylmethyl group, 1-isoquinolylmethyl group and the like can be mentioned.

The "C₁₋₆ alkyl group optionally substituted by 1 to 3 substituents selected from group E" is the above-defined "C₁₋₆ alkyl group" optionally substituted by 1 to 3 substituents selected from the above-defined "group E", which includes non-substituted alkyl group.

Specifically, methyl group, ethyl group, propyl group, isopropyl group, butyl group, isobutyl group, sec-butyl group, tert-butyl group, pentyl group, isopentyl group, tert-pentyl group, neopentyl group, 1-ethylpropyl group, hexyl group, trifluoromethyl group, hydroxymethyl group, 2-hydroxyethyl group, 3-hydroxypropyl group, 4-hydroxybutyl group, 1-hydroxy-1-methylethyl group, 1-hydroxypropan-2-yl group, 1,3-dihydroxypropan-2-yl group, 1-hydroxy-2-methylpropan-2-yl group, carboxymethyl group, ethoxycarbonylmethyl group, 2-carboxyethyl group, methoxymethyl group, methoxyethyl group, methoxyethoxyethyl group, ethoxycarbonylmethyl group, 2-ethoxycarbonylethyl group, 2-dimethylaminoethyl group, carbamoylmethyl group, methylcarbamoylmethyl group, sulfomethyl group, sulfamoylmethyl group, 2-sulfamoylethyl group, methylsulfamoylmethyl group and the like can be mentioned.

The "C₂₋₆ alkenyl group optionally substituted by 1 to 3 substituents selected from group E" is the above-defined "C₂₋₆ alkenyl group" optionally substituted by 1 to 3 substituents selected from the above-defined "group E", which includes non-substituted alkenyl group.

Specifically, vinyl group, allyl group, 1-propenyl group, isopropenyl group, 1-butenyl group, 2-butenyl group, 1,3-butadienyl group, 2-isopentenyl group, 3-isohexenyl group and 4-methyl-3-pentenyl group can be mentioned.

The "C₆₋₁₄ aryl group optionally substituted by 1 to 5 substituents selected from group E" is the above-defined "C₆₋₁₄ aryl group" optionally substituted by 1 to 5 substituents selected from the above-defined "group E", which includes non-substituted aryl group.

Specifically, phenyl group, naphthyl group, anthryl group, indenyl group, azulenyl group, fluorenyl group, phenanthryl group, 3-fluorophenyl group, 4-fluorophenyl group, 3-chlorophenyl group, 4-chlorophenyl group, 2,4-dichlorophenyl group, 3,5-dichlorophenyl group, 4-bromophenyl group, 4-nitrophenyl group, pentafluorophenyl group, 4-methylphenyl group, 4-tert-butylphenyl group, 2-trifluoromethylphenyl group, 4-trifluoromethylphenyl group, 4-(hydroxymethyl)phenyl group, 4-(methoxymethyl)phenyl group, 4-(2-carboxyethyl)phenyl group, 3-carboxyphenyl group, 4-carboxyphenyl group, 4-methoxyphenyl group, 3,4,5-trimethoxyphenyl group, 4-carbamoylphenyl group, 4-methylthiophenyl group, 4-(dimethylaminocarbonyl)phenyl group, 4-methylsulfonylphenyl group, 4-acetylaminophenyl group, 4-cyanophenyl group, 4-acetylphenyl group, 4-aminophenyl group, 4-dimethylaminophenyl group, 4-(methylsulfonylamino)phenyl group, 4-methylsulfinylphenyl group, 4-aminosulfonylphenyl group, 3-nitro-4-methoxyphenyl group, 4-nitro-3-methoxyphenyl group and 4-(tetrazol-5-yl)phenyl group can be mentioned.

The "C₃₋₈ cycloalkyl group optionally substituted by 1 to 5 substituents selected from group E" is the above-defined "C₃₋₈ cycloalkyl group" optionally substituted by 1 to 5 substituents selected from the above-defined "group E", which includes non-substituted cycloalkyl group.

Specifically, cyclopropyl group, cyclobutyl group, cyclopentyl group, cyclohexyl group, cycloheptyl group, 4-fluorocyclohexyl group, 2-methylcyclopentyl group, 3-methylcyclohexyl group, 4-methylcyclohexyl group, 4,4-dimethylcyclohexyl group, 3,5-dimethylcyclohexyl group, 4-tert-butylcyclohexyl group, 4-hydroxycyclohexyl group, 4-methoxycyclohexyl group and 2,3,4,5,6-pentafluorocyclohexyl group can be mentioned.

In addition, such group wherein the cyclopentyl group or cyclohexyl group is substituted by fluorine atom, chlorine atom, bromine atom, nitro group, methyl group, tert-butyl group, carboxyl group, trifluoromethyl group, hydroxymethyl group, methoxymethyl group, 2-carboxyethyl group, methoxy group, carbamoyl group, methylthio group, dimethylaminocarbonyl group, methylsulfonyl group or acetylamino group can be mentioned.

The "heterocyclic group optionally substituted by 1 to 5 substituents selected from group E" is the above-defined "heterocyclic group" optionally substituted by 1 to 5 substituents selected from the above-defined "group E", which includes non-substituted heterocyclic group.

Specifically, 2-pyridyl group, 3-pyridyl group, 4-pyridyl group, 3-fluoropyridin-4-yl group, 3-chloropyridin-4-yl group, 4-chloropyridin-3-yl group, pyrazinyl group, pyrimidinyl group, pyridazinyl group, 1,3,5-triazinyl group, pyrrolyl group, pyrazolyl group, imidazolyl group, 1,2,4-triazolyl group, tetrazolyl group, 2-thienyl group, 3-thienyl group, furyl group, oxazolyl group, 2-methyloxazol-4-yl group, isoxazolyl group, thiazolyl group, 2-methylthiazol-4-yl group, 2,5-dimethylthiazol-4-yl group, 2,4-dimethylthiazol-5-yl group, isothiazolyl group, thiadiazolyl group, pyrrolinyl group, pyrrolidinyl group, imidazolidinyl group, piperidyl group, N-methylpiperidin-4-yl group, N-(tert-butoxycarbonyl)piperidin-4-yl group, N-acetylpiperidin-4-yl group, N-methylsulfonylpiperidin-4-yl group, piperazinyl group, morpholinyl group, thiomorpholinyl group, tetrahydropyranyl group, quinolyl group, isoquinolyl group, quinazolinyl group, quinoxalinyl group, phthalazinyl group, cinnolinyl group, naphthyridinyl group, 5,6,7,8-tetrahydroquinolyl group, indolyl group, benzimidazolyl group, indolinyl group, benzofuranyl group, benzothienyl group, benzoxazolyl group, benzothiazolyl group, 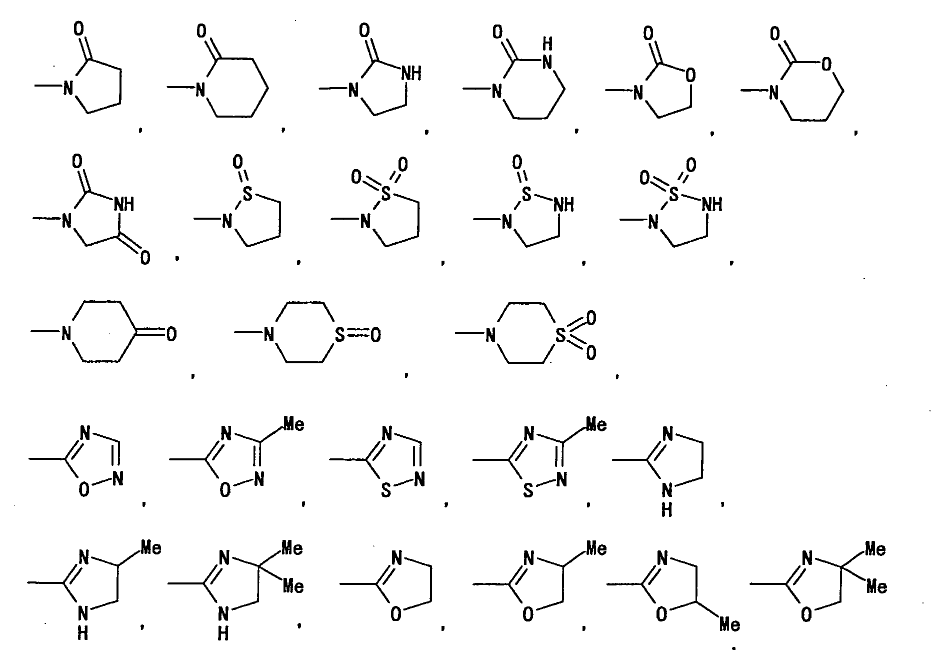 and the like can be mentioned.

In addition, such group wherein the 3, 4, 5 or 6-position of 2-pyridyl group, 2, 4, 5 or 6-position of 3-pyridyl group, 2, 3, 5 or 6-position of 4-pyridyl group, 3, 4 or 5-position of 2-thienyl group, and 2, 4 or 5-position of 3-thienyl group are substituted by fluorine atom, chlorine atom, bromine atom, nitro group, methyl group, tert-butyl group, carboxyl group, trifluoromethyl group, hydroxymethyl group, methoxymethyl group, 2-carboxyethyl group, methoxy group, carbamoyl group, methylthio group, dimethylaminocarbonyl group, methylsulfonyl group, amino group or acetylamino group can be mentioned.

The "carboxylic acid equivalent" means a bioisostere and may only be a substituent having a similar polar effect as carboxylic acid. Specifically, a chain substituent such as -CONHR^{105,}
(wherein R^{105,} is a hydroxyl group, a cyano group or a C₁₋₆ alkoxy group),
-SO₂R^{106,}
(wherein R^{106,} is a hydroxyl group, an amino group or a C₁₋₆ alkylamino group),
-NHCOR¹⁰⁷,
(wherein R¹⁰⁷, is an amino group or a C₁₋₆ alkylamino group) ,
-P(=O) (OH) (OR¹⁰⁹)
(wherein R¹⁰⁹ is a hydrogen atom or a substituent selected from the above-mentioned group C),
-P (=O) (OH) NR¹¹¹R¹¹²
(wherein R¹¹¹ and R¹¹² are each independently a hydrogen atom or a substituent selected from the above-mentioned group C),
-CONHCO-R¹¹³
(wherein R¹¹³ is a substituent selected from the above-mentioned group C),
-CONHSO₂-R¹¹⁴,
(wherein R¹¹⁴ is a substituent selected from the above-mentioned group C),
-SO₂NHCO-R¹¹⁵
(wherein R¹¹⁵ is a substituent selected from the above-mentioned group C) and the like, or a cyclic substituent such as a heterocyclic group having a hydrogen atom donor such as 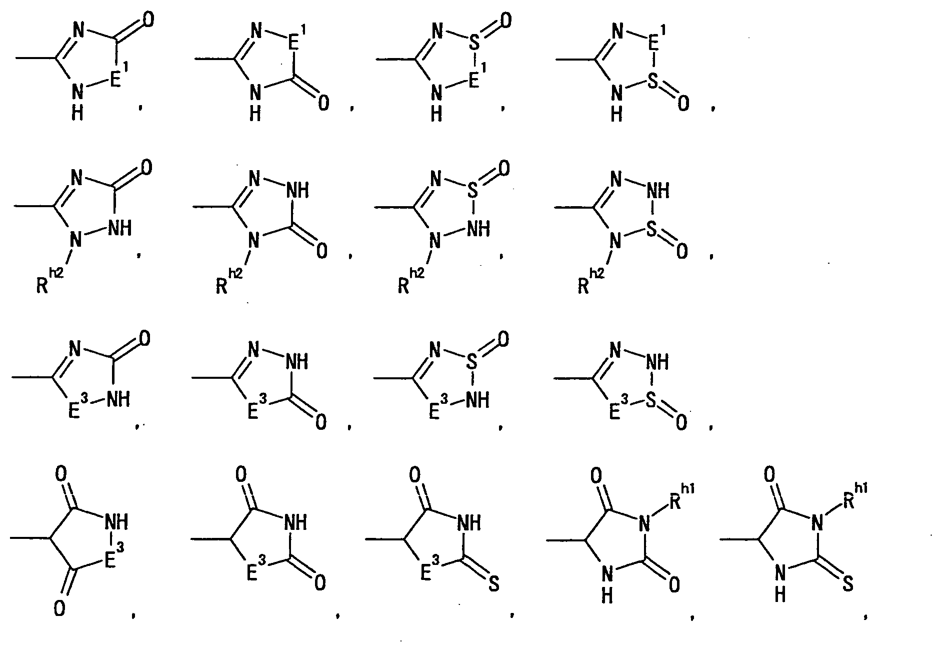 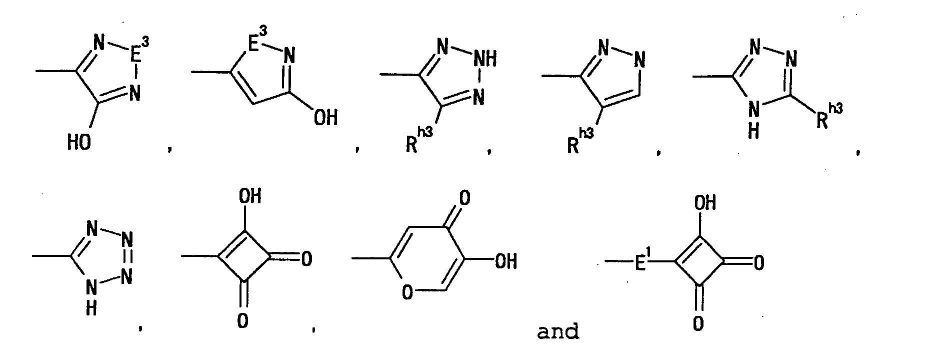 (wherein E¹ is an oxygen atom, a sulfur atom or N(-R^{h1}), R^{h1} is a hydrogen atom or a C₁₋₆ alkyl group, E³ is an oxygen atom or a sulfur atom, R^{h2} is a C₁₋₆ alkyl group, R^{h3} is an electron-withdrawing group such as a halogen atom, a cyano group, a C₁₋₆ alkyl group, a trifluoromethyl group, a formyl group, a chlorocarbonyl group, a nitro group, an acetyl group, an ethoxycarbonyl group, a carbamoyl group and the like) and the like, and said heterocyclic group substituted by an electron-withdrawing group and the like can be mentioned.

More specifically, -COOH, -COOEt, -COOPh, -COOBn,
-CONHCN, -CONHOH, -CONHOMe,
-SO₃H, -SO₂NH₂, -SO₂NHMe,
-NHCONH₂, -NHCON(Me)₂,
-P(=O)(OH)₂, -P(=O)(OH)(OEt) ,
-P(=O)(OH)NH₂, -P(=O)(OH)NHMe,
-CONHCOMe, -CONHCOBn,
-CONHSO₂Me, -CONHSO₂Ph,
-SO₂NHCOMe, -SO₂NHCOPh
wherein Me is a methyl group, Et is an ethyl group, Ph is a phenyl group and Bn is a benzyl group, 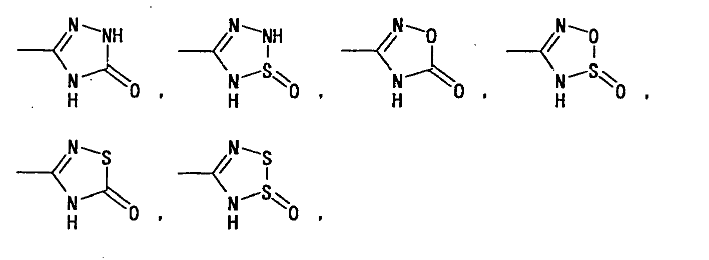 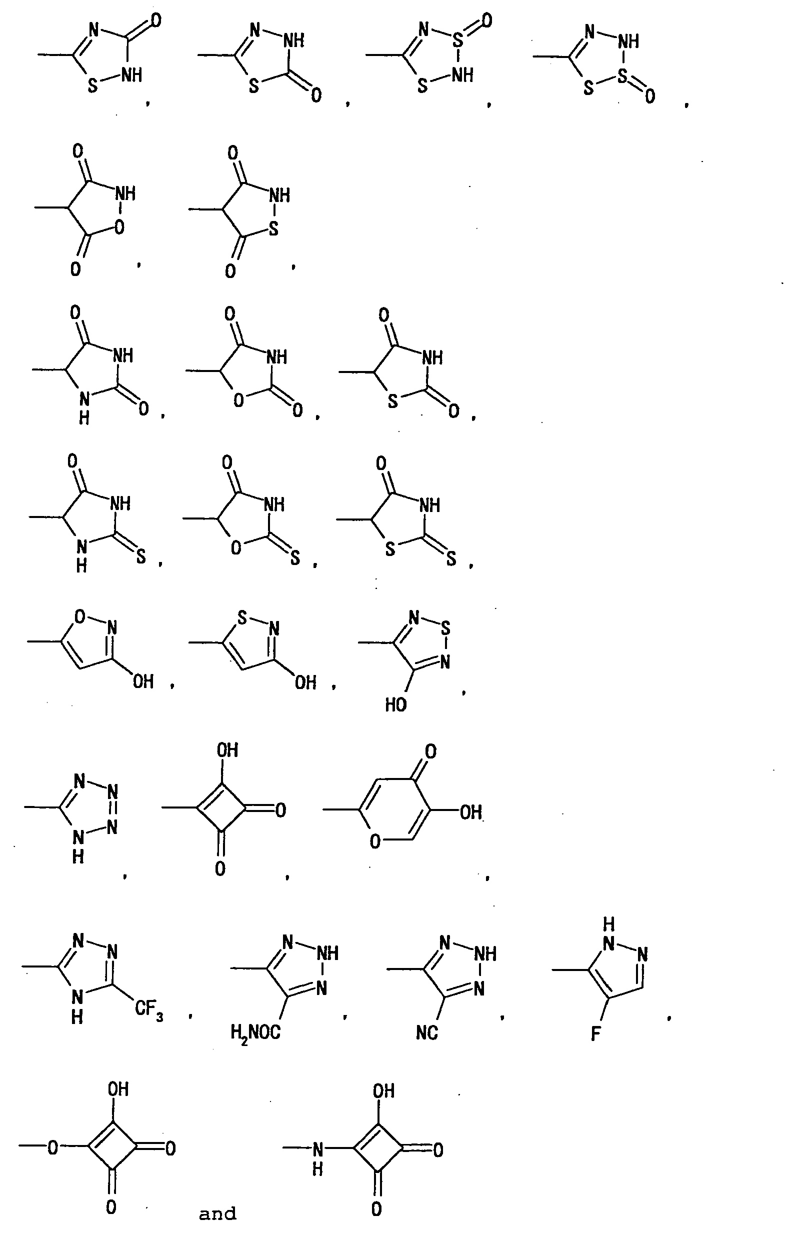 and the like can be mentioned.

One wherein 1 to 4 of G¹, G², G³ and G⁴ is a nitrogen atom is preferable. In addition, one wherein at least one of G¹ and G² is a nitrogen atom, or at least one of G¹ and G² is a heteroatom, and at least one of G³ and G⁴ is a heteroatom is preferable.

In the formula [I], the moiety 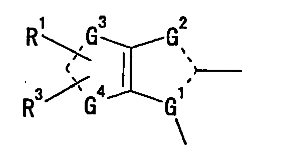 is preferably a fused ring selected from the group consisting of 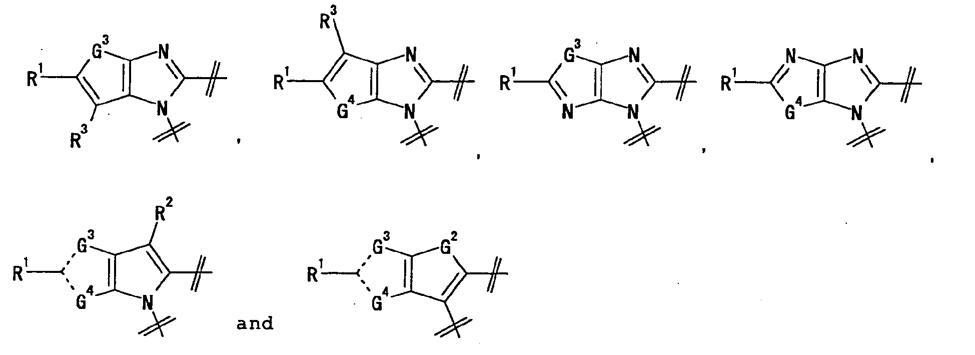 more preferably, a fused ring selected from the group consisting of 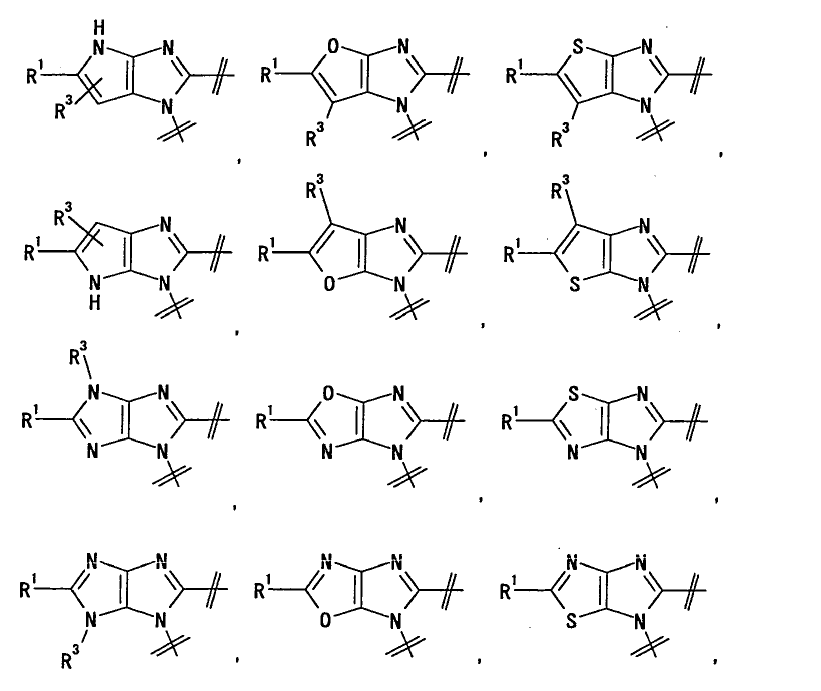 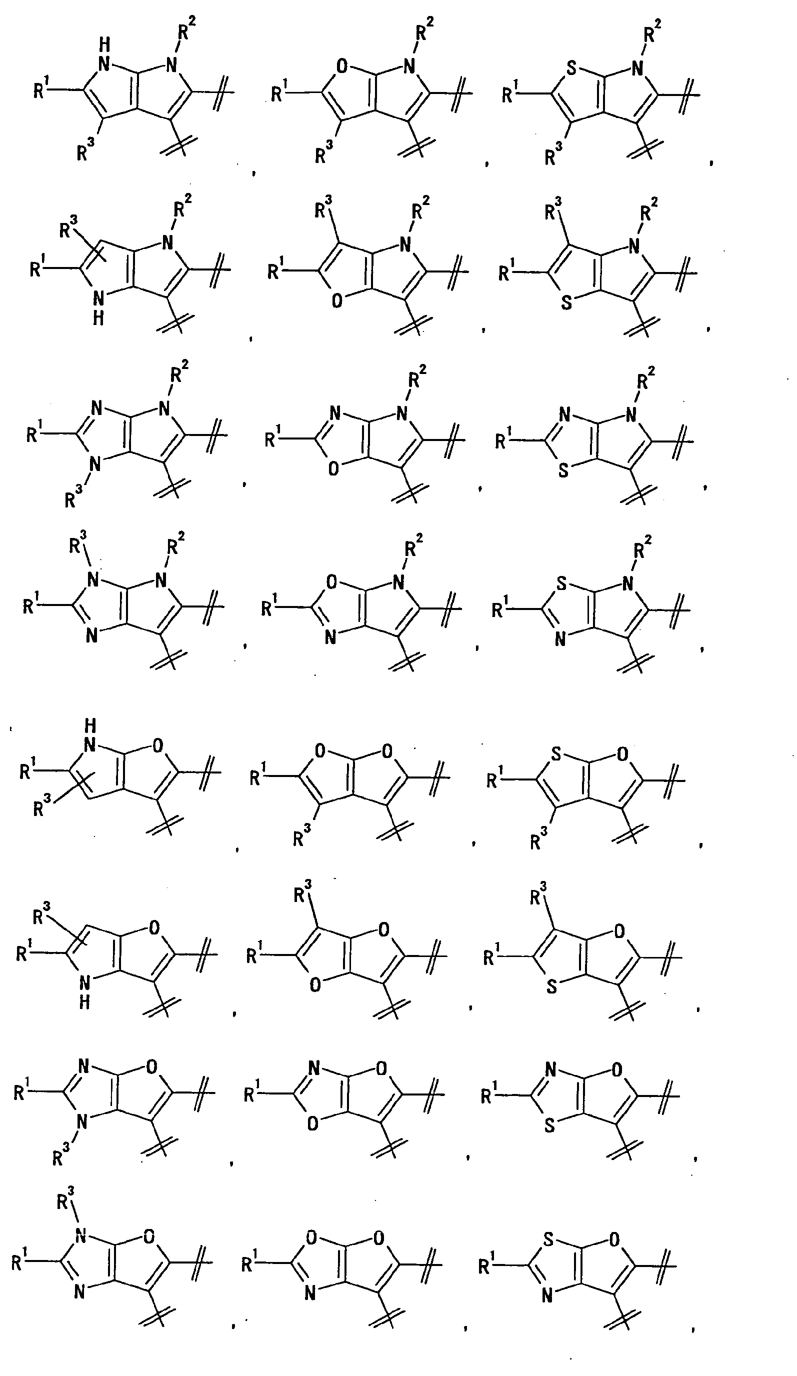 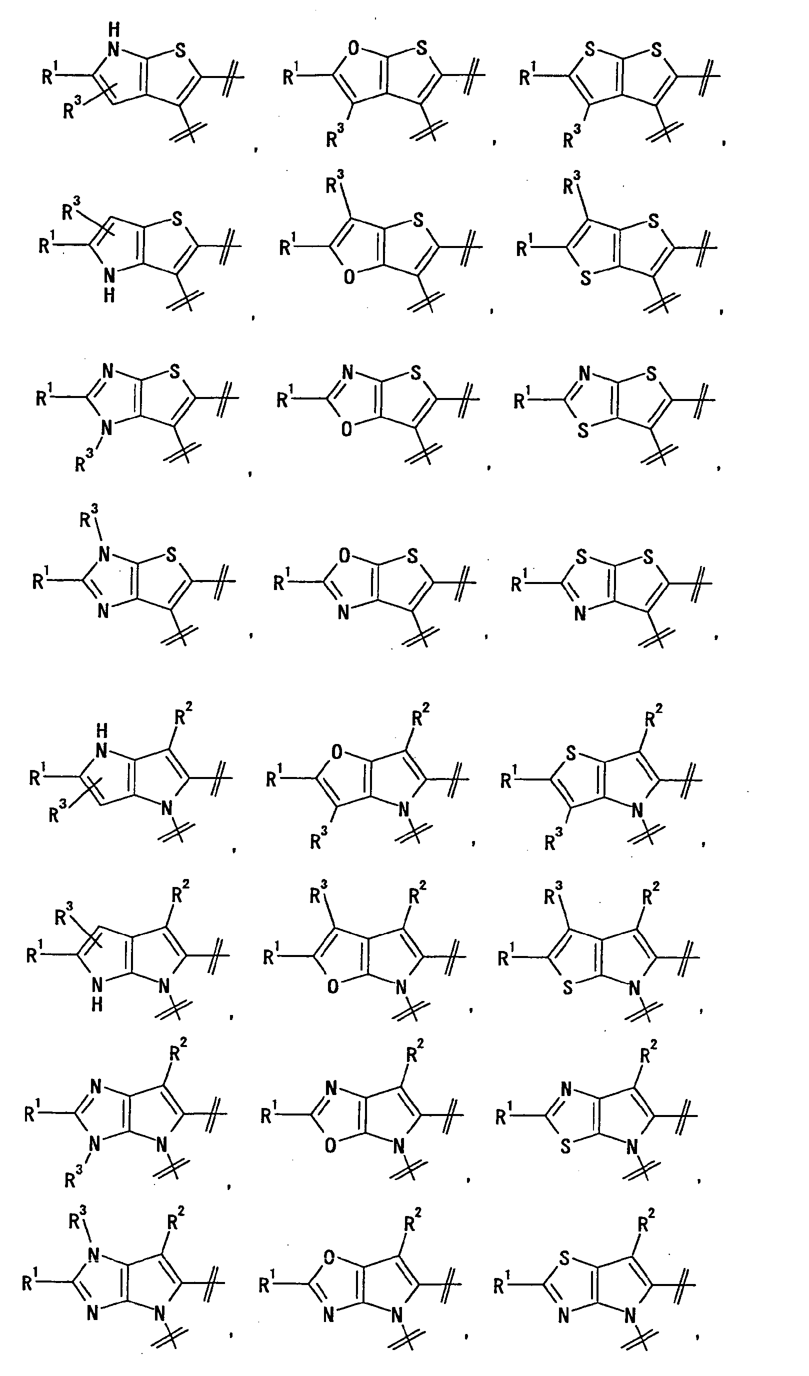 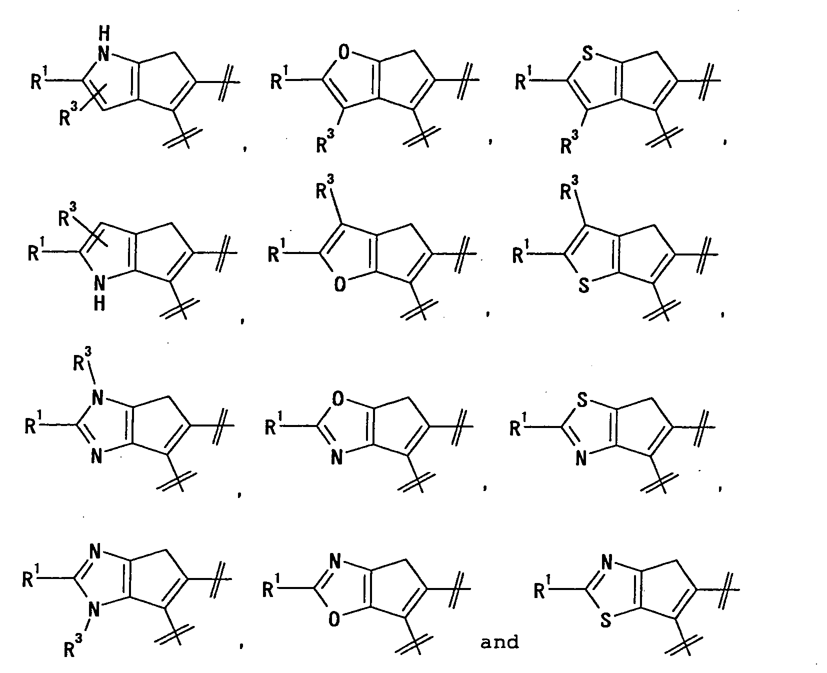 particularly preferably, a fused ring selected from the group consisting of

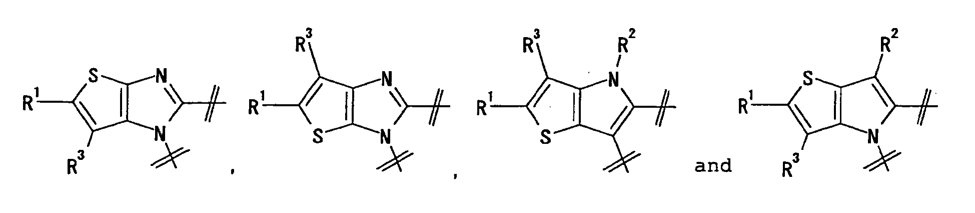

Moreover, in the formula [I], compounds represented by the following formula [I-1], [I-2], [I-3] and [I-4] are particularly preferable, and a compound represented by the formula [I-3] is most preferable. 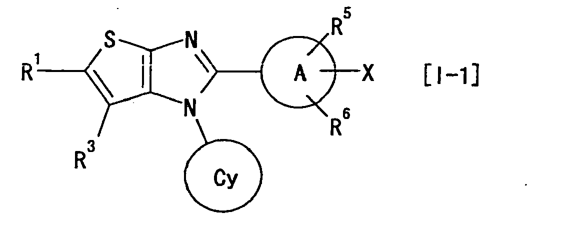 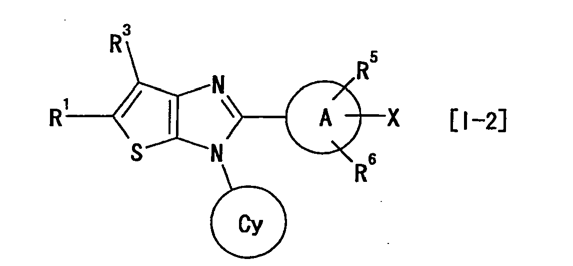 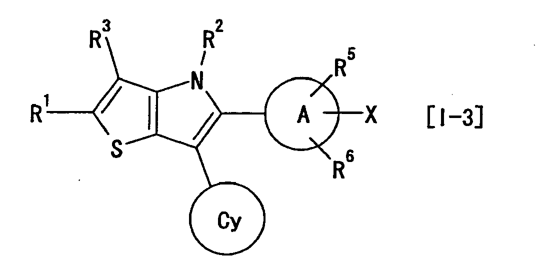 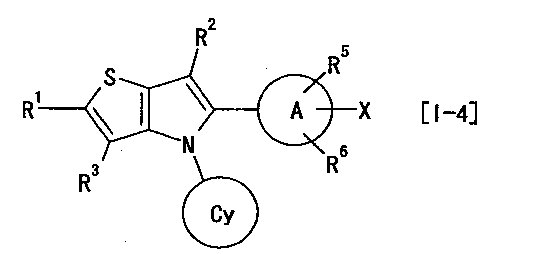 wherein each symbol is as defined above.

For R¹, a carboxyl group or the above-defined "carboxylic acid equivalent" is preferable, and carboxyl group is more preferable.

For R², hydrogen atom, phenylsulfonyl group, benzyloxycarbonyl group, allyl group, methyl group, ethyl group, isopropyl group, cyclohexyl group, 2,2,2-trifluoroethyl group, cyanomethyl group, nitromethyl group, 2-(2-methoxyethoxy)ethyl group, pivaloylmethyl group, ethoxycarbonylmethyl group, 3-(3-methylureido)propyl group, 2-(methylcarbamoyloxy)ethyl group, 2-(methylsulfanyl)ethyl group, 2-(methanesulfonyl)ethyl group, 2-(methylsulfamoyl)ethyl group, 2-hydroxy-2-methylpropyl group, methanesulfonylcarbamoylmethyl group, 3-(dimethylamino)-2-hydroxypropyl group, carbamoylmethyl group, methylcarbamoylmethyl group, isopropylcarbamoylmethyl group, dimethylcarbamoylmethyl group, 2-(dimethylcarbamoyl)ethyl group, 3-(dimethylcarbamoyl)propyl group, isobutylcarbamoylmethyl group, (1-ethylpropyl)carbamoylmethyl group, tert-butylcarbamoylmethyl group, (2,2-dimethylpropyl)carbamoylmethyl group, (3,3-dimethylbutyl)carbamoylmethyl group, (2,2,2-trifluoroethyl)carbamoylmethyl group, methoxycarbamoylmethyl group, 2-methoxyethylcarbamoylmethyl group, 3-methoxypropylcarbamoylmethyl group, 2-(methylsulfanyl)ethylcarbamoylmethyl group, carboxymethylcarbamoylmethyl group, 2-carboxyethylcarbamoylmethyl group, 3-carboxypropylcarbamoylmethyl group, carbamoylmethylcarbamoylmethyl group, 2-(dimethylamino)ethylcarbamoylmethyl group, N-[2-(dimethylamino)ethyl]-N-methylcarbamoylmethyl group, 3-(dimethylamino)propylcarbamoylmethyl group, 2-(acetylamino)ethylcarbamoylmethyl group, 2-hydroxyethyl group, 3-hydroxypropyl group, 2-methoxyethyl group, 2-(dimethylamino)ethyl group, carboxymethyl group, 2-(acetylamino)ethyl group, 3-(acetylamino)propyl group, 2-(methanesulfonylamino)ethyl group, 3-(methanesulfonylamino)propyl group, 2-[N-(methanesulfonyl)-N-methylamino]ethyl group, 3-(acetylsulfamoyl group)propyl group, 2-(3-methyl-2-butenyloxy)ethyl group, 2-(2-methoxyethoxy)ethylcarbamoylmethyl group, 2-(tetrahydropyran-2-yloxy)ethyl group, 2-(4-methylphenoxy)ethyl group, 3-(4-chlorophenylamino)propyl group, 2-(4-methylthiazol-2-ylamino)ethyl group, cyclopropylcarbamoylmethyl group, cyclobutylcarbamoylmethyl group, cyclopentylcarbamoylmethyl group, cyclohexylcarbamoylmethyl group, phenylcarbamoylmethyl group, benzylcarbamoylmethyl group, phenethylcarbamoylmethyl group, N-benzyl-N-methylcarbamoylmethyl group, 3-phenylpropylcarbamoylmethyl group, 4-phenylbutylcarbamoylmethyl group, 2-(3-chlorobenzyloxy)ethyl group, 3-(4-methylbenzylsulfanyl)propyl group, 2-(phenylacetylamino)ethyl group, 2-pyridylmethylcarbamoylmethyl group, 3-pyridylmethylcarbamoylmethyl group, 4-pyridylmethylcarbamoylmethyl group, 2-(pyridin-2-yl)ethylcarbamoylmethyl group, 2-(pyridin-3-yl)ethylcarbamoylmethyl group, 2-(pyridin-4-yl)ethylcarbamoylmethyl group, N-methyl-N-(pyridin-2-ylmethyl)carbamoylmethyl group, N-methyl-N-(2-(pyridin-2-yl)ethyl)carbamoylmethyl group, 3-(imidazol-1-yl)propylcarbamoylmethyl group, benzoylmethyl group, 2-(2,4-dimethylthiazol-5-yl)-2-oxoethyl group, 2-(3-methylisoxazol-4-yl)-2-oxoethyl group, 2-(3-methoxypyrrolidin-1-yl)-2-oxoethyl group, 2-(2-carboxypyrrolidin-1-yl)-2-oxoethyl group, 2-(2-carbamoylpyrrolidin-1-yl)-2-oxoethyl group, 2-oxo-2-piperidinoethyl group, 2-morpholino-2-oxoethyl group, 2-(3-methoxypiperidino)-2-oxoethyl group, 2-(4-methoxypiperidino)-2-oxoethyl group, 2-[4-(tert-butoxycarbonylamino)piperidino]-2-oxoethyl group, 2-[4-(dimethylamino)piperidino]-2-oxoethyl group, 2-oxo-2-(4-oxopiperidino)ethyl group, 2-(4-methylpiperazin-1-yl)-2-oxoethyl group, 2-(4-carboxypiperazin-1-yl)-2-oxoethyl group, 2-(4-isopropylpiperazin-1-yl)-2-oxoethyl group, 2-oxo-2-(thiomorpholin-4-yl)ethyl group, 2-oxo-2-(1-oxothiomorpholin-4-yl)ethyl group, 2-(1,1-dioxothiomorpholin-4-yl)-2-oxoethyl group, 2-(thiophen-2-ylcarbonylamino)ethyl group, 2-piperidinoethylcarbamoylmethyl group, 2-morpholinoethylcarbamoylmethyl group, 2-(1-methylpyrrolidin-2-yl)ethylcarbamoylmethyl group, 3-(2-oxopyrrolidin-1-yl)propylcarbamoylmethyl group and 2-(1-benzylpiperidin-4-yl)ethylcarbamoylmethyl group can be specifically mentioned.

In addition, benzyl group, phenethyl group, 3-phenylpropyl group, 2-methoxybenzyl group, 2-(dimethylamino)benzyl group, 3-methoxybenzyl group, 3-(dimethylamino)benzyl group, 3-phenoxybenzyl group, 4-fluorobenzyl group, 4-chlorobenzyl group, 4-methylbenzyl group, 4-hydroxybenzyl group, 4-methoxybenzyl group, 4-cyanobenzyl group, 4-(dimethylamino)benzyl group, 4-(methylcarbamoyl)benzyl group, 4-methanesulfonylbenzyl group, 2-pyridylmethyl group, 3-pyridylmethyl group, 4-pyridylmethyl group, 6-aminopyridin-3-ylmethyl group, 6-acetylaminopyridin-3-ylmethyl group, 2-piperidinoethyl group, 2-(piperazin-1-yl)ethyl group, 2-(4-phenoxypiperidino)ethyl group, 3-morpholinopropyl group, 1-methylimidazol-2-ylmethyl group, 4-tert-butylthiazol-2-ylmethyl group, 2-methylthiazol-4-ylmethyl group, 3,5-dimethylisoxazol-4-ylmethyl group, 5-methylisoxazol-3-ylmethyl group, [1,2,4]oxadiazol-3-ylmethyl group, 4,4-dimethyl-4,5-dihydrooxazol-2-ylmethyl group, 4-methyl-4H-[1,2,4]triazol-3-ylmethyl group, 1-methyl-1H-tetrazol-5-ylmethyl group, 2-methylpyrimidin-5-ylmethyl group, 5-methylthiophen-2-ylmethyl group, 2,5-dimethyloxazol-4-ylmethyl group, 5-methyl-4-methylcarbamoyloxazol-2-ylmethyl group, 2-methoxymethyl-5-methyloxazol-4-ylmethyl group, 2-(2-dimethylaminothiazol-4-yl)ethyl group, 2-phenyl-4-methylthiazol-5-ylmethyl group, 5-(dimethylaminomethyl)-[1,2,4]oxadiazol-3-ylmethyl group, 5-(acetylaminomethyl)-[1,2,4]oxadiazol-3-ylmethyl group, 2-(dimethylcarbamoylmethyl)-2H-tetrazol-5-ylmethyl group, 1-methylindol-3-ylmethyl group, phenyl-pyridin-2-ylmethyl group, benzhydrylcarbamoylmethyl group, 4-styrylbenzyl group, 2-(2-morpholino-2-oxoethoxy)ethyl group, 2-oxo-2-[4-(piperidinoacetyl)piperazin-1-yl]ethyl group, 2-oxo-2-[4-(pyrrolidin-1-yl)piperidino]ethyl group, 2-(2-phenoxyethylamino)ethyl group, 4-(morpholinocarbonyl)benzyl group, 3-(3-morpholinophenyl)propyl group, 3-ethynyloxybenzyl group, 2-{N-[3-(dimethylaminoacetylamino)benzyl]-N-methylamino}ethyl group, 2-(dibenzylamino)ethylcarbamoylmethyl group, 4-(2-dibenzylaminomethyl)cyclohexylmethyl group, 2-(morpholinoacetylamino)ethoxycarbonylmethyl group, 3-{1-[2-(2-methoxyethoxy)phenylacetyl]piperidin-4-ylmethylcarbamoyl}benzyl group, 2-(2-morpholinoethoxy)-5-{N-methyl-N-[4-(4-nitrophenylsulfonyl)benzoyl]amino}benzyl group, 2-(4-ethylpiperazin-1-yl)-2-oxoethyl group, 2-morpholinoethyl group, cyclohexylmethyl group, [N-methyl-N-(2-oxo-2-piperidinoethyl)carbamoyl]methyl group, 4-morpholinophenylcarbamoylmethyl group, 2-(4-cyclohexylpiperazin-1-yl)-2-oxoethyl group, 2-(4-cyclohexylpiperazin-1-yl)ethyl group, 2-(1,4'-bipiperidinyl-1'-yl)-2-oxoethyl group, 2-(1,4'-bipiperidinyl-1'-yl)ethyl group, 2-[N-methyl-N-(2-morpholinoethyl)amino]ethyl group, 2-[N-methyl-N-(2-piperidinoethyl) amino] ethyl group and 1-[N-methyl-N-(4-morpholinophenyl)carbamoyl]ethyl group can be mentioned.

For R²,
(1) a hydrogen atom,
(2) a C₁₋₆ alkyl group optionally substituted by 1 to 3 substituents selected from group E,
(3) 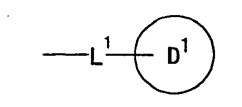 or
(4) 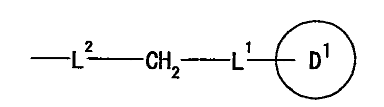 wherein L¹, L² and ring D¹ are as defined above, is preferable.

One of more preferable embodiments of R², a C₁₋₆ alkyl group optionally substituted by 1 to 3 substituents selected from group E can be mentioned and, for example, unsubstituted C₁₋₆ alkyl group such as methyl group, ethyl group and the like, and methyl group substituted by 1 to 3 substituents selected from group E can be mentioned. As used herein, the alkyl moiety is preferably a methyl group or an ethyl group, and more preferably, a methyl group.

Here, group E is preferably -OR^{e1}, -NR^{e3}R^{e4}, -CONR^{e6}R^{e7}, -COR^{e8}, -NR^{e9}CO-R^{e10}, -NR^{e11}SO₂-R^{e12}, -NR^{e19}-COOR^{e20} or -NR^{e21}-CONR^{e22}R^{e23}, more preferably, -OR^{e1}, -NR^{e3}R^{e4} or -CONR^{e6}R^{e7}, which is specifically methoxy group, dimethylamino group, dimethylcarbamoyl group, tert-butylcarbamoyl group and the like.

For group E, particularly preferred is -NR^{e3}R^{e4} (wherein R^{e3} and R^{e4} are each preferably a C₁₋₆ alkyl group optionally substituted by 1 to 3 substituents selected from group A).

As another more preferable embodiment of R², 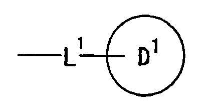 can be mentioned, wherein L¹ is preferably
a bond,
C₁₋₆ alkylene,
- (CHR^{L4})ᵤ₁-NR^{L1}-(CHR^{L5})ᵥ₁-,
- (CHR^{L4})ᵤ₁-CO-(CHR^{L5})ᵥ₁-,
-(CHR^{L4})ᵤ₁-CONR^{L2}-(CHR^{L5})ᵥ₁-,
- (CHR^{L4})ᵤ₁-NR^{L2}CO₂-(CHR^{L5})ᵥ₁-,
- (CHR^{L4})ᵤ₁-NR^{L2}CONR^{L3}-(CHR^{L5})ᵥ₁-,
-(CHR^{L4})ᵤ₁-NR^{L2}CO-(CHR^{L5})ᵥ₁- or
- (CHR^{L4})ᵤ₁-NR^{L2}SO₂-(CHR^{L5})ᵥ₁-,
more preferably,
a bond,
C₁₋₆ alkylene,
- (CHR^{L4})ᵤ₁-NR^{L1}-(CHR^{L5})ᵥ₁-,
-(CHR^{L4})ᵤ₁-CO-(CHR^{L5})ᵥ₁- or
- (CHR^{L4})ᵤ₁-CONR^{L2}-(CHR^{L5})ᵥ₁-,
further preferably,
- (CHR^{L4})ᵤ₁-CO-(CHR^{L5})ᵥ₁- or
- (CHR^{L4})ᵤ₁-CONR^{L2}-(CHR^{L5})ᵥ₁-.

Here, u1 is preferably 1 or 2, 1 is more preferable, and v1 is preferably 0, 1 or 2, and 0 is more preferable. For R^{L4} and R^{L5}, a hydrogen atom or a methyl group is preferable, and a hydrogen atom is more preferable.

Specifically, 2-morpholinoethyl group, 2-(4-methylpiperazin-1-yl)-2-oxoethyl group, 2-(4-ethylpiperazin-1-yl)-2-oxoethyl group, 2-morpholino-2-oxoethyl group, 2-[4-(dimethylamino)piperidino]-2-oxoethyl group, benzylcarbamoylmethyl group, 4-morpholinophenylcarbamoylmethyl group, 1-[N-methyl-N-(4-morpholinophenyl)carbamoyl]ethyl group, 2-(4-cyclohexylpiperazin-1-yl)ethyl group, 2-(4-cyclohexylpiperazin-1-yl)-2-oxoethyl group, 2-(1,4'-bipiperidinyl-1'-yl)ethyl group, 2-(1,4'-bipiperidinyl-1'-yl)-2-oxoethyl group, 2-[N-methyl-N-(2-piperidinoethyl)amino]ethyl group, 2-[N-methyl-N-(2-morpholinoethyl)amino]ethyl group and the like can be mentioned.

For R³, a hydrogen atom is preferable.

For ring Cy, a C₃₋₈ cycloalkyl group or a C₃₋₈ cycloalkenyl group is preferable and, for example, cyclohexyl group and cyclohexenyl group can be mentioned. A C₃₋₈ cycloalkyl group is more preferable, and a cyclohexyl group is most preferable.

For ring A, a C₆₋₁₄ aryl group is preferable, and a phenyl group is more preferable.

R⁵ and R⁶ are each independently preferably a hydrogen atom or a halogen atom, and more preferably, a hydrogen atom.

X is preferably a hydrogen atom, a halogen atom (e.g., fluorine atom, chlorine atom etc.), a C₁₋₆ alkyl group optionally substituted by 1 to 3 substituents selected from group A (e.g., methyl group, ethyl group and the like can be mentioned, preferably methyl group), -OR^{a11} (wherein R^{a11} is a hydrogen atom or a C₁₋₆ alkyl group optionally substituted by 1 to 3 substituents selected from group A. As preferable -OR^{a11}, methoxy group, ethoxy group and the like can be specifically mentioned, and more preferred is methoxy group) or 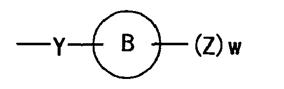 wherein each symbol is as defined above.

Y is preferably -(CH₂)ₘ-O-(CH₂)ₙ- (wherein each symbol is as defined above), more preferably -O-CH₂-.

Ring B is preferably a C₆₋₁₄ aryl group or a heterocyclic group comprising 1 to 4 heteroatoms selected from oxygen atom, nitrogen atom and sulfur atom, more preferably a C₆₋₁₄ aryl group, particularly preferably phenyl group.

Z is preferably 1 to 3 substituents selected from
(1) a hydrogen atom,
(2) a C₆₋₁₄ aryl group optionally substituted by 1 to 5 substituents selected from group D,
(3) a heterocyclic group optionally substituted by 1 to 5 substituents selected from group D,
(4) -(CH₂)ₜ-S(O)_{q}-R^{d2},
(5) -(CH₂)ₜ-NR^{d3}R^{d4} and
(6) -(CH₂)ₜ-NR^{d9}CO-R^{d10}
wherein each symbol is as defined above.

It is preferable that at least one of Z is a C₆₋₁₄ aryl group optionally substituted by 1 to 5 substituents selected from group D or a heterocyclic group optionally substituted by 1 to 5 substituents selected from group D. Here, as Z, 2-oxopyrrolidin-1-yl group, morpholino group, 4-methanesulfonylpiperidino group, 4-dimethylaminopiperidino group and the like can be specifically mentioned.

As X, 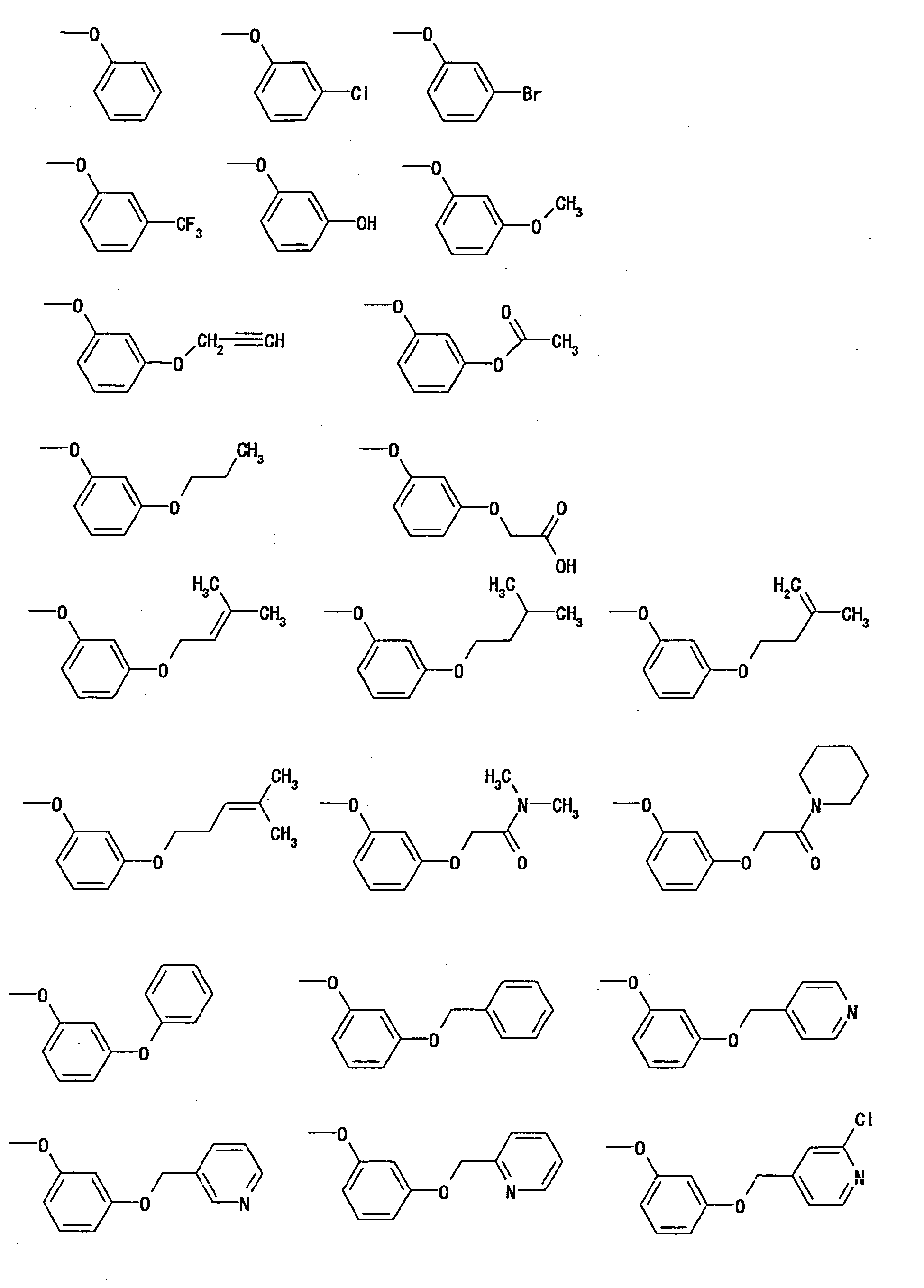 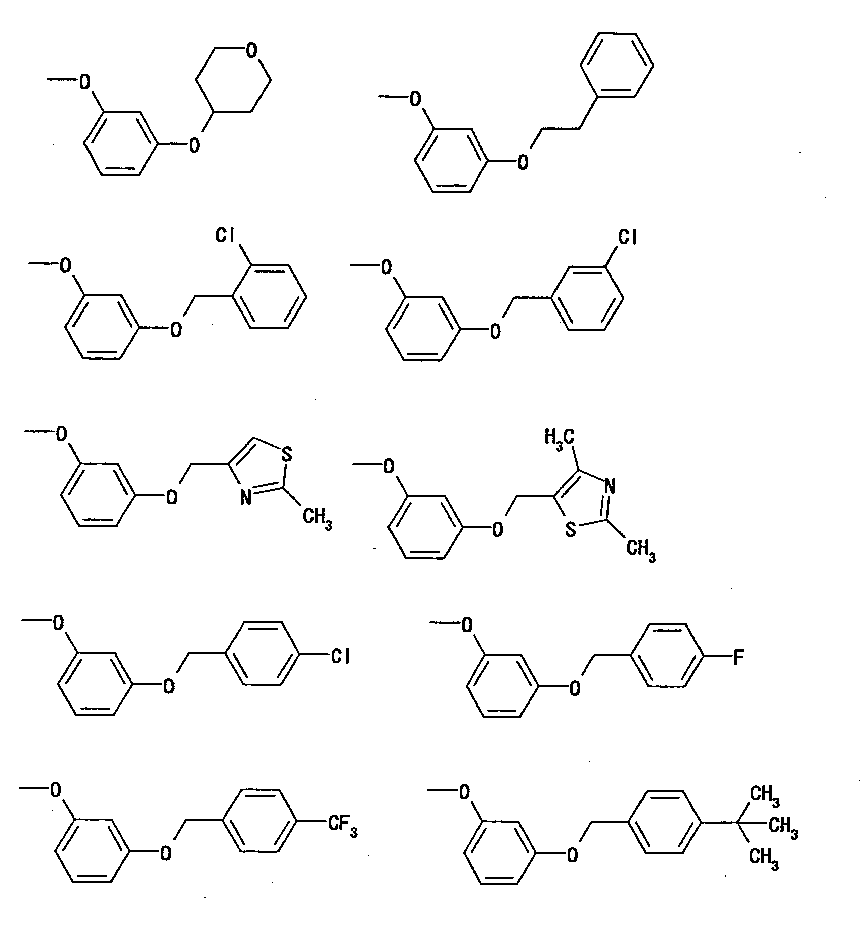 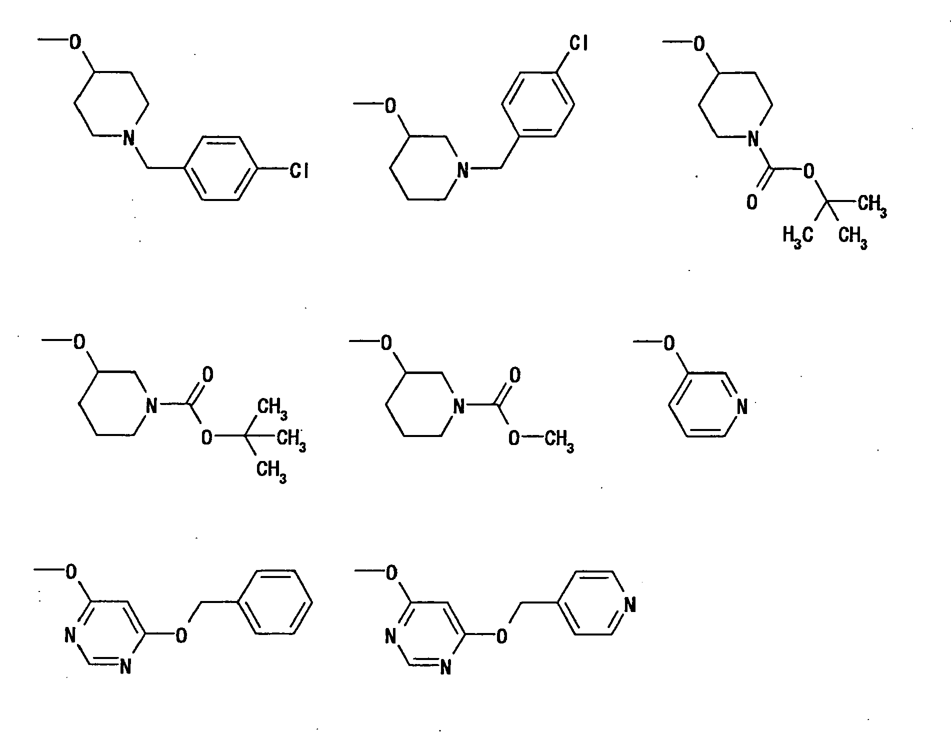 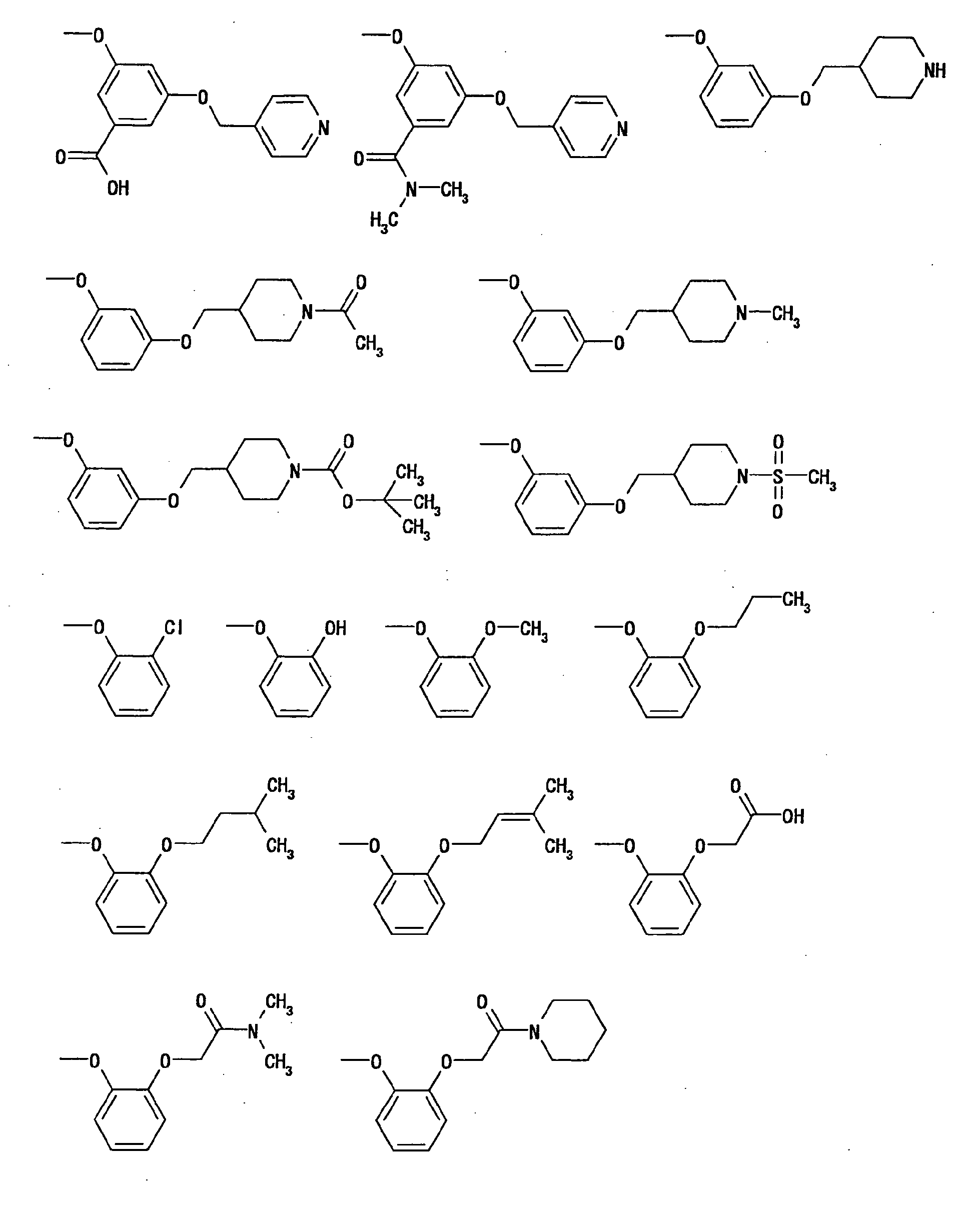 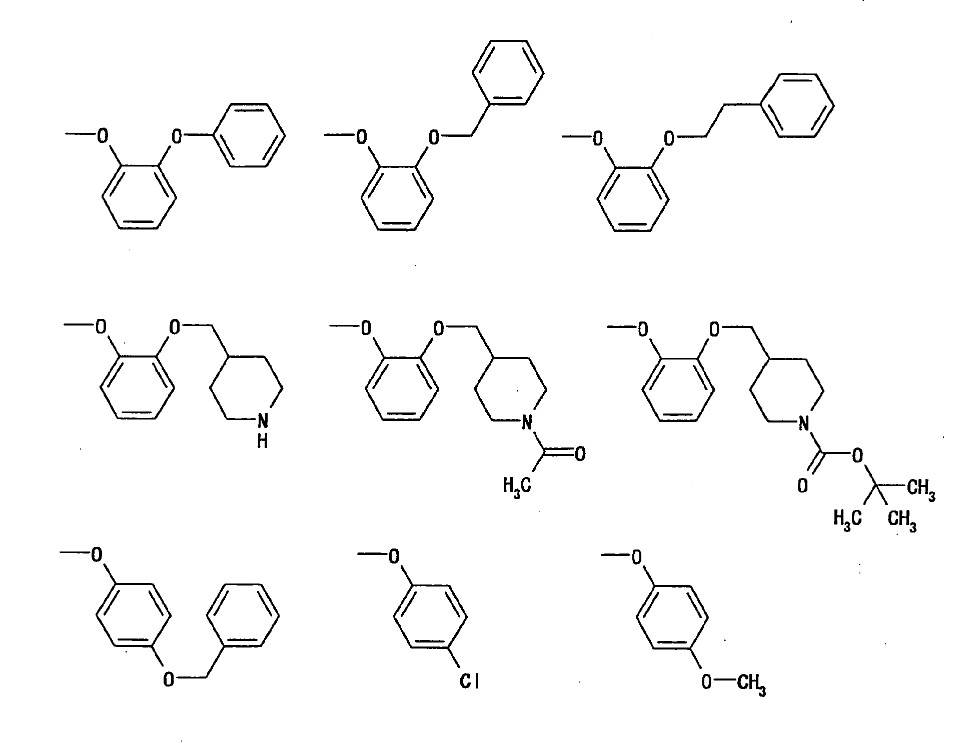 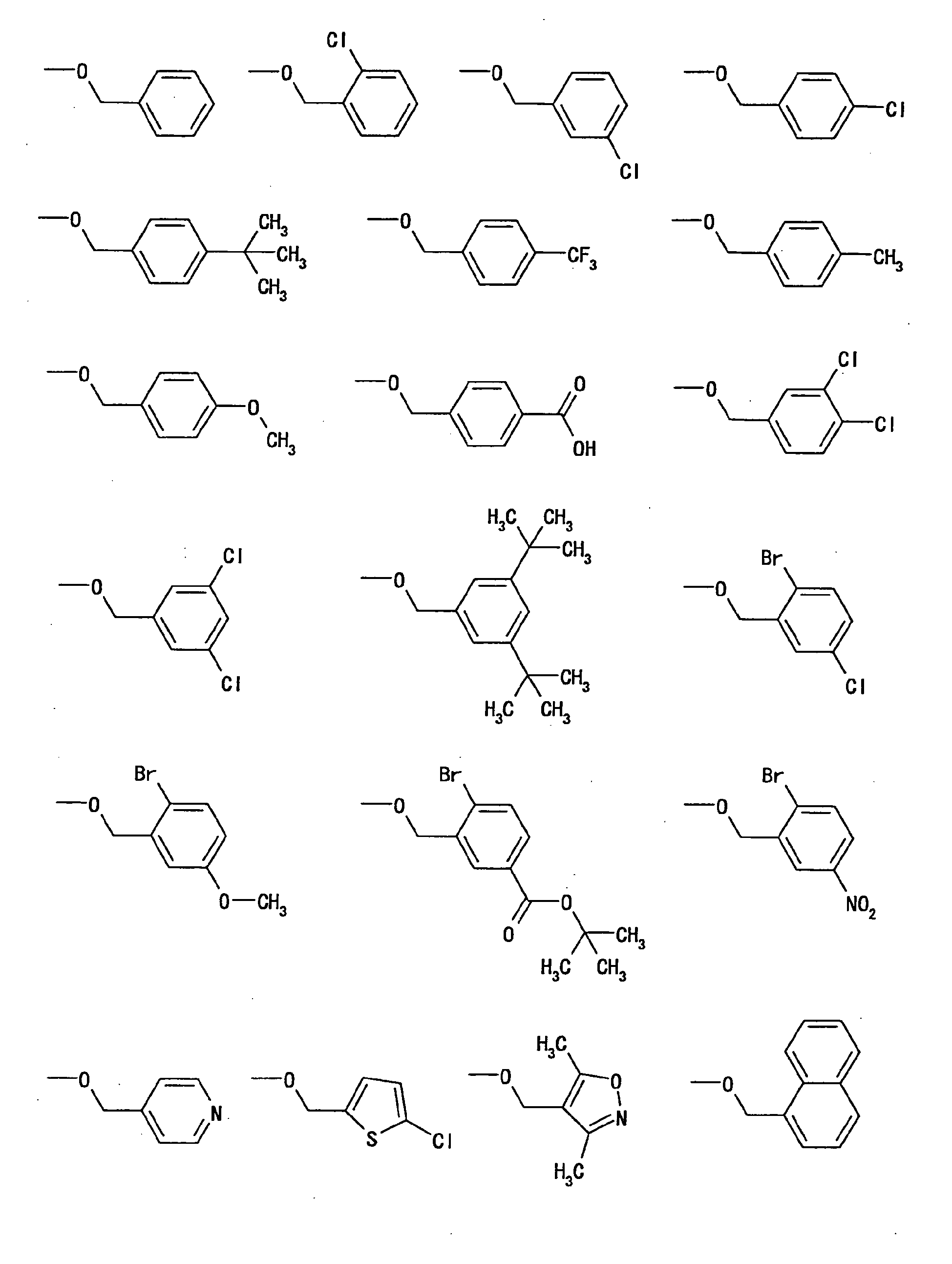 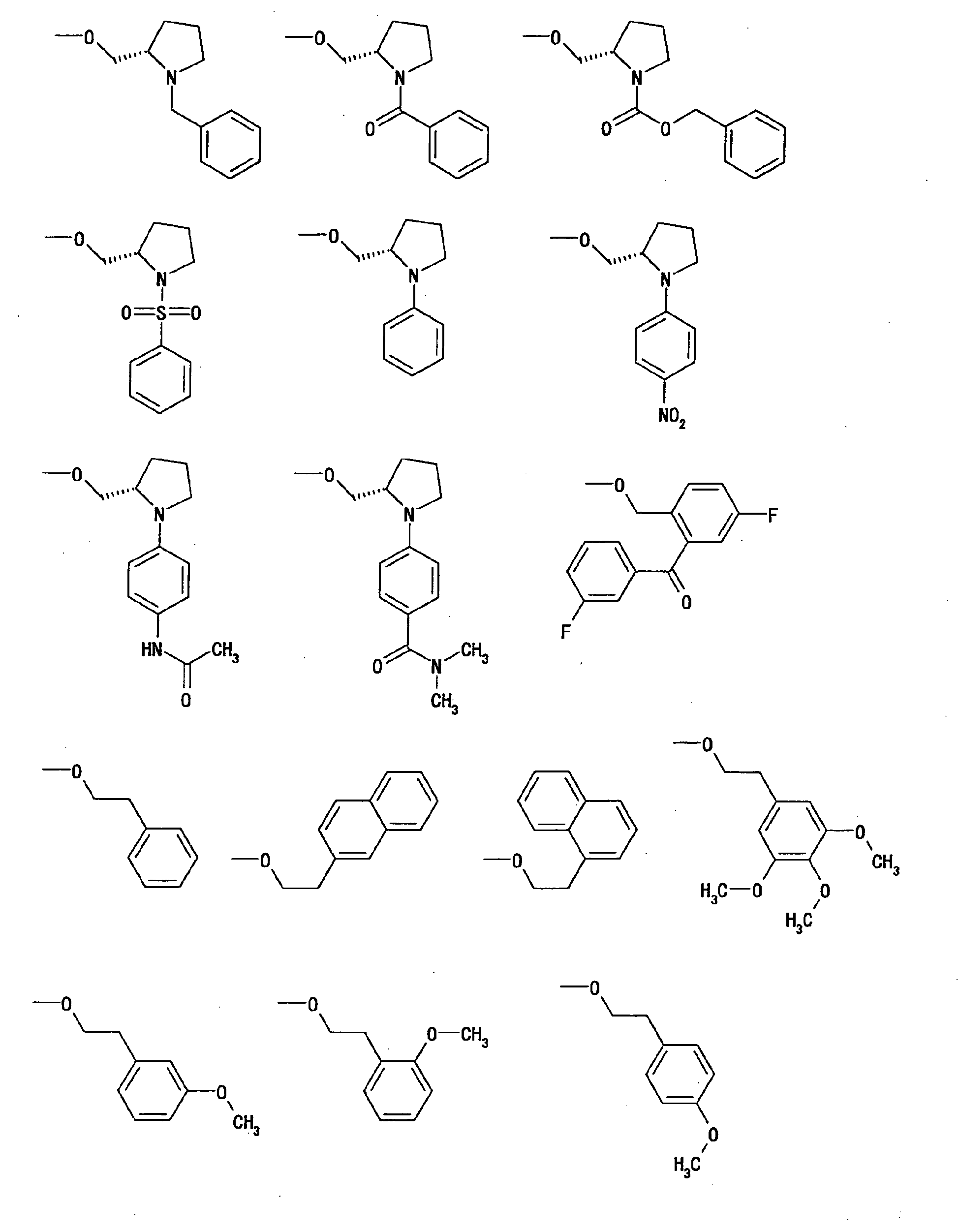 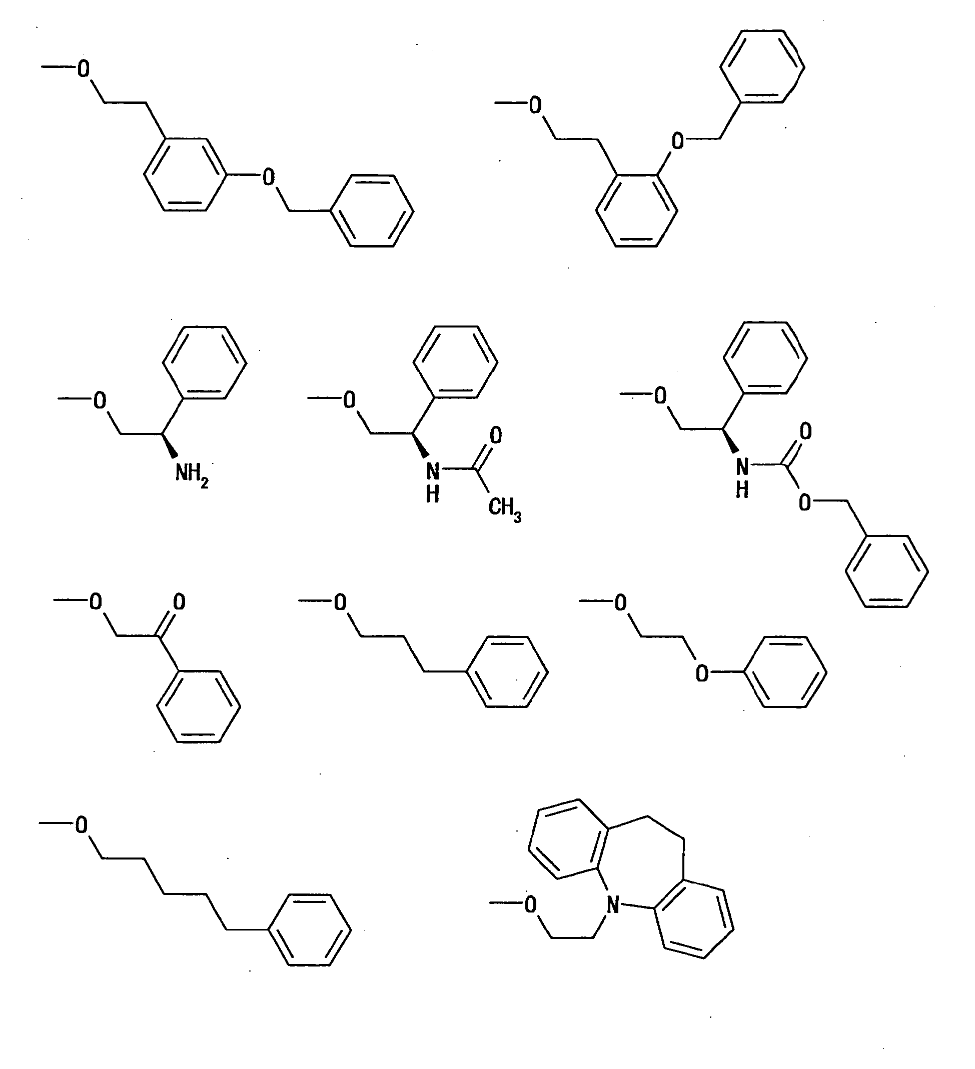 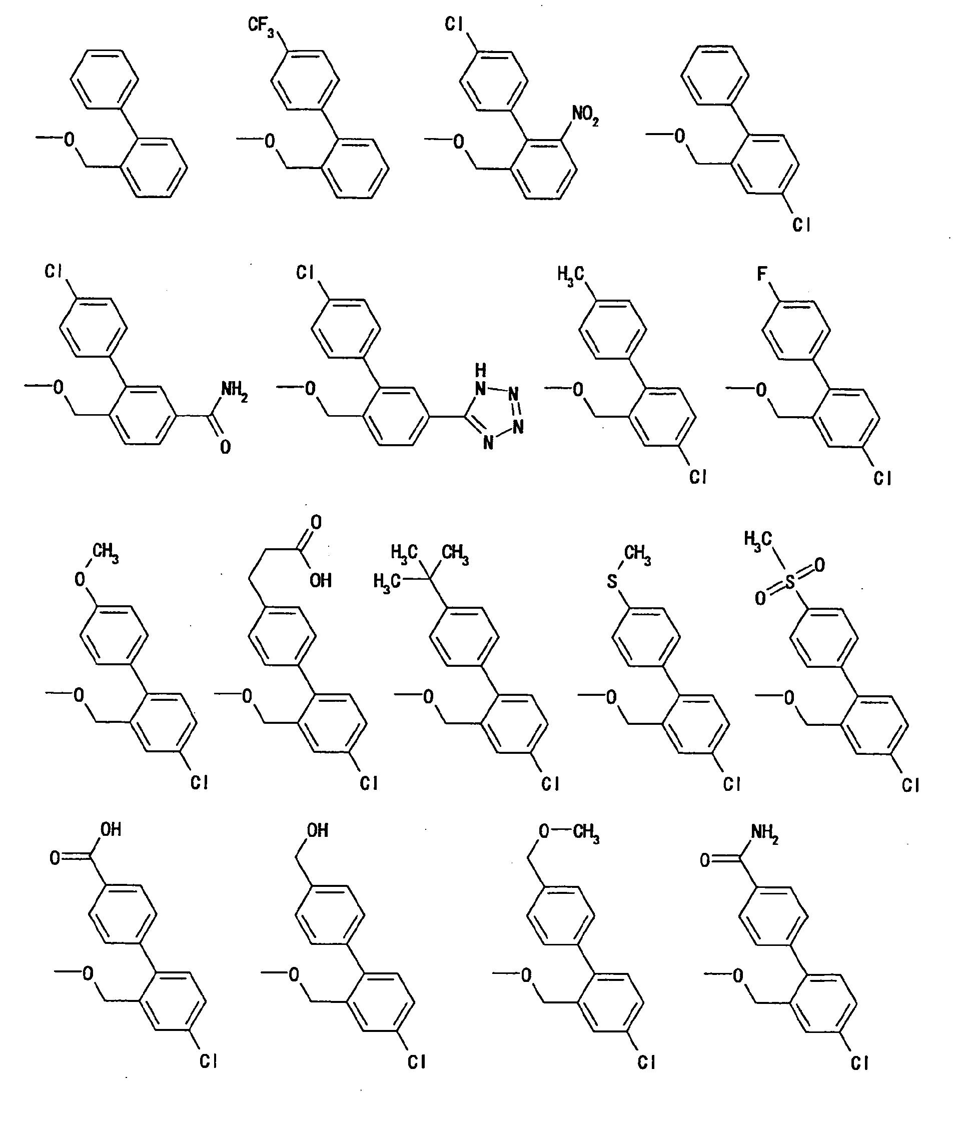 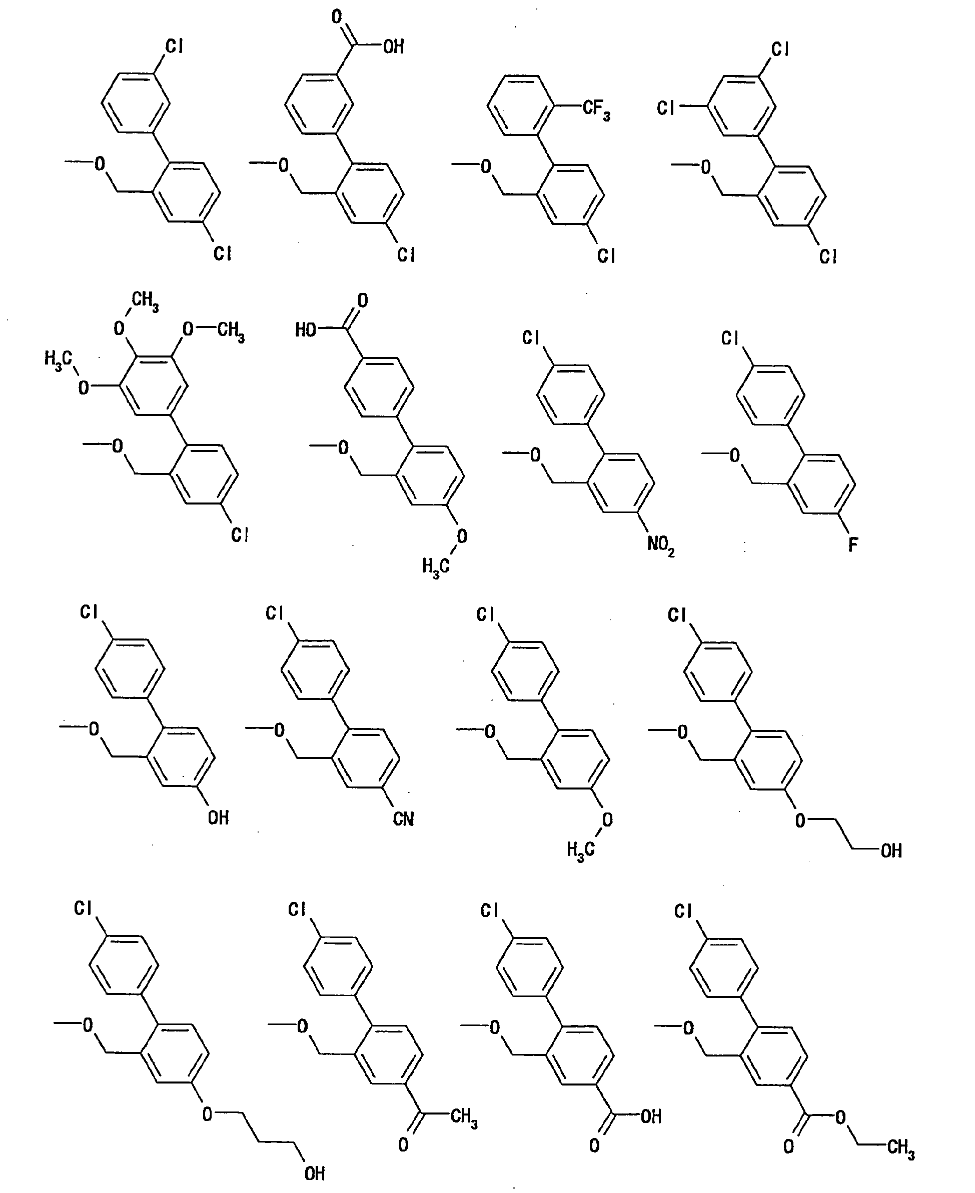 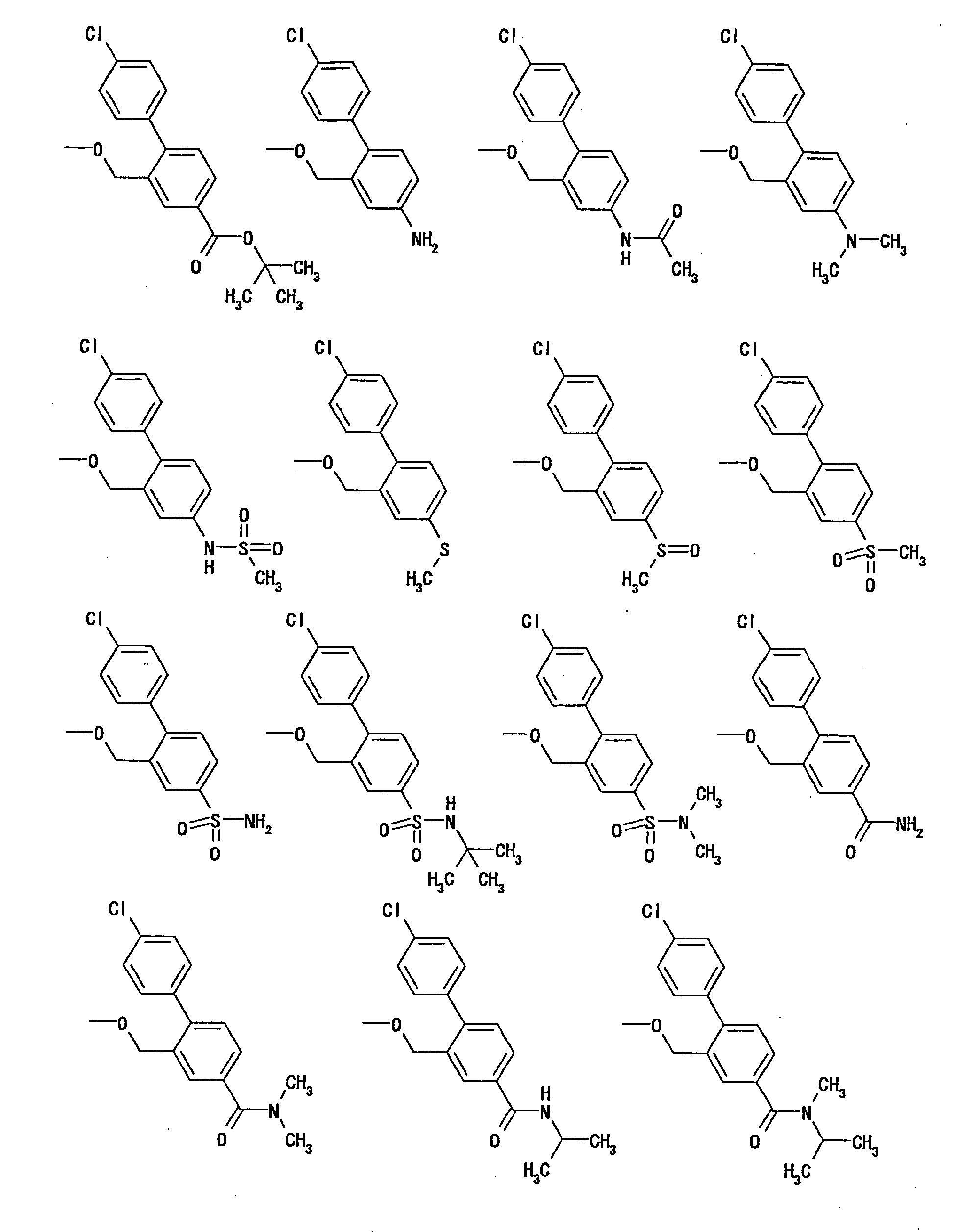 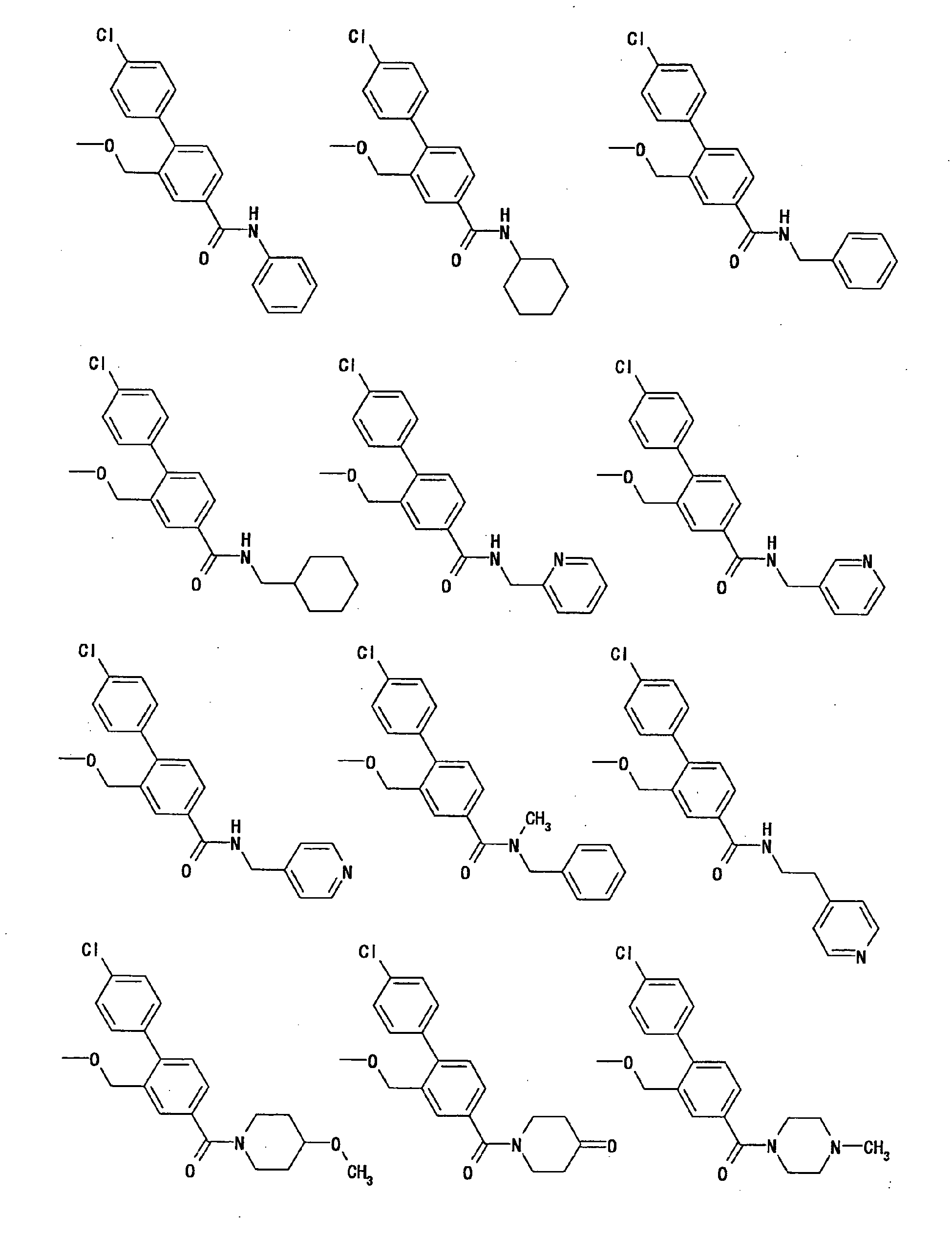 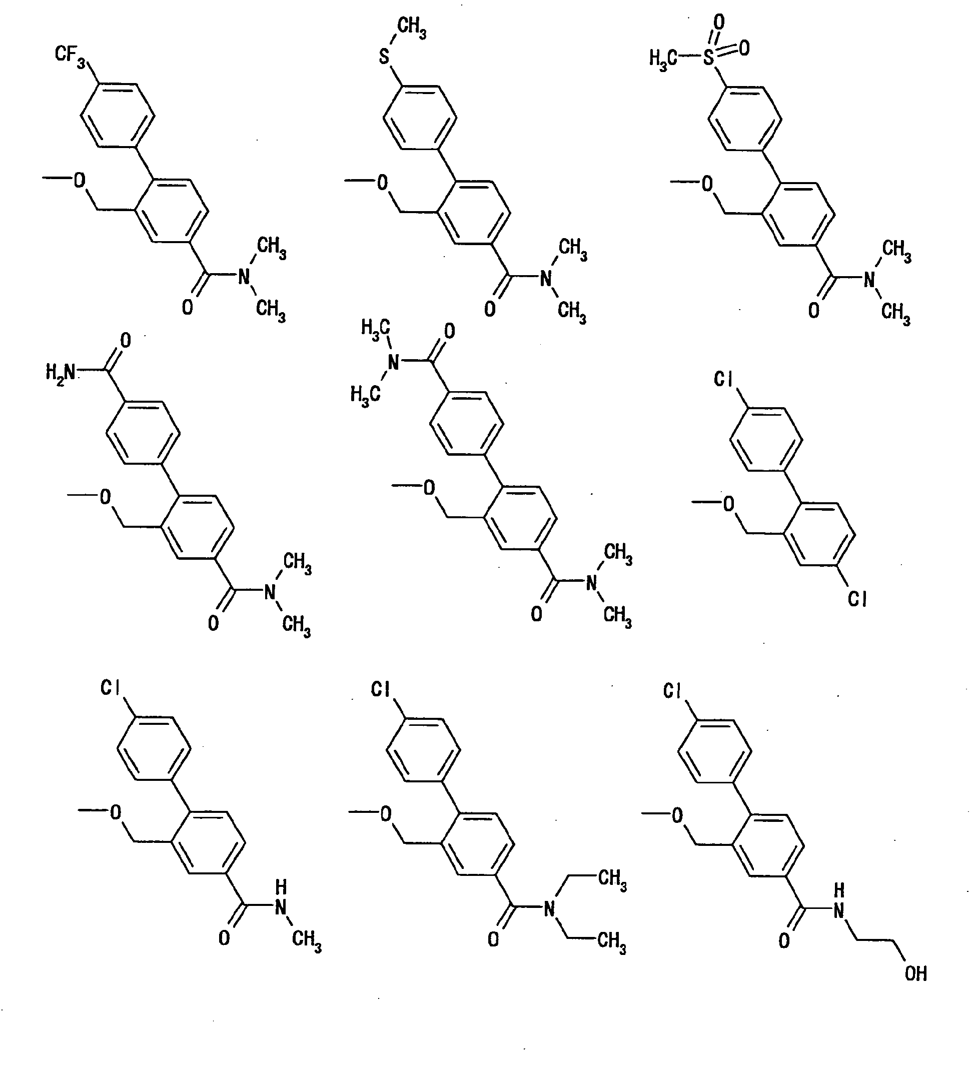 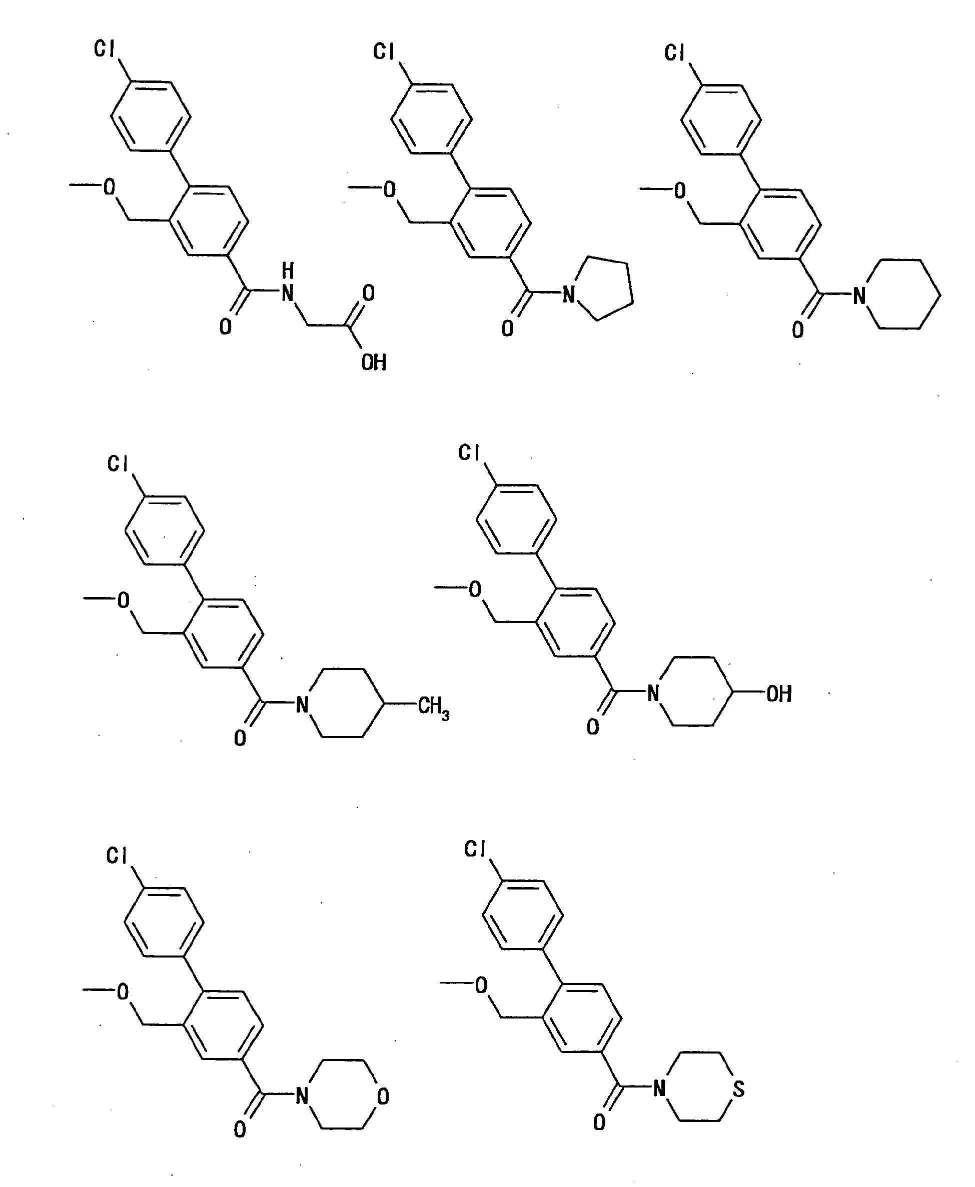 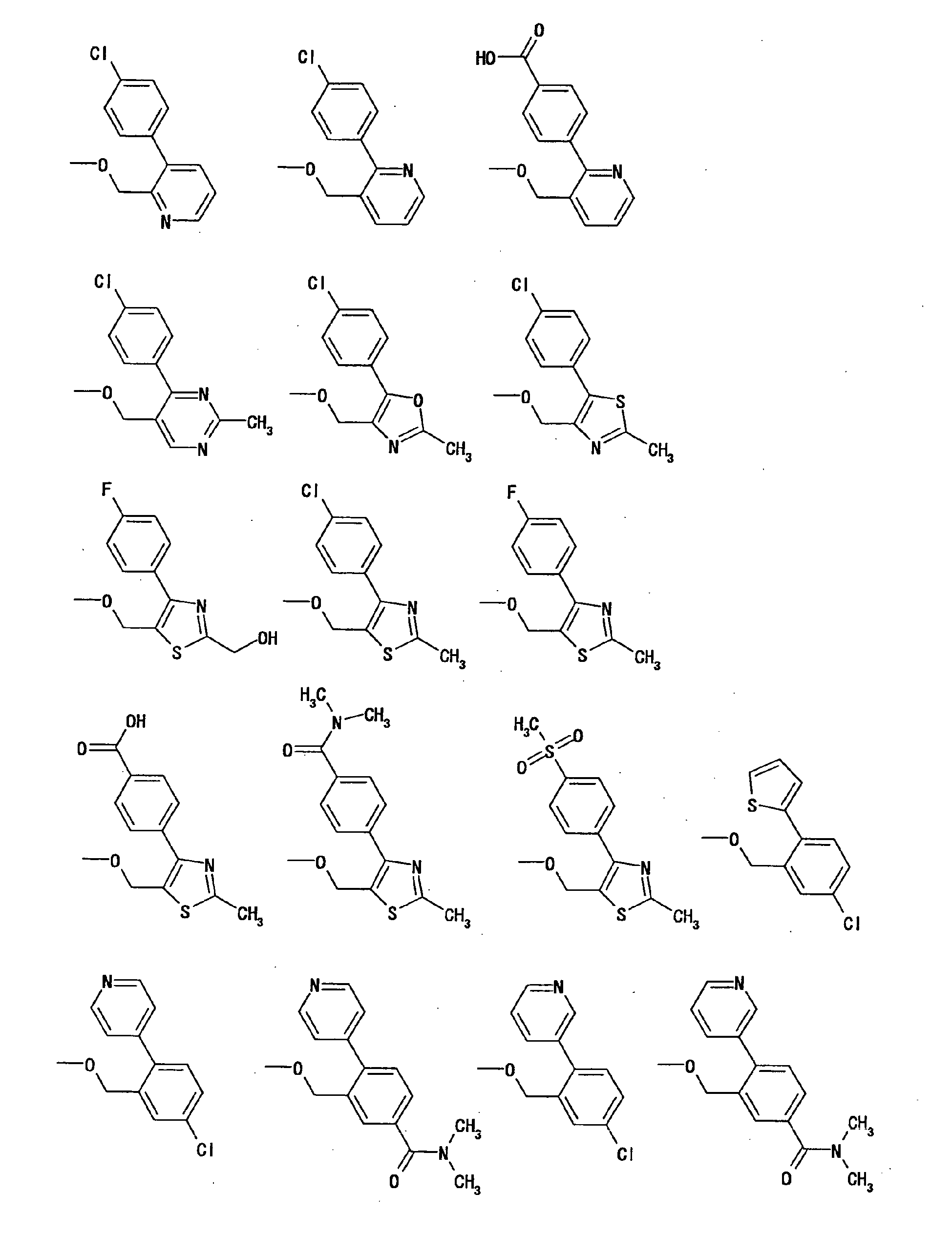 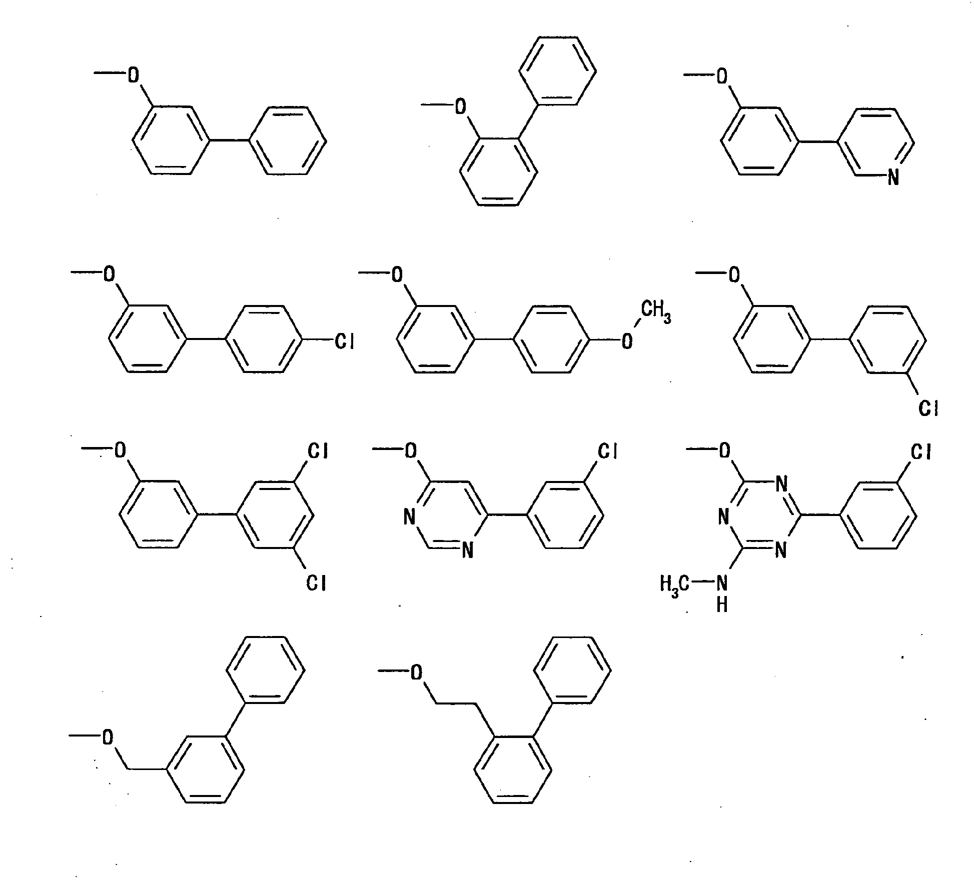 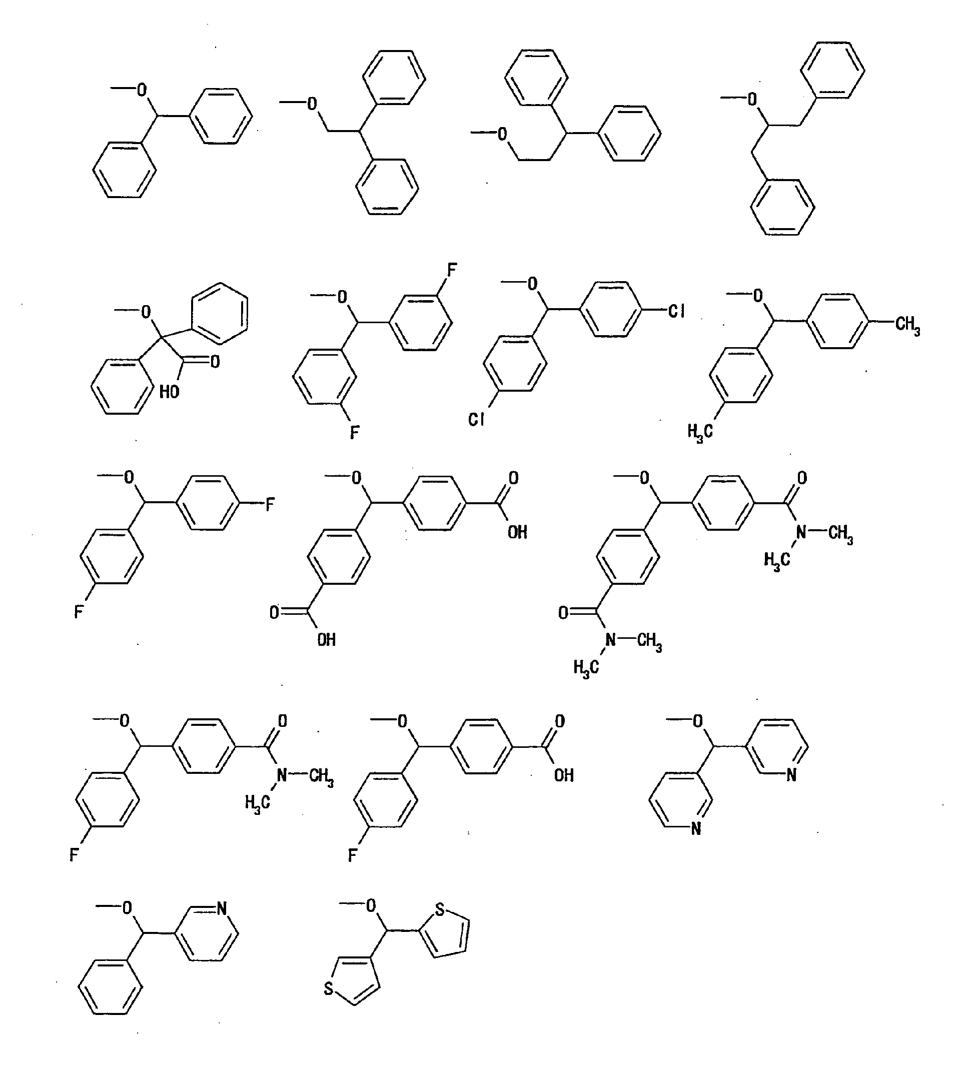 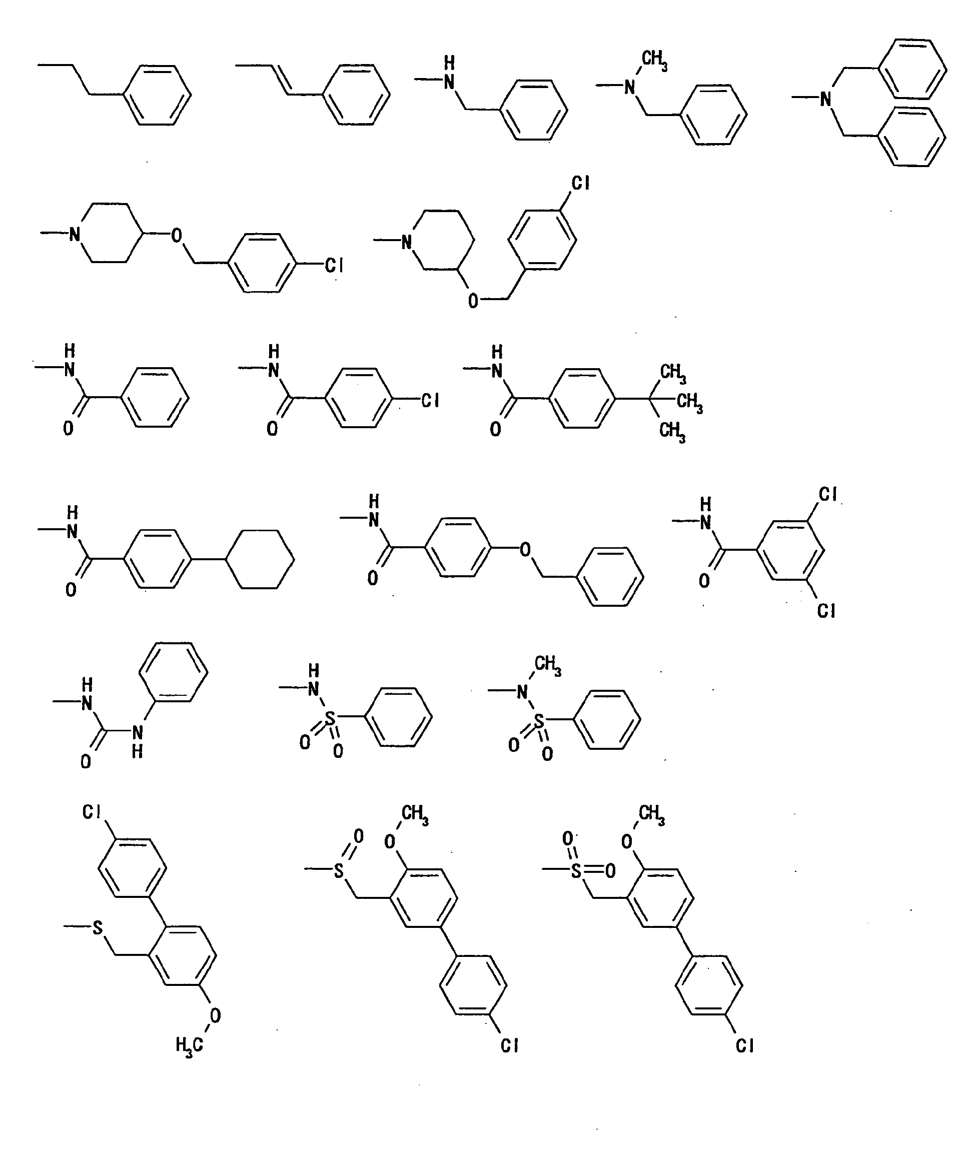 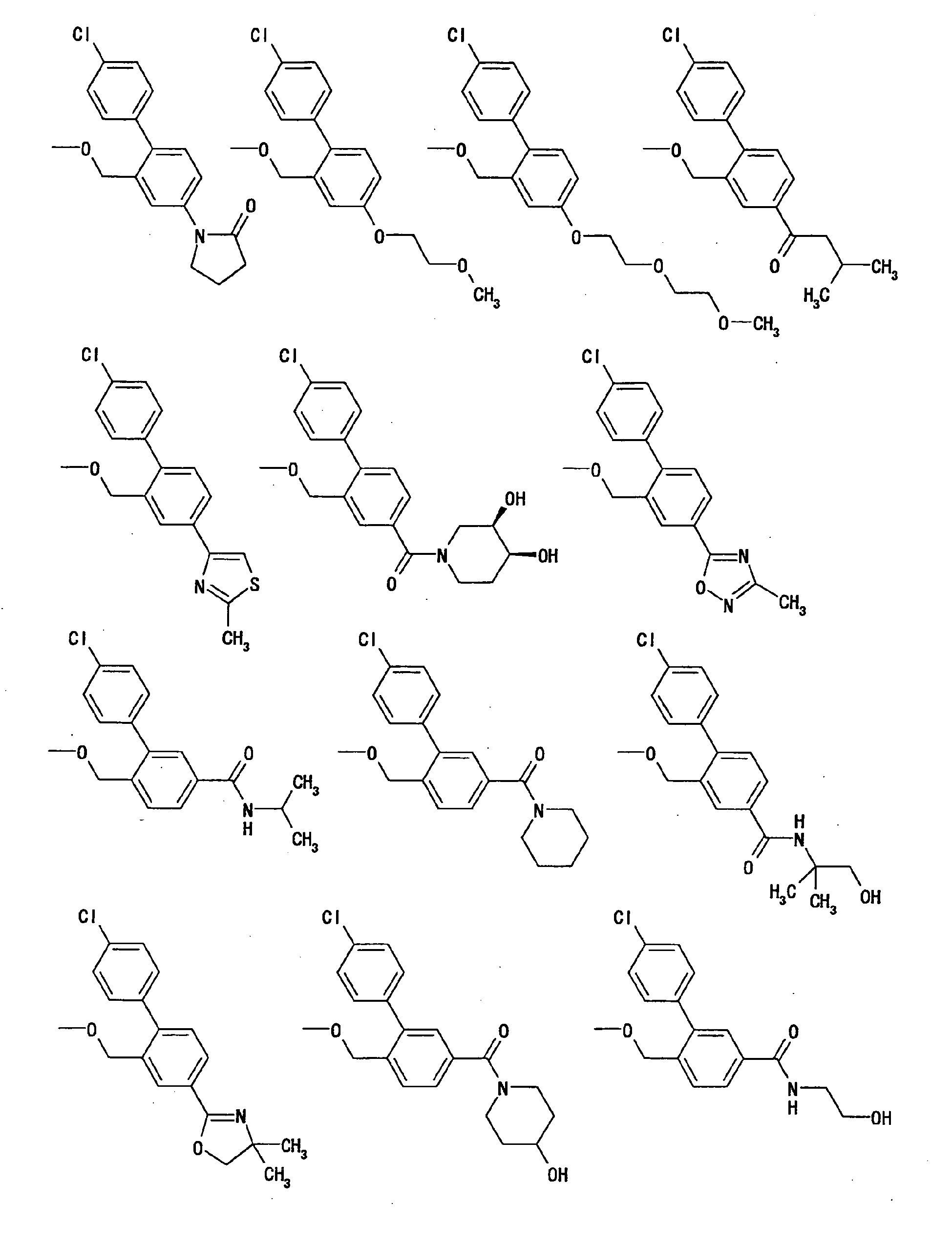 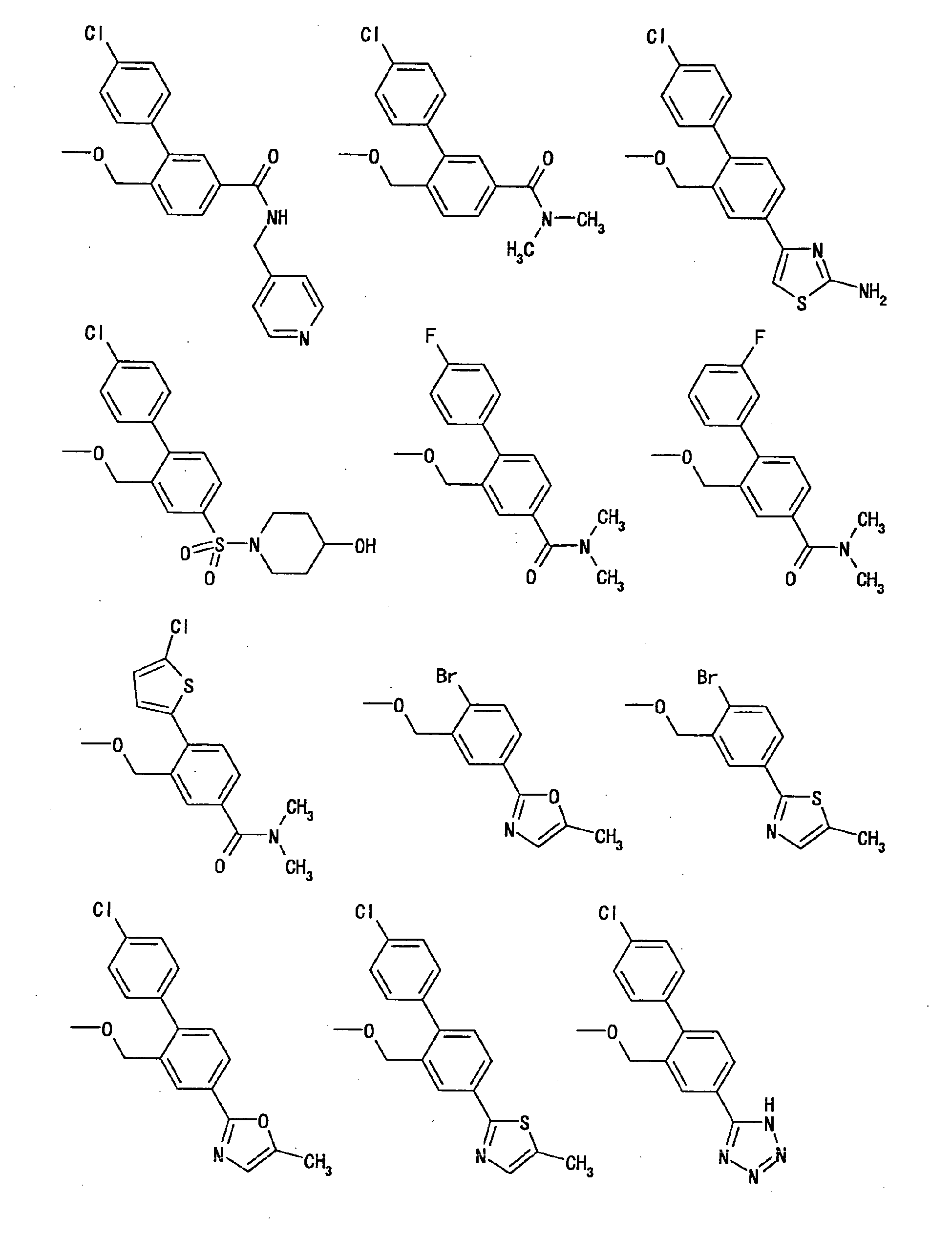 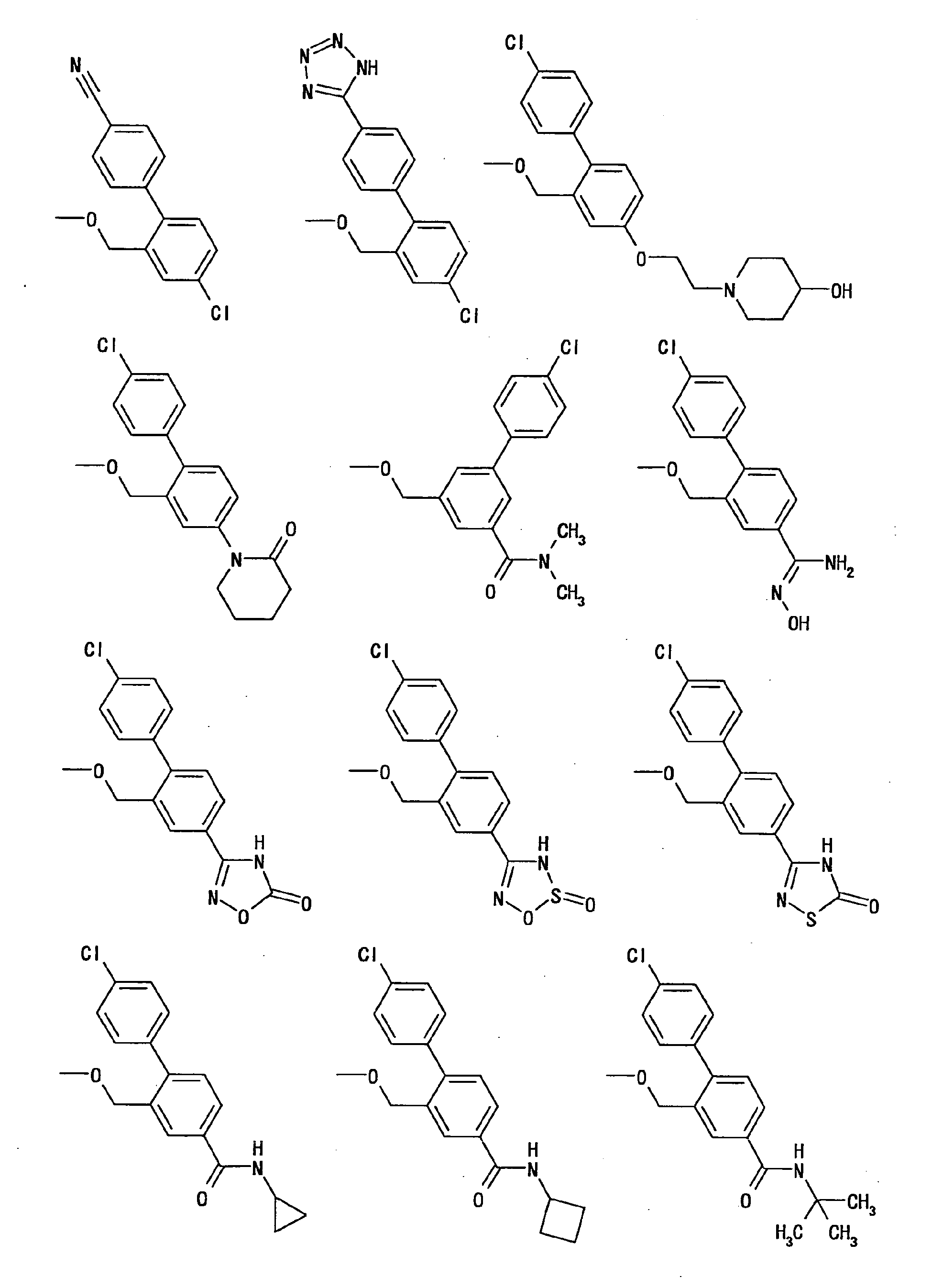 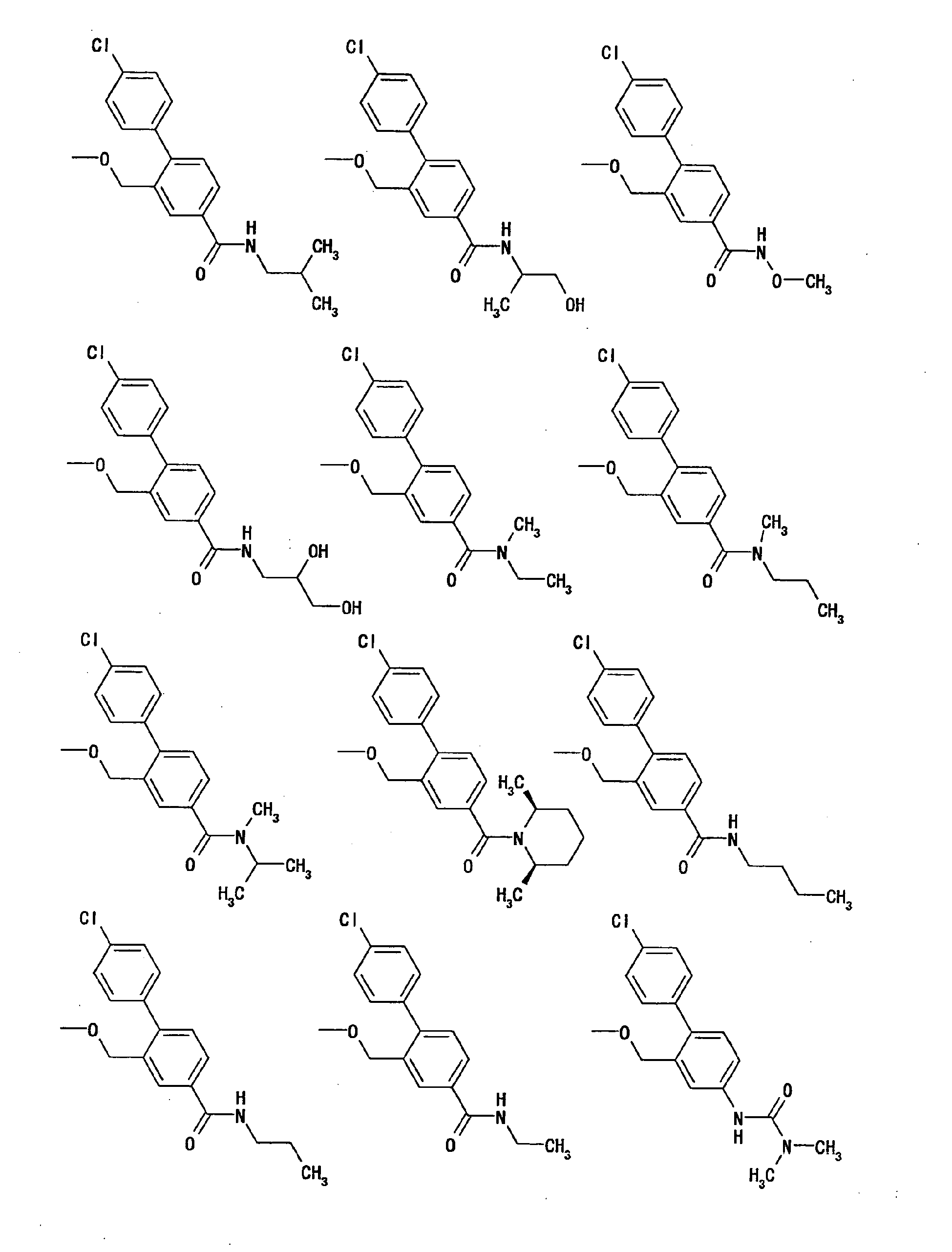 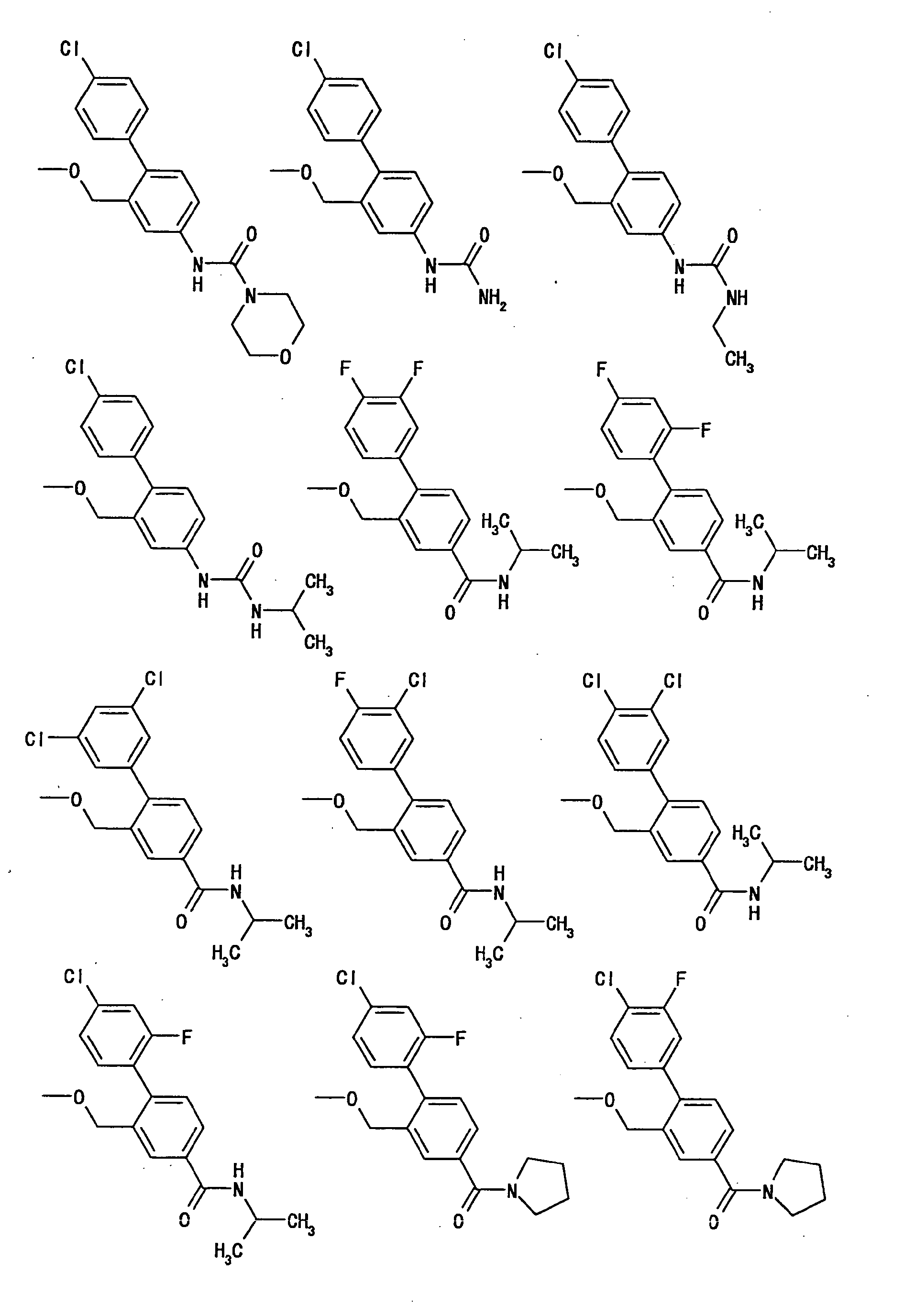 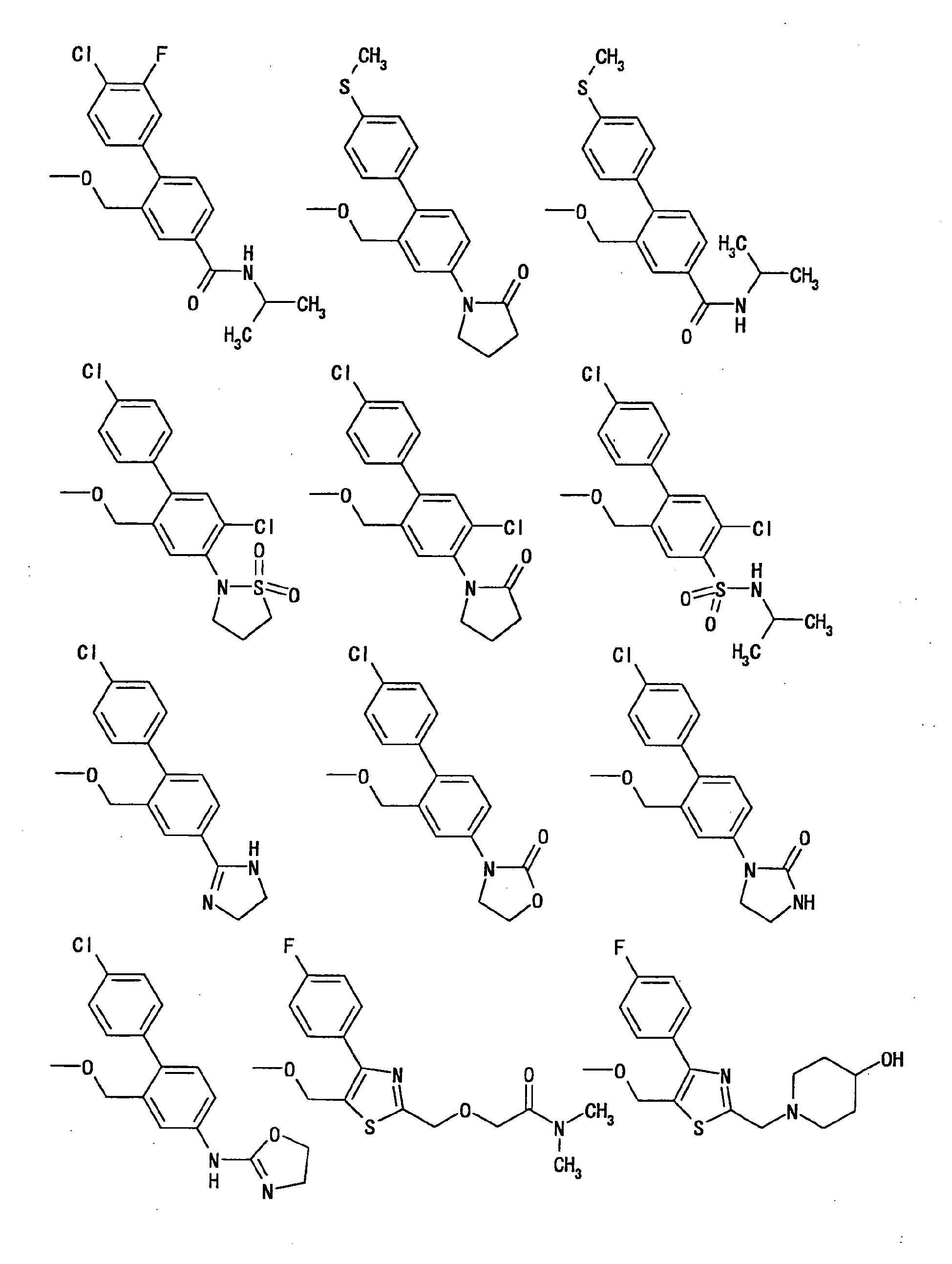 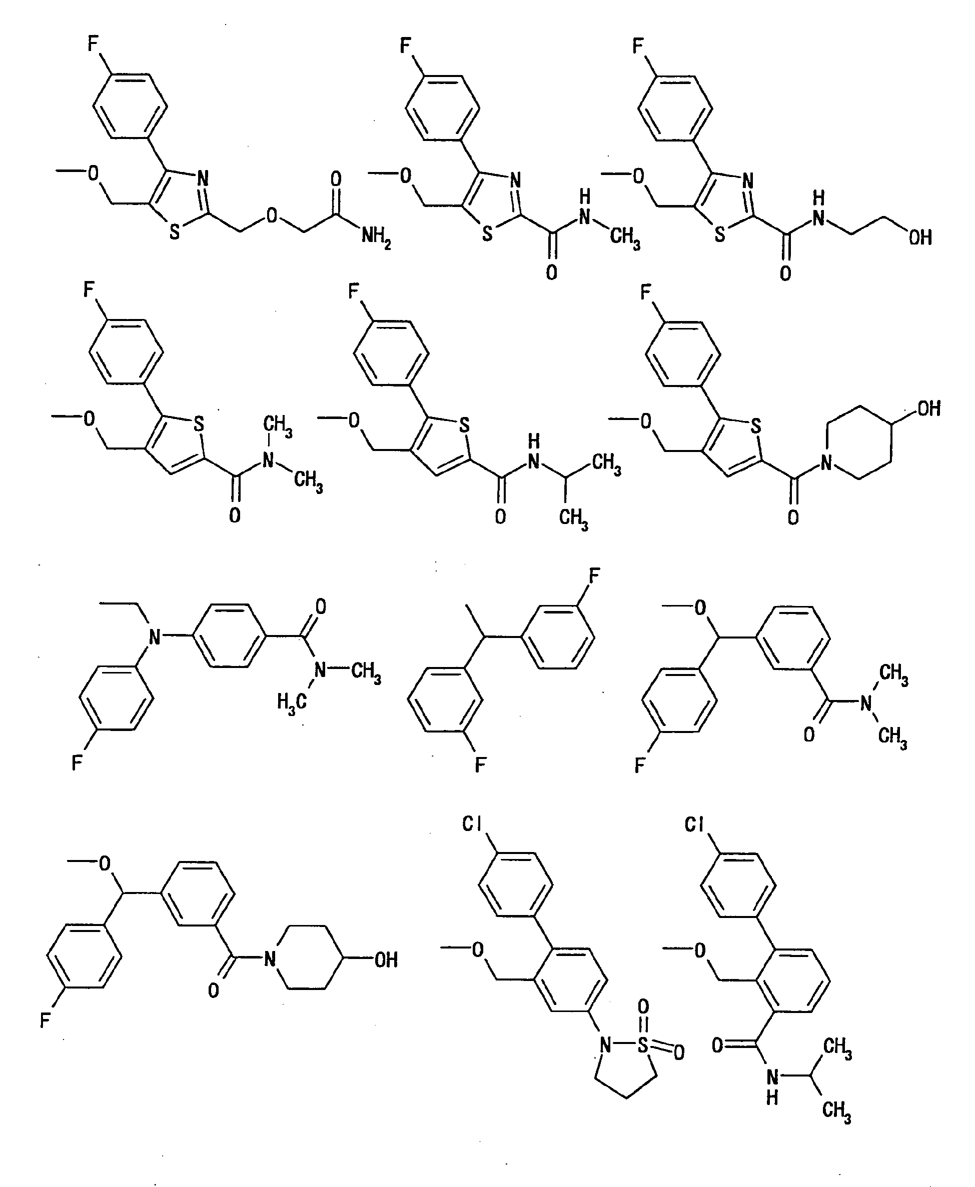 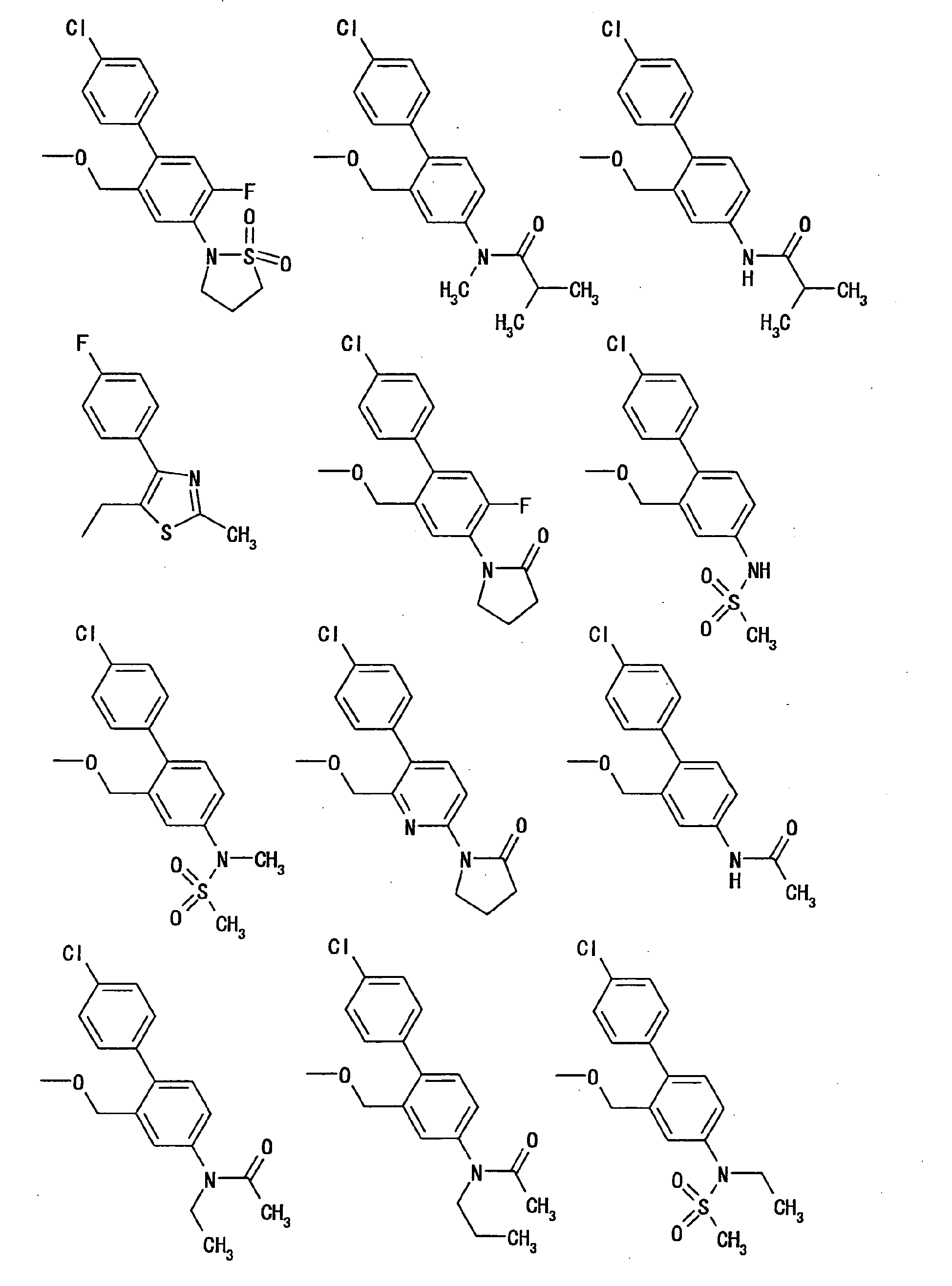 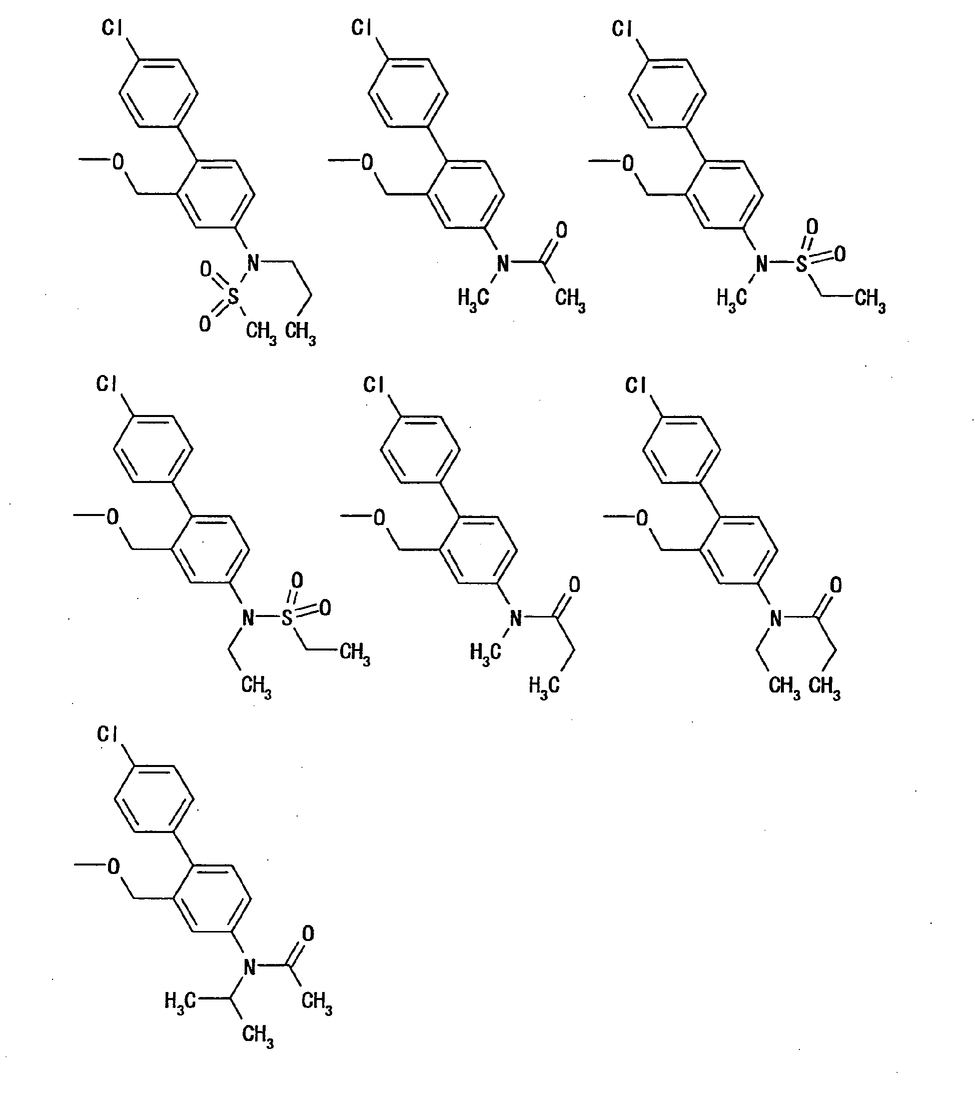 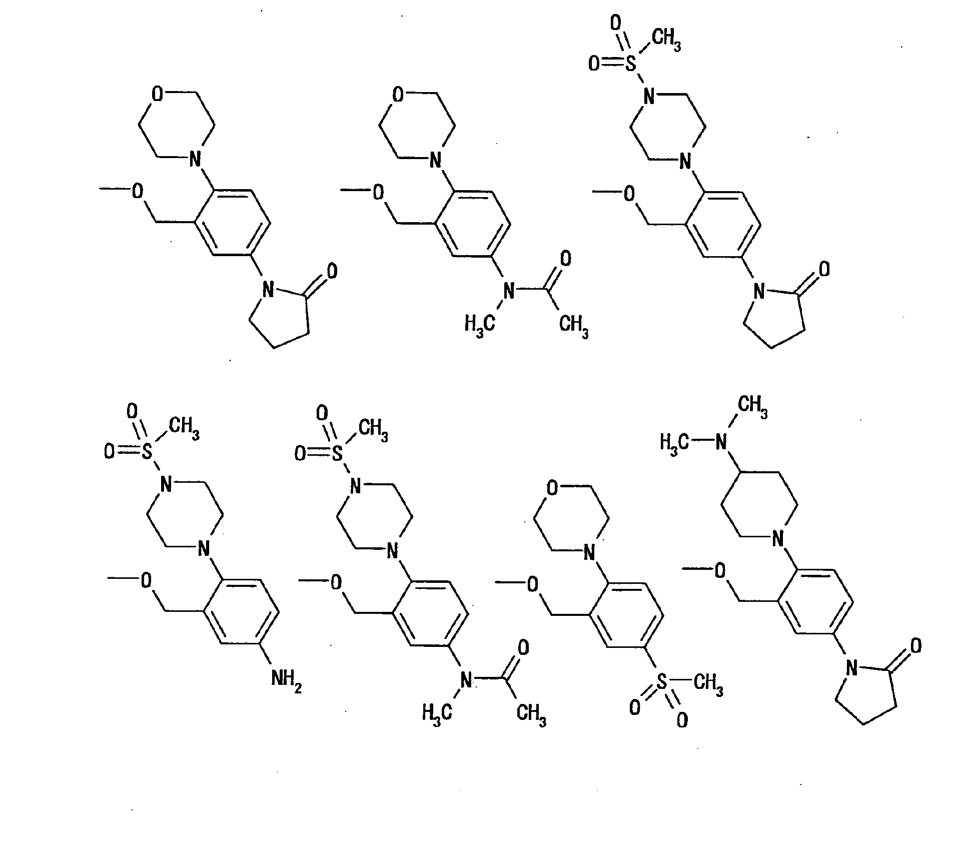 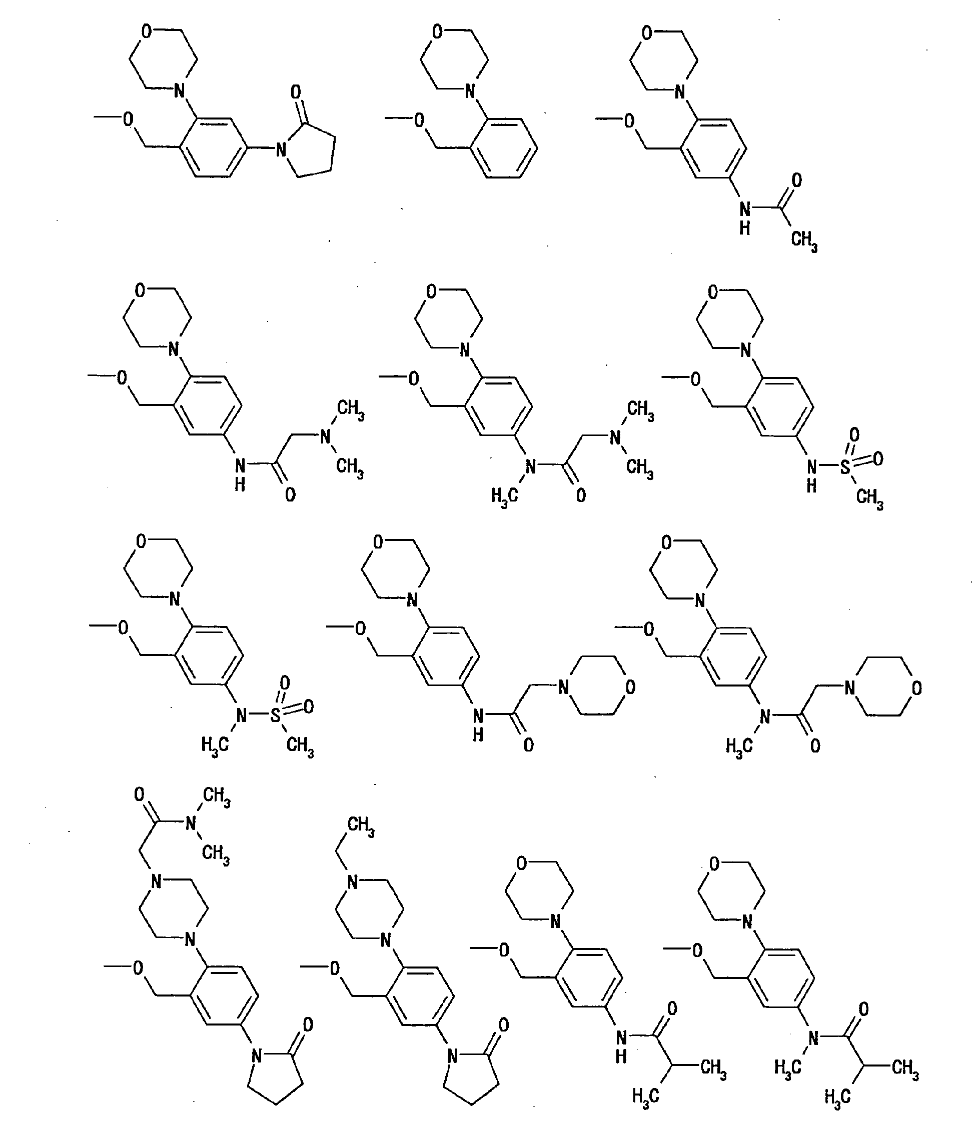 and the like can be specifically mentioned.

As a combination of R² and X,
when R² is a C₁₋₆ alkyl group optionally substituted by 1 to 3 substituents selected from group E, X is preferably 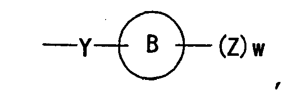 and when R² is 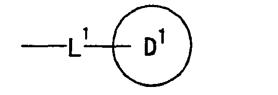 or 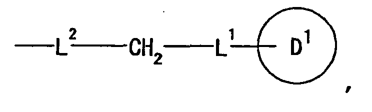 X is a hydrogen atom, a halogen atom (preferably, fluorine atom or chlorine atom), a C₁₋₆ alkyl group optionally substituted by 1 to 3 substituents selected from group A (preferably, methyl group or ethyl group) or -OR^{a11} (preferably, methoxy group or ethoxy group) is preferable, -OR^{a11} is more preferable.

The "carboxyl-protecting group" only needs to be suitable for reaction conditions, and is capable of protecting and deprotecting and may be, for example, methyl; substituted methyl group such as methoxymethyl, methylthiomethyl, 2-tetrahydropyranyl, methoxyethoxymethyl, benzyloxymethyl, phenacyl, diacylmethyl, phthalimidomethyl etc.; ethyl; substituted ethyl group such as 2,2,2-trichloroethyl, 2-chloroethyl, 2-(trimethylsilyl)ethyl, 2-methylthioethyl, 2-(p-toluenesulfonyl)ethyl, t-butyl etc.; benzyl; substituted benzyl group such as diphenylmethyl, triphenylmethyl, p-nitrobenzyl, 4-picolyl, p-methoxybenzyl, 2-(9,10-dioxo)anthrylmethyl etc.; silyl group such as trimethylsilyl, t-butyldimethylsilyl, phenyldimethylsilyl etc.; and the like.

The "pharmaceutically acceptable salt" may be any as long as it forms a non-toxic salt with a compound of the above-mentioned formula [I]. Such salt can be obtained by reacting the compound with an inorganic acid, such as hydrochloric acid, sulfuric acid, phosphoric acid, hydrobromic acid and the like; or an organic acid, such as oxalic acid, malonic acid, citric acid, fumaric acid, lactic acid, malic acid, succinic acid, tartaric acid, acetic acid, trifluoroacetic acid, gluconic acid, ascorbic acid, methylsulfonic acid, benzylsulfonic acid, meglumine acid and the like; or an inorganic base, such as sodium hydroxide, potassium hydroxide, calcium hydroxide, magnesium hydroxide, ammonium hydroxide and the like; or an organic base, such as methylamine, diethylamine, triethylamine, triethanolamine, ethylenediamine, tris(hydroxymethyl)methylamine, guanidine, choline, cinchonine and the like; with an amino acid, such as lysine, arginine, alanine and the like. The present invention encompasses water-retaining product, hydrate and solvate of each compound.

The compound represented by the above-mentioned formula [I] have various isomers. For example, E compound and Z compound are present as geometric isomer, and when the compound has asymmetric carbon atom(s), an enantiomer and a diastereomer are present as a stereoisomer due to the asymmetric carbon atom(s). A tautomer may be also present. The present invention encompasses all these isomers and mixtures thereof.

The present invention also encompasses a prodrug and a metabolite of each compound.

A "prodrug" means a derivative of the compound of the present invention, which is capable of chemical or metabolic decomposition, which shows inherent efficacy by reverting to the original compound after administration to a body, and which includes salts and complexes without a covalent bond.

A prodrug is utilized for, for example, improving absorption by oral administration, or targeting of a target site.

As the modification moiety, a functional group having high reactivity in the compound of the present invention can be mentioned such as hydroxyl group, carboxyl group, amino group, thiol group and the like.

As preferable embodiments of the compound of the present invention, a compound having fine pharmacological activity (e.g., a compound having strong polymerase inhibitory activity, a compound having strong inhibitory activity on enzyme complex comprising polymerase, a compound having strong HCV replicon-inhibitory activity, a compound having high anti-HCV activity in HCV infected cells and the like), a compound having fine bioavailability (e.g., a compound showing high oral absorbability, a compound having high cell-permeability, a compound stable to metabolic enzyme, a compound with low binding ability to protein and the like), a highly safe compound (e.g., a compound free of immunogenicity or showing low allergic response, a compound free of or low in increase in bilirubin value, a compound showing low P450 (CYP)-inhibitory activity and the like) and the like can be mentioned.

When the inventive compound is used as a pharmaceutical preparation, the inventive compound is generally admixed with pharmaceutically acceptable carriers, excipients, diluents, binders, disintegrators, stabilizers, preservatives, buffers, emulsifiers, aromatics, coloring agents, sweeteners, thickeners, correctives, solubilizers known per se, and other additives such as water, vegetable oil, alcohol such as ethanol, benzyl alcohol and the like, polyethylene glycol, glycerol triacetate, gelatin, lactose, carbohydrate such as starch and the like, magnesium stearate, talc, lanolin, petrolatum and the like, and prepared into a dosage form of tablets, pills, powders, granules, suppositories, injections, eye drops, liquids, capsules, troches, aerosols, elixirs, suspensions, emulsions, syrups and the like, which can be administered systemically or topically and orally or parenterally.

While the dose varies depending on the age, body weight, general condition, treatment effect, administration route and the like, it is from 0.01 mg to 3 g for an adult per dose, which is given one to several times a day.

The "prophylaxis of hepatitis C" means, for example, administration of a pharmaceutical agent to an individual found to carry an HCV by a test and the like but without a symptom of hepatitis C, or to an individual who shows an improved disease state of hepatitis after a treatment of hepatitis C, but who still carries an HCV and is associated with a risk of recurrence of hepatitis.

The therapeutic agent for hepatitis C of the present invention is expected to provide a synergistic effect when concurrently used with other antiviral agents, antiinflammatory agents or immunostimulants.

The medicaments with the prospect of synergistic effect include, for example, interferon-α, interferon-β, interferon-γ, interleukin-2, interleukin-8, interleukin-10, interleukin-12, TNFα, recombinant or modified products thereof, agonists, antibodies, vaccines, ribozymes, antisense nucleotides and the like.

As evidenced in the combination therapy of anti-HIV agents, which is also called a cocktail therapy, the combined use of various anti-virus agents against viruses showing frequent genetic mutations is expected to show effect for suppressing emergence and increase of drug tolerant viruses. For example, two or three agents from HCV-IRES inhibitors, HCV-NS3 protease inhibitors, HCV-NS2NS3 protease inhibitors, HCV-NS5A inhibitors and HCV polymerase inhibitor may be used in combination. Specifically, the combined use with Ribavirin(R), interferon-α (IFN-α, Roferon(R), Intron A(R), Sumiferon(R), MultiFeron(R), Infergen(R), Omniferon(R), Pegasys(R), PEG-Intron A(R)), interferon-β (Frone(R), Rebif(R), AvoneX(R), IFNβMOCHIDA(R)), interferon-ω, 1-β-L-ribofuranosyl-1H-1,2,4-triazole-3-carboxamide, 16α-bromo-3β-hydroxy-5α-androstan-17-one, 1H-imidazole-4-ethanamide dihydrochloride, HCV ribozyme Heptazyme(R), polyclonal antibody Civacir(R), lactoferrin GPX-400, (1S,2R,8R,8aR)-1,2,8-trihydroxyoctahydroindolizidinium chloride, HCV vaccine (MTH-68/B, Innivax C(R), Engerix B(R)), antisense oligonucleotide ISIS-14803, HCV-RNA transcriptase inhibitor VP-50406, tetrachlorodecaoxide (high concentration Oxoferin(R)), tetrahydrofuran-3-yl (S)-N-3-[3-(3-methoxy-4-oxazol-5-ylphenyl)ureido]benzylcarbamate, 4-amino-2-ethoxymethyl-α,α-dimethyl-1H-imidazo[4,5-c]quinoline-1-ethanol, interleukin-2 (Proleukin(R)), thymosin α1 and the like is exemplified, wherein (R) shows product names.

Furthermore, the combined use with the compounds disclosed in JP-A-08-268890, JP-A-10-101591, JP-A-07-069899, WO99/61613 and the like as HCV-IRES inhibitors; the compounds disclosed in WO98/22496, WO99/07733, WO99/07734, WO00/09543, WO00/09558, WO01/59929, WO98/17679, EP932617, WO99/50230, WO00/74768, WO97/43310, US5990276, WO01/58929, WO01/77113, WO02/8198, WO02/8187, WO02/8244, WO02/8256, WO01/07407, WO01/40262, WO01/64678, WO98/46630, JP-A-11-292840, JP-A-10-298151, JP-A-11-127861, JP-A-2001-103993, WO98/46597, WO99/64442, WO00/31129, WO01/32961, WO93/15730, US7832236, WO00/200400, WO02/8251, WO01/16379, WO02/7761 and the like as HCV protease inhibitors; the compounds disclosed in WO97/36554, US5830905, WO97/36866, US5633388, WO01/07027, WO00/24725 and the like as HCV helicase inhibitors; the compounds disclosed in WO00/10573, WO00/13708, WO00/18231, WO00/06529, WO02/06246, WO01/32153, WO01/60315, WO01/77091, WO02/04425, WO02/20497, WO00/04141 and the like as HCV polymerase inhibitors; the compounds disclosed in WO01/58877, JP-A-11-180981, WO01/12214 and the like as interferon agonists or enhancers; and the like is also exemplified.

In the case of combined administration, the compound of the present invention can be administered simultaneously with a pharmaceutical agent to be used in combination (hereinafter combination drug) or administered at certain time intervals. In the case of combined administration, a pharmaceutical composition containing the compound of the present invention and a combination drug can be administered. Alternatively, a pharmaceutical composition containing the compound of the present invention and a pharmaceutical composition containing a combination drug may be administered separately. The administration route of the compound of the present invention and that of the combination drug may be the same or different.

In the case of a combined administration, the compound of the present invention can be administered once a day or several times a day in a single dose of 0.1 mg to 1 g, or may be administered at a smaller dose. The combination drug can be administered at a dose generally used for the prevention or treatment of hepatitis C, for example, at a single dose of 0.2 mg to 0.8 mg. Alternatively, it may be administered at a smaller dose.

Inasmuch as HCV is known to be a virus associated with many genetic mutations, a compound effective for many genotypes is one of the preferable modes. If a compound ensures high blood concentration and sustention thereof when administered as a pharmaceutical agent to an animal infected with HCV, it is also one of the preferable modes. From these aspects, a compound having high inhibitory activity on both HCV type 1a and type 1b and high blood concentration is particularly preferable.

Examples of the Production Method of the compound to be used for the practice of the present invention are given in the following. However, the Production Method of the compound of the present invention is not limited to these examples.

Even if no directly corresponding disclosure is found in the following Production Methods, the steps may be modified for efficient production of the compound, such as introduction of a protecting group into a functional group with deprotection in a subsequent step, and changing the order of Production Methods and steps.

The treatment after reaction in each step may be conventional ones, for which typical methods, such as isolation and purification, crystallization, recrystallization, silica gel chromatography, preparative HPLC and the like, can be appropriately selected and combined.

### Production Method 1

By this production method, compound [1-5] wherein 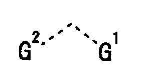 is N=C-N is produced.

### Production Method 1-1

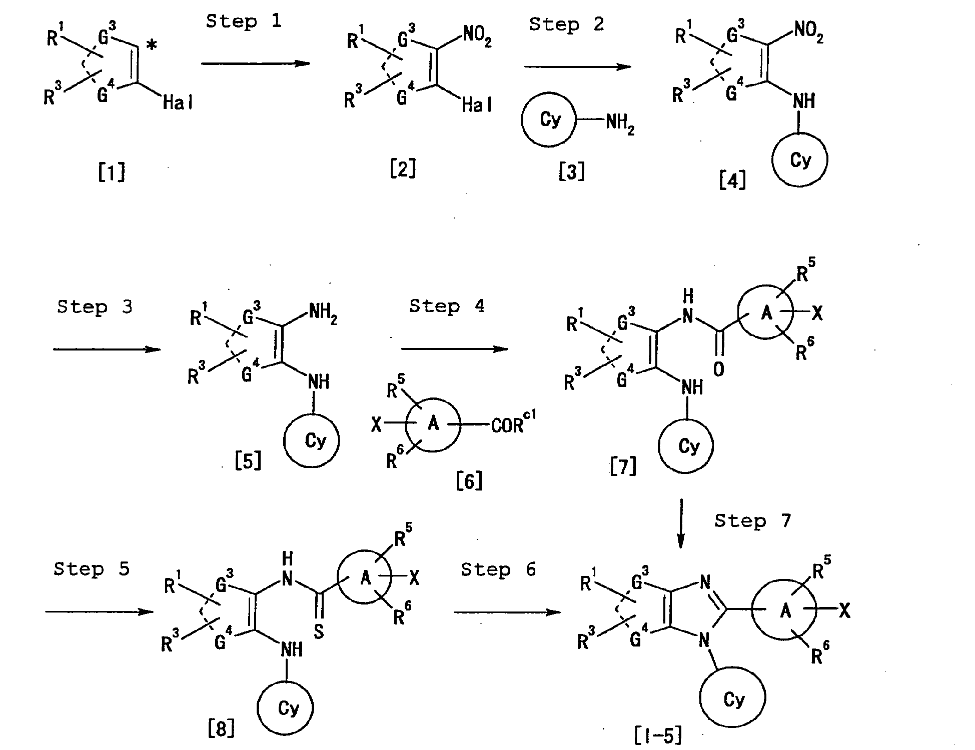

wherein Hal is a halogen atom such as chlorine atom, bromine atom and the like, R^{c1} is a halogen atom such as chlorine atom, bromine atom and the like or hydroxyl group, and other symbols are as defined above. Here, R¹ and R³ do not substitute at the position *.

### Step 1

Compound [2] can be obtained by nitrating compound [1] obtained by a conventional method or commercially available compound [1] with a nitrating agent such as nitric acid, fuming nitric acid, mixed acid of conc. nitric acid and conc. sulfuric acid, and the like at room temperature or under cooling.

### Step 2

Compound [4] can be obtained by reacting compound [2] with amine compound [3] in a solvent such as dimethyl sulfoxide (DMSO), N,N-dimethylformamide (DMF), acetonitrile, tetrahydrofuran (THF), toluene and the like, in the presence or absence of a base such as potassium carbonate, triethylamine, potassium tert-butoxide and the like at room temperature or under heating.

### Step 3

Compound [4] is hydrogenated in a solvent such as methanol, ethanol, THF, ethyl acetate, acetic acid, water and the like in the presence of a catalyst such as palladium carbon, palladium hydroxide, platinum oxide, Raney nickel and the like at room temperature or under heating to give compound [5]. In addition, compound [4] is reduced with a reducing agent such as zinc, iron, tin(II) chloride, sodium sulfite and the like, or reacted with hydrazine in the presence of iron(III) chloride to give compound [5]. Compound [5] can be also obtained by reacting compound [4] with sodium hydrosulfite under alkaline conditions.

### Step 4

Compound [5] is condensed with carboxylic acid compound [6] in a solvent such as DMF, acetonitrile, THF, chloroform, ethyl acetate, methylene chloride, toluene and the like using a condensing agent such as dicyclohexylcarbodiimide, 1-ethyl-3-(3-dimethylaminopropyl)carbodiimide hydrochloride, diphenylphosphoryl azide and the like and, where necessary, adding N-hydroxysuccinimide, 1-hydroxybenzotriazole and the like to give amide compound [7]. Alternatively, amide compound [7] can be obtained as follows. The carboxylic acid compound [6] is converted to an acid halide with thionyl chloride, oxalyl chloride and the like, or to an active ester of compound [6] (e.g., converting to a mixed acid anhydride with ethyl chlorocarbonate and the like), which is then reacted with compound [5] in the presence of a base such as triethylamine, potassium carbonate, pyridine and the like, or in an amine solvent such as pyridine and the like, to give amide compound [7].

### Step 5

Compound [8] can be obtained by reacting compound [7] with a thiocarbonylating agent such as Lawesson reagent, diphosphorus pentasulfide and the like in a solvent such as THF, 1,2-dimethoxyethane (DME), toluene, xylene, chloroform, methylene chloride, 1,4-dioxane and the like, at room temperature or under heating.

### Step 6

Compound [I-5] can be obtained by cyclizing compound [8] in the presence of a condensing agent such as dicyclohexylcarbodiimide, 1-ethyl-3-(3-dimethylaminopropyl)carbodiimide hydrochloride, diphenylphosphoryl azide and the like in a solvent such as DMF, acetonitrile, THF, chloroform, ethyl acetate, methylene chloride, toluene and the like at room temperature or under heating.

Compound [I-5] can be obtained by treating compound [8] with alkyl halide such as methyl iodide and the like in a solvent such as methanol, ethanol, chloroform and the like.

Compound [I-5] can be obtained by treating compound [8] with mercury oxide in a solvent such as ethanol, methanol and the like. Here, a catalytic amount of sulfur may be concurrently used.

This production method is particularly suitable when 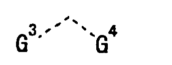 is C=C-S or S-C=C.

### Step 7

Compound [7] is heated in a solvent such as ethanol, methanol, toluene, DMF, chloroform and the like or without a solvent in the presence of an acid such as acetic acid, formic acid, hydrochloric acid, dilute sulfuric acid, phosphoric acid, polyphosphoric acid, p-toluenesulfonic acid and the like, a halogenating agent such as zinc chloride, phosphorus oxychloride, thionyl chloride and the like or an acid anhydride such as acetic anhydride and the like, to allow cyclization to give compound [I-5].

### Production Method 1-2

This production method is a different method to produce compound [I-5]. 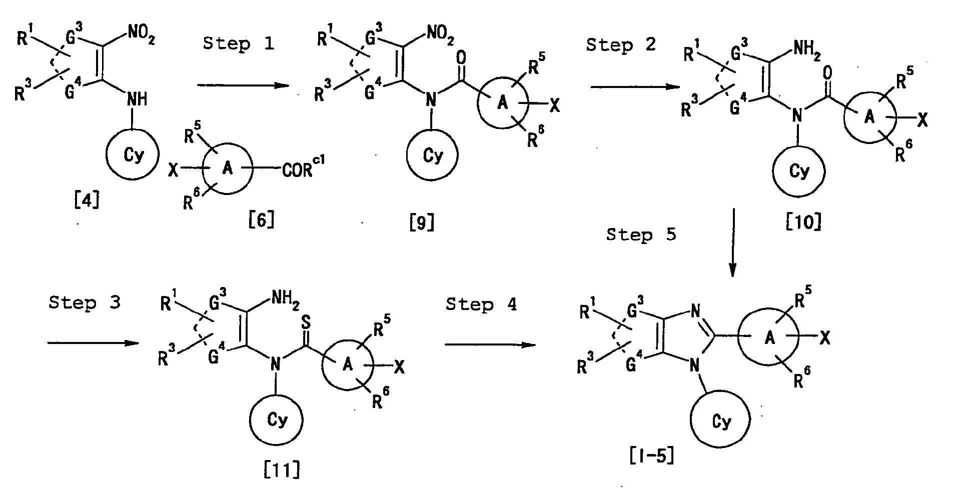 wherein each symbol is as defined above.

### Step 1

Compound [9] can be obtained by amide condensation of compound [4] obtained in the same manner as in Production Method 1-1, Step 2 with compound [6] in the same manner as in Production Method 1-1, Step 4.

### Step 2

Compound [10] can be obtained by reducing compound [9] in the same manner as in Production Method 1-1, Step 3.

### Step 3

Compound [11] can be obtained by reacting compound [10] with a thiocarbonylating agent in the same manner as in Production Method 1-1, Step 5.

### Step 4

Compound [I-5] can be obtained by cyclizing compound [11] in the same manner as in Production Method 1-1, Step 6.

### Step 5

Compound [I-5] can also be obtained by cyclizing compound [10] in the same manner as in Production Method 1-1, Step 7.

### Production Method 2

By this production method, a thienoimidazole compound wherein 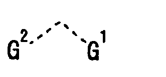 is N=C-N and 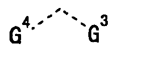 is C=C-S or S-C=C is produced. 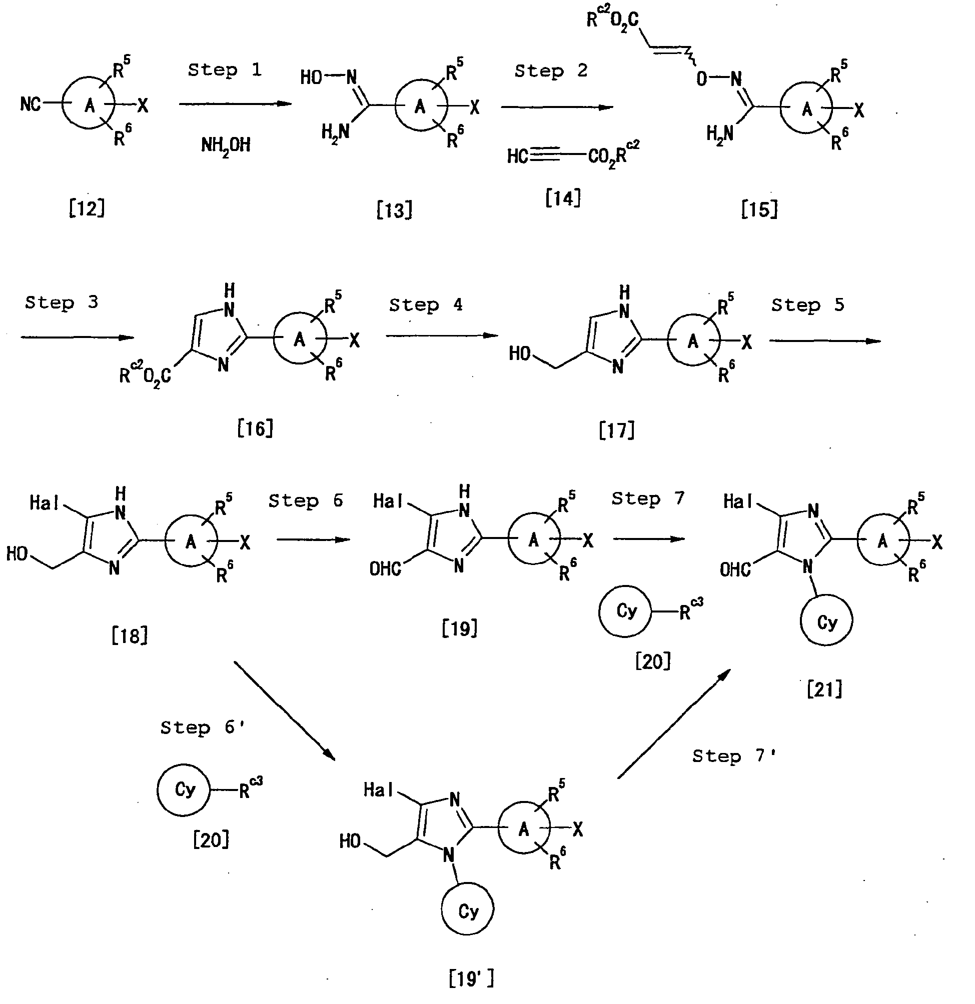 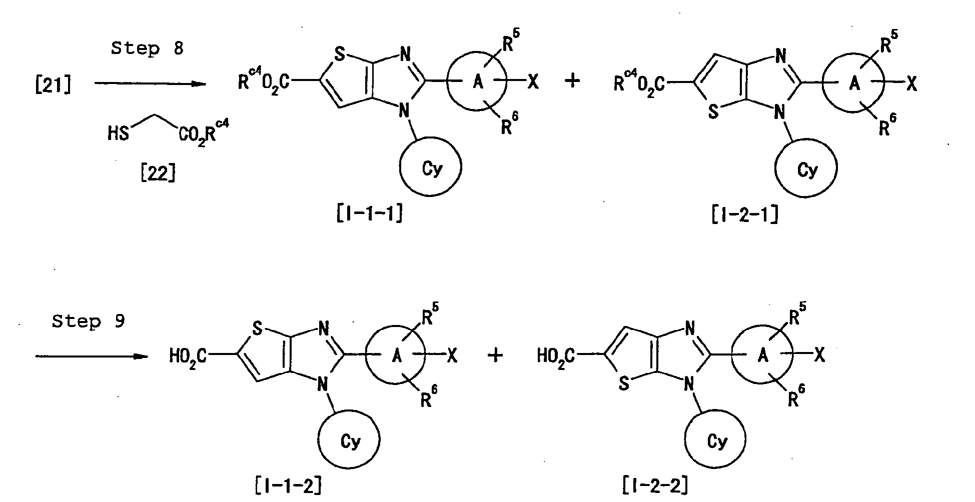 wherein R^{c2} and R^{c4} is a carboxyl-protecting group such as methyl group, ethyl group, phenyl group, benzyl group and the like, R^{c3} is a leaving group such as a halogen atom (e.g., chlorine atom, bromine atom and the like), a sulfonate (e.g., mesyloxy group, tosyloxy group and the like) and the like, and other symbols are as defined above.

### Step 1

Compound [13] can be obtained by reacting compound [12] obtained by a conventional method or commercially available compound [12] with hydroxylamine in a solvent such as water, methanol, ethanol, THF, DMF and the like at room temperature or under heating. When hydroxylamine hydrochloride and the like are used, the reaction is carried out in the presence of a base such as sodium carbonate, potassium carbonate, sodium hydrogen carbonate, sodium hydroxide, triethylamine and the like.

### Step 2

Compound [15] can be obtained by reacting compound [13] with propiolic acid ester [14] in a solvent such as methanol, ethanol, DMF, toluene, chloroform, methylene chloride, THF, dimethyl ether, acetonitrile and the like at room temperature or under heating.

### Step 3

Compound [16] can be obtained by cyclizing compound [15] with heating in a solvent such as diphenyl ether, decalin, naphthalene and the like, or without solvent.

### Step 4

Compound [17] can be obtained by reducing compound [16] by a conventional method.

For example, compound [17] can be obtained by reacting compound [16] in a solvent such as methanol, ethanol, THF, diethyl ether, 1,4-dioxane and the like in the presence of a reducing agent such as lithium aluminum hydride, sodium cyanoborohydride, lithium borohydride and the like under cooling to heating.

### Step 5

Compound [18] can be obtained by reacting compound [17] with a halogenating agent such as bromine, iodine, N-chlorosuccinimide, N-bromosuccinimide, N-iodosuccinimide and the like in a solvent such as DMF, acetonitrile, THF, chloroform, methylene chloride, ethyl acetate, acetone and the like at room temperature or under heating.

### Step 6

Compound [19] can be obtained by oxidizing compound [18] with an oxidizing agent such as oxalyl chloride, manganese dioxide, pyridinium chlorochromate, pyridinium dichromate, Collins reagent, Dess-Martin reagent and the like in a solvent such as DMF, DMSO, acetonitrile, THF, chloroform, methylene chloride, ethyl acetate, acetone and the like at room temperature or under heating. For example, an oxidizing method such as TPAP (tetrapropylammonium perruthenate) oxidation, DMSO oxidation and the like may be used. In addition, a treatment in the presence of sulfur trioxide in pyridine may be performed.

### Step 7

Compound [21] can be obtained by reacting compound [19] with compound [20] in a solvent such as DMF, DMSO, acetonitrile, ethanol, THF and the like in the presence of a base such as sodium hydride, sodium hydroxide, potassium hydroxide, potassium carbonate, sodium ethoxide, potassium tert-butoxide and the like at room temperature or under heating.

### Step 6'

Compound [19'] can be obtained by reacting compound [18] with compound [20] in the same manner as in Production Method 2, Step 7.

### Step 7'

Compound [21] can be obtained by oxidizing compound [19'] in the same manner as in Production Method 2, Step 6.

### Step 8

Compound [I-1-1] and its isomer, compound [I-2-1], can be obtained by reacting compound [21] with thioglycolic acid ester [22] in a solvent such as methanol, ethanol, toluene, THF, 1,4-dioxane, DMF and the like in the presence of a base such as sodium methoxide, sodium ethoxide and the like at room temperature or under heating.

Compound [I-1-1] and compound [I-2-1] can also be obtained by reacting compound [21] with thioglycolic acid ester [22] in a solvent such as DMF in the presence of potassium carbonate or lithium hydroxide at room temperature or under heating.

### Step 9

Compound [I-1-2] and compound [I-2-2] can be obtained by hydrolyzing compound [I-1-1] and compound [I-2-1], respectively, in a solvent such as methanol, ethanol, THF, 1,4-dioxane and the like, or in a mixed solvent thereof, or in a mixed solvent of such solvent and water, under basic conditions with sodium hydroxide, potassium hydroxide, potassium carbonate, lithium hydroxide and the like or under acidic conditions with hydrochloric acid, sulfuric acid and the like.

### Production Method 3

By this production method, a compound wherein 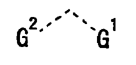 is N-C=C is produced.

### Production Method 3-1

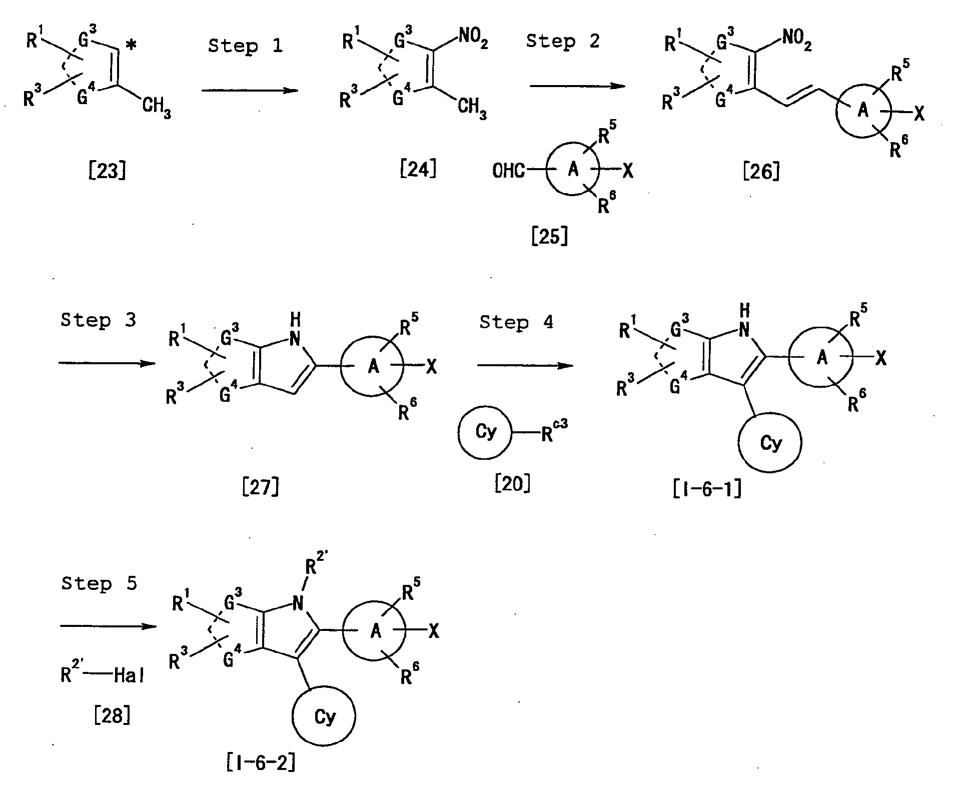

wherein R²' is a C₁₋₆ alkyl group optionally substituted by 1 to 3 substituents selected from group E and the like, and other symbols are as defined above. Here, R¹ and R³ do not substitute at the position *.

### Step 1

Compound [24] can be obtained by nitrating compound [23] obtained by a conventional method or commercially available compound [23] in the same manner as in Production Method 1-1, Step 1.

### Step 2

Compound [26] can be obtained by reacting compound [24] with aldehyde compound [25] in a solvent such as methanol, ethanol, DMF, DMSO, THF, 1,4-dioxane, toluene and the like, in the presence of a base such as pyrrolidine, diethylamine, potassium carbonate, sodium hydride, sodium methoxide, sodium ethoxide, potassium tert-butoxide and the like at room temperature or under heating.

### Step 3

Compound [27] can be obtained by cyclizing compound [26] with heating in a solvent such as ethanol, methanol, toluene, DMF, DMSO, pyridine, naphthalene, chloroform, acetic acid and the like or without solvent in the presence of phosphorous acid ester such as triethyl phosphite and the like.

### Step 4

Compound [I-6-1] can be obtained by reacting compound [27] with compound [20] in a solvent such as DMF, DMSO, 1,4-dioxane, acetonitrile, ethanol, THF and the like, in the presence of a base such as sodium hydride, sodium hydroxide, potassium hydroxide, potassium carbonate, cesium carbonate, sodium ethoxide, potassium tert-butoxide and the like under ice-cooling to heating.

### Step 5

Compound [I-6-2] can be obtained by reacting compound [I-6-1] with compound [28] in a solvent such as DMF, DMSO, acetonitrile, ethanol, THF and the like, in the presence of a base such as sodium hydride, sodium hydroxide, potassium hydroxide, potassium carbonate, sodium ethoxide, potassium tert-butoxide and the like, under ice-cooling to under heating.

In this Production Method, R²' may be any groups as long as it is bonded to nitrogen atom of fused ring via carbon atom, wherein C₁₋₆ alkyl group optionally substituted by 1 to 3 substituents selected from group E, as well as, for example, L² of -L²-ring D²-L¹-ring D¹ and -L²-CH₂-L¹-ring D¹, and L¹ of -L¹⁻(CHR^{L4})_{u1'}-L³-(CHR^{L5})ᵥ₁-ring D¹ and -L¹-ring D¹ may be C₁₋₆ alkylene, C₂₋₆ alkenylene, -(CHR^{L4})_{u1'}-O-(CHR^{L5})ᵥ₁-, -(CHR^{L4})_{u1'}-S-(CHR^{L5})ᵥ₁-, -(CHR^{L4})ᵤ₁,-NR^{L1}-(CHR^{L5})ᵥ₁-, -(CHR^{L4})_{u1'}-CO-(CHR^{L5})ᵥ₁-, -(CHR^{L4})_{u1'}-CONR^{L2}-(CHR^{L5})ᵥ₁-, -(CHR^{L4})_{u1'}-NR^{L2}CO₂-(CHR^{L5})ᵥ₁-, - (CHR^{L4})_{u1'}-NR^{L2}CONR^{L3}-(CHR^{L5})ᵥ₁-, -(CHR^{L4})ᵤ₁,-NR^{L2}CO-(CHR^{L5})ᵥ₁-, -(CHR^{L4})_{u1'}-NR^{L2}SO₂-(CHR^{L5})ᵥ₁-, -(CHR^{L4})_{u1'}-SO₂-(CHR^{L5})ᵥ₁- or - (CHR^{L4})ᵤ₁,-SO₂NR^{L2}-(CHR^{L5})ᵥ₁- wherein each symbol is as defined above. Here, u1' is an integer of 1 to 6.

In addition, Hal-R²' may be Hal-ring D¹ or Hal-ring D²-L¹⁻ring D¹.

### Production Method 3-2

For example, a production method when R^{2'} is -C₁₋₆ alkylene-COOR^{e5} or -C₁₋₆ alkylene-CONR^{e6}R^{e7} is as follows. 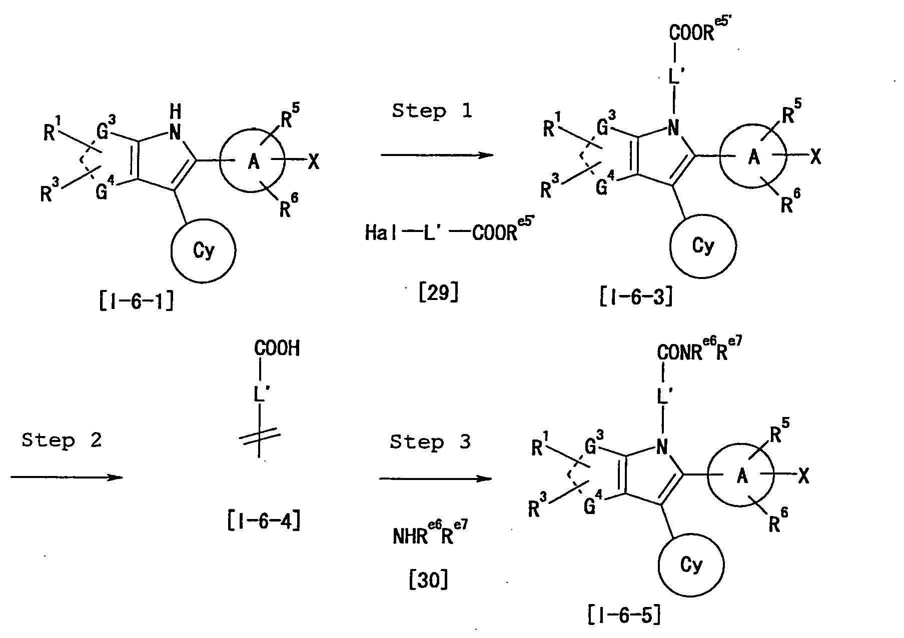 wherein L' is C₁₋₆ alkylene, R^{e5}' is a group selected from group F, and other symbols are as defined above.

### Step 1

Compound [I-6-3] can be obtained by reacting compound [I-6-1] with compound [29] in the same manner as in Production Method 3-1, Step 5.

### Step 2

Compound [I-6-4] can be obtained by eliminating R^{e5}' of compound [I-6-3] by a conventional method.

For example, when R^{e5}' is a tert-butyl group, compound [I-6-4] can be obtained by reacting compound [I-6-3] under mild conditions of using trifluoroacetic acid at room temperature and the like. In addition, compound [I-6-4] can also be obtained by treating compound [I-6-3] with hydrogen chloride or hydrochloric acid in a solvent such as ethyl acetate, 1,4-dioxane, alcohol and the like.

### Step 3

Carboxylic acid compound [I-6-4] is condensed with amine compound [30] in a solvent such as DMF, acetonitrile, THF, chloroform, ethyl acetate, methylene chloride, toluene and the like using a condensing agent such as dicyclohexylcarbodiimide, 1-ethyl-3-(3-dimethylaminopropyl)carbodiimide hydrochloride, diphenylphosphoryl azide and the like and, where necessary, adding N-hydroxysuccinimide, 1-hydroxybenzotriazole and the like to give amide compound [I-6-5]. Alternatively, amide compound [I-6-5] can be obtained as follows. The carboxylic acid compound [I-6-4] is converted to an acid halide with thionyl chloride, oxalyl chloride and the like, or to an active ester of carboxylic acid compound [I-6-4] (e.g., converting to a mixed acid anhydride with ethyl chlorocarbonate and the like), which is then reacted with amine compound [30] in the presence of a base such as triethylamine, potassium carbonate, sodium hydrogen carbonate, pyridine and the like, or in an amine solvent such as pyridine and the like, under ice-cooling or at room temperature to give amide compound [I-6-5].

### Production Method 4

By this production method, a compound wherein ring Cy is a C₃₋₈ cycloalkyl group is produced from a compound wherein ring Cy is a C₃₋₈ cycloalkenyl group. 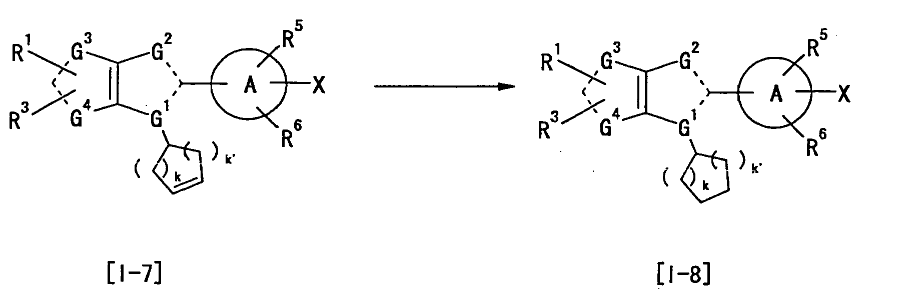 wherein k and k' are the same or different and each is 0 or an integer of 1 to 5, provided that k+k' is not 0, and other symbols are as defined above.

Compound [I-8] can be obtained by hydrogenation of compound [I-7] obtained in the same manner as in the above-mentioned Production Method in a solvent such as methanol, ethanol, THF, ethyl acetate, acetic acid, formic acid, water and the like, in the presence of a catalyst such as palladium carbon, palladium hydroxide, platinum oxide, Raney-nickel and the like, at room temperature or under heating.

### Production Method 5

In this Production Method, conversion of the substituents R¹, R² and R³ on the fused ring is shown. This Production Method is applicable irrespective of the position of substitution of the substituent.

### Production Method 5-1

### Conversion of carboxylic acid ester moiety to amide

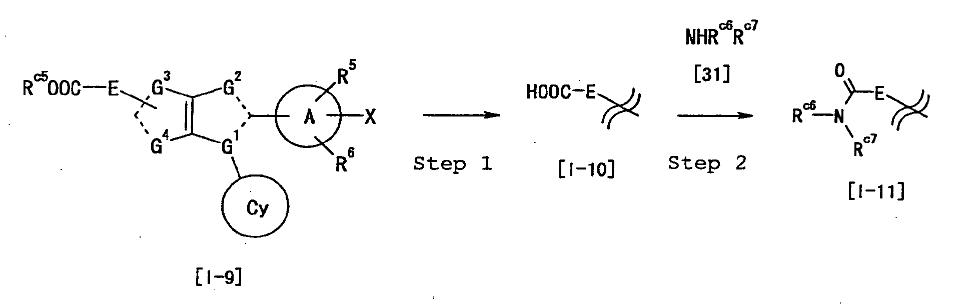

wherein E is a single bond, -(CH₂)ₛ-, -O-(CH₂)ₛ- or -NH-(CH₂)ₛ₋(wherein s is an integer of 1 to 6) , R^{c5}, R^{c6} and R^{c7} are C₁₋₆ alkyl group, and other symbols are as defined above.

### Step 1

Compound [I-9] obtained in the same manner as in the above-mentioned Production Method is subjected to hydrolysis in a solvent such as methanol, ethanol, THF, 1,4-dioxane and the like, or in a mixed solvent thereof, or in a mixed solvent of such solvent and water under basic conditions with sodium hydroxide, potassium hydroxide, potassium carbonate, lithium hydroxide and the like or under acidic conditions with hydrochloric acid, sulfuric acid and the like to give compound [I-10].

### Step 2

Compound [I-10] is reacted with compound [31] in the same manner as in Step 3 of Production Method 3-2 to give compound [I-11].

### Production Method 5-2

### Conversion of cyano group to substituted amidino group

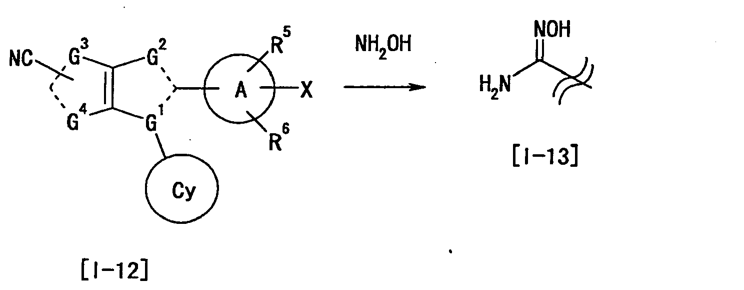

wherein each symbol is as defined above.

Compound [I-12] obtained in the same manner as in the above-mentioned Production Method is reacted with hydroxylamine in a solvent such as water, methanol, ethanol, THF, DMF and the like to give compound [I-13]. When a salt of hydroxylamine such as hydrochloride and the like is used, the reaction is carried out in the presence of a base such as sodium hydrogen carbonate, sodium hydroxide, triethylamine and the like.

### Production Method 5-3

### Conversion of sulfonic acid ester moiety to sulfonic acid

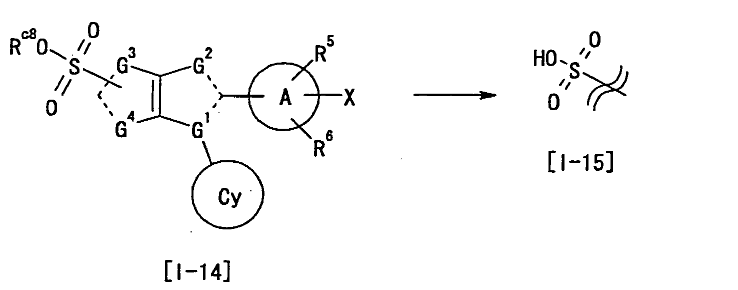

wherein R^{c8} is C₁₋₆ alkyl group, and other symbols are as defined above.

Compound [I-14] obtained in the same manner as in the above-mentioned Production Method is reacted with iodide salt such as sodium iodide, lithium iodide and the like, bromide salt such as sodium bromide, trimethylammonium bromide and the like, amine such as pyridine, trimethylamine, triazole and the like, phosphine such as triphenylphosphine and the like in a solvent such as DMF, DMSO, acetonitrile, methanol, ethanol, water and the like with heating to give compound [I-15].

### Production Method 6

This Production Method relates to convertion of the substituent X on ring A.

### Production Method 6-1

### Conversion of hydroxyl group to ether

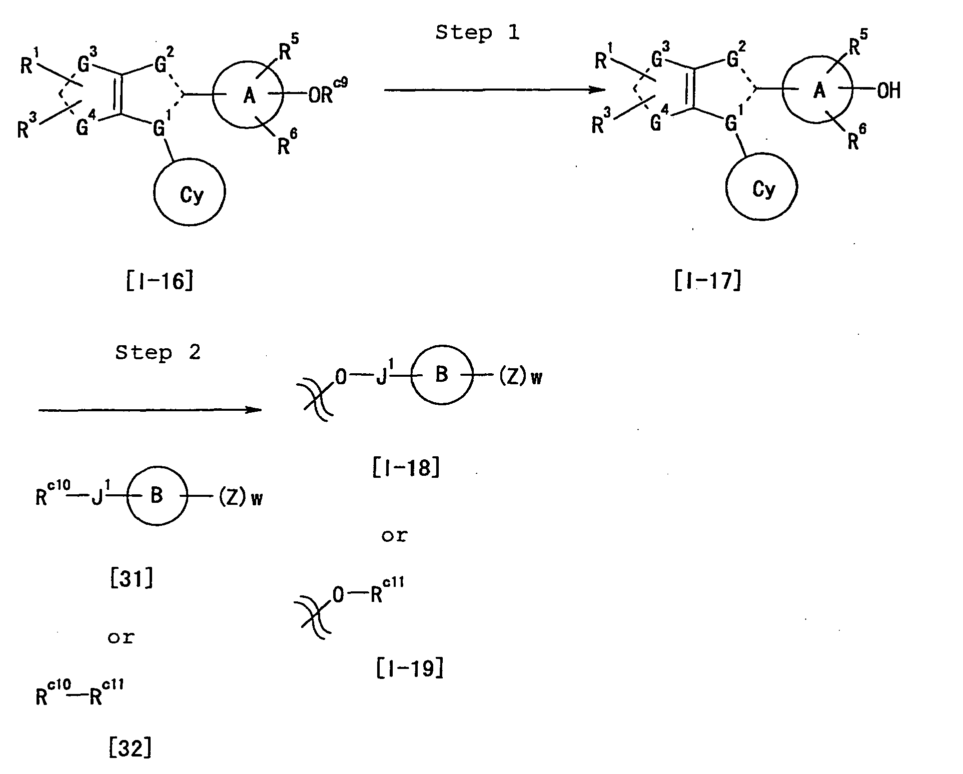

wherein R^{c9} is a hydroxyl-protecting group such as acetyl group, benzyl group and the like, R^{c10} is a halogen atom such as chlorine atom, bromine atom and the like, hydroxyl group, or a leaving group such as a sulfonate (e.g., mesyloxy group, tosyloxy group and the like), R^{c11} is an alkyl group optionally substituted by 1 to 3 substituents selected from group A corresponding to R^{a11}, J¹ is a bond, C₁₋₆ alkylene, C₂₋₆ alkenylene or *-(CH₂)ₘ-Y²-(CH₂)ₙ-, wherein * shows the side to be bonded to R^{c10}, m is an integer of 1 to 6, and other symbols are as defined above.

### Step 1

Compound [I-17] can be obtained by deprotection of compound [I-16] obtained in the same manner as in the above-mentioned Production Method, by a conventional method.

For example, when R^{c9} is acetyl group, compound [I-16] is hydrolyzed, in a solvent such as methanol, ethanol, THF, 1,4-dioxane and the like, or in a mixed solvent thereof, or in a mixed solvent of such solvent and water, under basic conditions with sodium hydroxide, potassium hydroxide, potassium carbonate, lithium hydroxide, sodium methoxide, sodium ethoxide and the like or under acidic conditions with hydrochloric acid, sulfuric acid and the like to give compound [I-17].

When R^{c9} is benzyl group, compound [I-16] is subjected to catalytic reduction in a solvent such as methanol, ethanol, THF, ethyl acetate, acetic acid, water and the like in the presence of palladium carbon, or by reacting with an acid such as hydrobromic acid and the like in a solvent such as acetic acid to give compound [I-17].

### Step 2

When R^{c10} of compound [31] is halogen atom, compound [I-17] is reacted with compound [31] in a solvent such as DMF, DMSO, acetonitrile, ethanol, THF and the like in the presence of a base such as sodium hydride, sodium hydroxide, potassium hydroxide, potassium carbonate, sodium ethoxide, potassium tert-butoxide and the like at room temperature or under heating to give compound [I-18].

When R^{c10} of compound [31] is hydroxyl group, the hydroxyl group of compound [31] is converted to halogen atom with thionyl chloride, phosphorus trichloride, phosphorus tribromide, carbon tetrabromide - triphenylphosphine, N-bromosuccinimide and the like and reacted with compound [I-17] by the aforementioned method to give compound [I-18]. In this case, compound [I-17] may be subjected to Mitsunobu reaction with compound [31] in a solvent such as DMF, acetonitrile, THF and the like using triphenylphosphine - diethyl azodicarboxylate and the like to give compound [I-18].

Compound [I-19] can be obtained in the same manner from compound [I-17] and compound [32].

### Production Method 6-2

### Conversion of nitro group to substituted amino group

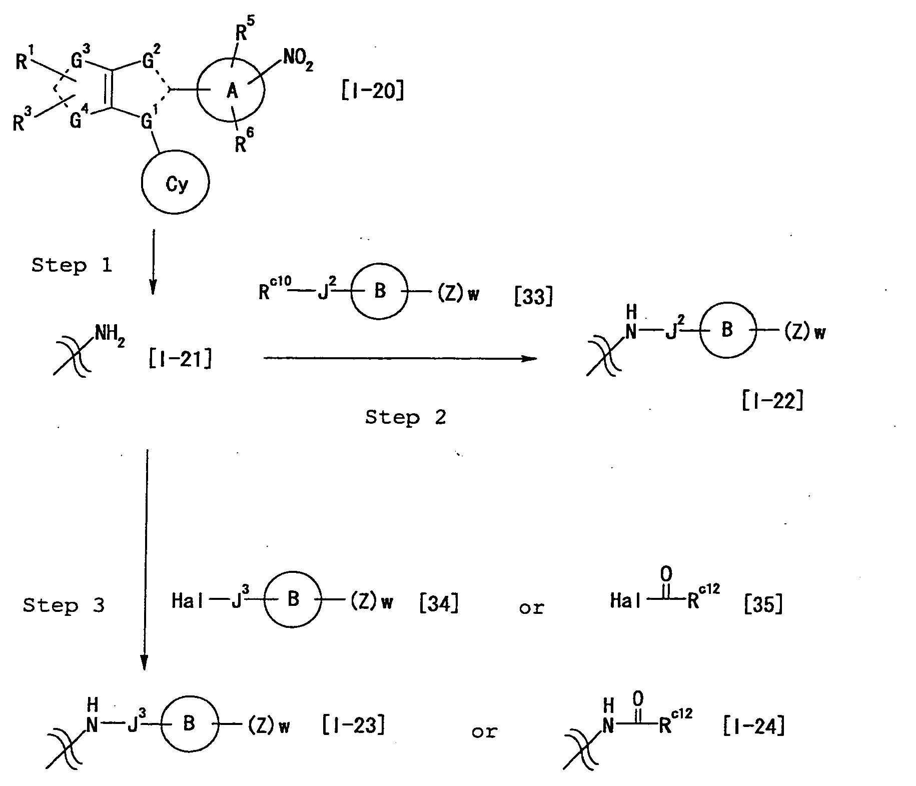

wherein R^{c12} is C₁₋₆ alkyl group, J² is -(CH₂)ₙ- or *-(CH₂)ₘ-Y²⁻(CH₂)ₙ- where m is an integer of 1 to 6, * shows the side to be bonded to R^{c10}, J³ is *-CO-(CH₂)ₘ-Y²-(CH₂)ₙ-, *-CO₂-(CH₂)ₘ-Y²- (CH₂)ₙ-, *-CONR^{y3}-(CH₂)ₘ-Y²-(CH₂)ₙ-, *-SO₂-(CH₂)ₘ-Y²-(CH₂)ₙ-, *-CO-(CH₂)ₙ-, *-CO₂-(CH₂)ₙ-, *-CONR^{y3}-(CH₂)ₙ- or *-SO₂-(CH₂)ₙ-, wherein * shows the side to be bonded to Hal, and other symbols are as defined above.

### Step 1

Compound [I-20] obtained in the same manner as in the above-mentioned Production Method is hydrogenated in a solvent such as methanol, ethanol, THF, ethyl acetate, acetic acid, water and the like in the presence of a catalyst such as palladium carbon, palladium hydroxide, platinum oxide, Raney nickel and the like at room temperature or under heating to give compound [I-21]. In addition, compound [I-20] is reduced with a reducing agent such as zinc, iron, tin(II) chloride, sodium sulfite and the like, or reacted with hydrazine in the presence of iron(III) chloride to give compound [I-21]. Compound [I-21] can be also obtained by reacting compound [I-20] with sodium hydrosulfite under alkaline conditions.

### Step 2

Compound [I-21] is alkylated with compound [33] in the same manner as in Step 2 of Production Method 6-1 to give compound [I-22].

### Step 3

When J³ of compound [34] is *-CO-(CH₂)ₘ-Y²-(CH₂)ₙ-, *-CO₂-(CH₂)ₘ-Y²-(CH₂)ₙ-, *-CONR^{y3}-(CH₂)ₘ-Y²-(CH₂)ₙ-, *-CO-(CH₂)ₙ-, *-CO₂-(CH₂)ₙ- or *-CONR^{y3}-(CH₂)ₙ-, compound [I-21] is reacted with compound [34] in a solvent such as DMF, acetonitrile, THF, chloroform, ethyl acetate, methylene chloride, toluene and the like in the presence of a base such as triethylamine, potassium carbonate, pyridine and the like, or in an amine solvent such as pyridine to give compound [I-23].

When J³ of compound [34] is *-SO₂-(CH₂)ₘ-Y²-(CH₂)ₙ- or *-SO₂-(CH₂)ₙ-, compound [I-21] is sulfonylated with compound [34] in the same manner as above to give compound [I-23].

Compound [I-21] is acylated with compound [35] in the same manner as above to give compound [I-24].

This Production Method is applied in the same manner as above to give disubstituted compounds (tertiary amine) of compound [I-22], compound [I-23] and compound [I-24].

### Production Method 6-3

### Conversion of carboxylic acid moiety to amide

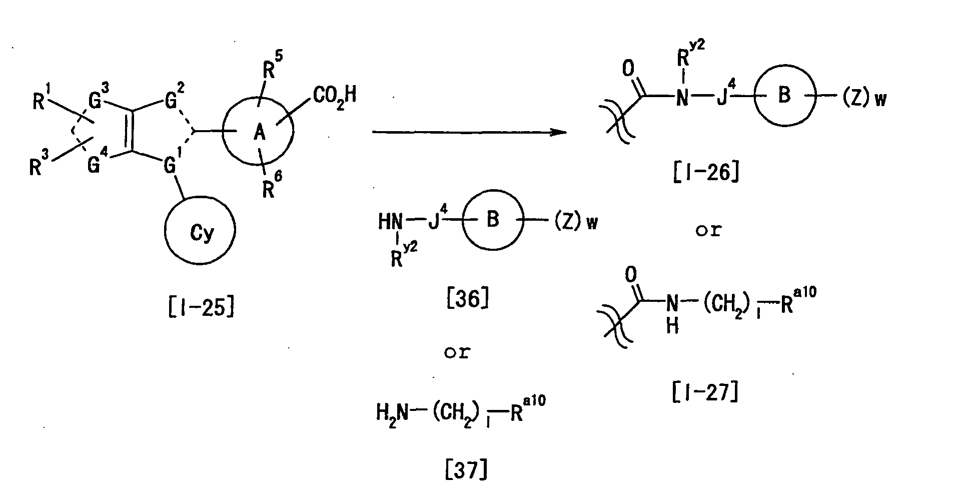

wherein J⁴ is -(CH₂)ₙ- or #- (CH₂)ₘ-Y²-(CH₂)ₙ- wherein # shows the side to be bonded to amine, and other symbols are as defined above.

The carboxylic acid compound [I-25] obtained in the same manner as in the above-mentioned Production Method is condensed with amine compound [36] in a solvent such as DMF, acetonitrile, THF, chloroform, ethyl acetate, methylene chloride, toluene and the like using a condensing agent such as dicyclohexylcarbodiimide, 1-ethyl-3-(3-dimethylaminopropyl)carbodiimide hydrochloride, diphenylphosphoryl azide and the like and, where necessary, adding N-hydroxysuccinimide, 1-hydroxybenzotriazole and the like to give amide compound [I-26]. Alternatively, amide compound [I-26] can be obtained as follows. The carboxylic acid compound [I-25] is converted to an acid halide with thionyl chloride, oxalyl chloride and the like, or to an active ester of carboxylic acid compound [I-25] (e.g., converting to a mixed acid anhydride with ethyl chlorocarbonate and the like), which is then reacted with amine compound [36] in the presence of a base such as triethylamine, potassium carbonate, pyridine, 4-(dimethylamino)pyridine and the like, or in an amine solvent such as pyridine, or in the presence of acetic acid and sodium acetate in an equivalent amount ratio, to give amide compound [I-26].

Compound [I-27] can be obtained by reacting carboxylic acid compound [I-25] with amine compound [37] in the same manner as above.

### Production Method 7

In this Production Method, additional substituent(s) is(are) introduced into ring B.

### Production Method 7-1

### Direct bonding of ring Z" to ring B

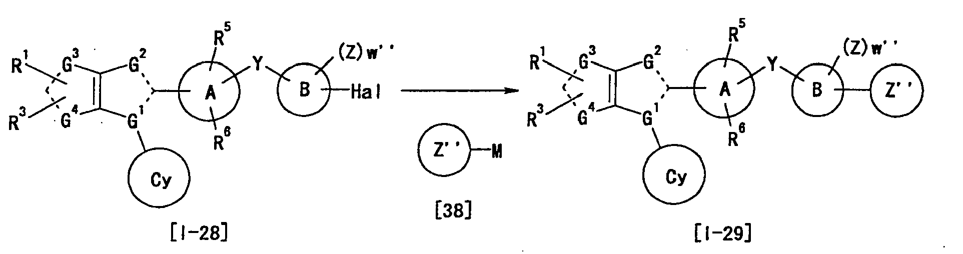

wherein ring Z"-M is aryl metal compound, ring Z" moiety is optionally substituted C₆₋₁₄ aryl or optionally substituted heterocyclic group corresponding to substituent Z, and the metal moiety contains boron, zinc, tin, magnesium and the like, such as phenylboronic acid, 4-chlorophenylboronic acid, w" is 0, 1 or 2, and other symbols are as defined above.

Compound [I-28] obtained in the same manner as in the above-mentioned Production Method is reacted with aryl metal compound [38] in a solvent such as DMF, acetonitrile, 1,2-dimethoxyethane, THF, toluene, water and the like in the presence of a palladium catalyst such as tetrakis(triphenylphosphine)palladium, bis(triphenylphosphine)palladium(II) dichloride, palladium acetate - triphenylphosphine and the like, a nickel catalyst such as nickel chloride, [1,3-bis(diphenylphosphino)propane]nickel(II) chloride and the like, and a base such as potassium carbonate, potassium hydrogen carbonate, sodium hydrogen carbonate, potassium phosphate, triethylamine, potassium fluoride, sodium hydrogen phosphate, cesium carbonate and the like at room temperature or under heating, to give compound [I-29].

### Production Method 7-2

### Conversion of hydroxyl group to ether

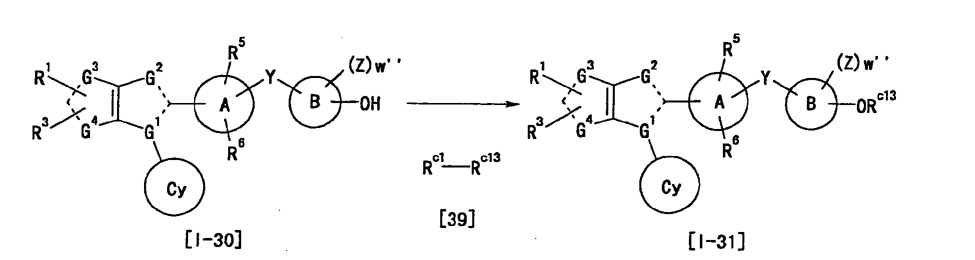

wherein R^{c13} is -R^{d1} or -(CH₂)ₚ-COR^{d25} corresponding to substituent Z, and other symbols are as defined above.

### Step 1

Compound [I-30] obtained in the same manner as in the above-mentioned Production Method is reacted with compound [39] in the same manner as in Step 2 of Production Method 6-1 to give compound [I-31].

### Production Method 7-3

### Synthesis in advance of ring B part such as compound [31] in Production Method 6-1

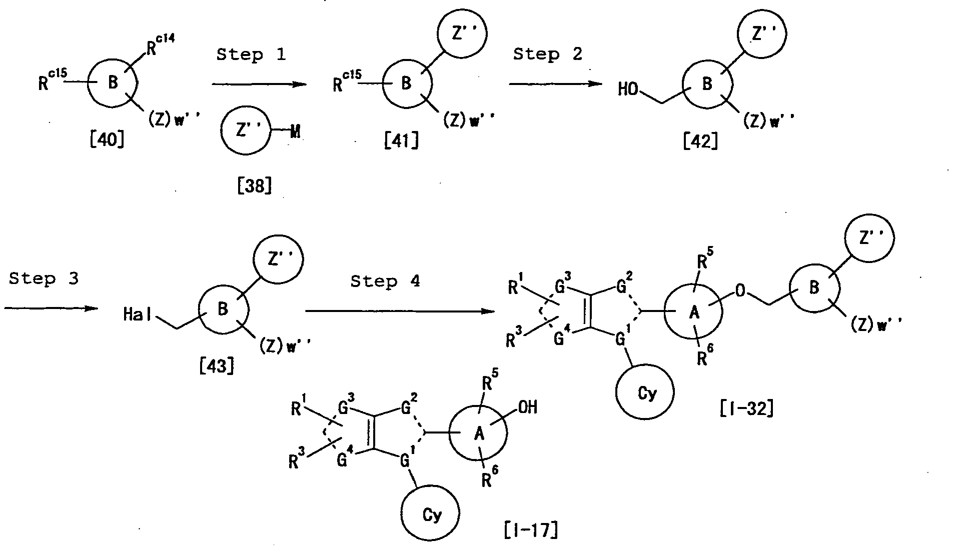

wherein R^{c14} is leaving group such as chlorine atom, bromine atom, iodine atom, trifluoromethanesulfonyloxy group, p-toluenesulfonyloxy group, methanesulfonyloxy group and the like, R^{c15} is formyl group, carboxyl group or carboxylic acid ester such as methoxycarbonyl group, ethoxycarbonyl group, tert-butoxycarbonyl group and the like, and other symbols are as defined above.

### Step 1

Commercially available compound [40] or compound [40] obtained by a conventional method is reacted with aryl metal compound [38] in the same manner as in Production Method 7-1 to give compound [41].

### Step 2

Compound [41] obtained in the same manner as in the above-mentioned Production Method is reduced according to a conventional method to give compound [42].

For example, compound [41] is reacted with in a solvent such as methanol, ethanol, THF and the like in the presence of a reducing agent such as lithium aluminum hydride, sodium borohydride and the like under cooling to heating to give compound [42].

### Step 3

Compound [42] obtained in the same manner as in the above-mentioned Production Method is reacted in a solvent such as 1,4-dioxane, diethyl ether, THF, methylene chloride, chloroform, toluene and the like with a halogenating agent, such as phosphorus halide (e.g., phosphorus pentachloride, phosphorus tribromide and the like), thionyl chloride and the like, to give compound [43]. For an accerelated reaction, the reaction may be carried out in the presence of a tertiary amine such as triethylamine, DMF, pyridine and the like, or under heating.

### Step 4

Compound [42] or [43] obtained in the same manner as in the above-mentioned Production Method is reacted with compound [I-17] in the same manner as in Step 2 of Production Method 6-1 to give compound [I-32].

### Production Method 7-4

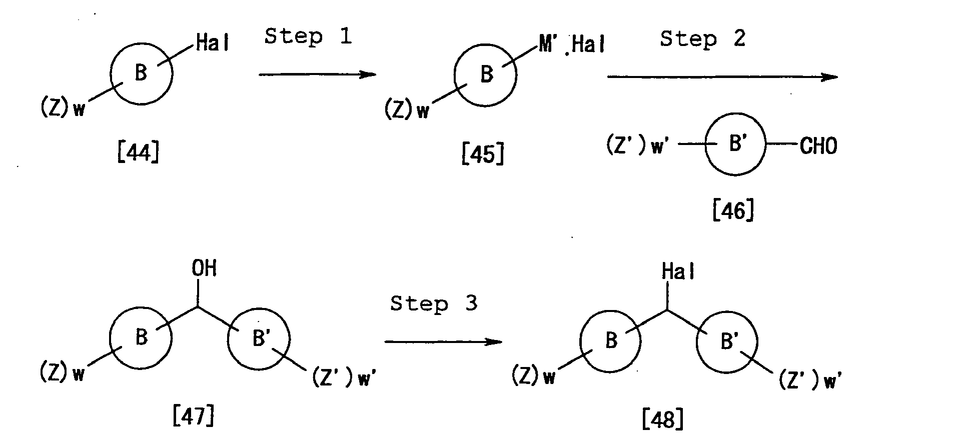

wherein M' is a metal such as magnesium, lithium, zinc and the like, and other symbols are as defined above.

### Step 1

Commercially available compound [44] or compound [44] obtained by a conventional method is converted to aryl metal reagent by a conventional method to give compound [45].

For example, when M' is magnesium, magnesium is reacted with compound [44] in a solvent such as THF, diethyl ether, benzene, toluene and the like, preferably THF, from cooling to heating preferably at -100°C to 100°C to give compound [45].

### Step 2

Compound [45] obtained in the same manner as in the above-mentioned Production Method is reacted with compound [46] to give compound [47].

Compound [45] is reacted with compound [46] in a solvent such as diethyl ether, benzene, toluene, THF and the like, preferably THF, from cooling to room temperature, preferably at -100°C to 30°C to give compound [47].

### Step 3

Compound [47] obtained in the same manner as in the above-mentioned Production Method is halogenated in the same manner as in Step 3 of Production Method 7-3 to give compound [48].

Compound [47] is reacted with thionyl chloride and pyridine preferably in toluene solvent to give compound [48].

When compound [48] is symmetric, namely, when the ring B-(Z)w moiety and the ring B'-(Z')w' moiety are the same, compound [45] is reacted with formate such as methyl formate, ethyl formate and the like, preferably ethyl formate, in a solvent such as diethyl ether, benzene, toluene, THF and the like, preferably THF, from cooling to room temperature, preferably at -100°C to 30°C, to give compound [48].

### Production Method 7-5

### Method including steps to introduce a protecting group into a functional group

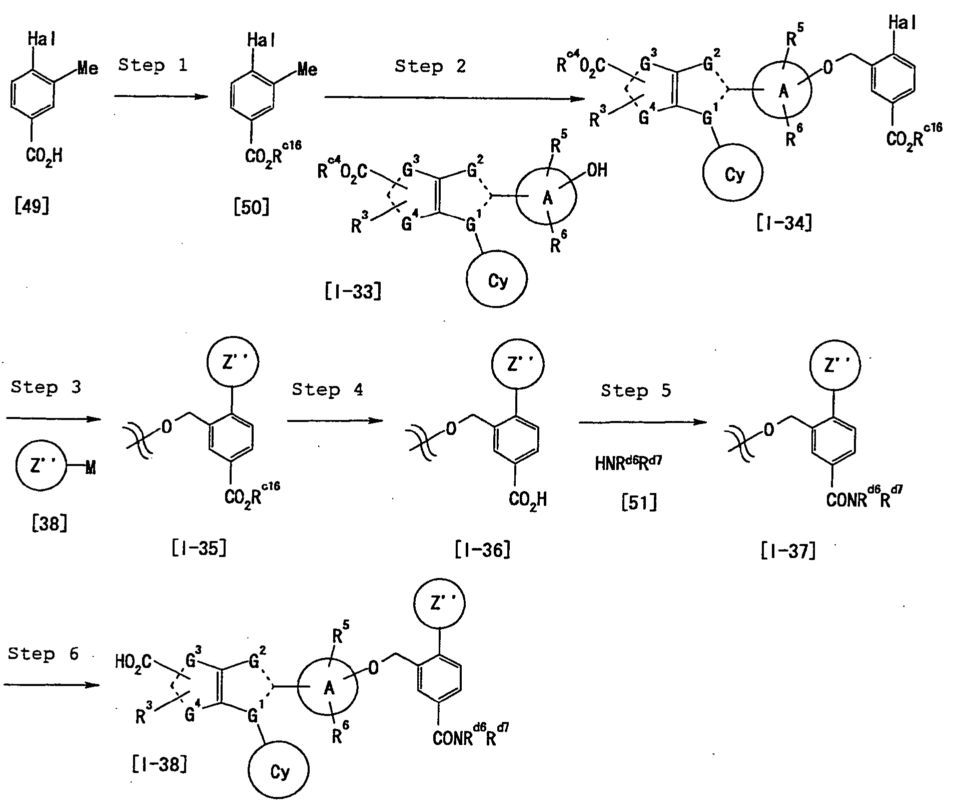

wherein R^{c4} is carboxyl-protecting group such as methyl group and the like, R^{c16} is carboxyl-protecting group such as tert-butyl group and the like, and other symbols are as defined above.

### Step 1

Commercially available compound [49] or compound [49] obtained by a conventional method is protected by a conventional method to give compound [50].

For example, when R^{c16} is tert-butyl group, compound [49] is converted to acid halide with thionyl chloride, oxalyl chloride and the like in a solvent such as THF, chloroform, methylene chloride, toluene and the like, and reacted with potassium tert-butoxide to give compound [50].

### Step 2

The methyl group of compound [50] obtained in the same manner as in the above-mentioned Production Method is converted to bromomethyl group with N-bromosuccinimide and N,N'-azobisisobutyronitrile and reacted with compound [I-33] in the same manner as in Step 2 of Production Method 6-1 to give compound [I-34].

### Step 3

Compound [I-34] obtained in the same manner as in the above-mentioned Production Method is reacted with aryl metal compound [38] in the same manner as in Production Method 7-1 to give compound [I-35].

### Step 4

The R^{c16} of the compound [I-35] obtained in the same manner as in the above-mentioned Production Method is removed by a conventional method to give compound [I-36].

The carboxyl-protecting group can be removed by a conventional deprotection method according to the protecting group. In this Step, the conditions free from reaction of R^{c4} are preferable. For example, when R^{c16} is tert-butyl group, compound [I-35] is treated with trifluoroacetic acid in a solvent such as methylene chloride, chloroform and the like to give compound [I-36]. In addition, compound [I-36] can also be obtained by treating compound [I-35] with hydrogen chloride or hydrochloric acid in a solvent such as ethyl acetate, 1,4-dioxane, alcohol and the like.

### Step 5

Compound [I-36] obtained in the same manner as in the above-mentioned Production Method is subjected to amide condensation with compound [51] in the same manner as in Production Method 6-3 to give compound [I-37].

### Step 6

Compound [I-37] obtained in the same manner as in the above-mentioned Production Method is deprotected in the same manner as in Step 1 of Production Method 5-1 to give compound [I-38].

As used herein, R^{c4} is preferably a protecting group that does not react during the Step 1 through Step 5 but removed in this Step.

For example, when R^{c4} is methyl group, compound [I-37] is reacted in an alcohol solvent such as methanol, ethanol, n-propanol, isopropanol and the like or a mixed solvent of alcohol solvent and water in the presence of a base such as potassium carbonate, sodium carbonate, lithium hydroxide, sodium hydroxide, potassium hydroxide and the like from cooling to heating for deprotection, followed by acidifying the reaction solution to give compound [I-38].

### Production Method 7-6

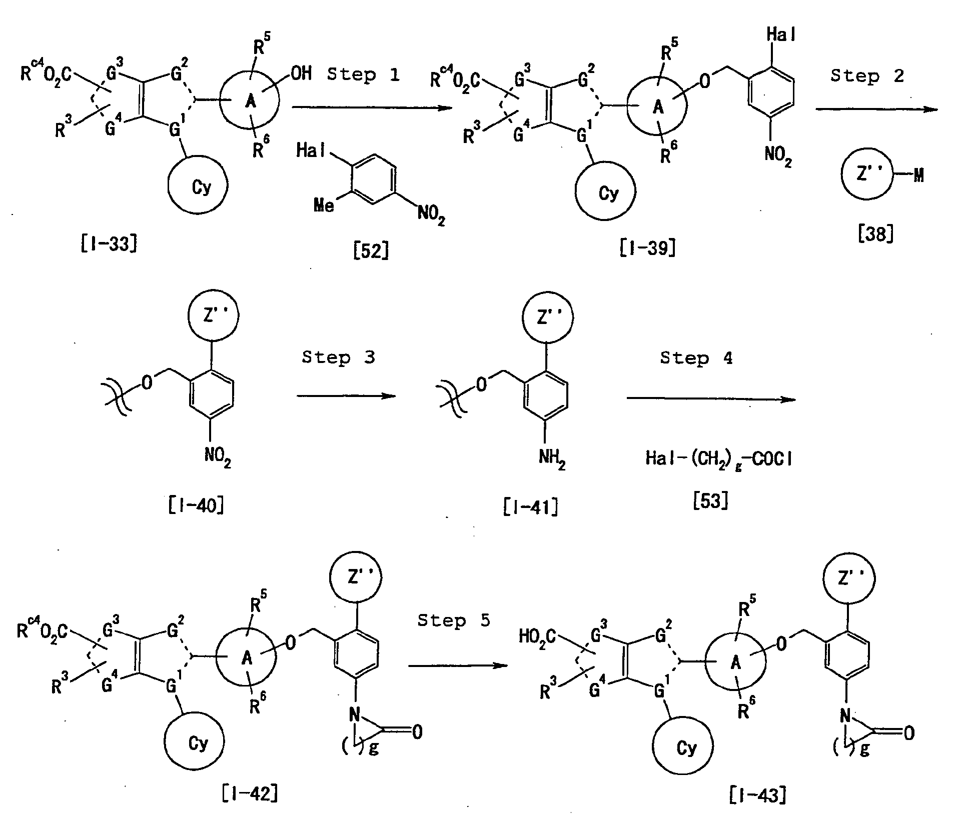

wherein g is an integer of 1 to 5, and other sumbols are as defined above.

### Step 1

Compound [I-33] obtained in the same manner as in the above-mentioned Production Method is reacted with toluene derivative [52] in the same manner as in Step 2 of Production Method 7-5 to give compound [I-39].

### Step 2

Compound [I-39] obtained in the same manner as in the above-mentioned Production Method is reacted with aryl metal compound [38] in the same manner as in Production Method 7-1 to give compound [I-40].

### Step 3

Compound [I-40] obtained in the same manner as in the above-mentioned Production Method is reduced in the same manner as in Step 1 of Production Method 6-2 to give compound [I-41].

### Step 4

Compound [I-41] obtained in the same manner as in the above-mentioned Production Method is amide condensed with compound [53] in the same manner as in Production Method 6-3, which is then subjected to cyclization in a solvent such as DMF, acetonitrile, THF, toluene and the like in the presence or absence of a base such as potassium carbonate, triethylamine, potassium tert-butoxide and the like at room temperature or under heating, to give compound [I-42].

### Step 5

Compound [I-42] obtained in the same manner as in the above-mentioned Production Method is deprotected in the same manner as in Step 1 of Production Method 5-1 to give compound [I-43].

### Production Method 7-7

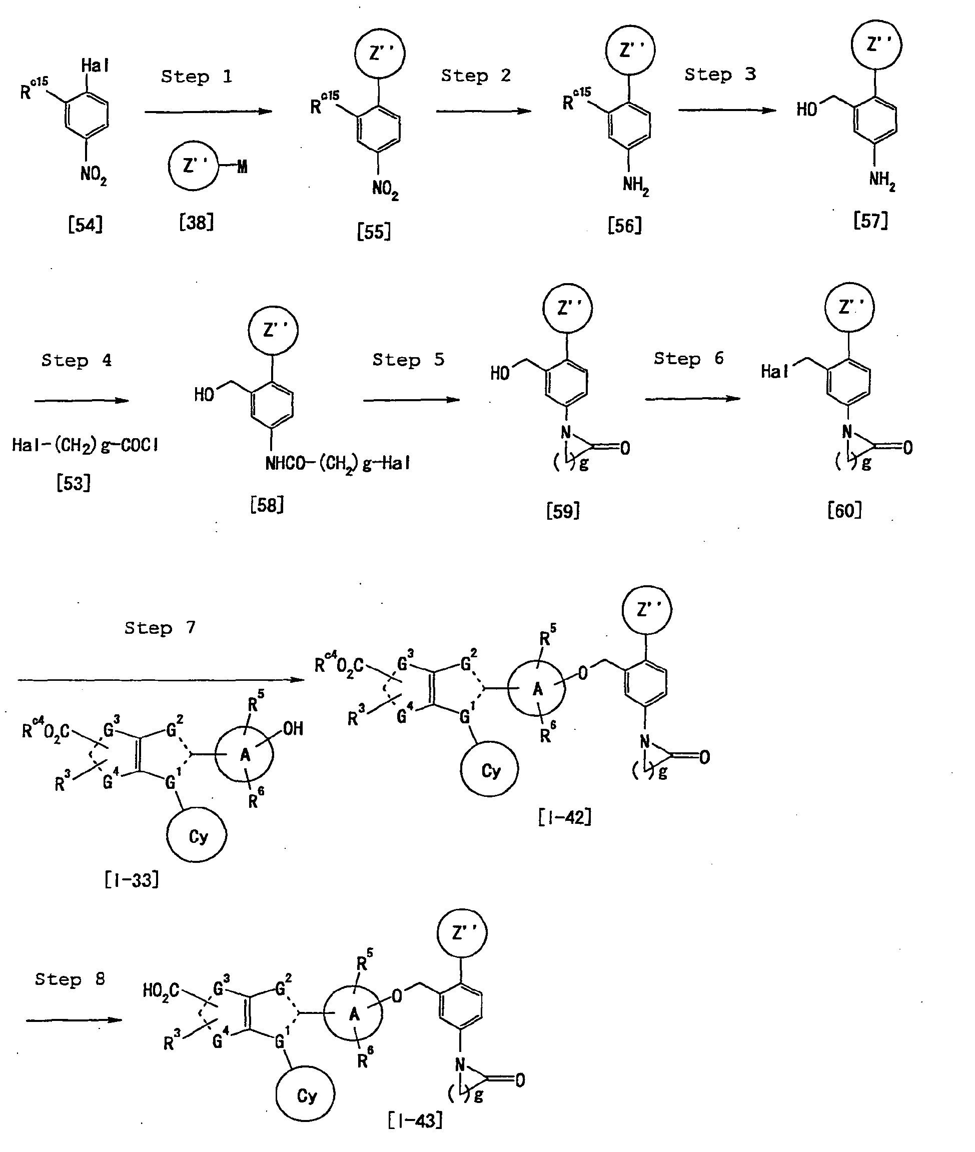

wherein each symbol is as defined above.

### Step 1

Commercially available compound [54] or compound [54] obtained by a conventional method is reacted with compound [38] in the same manner as in Production Method 7-1 to give compound [55].

### Step 2

Compound [55] obtained in the same manner as in the above-mentioned Production Method is reduced in the same manner as in Step 1 of Production Method 6-2 to give compound [56].

### Step 3

Compound [56] obtained in the same manner as in the above-mentioned Production Method is reduced in the same manner as in Step 2 of Production Method 7-3 to give compound [57].

### Step 4

Compound [57] obtained in the same manner as in the above-mentioned Production Method is reacted with compound [53] in a solvent such as DMF, acetonitrile, THF, chloroform, ethyl acetate, methylene chloride, toluene and the like to give compound [58]. To enhance the reaction selectivity for amino group, acetic acid and sodium acetate may be added in an equivalent amount ratio.

### Step 5

Compound [58] obtained in the same manner as in the above-mentioned Production Method is subjected to cyclization in a solvent such as ethanol, DMF, acetonitrile, THF, toluene, water and the like in the presence or absence of a base such as potassium hydroxide, potassium carbonate, triethylamine, potassium tert-butoxide and the like at room temperature or under heating, to give compound [59].

### Step 6

Compound [59] obtained in the same manner as in the above-mentioned Production Method is halogenated in the same manner as in Step 3 of Production Method 7-3 to give compound [60].

### Step 7

Compound [60] obtained in the same manner as in the above-mentioned Production Method is reacted in the same manner as in Step 2 of Production Method 6-1 with compound [I-33] obtained in the same manner as in the above-mentioned Production Method to give compound [I-42].

### Step 8

Compound [I-42] obtained in the same manner as in the above-mentioned Production Method is deprotected in the same manner as in Step 1 of Production Method 5-1 to give compound [I-43].

### Production Method 7-8

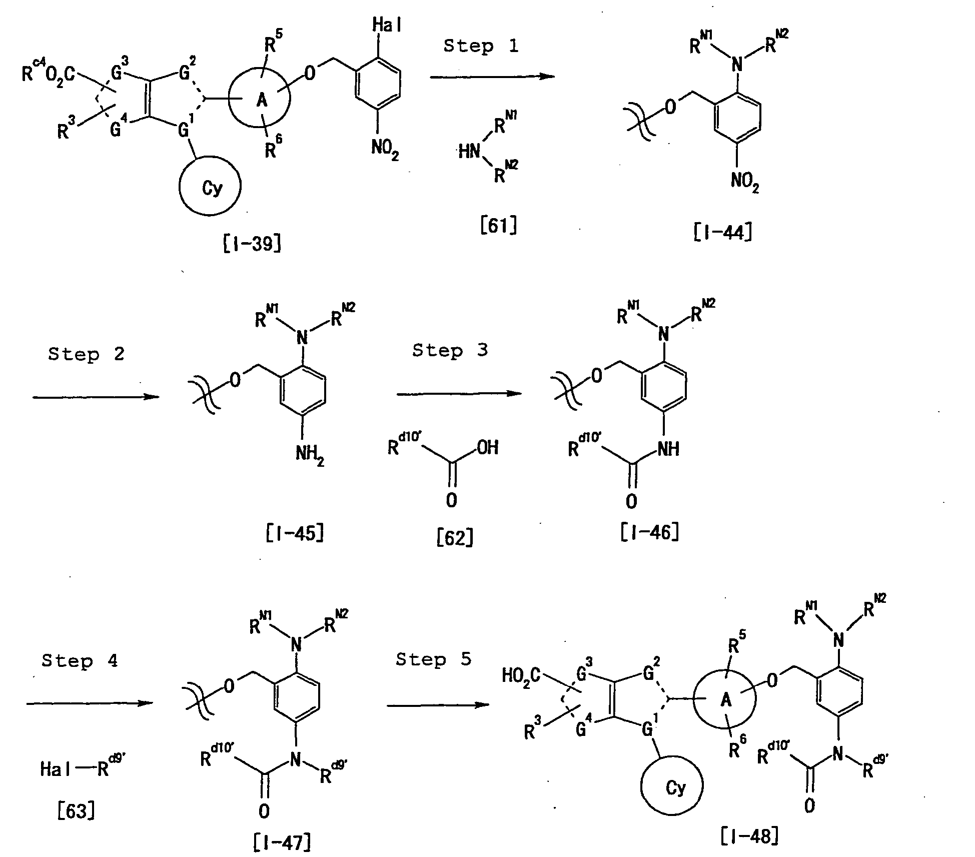

wherein R^{N1} and R^{N2} are the same or different and each is hydrogen atom or a group selected from group F, or R^{N1} and R^{N2} are linked to form a heterocycle containing NH such as piperidino group, 1-piperazinyl group, morpholino group and the like, R^{d10}, is a group selected from group F, R^{d9}, is a C₁₋₆ alkyl group optionally substituted by 1 to 3 substituents selected from group A, and other symbols are as defined above.

### Step 1

Compound [I-39] obtained in the same manner as in the above-mentioned Production Method is reacted with amine compound [61] in the same manner as in Step 2 of Production Method 1-1 to give compound [I-44].

### Step 2

Compound [I-44] is reduced in the same manner as in Step 3 of Production Method 1-1 to give compound [I-45].

### Step 3

Compound [I-45] is reacted with carboxylic acid compound [62] in the same manner as in Production Method 6-3 to give compound [I-46].

### Step 4

Compound [I-46] is alkylated with compound [63] in the same manner as in Step 5 of Production Method 3-1 to give compound [I-47].

### Step 5

Compound [I-47] is deprotected in the same manner as in Step 9 of Production Method 2 to give compound [I-48].

### Production Method 7-9

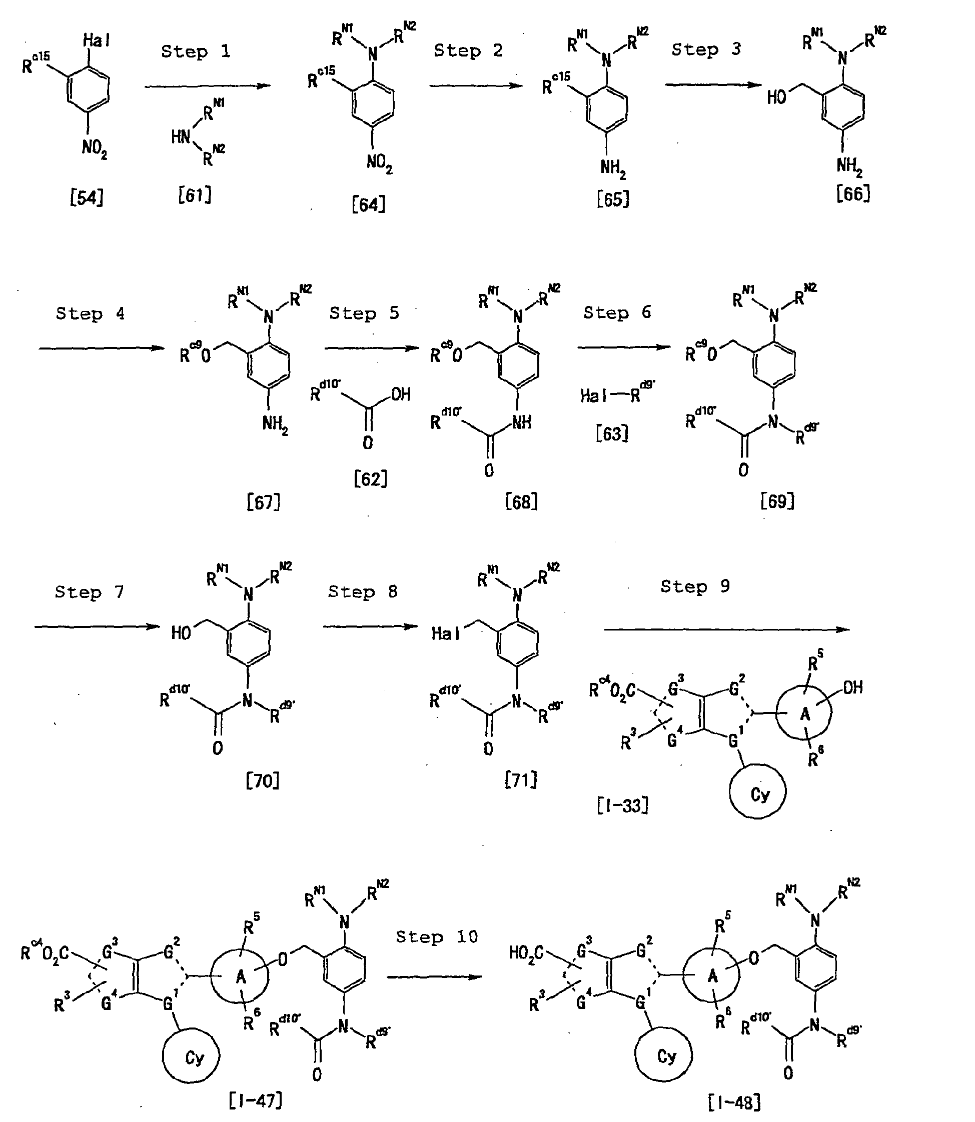

wherein each symbol is as defined above.

### Step 1

Commercially available compound [54] or compound [54] obtained by a conventional method is reacted with amine compound [61] in the same manner as in Step 2 of Production Method 1-1 to give compound [64].

### Step 2

Compound [64] is reduced in the same manner as in Step 3 of Production Method 1-1 to give compound [65].

### Step 3

Compound [65] is reduced in the same manner as in Step 2 of Production Method 7-3 to give compound [66].

### Step 4

The hydroxyl group of the compound [66] is protected by a conventional method to give compound [67].

For protection, for example, when R^{c9} is acetyl group, the compound [66] is reacted with acetic anhydride in the presence of pyridine or tertiary amine at room temperature to heating, when R^{c9} is benzyl group, the compound [66] is heated under reflux with benzyl chloride in benzene in the presence of a base such as potassium hydroxide and the like, when R^{c9} is tert-butyldiphenylsilyl group, the compound [66] is treated with tert-butyldiphenylsilyl chloride and imidazole at room temperature in DMF, and the like.

In addition, desired R^{d10}'-CO group may be introduced as a hydroxyl-protecting group in the next Step 5 without going through this step.

### Step 5

Compound [67] is reacted with carboxylic acid compound [62] in the same manner as in Production Method 6-3 to give compound [68].

### Step 6

Compound [68] is alkylated with compound [63] in the same manner as in Step 5 of Production Method 3-1 to give compound [69].

### Step 7

Compound [69] is deprotected in the same manner as in Step 1 of Production Method 6-1 to give compound [70].

### Step 8

Compound [70] is halogenated in the same manner as in Step 3 of Production Method 7-3 to give compound [71].

### Step 9

Compound [71] is reacted in the same manner as in Step 2 of Production Method 6-1 with compound [I-33] obtained in the same manner as in the above-mentioned Production Method to give compound [I-47].

### Step 10

Compound [I-47] is deprotected in the same manner as in Step 9 of Production Method 2 to give compound [I-48].

In the compounds of the formula [I], a desired heterocyclic group (including carboxylic acid equivalent) can be formed according to a method similar to the methods disclosed in known publications. Examples of such heterocyclic group and reference publications are recited in the following.

5-oxo-Δ²-1,2,4-oxadiazolin-3-yl group (or 2,5-dihydro-5-oxo-4H-1,2,4-oxadiazol-3-yl group), 5-oxo-Δ²-1,2,4-thiadiazolin-3-yl group (or 2,5-dihydro-5-oxo-4H-1,2,4-thiadiazol-3-yl group), 2-oxo-Δ³-1,2,3,5-oxathiadiazolin-4-yl group (or 2-oxo-Δ³-1,2,3,5-oxathiadiazol-4-yl group): Journal of Medicinal Chemistry, 39(26), 5228-35, 1996, based on [I-13], for example, 5-oxo-Δ²⁻1,2,4-oxadiazolin-3-yl group, 5-thioxo-Δ²-1,2,4-oxadiazolin-3-yl group can be formed.
5-oxo-Δ²-1,2,4-triazolin-3-yl group: J Org Chem, 61(24), 8397-8401, 1996,
1-oxo-Δ³-1,2,3,5-thiatriazolin-4-yl group: Liebigs Ann Chem, 1376, 1980,
3-oxo-Δ⁴-1,2,4-oxadiazolin-5-yl group: EP145095,
5-oxo-Δ²-1,3,4-oxadiazolin-2-yl group: J Org Chem, 20, 412, 1955,
5-oxo-Δ³-1,2,4-dioxazolin-3-yl group: J Prakt Chem, 314, 145, 1972,
3-oxo-Δ⁴-1,2,4-thiadiazolin-5-yl group: JP-A-61-275271,
5-oxo-Δ³-1,2,4-dithiazolin-3-yl group: J Org Chem, 61(19), 6639-6645, 1996,
2-oxo-Δ⁴-1,3,4-dioxazolin-5-yl group: J Org Chem, 39, 2472, 1974,
2-oxo-Δ⁴-1,3,4-oxathiazolin-5-yl group: J Med Chem, 35(20), 3691-98, 1992,
5-oxo-Δ²-1,3,4-thiadiazolin-2-yl group: J Prakt Chem, 332(1), 55, 1990,
5-oxo-Δ²-1,4,2-oxathiazolin-3-yl group: J Org Chem, 31, 2417, 1966,
2-oxo-Δ⁴-1,3,4-dithiazolin-5-yl group: Tetrahedron Lett, 23, 5453, 1982,
2-oxo-Δ⁴-1,3,2,4-dioxathiazolin-5-yl group: Tetrahedron Lett, 319, 1968,
3,5-dioxoisoxazolidin-4-yl group: Helv Chim Acta, 1973, 48, 1965,
2,5-dioxoimidazolidin-4-yl group: Heterocycles, 43(1), 49-52, 1996,
5-oxo-2-thioxoimidazolidin-4-yl group: Heterocycles, 5, 391, 1983,
2,4-dioxooxazolidin-5-yl group: J Am Chem Soc, 73, 4752, 1951, 4-oxo-2-thioxooxazolidin-5-yl group: Chem Ber, 91, 300, 1958, 2,4-dioxothiazolidin-5-yl group: JP-A-57-123175,
4-oxo-2-thioxothiazolidin-5-yl group: Chem Pharm Bull, 30, 3563, 1982.

### Examples

The fused ring compounds of the formula [I] of the present invention and production methods thereof are explained in detail in the following by way of Examples. It is needless to say that the present invention is not limited by these Examples. In the Examples, Me means methyl group, Et means ethyl group, and Ac means acetyl group.

### Preparation Example 1

### Step 1: Production of methyl 2-chloro-5-nitrobenzoate

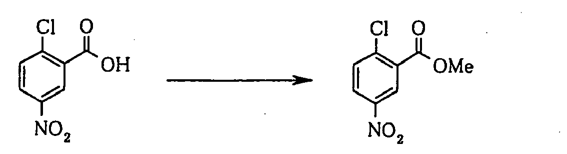

To a solution of 2-chloro-5-nitrobenzoic acid (30 g, 148.8 mmol) in methanol (150 ml) was added conc. sulfuric acid (2 ml), and the mixture was heated under reflux for 24 hr. After allowing to cool to room temperature, water was added, and the mixture was extracted with ethyl acetate. The organic layer was washed successively with water, saturated aqueous sodium hydrogen carbonate solution and saturated brine, and dried over anhydrous sodium sulfate. After filtration, the solvent was evaporated under reduced pressure to give methyl 2-chloro-5-nitrobenzoate (31.3 g, yield 97.9%) as a crude product. The obtained crude product was used for Step 2 without further purification.

### Step 2: Production of methyl 2-morpholino-5-nitrobenzoate

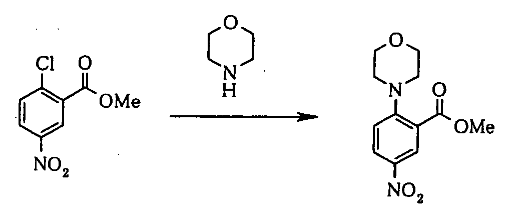

To a suspension of methyl 2-chloro-5-nitrobenzoate (13.7 g, 63.7 mmol) obtained as a crude product in Step 1 and potassium carbonate (11 g, 79.5 mmol) in dimethyl sulfoxide (50 ml) was added morpholine (6.2 ml, 71.1 mmol), and the mixture was stirred at 60°C for 4 hr. After allowing to cool to room temperature, water was added and the mixture was stirred under ice-cooling for 30 min. The precipitated solid was collected by filtration, washed with water and vacuum dried to give methyl 2-morpholino-5-nitrobenzoate (16.6 g, yield 97.8%). The obtained crude product was used for Step 3 without further purification. Step 3: Production of methyl 5-amino-2-morpholinobenzoate

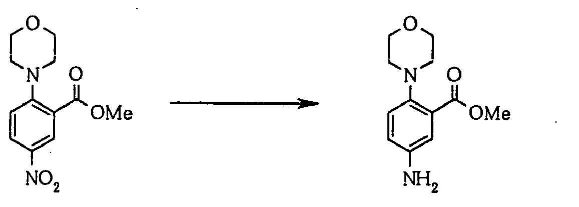

A suspension of methyl 2-morpholino-5-nitrobenzoate (16.6 g, 62.4 mmol) obtained as a crude product in Step 2, reduced iron (17.4 g, 0.3 mol) and ammonium chloride (16.6 g, 0.3 mol) in tetrahydrofuran (160 ml), ethanol (320 ml) and water (80 ml) was heated under reflux at 100°C for 2 hr. After allowing to cool to room temperature, the mixture was filtered through celite and washed with ethanol. The solvent of the filtrate was evaporated under reduced pressure. A saturated aqueous sodium hydrogen carbonate solution was added and the mixture was extracted with ethyl acetate. The organic layer was washed with saturated brine, and dried over anhydrous sodium sulfate. After filtration, the solvent was evaporated under reduced pressure. The residue was purified by silica gel column chromatography (n-hexane:ethyl acetate=2:3) to give methyl 5-amino-2-morpholinobenzoate (11.0 g, yield 74.9%).

### Step 4: Production of (5-amino-2-morpholinophenyl)methanol

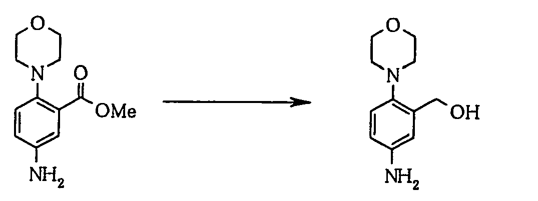

To a solution of methyl 5-amino-2-morpholinobenzoate (11.0 g, 46.6 mmol) in tetrahydrofuran (110 ml) was added lithium aluminum hydride (2.1 g, 55.3 mmol) with stirring under ice-cooling, and the mixture was stirred for 1 hr. Water (2.2 ml), 4N aqueous sodium hydroxide solution (2.2 ml) and water (6.2 ml) were added to the reaction mixture with stirring under ice-cooling. The mixture was filtered through celite, and the solvent of the filtrate was evaporated under reduced pressure. n-Hexane was added to the residue, and the precipitated solid was collected by filtration, washed with n-hexane and vacuum dried to give (5-amino-2-morpholinophenyl)methanol (8.3 g, yield 86.3%).
¹H-NMR(400MHz, δppm, DMSO-d₆): 6.80(1H, d, J=8.4Hz), 6.67(1H, d, J=2.5Hz), 6.40(1H, dd, J=8.4, 2.5Hz), 4.90(1H, t, J=5.5Hz), 4.77(2H, brs), 4.47(2H, d, J=5.5Hz), 3.70-3.60(4H, m), 2.70-2.60(4H, m).

### Step 5: Production of 5-acetylamino-2-morpholinobenzyl acetate

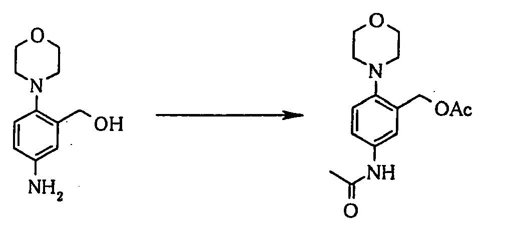

To a solution of (5-amino-2-morpholinophenyl)methanol (880 mg, 4.23 mmol) in tetrahydrofuran (10 ml) were added triethylamine (1.3 ml, 9.3 mmol) and 4-(dimethylamino)pyridine (5 mg). To the mixture was added acetic anhydride (0.84 ml, 8.9 mmol) under ice-cooling and the mixture was stirred for 2 hr. Water was added to the reaction mixture, and the mixture was extracted with ethyl acetate. The organic layer was washed successively with water, saturated aqueous sodium hydrogen carbonate solution and saturated brine, and dried over anhydrous sodium sulfate. After filtration, the solvent was evaporated under reduced pressure, and n-hexane was added to the residue. The precipitated solid was collected by filtration, washed with n-hexane and vacuum dried to give 5-acetylamino-2-morpholinobenzyl acetate (1.05 g, yield 85.5%).

### Step 6: Production of 5-(N-acetyl-N-methylamino)-2-morpholinobenzyl acetate

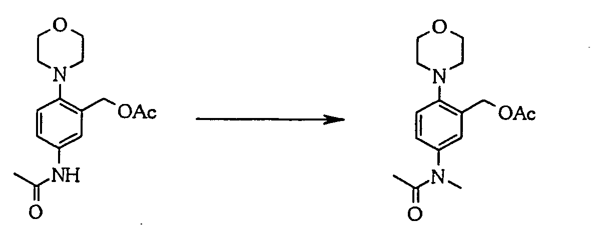

To a solution of 5-acetylamino-2-morpholinobenzyl acetate (1.05 g, 3.59 mmol) in N,N-dimethylformamide (10 ml) was added sodium hydride (60% in oil, 187 mg, 4.67 mmol) with stirring under ice-cooling, and the mixture was stirred for 1 hr. To the reaction mixture was added methyl iodide (0.33 ml, 5.3 mmol) under ice-cooling, and the mixture was stirred at room temperature for 1 hr. Water was added to the reaction mixture, and the mixture was extracted with ethyl acetate. The organic layer was washed successively with water and saturated brine, and dried over anhydrous sodium sulfate. After filtration, the solvent was evaporated under reduced pressure to give 5-(N-acetyl-N-methylamino)-2-morpholinobenzyl acetate as a crude product. The obtained crude product was used for Step 7 without further purification.

### Step 7: Production of N-(3-hydroxymethyl-4-morpholinophenyl)-N-methylacetamide

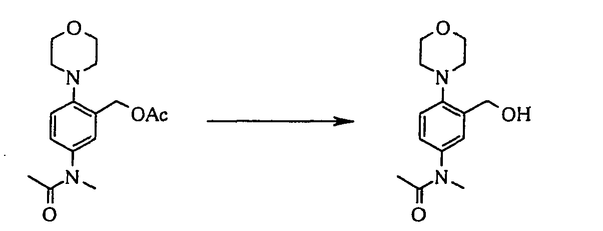

To a solution of 5-(N-acetyl-N-methylamino)-2-morpholinobenzyl acetate obtained as a crude product in Step 6 in methanol (10 ml) was added potassium carbonate (500 mg, 3.6 mmol), and the mixture was stirred at room temperature for 2 hr. The solvent of the reaction mixture was evaporated under reduced pressure, water was added to the residue, and the mixture was extracted with ethyl acetate. The organic layer was washed successively with water and saturated brine, and dried over anhydrous sodium sulfate. After filtration, the solvent was evaporated under reduced pressure. The residue was purified by silica gel column chromatography (chloroform:methanol=20:1) to give N-(3-hydroxymethyl-4-morpholinophenyl)-N-methylacetamide (650 mg, yield 68.6%).
¹H-NMR(300MHz, δppm, CDCl₃): 7.26-7.19(1H, m) , 7.12-7.05(2H, m), 4.80(2H, brs), 4.43(1H, brs), 3.90-3.85(3H, brs), 3.24(3H, brs), 3.05-2.85 (4H, m), 1.88(3H, brs).

### Example 1

### Production of methyl 3-cyclohexyl-2-(4-hydroxyphenyl)-3H-thieno[2,3-d]imidazole-5-carboxylate

### Step 1: Production of methyl 5-chlorothiophene-2-carboxylate

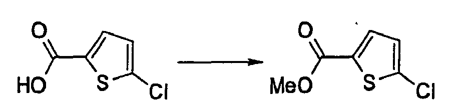

To a solution of 5-chlorothiophene-2-carboxylic acid (5.07 g, 31.2 mmol) in methanol (30 ml) was added conc. sulfuric acid (2 ml), and the mixture was heated under reflux for 6 hr. After allowing to cool to room temperature, water was added to the reaction mixture, and the mixture was extracted with ethyl acetate. The organic layer was washed successively with water, saturated aqueous sodium hydrogen carbonate solution, and saturated brine, and dried over anhydrous magnesium sulfate. After filtration, the solvent was evaporated under reduced pressure to give methyl 5-chlorothiophene-2-carboxylate (4.99 g, yield 91%) as a crude product.
¹H-NMR(400MHz, δppm, DMSO-d₆): 7.58(1H, d, J=8.0Hz), 6.93(1H, d, J=4.0Hz), 3.87(3H, s).

### Step 2: Production of methyl 5-chloro-4-nitrothiophene-2-carboxylate

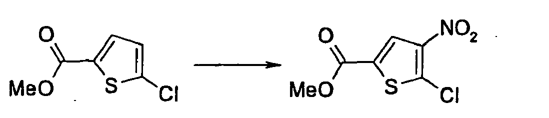

Fuming nitric acid (15 ml) was cooled with ice and saturated brine, methyl 5-chlorothiophene-2-carboxylate (4.99 g, 28.3 mmol) was added by small portions, and the mixture was stirred for 1 hr. Ice water was added to the reaction mixture and the precipitated solid was collected by filtration, washed with water and vacuum dried to give methyl 5-chloro-4-nitrothiophene-2-carboxylate (3.88 g, yield 58%).
¹H-NMR(400MHz, δppm, DMSO-d₆): 8.17(1H, s).

### Step 3: Production of methyl 5-cyclohexylamino-4-nitrothiophene-2-carboxylate

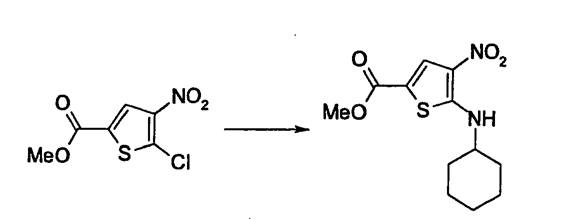

To a solution of methyl 5-chloro-4-nitrothiophene-2-carboxylate (2.35 g, 9.89 mmol) in dimethyl sulfoxide (12 ml) was added cyclohexylamine (2.16 g, 21.8 mmol) and the mixture was stirred at room temperature for 1 hr. Water was added to the reaction mixture and the precipitated solid was collected by filtration, washed with water and vacuum dried to give methyl 5-cyclohexylamino-4-nitrothiophene-2-carboxylate (2.94 g, yield 106%).
¹H-NMR(400MHz, δppm, DMSO-d₆): 8.95(1H, brs), 7.83(1H, s), 3.79(3H, s), 3.31(1H, brs), 1.91-1.98(2H, m), 1.69-1.77(2H, m), 1.53-1.65(3H, m), 1.29-1.42(2H, m), 1.09-1.22(1H, m).

### Step 4: Production of 4-acetoxybenzoyl chloride

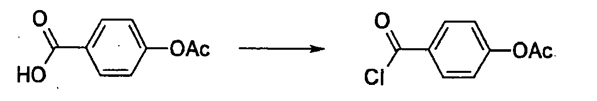

To a solution of 4-acetoxybenzoic acid (1.96 g, 10.9 mmol) in chloroform (40 ml) was added oxalyl dichloride (1.42 ml, 16.3 mmol), a catalytic amount of N,N-dimethylformamide was added dropwise, and the mixture was stirred at room temperature for 1 hr. The solvent was evaporated under reduced pressure to give 4-acetoxybenzoyl chloride as a crude product. The obtained crude product was used for Step 6 without further purification. Step 5: Production of methyl 4-amino-5-cyclohexylaminothiophene-2-carboxylate

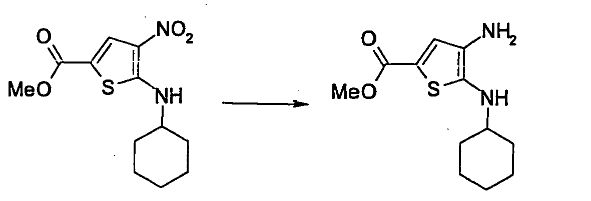

A suspension of methyl 5-cyclohexylamino-4-nitrothiophene-2-carboxylate (2.94 g, 10.3 mmol), reduced iron (2.89 g, 51.7 mmol) and ammonium chloride (2.77 g, 51.7 mmol) in tetrahydrofuran (30ml), ethanol (60 ml) and water (25 ml) was heated under an argon atmosphere at 100°C for 2 hr. After allowing to cool to room temperature, tetrahydrofuran (50 ml) and ethanol (50 ml) were added to the reaction mixture. The mixture was filtered through celite, and washed with tetrahydrofuran. The filtrate was evaporated under reduced pressure to give methyl 4-amino-5-cyclohexylaminothiophene-2-carboxylate. The obtained crude product was used for Step 6 without further purification.

### Step 6: Production of methyl 4-(4-acetoxybenzoylamino)-5-cyclohexylaminothiophene-2-carboxylate

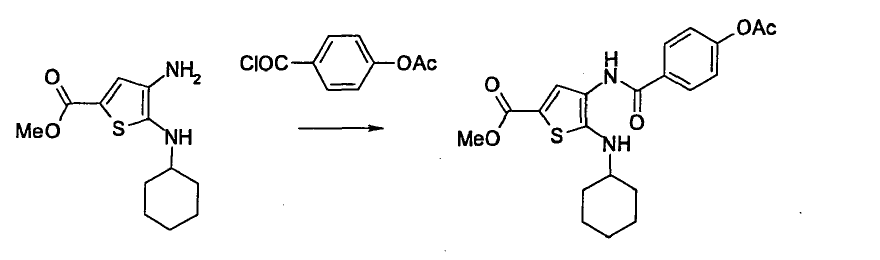

To a solution of methyl 4-amino-5-cyclohexylaminothiophene-2-carboxylate obtained in Step 5 in pyridine (10 ml) was added dropwise under ice-cooling a solution of 4-acetoxybenzoyl chloride obtained in Step 4 in chloroform (10 ml), and the mixture was stirred for 15 min. The solvent was evaporated under reduced pressure, and the residue was purified by silica gel column chromatography (n-hexane:ethyl acetate=2:1-3:2) to give methyl 4-(4-acetoxybenzoylamino)-5-cyclohexylaminothiophene-2-carboxylate (2.29 g, yield 53%).
¹H-NMR(400MHz, δppm, DMSO-d₆): 9.66(1H, s), 7.97 (2H, d, J=8.0Hz), 7.68(1H, s), 7.27(2H, d, J=8.0Hz), 6.62(2H, d, J=8.0Hz), 3.71(3H, s), 3.07(1H, brs), 2.30(3H, s), 1.97-2.04(2H, m), 1.69-1. 77 (2H, m), 1.55-1.64(1H, m), 1.11-1.39(5H, m).

### Step 7: Production of methyl 4-(4-acetoxythiobenzoylamino)-5-cyclohexylaminothiophene-2-carboxylate

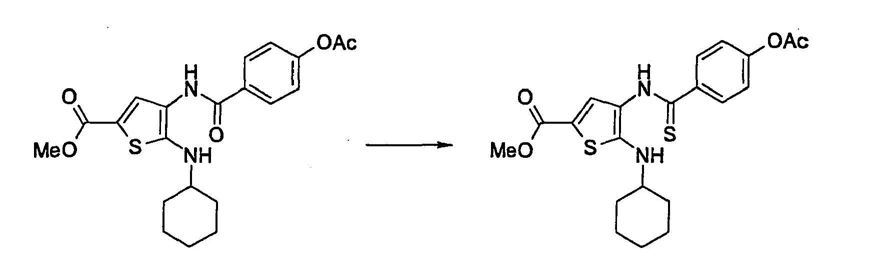

To a solution of methyl 4-(4-acetoxybenzoylamino)-5-cyclohexylaminothiophene-2-carboxylate (2.51 g, 6.03 mmol) in tetrahydrofuran (40 ml) was added Lawesson reagent (2.44 g, 6.03 mmol), and the mixture was heated under reflux for 1 hr. After allowing to cool to room temperature, a saturated aqueous sodium hydrogen carbonate solution was added to the reaction mixture and the mixture was extracted with ethyl acetate. The organic layer was washed successively with water, saturated aqueous sodium hydrogen carbonate solution, and saturated brine, and dried over anhydrous magnesium sulfate. After filtration, the solvent was evaporated under reduced pressure. The residue was purified by silica gel column chromatography (n-hexane:ethyl acetate=2:1-3:2) to give methyl 4-(4-acetoxythiobenzoylamino)-5-cyclohexylaminothiophene-2-carboxylate (2.14 g, yield 82%).
¹H-NMR(400MHz, δppm, DMSO-d₆): 11.03(1H, s), 7.94(2H, d, J=8.0Hz), 7.77(1H, s), 7.21(2H, d, J=8.0Hz), 6.69(1H, d, J=8.0Hz), 3.72 (3H, s), 3.09(1H, brs), 2. 30 (3H, s), 1.95-2.03 (2H, m), 1.67-1.77(2H, m), 1.54-1.63(1H, m), 1.08-1.38(5H, m).

### Step 8: Production of methyl 2-(4-acetoxyphenyl)-3-cyclohexyl-3H-thieno[2,3-d]imidazole-5-carboxylate

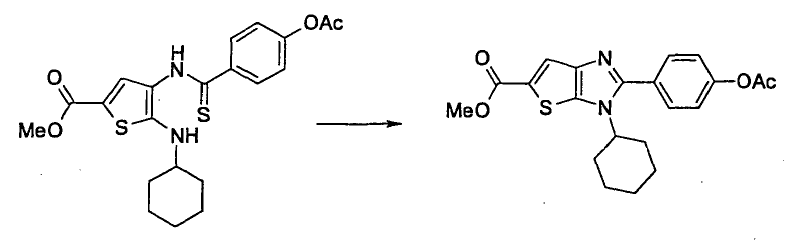

To a solution of methyl 4-(4-acetoxythiobenzoylamino)-5-cyclohexylaminothiophene-2-carboxylate (2.14 g, 4.95 mmol) in acetonitrile (40 ml) was added 1-ethyl-3-(3'-dimethylaminopropyl)carbodiimide hydrochloride (WSC-HCl) (2.70 g, 14.1 mmol) and the mixture was stirred overnight at room temperature. After heating under reflux for 2 hr, and the mixture was allowed to cool to room temperature. WSC-HCl (2.70 g, 14.1 mmol) was further added, and the mixture was stirred at room temperature for 1 hr and further heated under reflux for 2 hr. After allowing to cool to room temperature, a saturated aqueous sodium hydrogen carbonate solution was added to the reaction mixture, and the mixture was extracted with ethyl acetate. The organic layer was washed successively with water, saturated aqueous sodium hydrogen carbonate solution, and saturated brine, and dried over anhydrous magnesium sulfate. After filtration, the solvent was evaporated under reduced pressure. The residue was purified by silica gel column chromatography (n-hexane:ethyl acetate=2:1). The obtained solid was washed with a mixed solvent of n-hexane:ethyl acetate=1:1 to give methyl 2-(4-acetoxyphenyl)-3-cyclohexyl-3H-thieno[2,3-d]imidazole-5-carboxylate (593 mg, yield 32%).
¹H-NMR(300MHz, δppm, CDCl₃): 7.91(1H, s), 7.63(2H, d, J=9.0Hz), 7.27(2H, d, J=9.0Hz), 4.28-4.38(1H, m), 3.92(3H, s), 2.35(3H, s), 1.86-2.12(6H, m), 1.71-1.81(1H, m), 1.24-1.45(3H, m). Step 9: Production of methyl 3-cyclohexyl-2-(4-hydroxyphenyl)-3H-thieno[2,3-d]imidazole-5-carboxylate

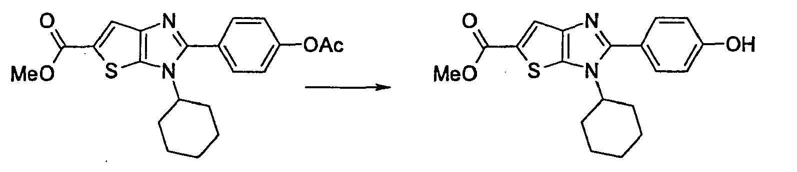

To a solution of methyl 2-(4-acetoxyphenyl)-3-cyclohexyl-3H-thieno[2,3-d]imidazole-5-carboxylate (100 mg, 0.251 mmol) in methanol (4 ml) was added potassium carbonate (104 mg, 0.753 mmol), and the mixture was stirred at room temperature for 1 hr. The solvent was evaporated under reduced pressure, and saturated aqueous ammonium chloride solution was added to the residue. The precipitated solid was collected by filtration, washed with water and vacuum dried to give methyl 3-cyclohexyl-2-(4-hydroxyphenyl)-3H-thieno[2,3-d]imidazole-5-carboxylate (80 mg, yield 89%). The obtained crude product was used for Example 2 without further purification.
¹H-NMR(400MHz, δppm, DMSO-d₆): 9.95(1H, s), 7.89(1H, s), 7.44(2H, d, J=8.0Hz), 6.91(2H, d, J=8.0Hz), 4.21-4.31(1H, m), 3.84(3H, s), 1.78-2.00(6H, m), 1.59-1.69(1H, m), 1.20-1.38(3H, m).

### Example 2

### Production of methyl 2-{4-[4'-chloro-4-(2-oxopyrrolidin-1-yl)biphenyl-2-ylmethoxy]phenyl}-3-cyclohexyl-3H-thieno[2,3-d]imidazole-5-carboxylate

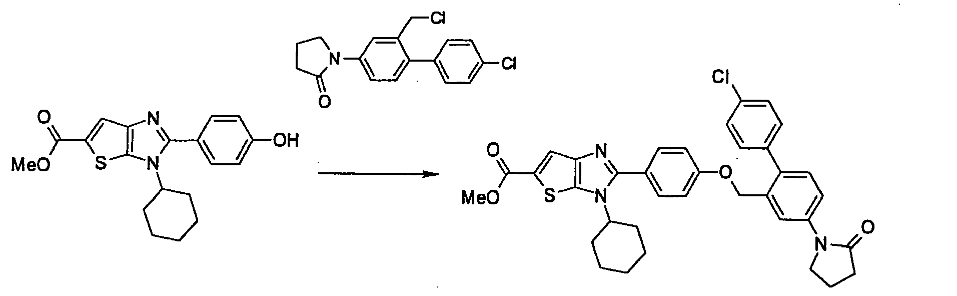

To a solution of methyl 3-cyclohexyl-2-(4-hydroxyphenyl)-3H-thieno[2,3-d]imidazole-5-carboxylate (70 mg, 0.196 mmol) in N,N-dimethylformamide (2 ml) were added potassium carbonate (41 mg, 0.294 mmol) and 1-(4'-chloro-2-chloromethylbiphenyl-4-yl)pyrrolidin-2-one (see WO03/000254) (79 mg, 0.245 mmol), and the mixture was stirred at 80°C for 2 hr. After allowing to cool to room temperature, water was added to the reaction mixture and the precipitated solid was collected by filtration. The obtained solid was washed with water, vacuum dried and purified by silica gel column chromatography (n-hexane:ethyl acetate=1:2-1:3) to give methyl 2-{4-[4'-chloro-4-(2-oxopyrrolidin-1-yl)biphenyl-2-ylmethoxy]phenyl}-3-cyclohexyl-3H-thieno[2,3-d]imidazole-5-carboxylate (102 mg, yield 82%).
¹H-NMR(400MHz, δppm, DMSO-d₆): 7.96(1H, d, J=4.0Hz), 7.91(1H,s), 7.76(1H, dd, J=8.0, 4.0Hz), 7.54(2H, d, J=8.0Hz), 7.46(4H, dd, J=16.0, 12.0Hz), 7.36(1H, d, J=8.0Hz), 7.08(2H, d, J=12.0Hz), 5.04(2H, s), 4.21-4.30(1H, m), 3.90(3H, t, J=6.0Hz), 3.84(3H, s), 2.53(2H, t, J=8.0Hz), 2.06-2.13(2H, m), 1.78-2.01(6H, m), 1.61-1.68(1H, m), 1.21-1.39(3H, m).

### Example 3

### Production of 2-{4-[4'-chloro-4-(2-oxopyrrolidin-1-yl)biphenyl-2-ylmethoxy]phenyl}-3-cyclohexyl-3H-thieno[2,3-d]imidazole-5-carboxylic acid hydrochloride

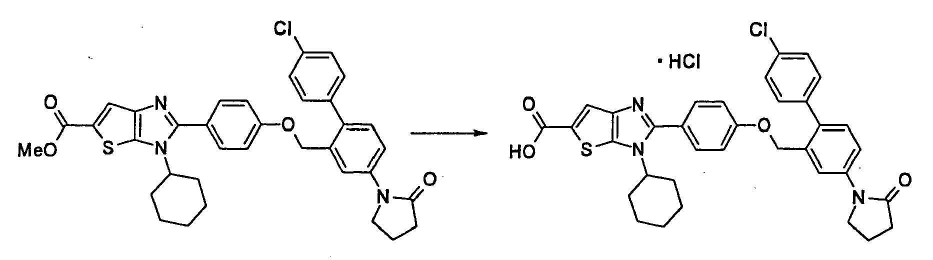

To a solution of methyl 2-{4-[4'-chloro-4-(2-oxopyrrolidin-1-yl)biphenyl-2-ylmethoxy]phenyl}-3-cyclohexyl-3H-thieno[2,3-d]imidazole-5-carboxylate (100 mg, 0.156 mmol) in a mixture of tetrahydrofuran (2 ml) and methanol (2 ml) was added 4N aqueous sodium hydroxide solution (0.2 ml), and the mixture was stirred at 80°C for 2 hr. After allowing to cool to room temperature, the mixture was neutralized with 2N aqueous hydrochloric acid. Saturated brine was added, and the mixture was extracted with ethyl acetate and tetrahydrofuran (1:1). The organic layer was washed with saturated brine, and dried over anhydrous magnesium sulfate. After filtration, the solvent was evaporated under reduced pressure. The residue was dissolved in tetrahydrofuran, and a solution of 4N hydrochloric acid in ethyl acetate (0.2 ml) was added. The solvent was evaporated under reduced pressure to give 2-{4-[4'-chloro-4-(2-oxopyrrolidin-1-yl)biphenyl-2-ylmethoxy]phenyl}-3-cyclohexyl-3H-thieno[2,3-d]imidazole-5-carboxylic acid hydrochloride (91 mg, yield 88%).
¹H-NMR(300MHz, δppm, DMSO-d₆): 7.98(1H, d, J=2.4Hz), 7.88(1H, s), 7.76(1H, dd, J=8.4, 2.4Hz), 7.59(2H, d, J=8.7Hz), 7.47(4H, dd, J=11.7, 8.7Hz), 7.38(1H, d, J=8.4Hz), 7.12(2H, d, J=8.7Hz), 5.06(2H, s), 4.19-4.36(1H, m), 3.90(2H, t, J=7.1Hz), 2.53(2H, t, J=9.0Hz), 1.74-2.17(8H, m), 1.61-1.69(1H, m), 1.21-1.42(3H, m); MS 627(M+1).

### Example 6

### Production of methyl 2-(4-benzyloxy-2-fluorophenyl)-1-cyclohex-2-enyl-1H-thieno[2,3-d]imidazole-5-carboxylate

### Step 1: Production of 4-benzyloxy-2-fluorobenzonitrile

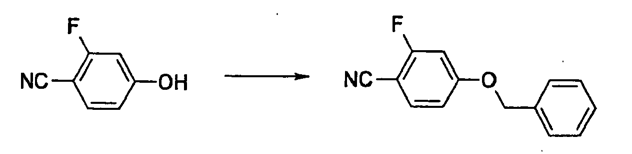

To a solution of 2-fluoro-4-hydroxybenzonitrile (20.0 g, 146 mmol) in N,N-dimethylformamide (100 ml) were added potassium carbonate (26.2 g, 190 mmol) and benzyl bromide (19 ml, 160 mmol) and the mixture was stirred at 80°C for 2 hr. After allowing to cool to room temperature, water was added to the reaction mixture and the precipitated solid was collected by filtration and washed successively with water and hexane. The obtained solid was washed with a mixed solvent of n-hexane:ethyl acetate=5:1 to give 4-benzyloxy-2-fluorobenzonitrile (25.8 g, yield 78%).
¹H-NMR(400MHz, δppm, DMSO-d₆): 7.84(1H, t, J=8.4Hz), 7. 34-7.47(5H, m), 7.25(1H, dd, J=12.0, 2.0Hz), 7.05(1H, dd, J=8.8, 2.8Hz), 5.22 (3H, s).

### Step 2: Production of 4-benzyloxy-2-fluoro-N-hydroxybenzamidine

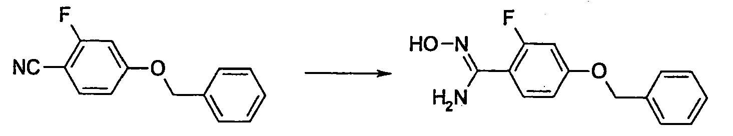

To a suspension of 4-benzyloxy-2-fluorobenzonitrile (15.0 g, 66 mmol) in water (150 ml) and ethanol (150 ml) were added hydroxylamine hydrochloride (6.42 g, 92.4 mmol) and sodium carbonate (10.5 g, 99.0 mmol), and the mixture was heated under reflux for 18 hr. The reaction mixture was appropriately evaporated under reduced pressure and the residue was extracted with chloroform. The organic layer was washed with saturated brine and dried over anhydrous magnesium sulfate. After filtration, the solvent was evaporated under reduced pressure, and the obtained solid was washed with a mixed solvent of n-hexane:ethyl acetate=1:1 to give 4-benzyloxy-2-fluoro-N-hydroxybenzamidine (14.7 g, yield 86%).
¹H-NMR(400MHz, δppm, DMSO-d₆): 9.51(1H, s), 7.31-7.45 (6H, m), 6.92(1H, dd, J=12.8, 2.0Hz), 6.85(1H, dd, J=8.8, 2.8Hz), 5.71(2H, s), 5.14(2H, s).

### Step 3: Production of methyl 3-[(4-benzyloxy-2-fluorobenzimidoyl)aminooxy]acrylate

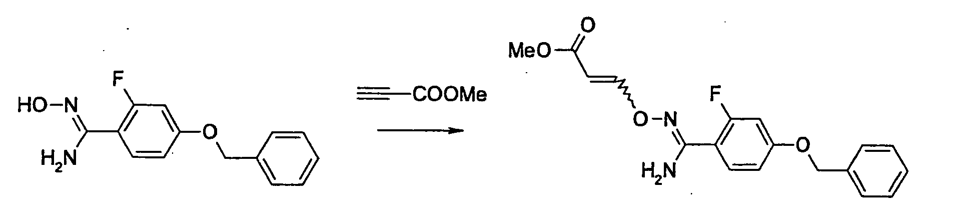

To a solution of 4-benzyloxy-2-fluoro-N-hydroxybenzamidine (14.5 g, 55.7 mmol) in methanol (145 ml) was added methyl propiolate (6.09 g, 72.4 mmol), and the mixture was heated under reflux for 12 hr. The solvent was evaporated under reduced pressure, and the obtained solid was washed with a mixed solvent of n-hexane:ethyl acetate=10:1 to give methyl 3-[(4-benzyloxy-2-fluorobenzimidoyl)aminooxy]acrylate (13.5 g, yield 70%).
¹H-NMR(400MHz, δppm, DMSO-d₆): 7.77 and 6.84-6.93 (total 2H, d and m, J=12.0Hz), 7.32-7.46(6H, m), 6.97-7.05(1H, m), 6.48(1H, brs), 5.49 and 4.83(total 1H, d and d, J=12.4Hz and J=7.2Hz), 5.18(2H, s), 3.61 and 3.60(total 3H, s).

### Step 4: Production of methyl 2-(4-benzyloxy-2-fluorophenyl)-1H-imidazole-4-carboxylate

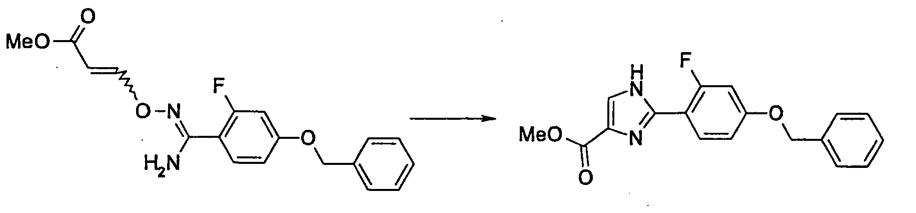

A solution of methyl 3-[(4-benzyloxy-2-fluorobenzimidoyl)aminooxy]acrylate (6.00 g, 17.4 mmol) in biphenyl ether (30 ml) was stirred at 220°C for 15 min. After rapidly coling to room temperature, n-hexane (90 ml) was added to the reaction mixture. The precipitated solid was collected by filtration and washed with n-hexane. The obtained solid was washed with a mixed solvent of n-hexane:ethyl acetate=1:1 to give methyl 2-(4-benzyloxy-2-fluorophenyl)-1H-imidazole-4-carboxylate (3.43 g, yield 60%).
¹H-NMR(400MHz, δppm, DMSO-d₆): 12.65(1H, brs), 7.89(1H, s), 7.87(1H, t, J=8.0Hz), 7.33-7.48(5H, m), 7.08(1H, dd, J=13.2, 2.4Hz), 6.99(1H, dd, J=8.8, 2.4Hz), 5.18(2H, s), 3.77(3H, s).

### Step 5: Production of [2-(4-benzyloxy-2-fluorophenyl)-1H-imidazol-4-yl]methanol

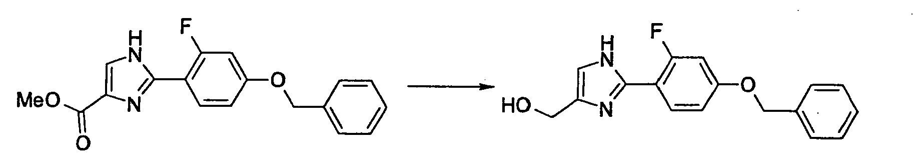

To a solution of methyl 2-(4-benzyloxy-2-fluorophenyl)-1H-imidazole-4-carboxylate (5.57 g, 17.1 mmol) in tetrahydrofuran (340 ml) was added lithium aluminum hydride (1.94 g, 6.03 mmol) with stirring under ice-cooling, and the mixture was stirred for 3 hr. Saturated aqueous ammonium chloride solution was added to the reaction mixture, and the mixture was extracted with ethyl acetate. The organic layer was washed successively with saturated aqueous ammonium chloride solution and saturated brine, and dried over anhydrous magnesium sulfate. After filtration, the solvent was evaporated under reduced pressure, and the obtained solid was washed with a mixed solvent of n-hexane:ethyl acetate=1:1 to give [2-(4-benzyloxy-2-fluorophenyl)-1H-imidazol-4-yl]methanol (4.31 g, yield 84%).
¹H-NMR(400MHz, δppm, DMSO-d₆): 11.82(1H, brs), 7.85(1H, t, J=8.8Hz), 7.32-7.47(5H, m), 7.03(1H, dd, J=13.2, 2.4Hz), 6.94(1H, dd, J=8.8, 2.8Hz), 5.16(2H, s), 4.87(1H, brs), 4.41(2H, brs).

### Step 6: Production of [2-(4-benzyloxy-2-fluorophenyl)-5-chloro-1H-imidazol-4-yl]methanol

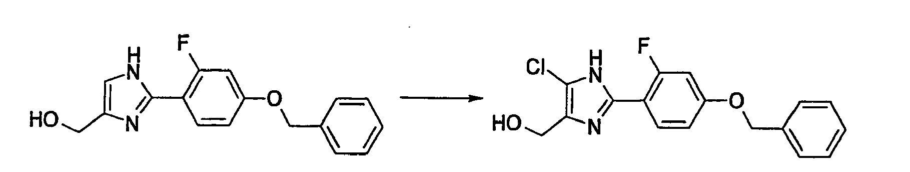

To a solution of [2-(4-benzyloxy-2-fluorophenyl)-1H-imidazol-4-yl]methanol (2.00 g, 6.70 mmol) in a mixture of tetrahydrofuran (80 ml), ethyl acetate (20 ml) and acetone (20 ml) was added N-chlorosuccinimide (1.03 g, 7.71 mmol) under ice-cooling, and the mixture was stirred for 1 hr, stirred at room temperature for 2 hr and then at 55°C for 4 hr. After allowing to cool to room temperature, water was added to the reaction mixture, and the mixture was stirred for 1 hr and extracted with ethyl acetate. The organic layer was washed successively with water and saturated brine, and dried over anhydrous magnesium sulfate. After filtration, the solvent was evaporated under reduced pressure, and the obtained solid was washed with ethyl acetate to give [2-(4-benzyloxy-2-fluorophenyl)-5-chloro-1H-imidazol-4-yl]methanol (1.44 g, yield 65%).
¹H-NMR(400MHz, δppm, DMSO-d₆): 12.38(1H, s), 7.77(1H, t, J=9.0Hz), 7.32-7.47(5H, m), 7.06(1H, dd, J=12.8, 2.4Hz), 6.96(1H, dd, J=8.8, 2.4Hz), 5.17(2H, s), 5.11(1H, t, J=5.6Hz), 4.42(2H, d, J=5.2Hz).

### Step 7: Production of 2-(4-benzyloxy-2-fluorophenyl)-5-chloro-1H-imidazole-4-carbaldehyde

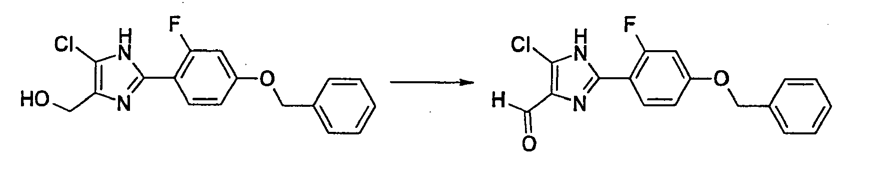

To a solution of [2-(4-benzyloxy-2-fluorophenyl)-5-chloro-1H-imidazol-4-yl]methanol (1.44 g, 4.33 mmol) in a mixture of ethyl acetate (60 ml) and chloroform (30 ml) was added manganese dioxide (2.21 g, 21.6 mmol) and the mixture was stirred at 80°C for 12 hr. After allowing to cool to room temperature, the mixture was filtered and the solvent was evaporated under reduced pressure. The residue was purified by silica gel column chromatography (n-hexane:ethyl acetate=1:1) and concentrated, and the obtained solid was washed with a mixed solvent of n-hexane:ethyl acetate=10:1 to give 2-(4-benzyloxy-2-fluorophenyl)-5-chloro-1H-imidazole-4-carbaldehyde (1.09 g, yield 76%).
¹H-NMR(400MHz, δppm, DMSO-d₆): 13.61(1H, brs), 9.71(1H, s), 7.84(1H, t, J=8.8Hz), 7.33-7.48(5H, m), 7.12(1H, dd, J=12.8, 2.4Hz), 7.00(1H, dd, J=8.8, 2.4Hz), 5.20(2H, s).

### Step 8: Production of 2-(4-benzyloxy-2-fluorophenyl)-5-chloro-3-cyclohex-2-enyl-3H-imidazole-4-carbaldehyde

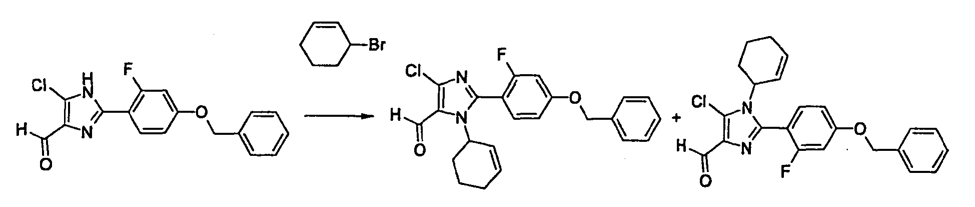

To a solution of 2-(4-benzyloxy-2-fluorophenyl)-5-chloro-1H-imidazole-4-carbaldehyde (1.20 g, 3.63 mmol) in N,N-dimethylformamide (12 ml) were added potassium carbonate (5.02 g, 36.3 mmol) and 3-bromocyclohexene (626 µl, 5.44 mmol), and the mixture was stirred at 90°C for 6 hr, during which 3-bromocyclohexene (626 µl, 5.44 mmol) was added 3 times. After allowing to cool to room temperature, water was added to the reaction mixture, and the mixture was extracted with ethyl acetate. The organic layer was washed with saturated brine, and dried over anhydrous magnesium sulfate. After filtration, the solvent was evaporated under reduced pressure. The residue was purified by silica gel column chromatography (n-hexane:ethyl acetate=5:1-4:1-3:1) to give 2-(4-benzyloxy-2-fluorophenyl)-5-chloro-3-cyclohex-2-enyl-3H-imidazole-4-carbaldehyde (534 mg, yield 36%) and 2-(4-benzyloxy-2-fluorophenyl)-5-chloro-1-cyclohex-2-enyl-1H-imidazole-4-carbaldehyde (235 mg, yield 16%). 2-(4-benzyloxy-2-fluorophenyl)-5-chloro-3-cyclohex-2-enyl-3H-imidazole-4-carbaldehyde
¹H-NMR(400MHz, δppm, DMSO-d₆) : 9.83(1H, s), 7.33-7.54(6H, m), 7.12(1H, dd, J=12.0, 2.4Hz), 7.00(1H, dd, J=8.4, 2.0Hz), 5.71(2H, brd, J=9.6Hz), 5.54(2H, brd, J=10.4Hz), 5.21(2H, s), 5.08(1H, brs), 1.90-2.08(4H, m), 1.72-1.82(1H, m), 1.47-1.60(1H, m).
2-(4-benzyloxy-2-fluorophenyl)-5-chloro-1-cyclohex-2-enyl-1H-imidazole-4-carbaldehyde
¹H-NMR(400MHz, δppm, DMSO-d₆): 9.81(1H, s), 7.33-7.50 (6H, m), 7.12(1H, dd, J=12.0, 2.0Hz), 7.01(1H, dd, J=8.8, 2.4Hz), 5.84(1H, brd, J=9.2Hz), 5.60(1H, brd, J=10.8Hz), 5.20(2H, s), 4.78(1H, brs), 1.93-2.11(4H, m), 1.77-1.84(1H, m), 1.49-1.62(1H, m).

### Step 9: Production of methyl 2-(4-benzyloxy-2-fluorophenyl)-1-cyclohex-2-enyl-1H-thieno[2,3-d]imidazole-5-carboxylate

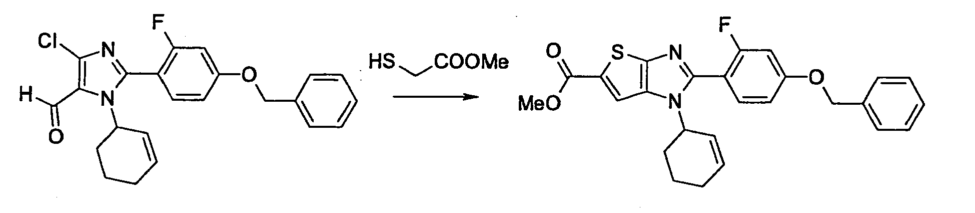

To a solution of sodium methoxide (207 mg, 3.83 mmol) in methanol (5 ml) was added methyl thioglycolate (343 µl, 3.83 mmol), and the mixture was stirred at room temperature for 1 hr. A solution of 2-(4-benzyloxy-2-fluorophenyl)-5-chloro-3-cyclohex-2-enyl-3H-imidazole-4-carbaldehyde (525 mg, 1.28 mmol) in methanol (6 ml) was added, and the mixture was heated under reflux for 9 hr. After allowing to cool to room temperature, a saturated aqueous sodium hydrogen carbonate solution was added to the reaction mixture, and the mixture was extracted with ethyl acetate. The organic layer was washed successively with a saturated aqueous sodium hydrogen carbonate solution and saturated brine, and dried over anhydrous magnesium sulfate. After filtration, the solvent was evaporated under reduced pressure. The residue was purified by silica gel column chromatography (n-hexane:ethyl acetate=4:1-3:1) and the obtained solid was washed with a mixed solvent of n-hexane:ethyl acetate=4:1 to give methyl 2-(4-benzyloxy-2-fluorophenyl)-1-cyclohex-2-enyl-1H-thieno[2,3-d]imidazole-5-carboxylate (179 mg, yield 30%).
¹H-NMR(400MHz, δppm, DMSO-d₆): 7.67(1H, s), 7.51(1H, t, J=8.4Hz), 7.33-7.46(5H, m), 6.91(1H, dd, J=8.8, 2.4Hz), 6.81(1H, dd, J=12.0, 2.4Hz), 6.05-6.11(1H, m), 5.69(1H, brd, J=10.4Hz), 5.12(2H, s), 4.77(1H, brs), 3.90(3H, s), 1.85-2.31(5H, m), 1.60-1.72(1H, m).

### Example 7

### Production of 2-(4-benzyloxy-2-fluorophenyl)-1-cyclohex-2-enyl-1H-thieno[2,3-d]imidazole-5-carboxylic acid hydrochloride

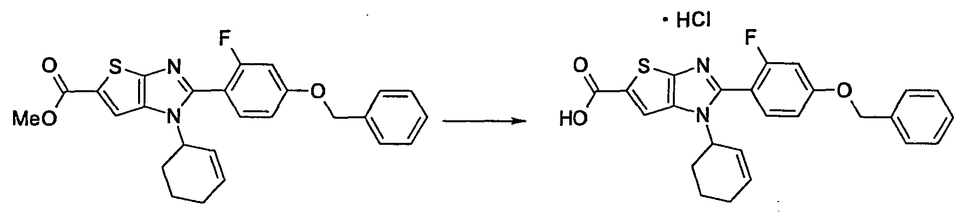

To a solution of methyl 2-(4-benzyloxy-2-fluorophenyl)-1-cyclohex-2-enyl-1H-thieno[2,3-d]imidazole-5-carboxylate (50 mg, 0.108 mmol) in a mixture of tetrahydrofuran (2 ml) and methanol (2 ml) was added 4N aqueous sodium hydroxide solution (2 ml) and the mixture was stirred at room temperature for 5 hr. The mixture was neutralized with 2N aqueous hydrochloric acid, saturated brine was added, and the mixture was extracted with ethyl acetate. The organic layer was washed with saturated brine and dried over anhydrous magnesium sulfate. After filtration, the solvent was evaporated under reduced pressure. The residue was dissolved in tetrahydrofuran, a solution of 4N hydrochloric acid in ethyl acetate (0.2 ml) was added, and the solvent was evaporated under reduced pressure to give 2-(4-benzyloxy-2-fluorophenyl)-1-cyclohex-2-enyl-1H-thieno[2,3-d]imidazole-5-carboxylic acid hydrochloride (44 mg, yield 84%).
¹H-NMR(400MHz, δppm, DMSO-d₆): 7.61(1H, s), 7.56(1H, t, J=8.6Hz), 7.34-7.50(5H, m), 7.15(1H, dd, J=12.0, 2.8Hz), 7.05(1H, dd, J=8.8, 2.4Hz), 6.06-6.13(1H, m), 5.68(1H, brd, J=10.0Hz), 5.21(2H, s), 4.73(1H, brs), 2.17-2.32(1H, m), 1.73-2.11(4H, m), 1.54-1.66(1H, m); MS 449(M+1).

### Example 9

### Production of ethyl 5-(4-benzyloxyphenyl)-6-cyclohex-2-enyl-4H-thieno[3,2-b]pyrrole-2-carboxylate

### Step 1: Production of methyl 5-methylthiophene-2-carboxylate

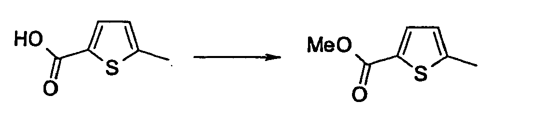

To a solution of 5-methylthiophene-2-carboxylic acid (40.3 g, 282 mmol) in N,N-dimethylformamide (500 ml) were added potassium carbonate (43 g, 310 mmol) and methyl iodide (19.3 ml, 310 mmol). The mixture was stirred at room temperature for 4 hr and diethyl ether (1.0 L) was added. The organic layer was washed successively with water (500 ml x 3) and saturated brine (200 ml) and dried over magnesium sulfate. Filtration and concentration gave methyl 5-methylthiophene-2-carboxylate (40.8 g, yield 93%).
¹H-NMR(300MHz, δppm, CDCl₃): 7.61(1H, d, J=3.6Hz), 6.76(1H, d, J=3.6Hz), 3.85(3H, s), 2.52(3H, s).

### Step 2: Production of methyl 4-nitro-5-methylthiophene-2-carboxylate

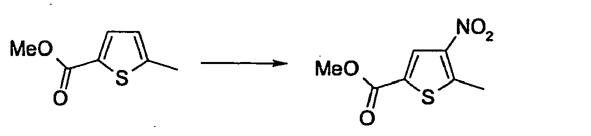

To a solution of methyl 5-methylthiophene-2-carboxylate (40.8 g, 260 mmol) in conc. sulfuric acid (400 ml) was added dropwise under ice-cooling a solution of fuming nitric acid (16.5 ml, 391 mmol) in conc. sulfuric acid (100 ml) at an inside temperature not exceeding 5°C. After the completion of the dropwise addition, the mixture was stirred under ice-cooling for 30 min and gradually poured into ice (1 kg). The precipitated solid was washed with water (500 ml x 6) and dried under reduced pressure to give methyl 4-nitro-5-methylthiophene-2-carboxylate (35.1 g, yield 67%).
¹H-NMR(300MHz, δppm, CDCl₃): 8.20(1H, s) , 3.91 (3H, s), 2. 84 (3H, s) .

### Step 3: Production of ethyl (E)-5-[2-(4-benzyloxyphenyl)vinyl]-4-nitrothiophene-2-carboxylate

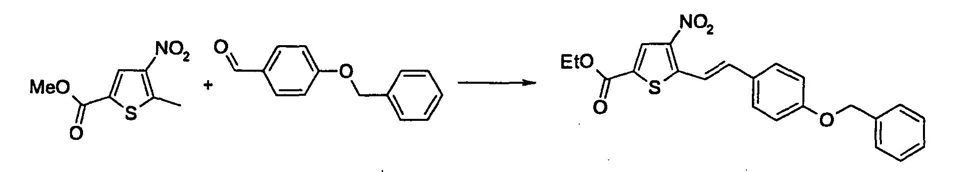

To a solution of methyl 4-nitro-5-methylthiophene-2-carboxylate (10 g, 50 mmol) in ethanol (100 ml) were added 4-benzyloxybenzaldehyde (15.8 g, 75 mmol) and pyrrolidine (6.3 ml, 75 mmol). After reaction with heating under reflux for 20 hr, the mixture was allowed to cool to room temperature. The precipitated solid was washed with ethanol (10 ml x 3) and dried under reduced pressure to give ethyl (E)-5-[2-(4-benzyloxyphenyl)vinyl]-4-nitrothiophene-2-carboxylate (16.7 g, yield 84%).
¹H-NMR(400MHz, δppm, CDCl₃): 8.18(1H, s), 7.99(1H, d, J=16Hz), 7.52(2H, d, J=8.8Hz), 7.44-7.28(6H, m), 6.99(2H, d, J=8.8Hz), 5.11(2H, s), 4.37(2H, q, J=7.0Hz), 1.40(3H, t, J=7.0Hz).

### Step 4: Production of ethyl 5-(4-benzyloxyphenyl)-4H-thieno[3,2-b]pyrrole-2-carboxylate

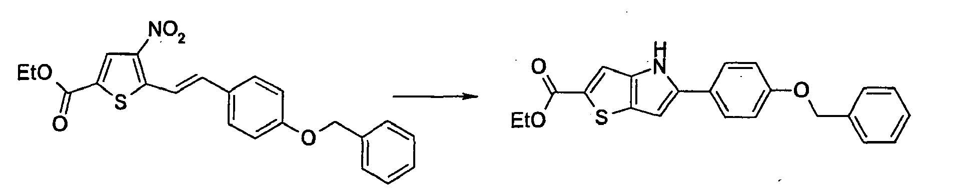

A solution of ethyl (E)-5-[2-(4-benzyloxyphenyl)vinyl]-4-nitrothiophene-2-carboxylate (16.7 g, 42 mmol) in triethyl phosphite (100 ml) was stirred at 180°C for 3 hr. After concentration under reduced pressure, chloroform (300 ml) was added to the residue. The organic layer was washed with water (100 ml x 2), and dried over magnesium sulfate. After filtration and concentration, and the obtained residue was purified by silica gel column chromatography (ethyl acetate). The obtained solid was washed with diethyl ether (5 ml x 3) to give ethyl 5-(4-benzyloxyphenyl)-4H-thieno[3,2-b]pyrrole-2-carboxylate (1.7 g, yield 10%).
¹H-NMR(400MHz, δppm, CDCl₃): 8.49(1H, brs), 7.66(1H, s), 7.48(2H, d, J=8.6Hz), 7.46-7.30(5H, m), 7.02(2H, d, J=8.6Hz), 6.62(1H, d,J=1.8Hz), 5.10(2H, s), 4.35(2H, q, J=6.9Hz), 1.38(3H, t, J=6.9Hz).

### Step 5: Production of ethyl 5-(4-benzyloxyphenyl)-6-cyclohex-2-enyl-4H-thieno[3,2-b]pyrrole-2-carboxylate

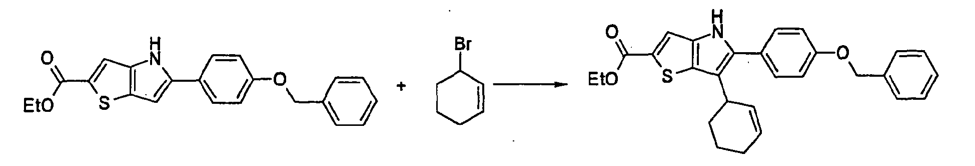

To a solution of ethyl 5-(4-benzyloxyphenyl)-4H-thieno[3,2-b]pyrrole-2-carboxylate (711 mg, 2 mmol) in tetrahydrofuran (10 ml) was added sodium hydride (60% in oil, 118 mg, 3 mmol) under ice-cooling and the mixture was stirred for 15 min. 3-Bromocyclohexene (0.34 ml, 3 mmol) was added and the mixture was stirred under ice-cooling for 4 hr. Ethyl acetate (50 ml) was added, and the organic layer was washed with water (20 ml x 3), and dried over magnesium sulfate. After filtration and concentration, the obtained residue was purified by silica gel column chromatography (n-hexane:ethyl acetate=90:10-80:20) to give ethyl 5-(4-benzyloxyphenyl)-6-cyclohex-2-enyl-4H-thieno[3,2-b]pyrrole-2-carboxylate (600 mg, yield 64%).

¹H-NMR(400MHz, δppm, CDCl₃): 8.29(1H, s), 7.63(1H, s), 7.46-7.30(7H, m), 7.04(2H, d, J=8.8Hz), 5.99-5.89(1H, m), 5.70-5.62(1H, m), 5.11(2H, s), 4.34(2H, q, J=7.2Hz), 3.66-3.57(1H, m), 2.25-1.60(6H, m), 1.38(3H, t, J=7.2Hz).

### Example 10

### Production of ethyl 5-(4-benzyloxyphenyl)-6-cyclohex-2-enyl-4-methyl-4H-thieno[3,2-b]pyrrole-2-carboxylate

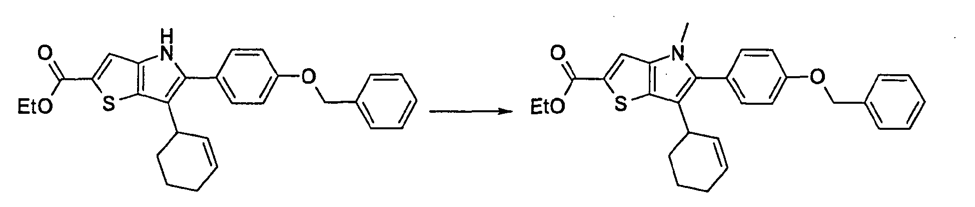

To a solution of ethyl 5-(4-benzyloxyphenyl)-6-cyclohex-2-enyl-4H-thieno[3,2-b]pyrrole-2-carboxylate (500 mg, 1.13 mmol) in N,N-dimethylformamide (5 ml) was added sodium hydride (60% in oil, 68 mg, 1.7 mmol) under ice-cooling and the mixture was stirred for 15 min. Methyl iodide (0.11 ml, 1.7 mmol) was added and the mixture was warmed to room temperature and stirred for 2 hr. Ethyl acetate (70 ml) was added, and the organic layer was washed successively with water (10 ml x 3) and saturated brine (10 ml) and dried over magnesium sulfate. Filtration and concentration gave ethyl 5-(4-benzyloxyphenyl)-6-cyclohex-2-enyl-4-methyl-4H-thieno[3,2-b]pyrrole-2-carboxylate as a crude product. The obtained compound was used for Example 11 without further purification.
¹H-NMR(400MHz, δppm, CDCl₃): 7.67(1H, s), 7.48-7.32(5H, m), 7.26(2H, d, J=8.8Hz), 7.06(2H, d, J=8.8Hz), 5.95-5.82(1H, m), 5.68-5.62(1H, m), 5.12(2H, s), 4.34(2H, q, J=7.0Hz), 3.55(3H, s), 3.38-3.30(1H, m), 2.25-1.50(6H, m), 1.38(3H, t, J=7.0Hz).

### Example 11

### Production of ethyl 5-(4-benzyloxyphenyl)-6-cyclohexyl-4-methyl-4H-thieno[3,2-b]pyrrole-2-carboxylate

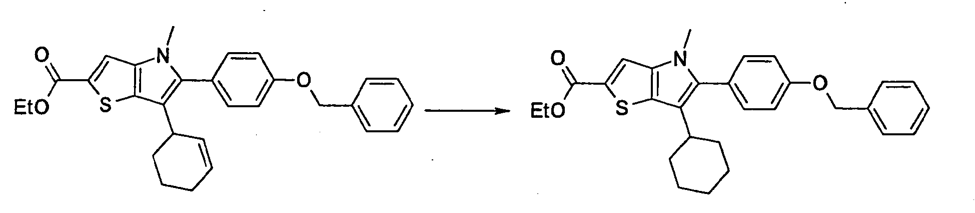

To a solution of ethyl 5-(4-benzyloxyphenyl)-6-cyclohex-2-enyl-4-methyl-4H-thieno[3,2-b]pyrrole-2-carboxylate in a mixture of methanol (5 ml) and tetrahydrofuran (5 ml) was added 20% palladium hydroxide/carbon (59 mg, 0.11 mmol). After stirring overnight at room temperature under hydrogen atmosphere under normal pressure, the mixture was filtered through celite, and the filtrate was concentrated to give ethyl 5-(4-benzyloxyphenyl)-6-cyclohexyl-4-methyl-4H-thieno[3,2-b]pyrrole-2-carboxylate as a crude product. The obtained compound was used for Example 13 without further purification.
¹H-NMR(400MHz, δppm, CDCl₃): 7.67(1H, s), 7.49-7.32(5H, m), 7.24(2H, d, J=8.6Hz), 7.06(2H, d, J=8.6Hz), 5.12(2H, s), 4.35(2H, q, J=7.2Hz), 3.53(3H, s), 2.55-2.42(1H, m), 1.88-1.56(6H, m), 1.39(3H, t, J=7.2Hz), 1.35-1.15(4H, m).

### Example 13

### Production of ethyl 6-cyclohexyl-5-(4-hydroxyphenyl)-4-methyl-4H-thieno[3,2-b]pyrrole-2-carboxylate

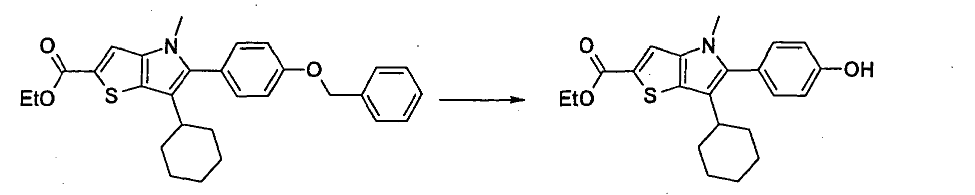

After stirring a solution of ethyl 5-(4-benzyloxyphenyl)-6-cyclohexyl-4-methyl-4H-thieno[3,2-b]pyrrole-2-carboxylate in 25% hydrobromic acid-acetic acid (5 ml) at room temperature for 1 hr, ethyl acetate (50 ml) was added, and the organic layer was washed successively with water (20 ml x 3) and saturated brine (20 ml), and dried over magnesium sulfate. After filtration and concentration, the obtained residue was purified by silica gel column chromatography (n-hexane:ethyl acetate=80:20-50:50) to give ethyl 6-cyclohexyl-5-(4-hydroxyphenyl)-4-methyl-4H-thieno[3,2-b]pyrrole-2-carboxylate (400 mg, yield 96%).
¹H-NMR(400MHz, δppm, CDCl₃): 7.67(1H, s), 7.19(2H, d, J=8.3Hz), 6.92(2H, d, J=8.3Hz), 4.91(1H, s), 4.35(2H, q, J=7.2Hz), 3.52(3H, s), 2.55-2.40(1H, m), 1.90-1.63(6H, m), 1.39(3H, t, J=7.2Hz), 1.32-1.15(4H, m).

### Example 16

### Production of ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate

### Step 1: Production of N-(3-chloromethyl-4-morpholinophenyl)-N-methylacetamide

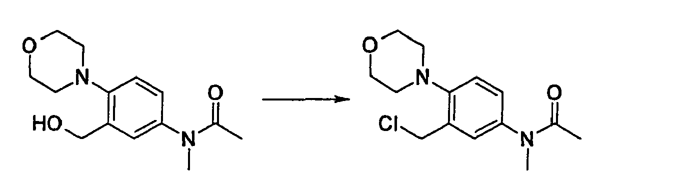

To a solution of N-(3-hydroxymethyl-4-morpholinophenyl)-N-methylacetamide (106 mg, 0.4 mmol) in chloroform (3 ml) were added triethylamine (0.11 ml, 0.8 mmol) and thionyl chloride (58 µl, 0.8 mmol). The mixture was stirred at room temperature for 2 hr and ethyl acetate (30 ml) was added. The organic layer was washed successively with saturated aqueous sodium hydrogen carbonate solution (20 ml x 2) and saturated brine (20 ml), and dried over magnesium sulfate. Filtration and concentration gave N-(3-chloromethyl-4-morpholinophenyl)-N-methylacetamide as a crude product. The obtained compound was used for Step 2 without further purification.

### Step 2: Production of ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate

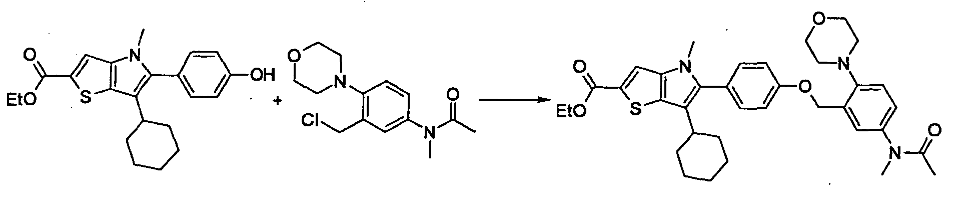

To a solution of ethyl 6-cyclohexyl-5-(4-hydroxyphenyl)-4-methyl-4H-thieno[3,2-b]pyrrole-2-carboxylate (100 mg, 0.27 mmol) in N,N-dimethylformamide (5 ml) was added N-(3-chloromethyl-4-morpholinophenyl)-N-methylacetamide and potassium carbonate (75 mg, 0.54 mmol). After stirring at 100°C for 4 hr, ethyl acetate (30 ml) was added, and the organic layer was washed successively with water (20 ml x 3) and saturated brine (20 ml) and dried over magnesium sulfate. After filtration and concentration, the obtained residue was purified by silica gel column chromatography (ethyl acetate) to give ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate (92 mg, yield 55%).
¹H-NMR(400MHz, δppm, CDCl₃): 7.66(1H, s), 7.36(1H, s), 7.23(2H, d, J=8.8Hz), 7.18-7.09(2H, m), 7.01(2H, d, J=8.8Hz), 5.22(2H, s), 4.34(2H, q, J=7.2Hz), 3.88-3.60(4H, m), 3.51(3H, s), 3.23(3H, s), 3.20-2.95(4H, m), 2.48-2.40(1H, m), 1.85-1.58(6H, m), 1.79(3H, s), 1.38(3H, t, J=7.2Hz), 1.28-1.15(4H, m).

### Example 17

### Production of 5-{4-[5-(N-acetyl-N-methylamino)-2-morpholinobenzyloxy]phenyl}-6-cyclohexyl-4-methyl-4H-thieno[3,2-b]pyrrole-2-carboxylic acid hydrochloride

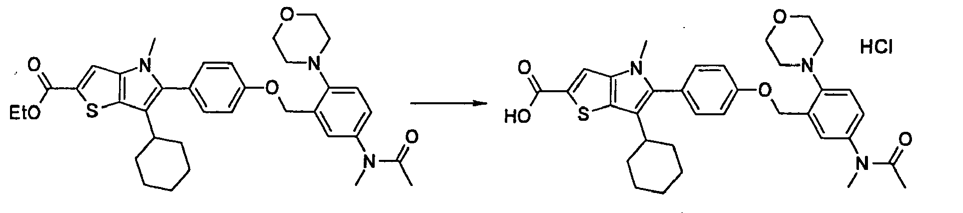

To a solution of ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate (92 mg, 0.15 mmol) in a mixture of methanol (2 ml) and tetrahydrofuran (2 ml) was added a 4N aqueous sodium hydroxide solution (2 ml). After stirring overnight at room temperature, the reaction solution was concentrated. The obtained residue was acidified with 1N aqueous hydrochloric acid solution. The precipitated solid was washed with water (5 ml x 3), dried under reduced pressure and dissolved in ethyl acetate (2 ml). A 4N hydrochloric acid-ethyl acetate solution (1 ml, 4 mmol) was added. The reaction solution was concentrated and diethyl ether (4 ml) and n-hexane (2 ml) were added. The precipitated solid was washed with a mixed solvent of diethyl ether:n-hexane=1:2 and dried under reduced pressure to give 5-{4-[5-(N-acetyl-N-methylamino)-2-morpholinobenzyloxy]phenyl}-6-cyclohexyl-4-methyl-4H-thieno[3,2-b]pyrrole-2-carboxylic acid hydrochloride (70 mg, yield 78%).
¹H-NMR(400MHz, δppm, DMSO-d₆): 12.58(1H, brs), 7.78(1H, s), 7.36(1H, d, J=2.6Hz), 7.32-7.24(3H, m), 7.22(1H, d, J=8.2Hz), 7.09(2H, d, J=8.6Hz), 5.22(2H, s), 3.76-3.71 (4H, m), 3.49(3H, s), 3.09 (3H, s), 2.94-2.87(4H, m), 2.55-2.51(1H, m), 2.06(3H, s), 1.76-1.50(7H, m), 1.26-1.14(3H, m); MS 602(M+1).

In the same manner as in Examples 1 to 3, 6, 7, 9 to 11, 13, 16 and 17, and using other conventional methods where necessary, the following compounds of Examples were produced. 2-(4-benzyloxyphenyl)-3-cyclohexyl-3H-thieno[2,3-d]imidazole-5-carboxylic acid hydrochloride (Example 4),
3-cyclohexyl-2-{4-[2-morpholino-5-(2-oxopyrrolidin-1-yl)benzyloxy]phenyl}-3H-thieno[2,3-d]imidazole-5-carboxylic acid (Example 5), 5-(4-benzyloxyphenyl)-4,6-dicyclohexyl-4H-thieno[3,2-b]pyrrole-2-carboxylic acid (Example 8),
5-(4-benzyloxyphenyl)-6-cyclohexyl-4-methyl-4H-thieno[3,2-b]pyrrole-2-carboxylic acid (Example 12),
5-(4-benzyloxyphenyl)-6-cyclohexyl-4-dimethylcarbamoylmethyl-4H-thieno[3,2-b]pyrrole-2-carboxylic acid (Example 14),
6-cyclohexyl-4-methyl-5-{4-[2-morpholino-5-(2-oxopyrrolidin-1-yl)benzyloxy]phenyl}-4H-thieno[3,2-b]pyrrole-2-carboxylic acid hydrochloride (Example 15),
6-cyclohexyl-5-{4-[2-(4-methanesulfonylpiperazin-1-yl)-5-(2-oxopyrrolidin-1-yl)benzyloxy]phenyl}-4-methyl-4H-thieno[3,2-b]pyrrole-2-carboxylic acid hydrochloride (Example 18),
5-{4-[5-amino-2-(4-methanesulfonylpiperazin-1-yl)benzyloxy]phenyl}-6-cyclohexyl-4-methyl-4H-thieno[3,2-b]pyrrole-2-carboxylic acid hydrochloride (Example 19),
5-{4-[5-(N-acetyl-N-methylamino)-2-(4-methanesulfonylpiperazin-1-yl)benzyloxy]phenyl}-6-cyclohexyl-4-methyl-4H-thieno[3,2-b]pyrrole-2-carboxylic acid hydrochloride (Example 20),
6-cyclohexyl-4-dimethylcarbamoylmethyl-5-(4-methoxyphenyl)-4H-thieno[3,2-b]pyrrole-2-carboxylic acid (Example 21),
6-cyclohexyl-5-[4-(5-methanesulfonyl-2-morpholinobenzyloxy)phenyl]-4-methyl-4H-thieno[3,2-b]pyrrole-2-carboxylic acid hydrochloride (Example 23),
ethyl 6-cyclohexyl-4-methyl-5-{4-[2-morpholino-5-(2-oxopyrrolidin-1-yl)benzyloxy]phenyl}-4H-thieno[3,2-b]pyrrole-2-carboxylate hydrochloride (Example 24),
6-cyclohexyl-5-(4-methoxyphenyl)-4-morpholinocarbonylmethyl-4H-thieno[3,2-b]pyrrole-2-carboxylic acid (Example 25),
6-cyclohexyl-4-(4-ethylpiperazin-1-yl)carbonylmethyl-5-(4-methoxyphenyl)-4H-thieno[3,2-b]pyrrole-2-carboxylic acid (Example 26) ,
6-cyclohexyl-4-(4-dimethylaminopiperidino)carbonylmethyl-5-(4-methoxyphenyl)-4H-thieno[3,2-b]pyrrole-2-carboxylic acid (Example 27) ,
6-cyclohexyl-5-[4-(5-isobutyrylamino-2-morpholinobenzyloxy)phenyl]-4-methyl-4H-thieno[3,2-b]pyrrole-2-carboxylic acid hydrochloride (Example 29),
6-cyclohexyl-5-{4-[5-(N-isobutyryl-N-methylamino)-2-morpholinobenzyloxy]phenyl}-4-methyl-4H-thieno[3,2-b]pyrrole-2-carboxylic acid hydrochloride (Example 30),
6-cyclohexyl-4-methyl-5-[4-(2-morpholinobenzyloxy)phenyl]-4H-thieno[3,2-b]pyrrole-2-carboxylic acid hydrochloride (Example 31) ,
4-(benzylcarbamoylmethyl)-6-cyclohexyl-5-(4-methoxyphenyl)-4H-thieno[3,2-b]pyrrole-2-carboxylic acid (Example 32),
4-(tert-butylcarbamoylmethyl)-6-cyclohexyl-5-(4-methoxyphenyl)-4H-thieno[3,2-b]pyrrole-2-carboxylic acid (Example 33),
6-cyclohexyl-4-methyl-5-{4-[2-morpholino-4-(2-oxopyrrolidin-1-yl)benzyloxy]phenyl}-4H-thieno[3,2-b]pyrrole-2-carboxylic acid hydrochloride (Example 34),
5-(2-benzyloxyphenyl)-6-cyclohexyl-4-methyl-4H-thieno[3,2-b]pyrrole-2-carboxylic acid (Example 35),
5-(2-benzyloxyphenyl)-6-cyclohexyl-4-dimethylcarbamoylmethyl-4H-thieno[3,2-b]pyrrole-2-carboxylic acid (Example 36),
5-[4-(1-tert-butoxycarbonylpiperidin-3-yloxy)phenyl]-6-cyclohexyl-4-dimethylcarbamoylmethyl-4H-thieno[3,2-b]pyrrole-2-carboxylic acid (Example 37),
6-cyclohexyl-4-dimethylcarbamoylmethyl-5-[4-(2-morpholinobenzyloxy)phenyl]-4H-thieno[3,2-b]pyrrole-2-carboxylic acid (Example 38),
5-[4-(1-tert-butoxycarbonylpiperidin-4-yloxy)phenyl]-6-cyclohexyl-4-dimethylcarbamoylmethyl-4H-thieno[3,2-b]pyrrole-2-carboxylic acid (Example 39),
6-cyclohexyl-5-{4-[5-(2-dimethylaminoacetylamino)-2-morpholinobenzyloxy]phenyl}-4-methyl-4H-thieno[3,2-b]pyrrole-2-carboxylic acid dihydrochloride (Example 40),
6-cyclohexyl-4-methyl-5-{4-[2-morpholino-5-(2-morpholinoacetylamino)benzyloxy]phenyl}-4H-thieno[3,2-b]pyrrole-2-carboxylic acid dihydrochloride (Example 41),
6-cyclohexyl-4-dimethylcarbamoylmethyl-5-(4-phenoxyphenyl)-4H-thieno[3,2-b]pyrrole-2-carboxylic acid (Example 42),
6-cyclohexyl-5-(4-{5-[N-(2-dimethylaminoacetyl)-N-methylamino]-2-morpholinobenzyloxy}phenyl)-4-methyl-4H-thieno[3,2-b]pyrrole-2-carboxylic acid dihydrochloride (Example 43),
5-(4-benzyloxyphenyl)-4-(tert-butylcarbamoylmethyl)-6-cyclohexyl-4H-thieno[3,2-b]pyrrole-2-carboxylic acid (Example 44) ,
4-benzyl-5-(4-benzyloxyphenyl)-6-cyclohexyl-4H-thieno[3,2-b]pyrrole-2-carboxylic acid (Example 45),
5-(4-benzyloxyphenyl)-6-cyclohexyl-4-(2-dimethylaminoethyl)-4H-thieno[3,2-b]pyrrole-2-carboxylic acid (Example 46),
5-(4-benzyloxyphenyl)-6-cyclohexyl-4-(2-morpholinoethyl)-4H-thieno[3,2-b]pyrrole-2-carboxylic acid (Example 47),
5-(4-benzyloxyphenyl)-6-cyclohexyl-4-cyclohexylmethyl-4H-thieno[3,2-b]pyrrole-2-carboxylic acid (Example 48),
5-(4-benzyloxyphenyl)-6-cyclohexyl-4-(2-methoxyethyl)-4H-thieno[3,2-b]pyrrole-2-carboxylic acid (Example 49),
5-(4-benzyloxyphenyl)-6-cyclohexyl-4-{[N-methyl-N-(2-oxo-2-piperidinoethyl)carbamoyl]methyl}-4H-thieno[3,2-b]pyrrole-2-carboxylic acid (Example 50),
5-(4-benzyloxyphenyl)-6-cyclohexyl-4-[(4-morpholinophenylcarbamoyl)methyl]-4H-thieno[3,2-b]pyrrole-2-carboxylic acid hydrochloride (Example 51),
5-(4-benzyloxyphenyl)-6-cyclohexyl-4-[2-(4-cyclohexylpiperazin-1-yl)-2-oxoethyl]-4H-thieno[3,2-b]pyrrole-2-carboxylic acid hydrochloride (Example 52),
5-(4-benzyloxyphenyl)-6-cyclohexyl-4-[2-(4-cyclohexylpiperazin-1-yl)ethyl]-4H-thieno[3,2-b]pyrrole-2-carboxylic acid dihydrochloride (Example 53),
5-(4-benzyloxyphenyl)-4-[2-(1,4'-bipiperidinyl-1'-yl)-2-oxoethyl]-6-cyclohexyl-4H-thieno[3,2-b]pyrrole-2-carboxylic acid hydrochloride (Example 54),
5-(4-benzyloxyphenyl)-4-[2-(1,4'-bipiperidinyl-1'-yl)ethyl]-6-cyclohexyl-4H-thieno[3,2-b]pyrrole-2-carboxylic acid dihydrochloride (Example 55),
6-cyclohexyl-4-(2-dimethylaminoethyl)-5-[4-(5-methanesulfonyl-2-morpholinobenzyloxy)phenyl]-4H-thieno[3,2-b]pyrrole-2-carboxylic acid dihydrochloride (Example 56),
6-cyclohexyl-4-methyl-5-(4-{5-[N-methyl-N-(2-morpholinoacetyl)amino]-2-morpholinobenzyloxy}phenyl)-4H-thieno[3,2-b]pyrrole-2-carboxylic acid dihydrochloride (Example 57),
5-(4-benzyloxyphenyl)-6-cyclohexyl-4-{2-[N-methyl-N-(2-morpholinoethyl)amino]ethyl}-4H-thieno[3,2-b]pyrrole-2-carboxylic acid dihydrochloride (Example 58),
5-(4-benzyloxyphenyl)-6-cyclohexyl-4-{2-[N-methyl-N-(2-piperidinoethyl)amino]ethyl}-4H-thieno[3,2-b]pyrrole-2-carboxylic acid dihydrochloride (Example 59),
5-(4-benzyloxyphenyl)-6-cyclohexyl-4-{1-[N-methyl-N-(4-morpholinophenyl)carbamoyl]ethyl}-4H-thieno[3,2-b]pyrrole-2-carboxylic acid hydrochloride (Example 60).

In the same manner as in the aforementioned Examples, and using other conventional methods where necessary, the following compounds can be produced. 6-cyclohexyl-5-(4-methoxyphenyl)-4-(4-methylpiperazin-1-yl)carbonylmethyl-4H-thieno[3,2-b]pyrrole-2-carboxylic acid hydrochloride (Example 22),
6-cyclohexyl-5-{4-[2-(4-dimethylaminopiperidino)-5-(2-oxopyrrolidin-1-yl)benzyloxy]phenyl}-4-methyl-4H-thieno[3,2-b]pyrrole-2-carboxylic acid dihydrochloride (Example 28)

The structural formulas and physicochemical data of the Example compounds are shown in Tables 1-17.

**Table 1**

| Example | Formula | ¹H-NMR, δppm | MS |
|---|---|---|---|
| 4 | 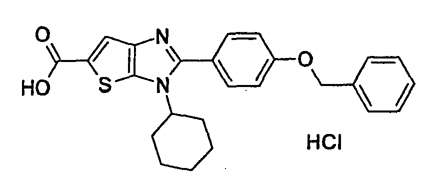 | 400MHz,DMSO-d6 7.91 (1H,s), 7.65(2H,d,J=9.0Hz), 7.52-7.34(5H,m), 7.25(2H,d,J=9.0Hz), 5.23(2H,s), 4.37-4.24(1H,m), 2.10-1.78(6H,m), 1.69-1.59(1H,m), 1.42-1.21(3H,m). | 433(M+1) |
| 5 | 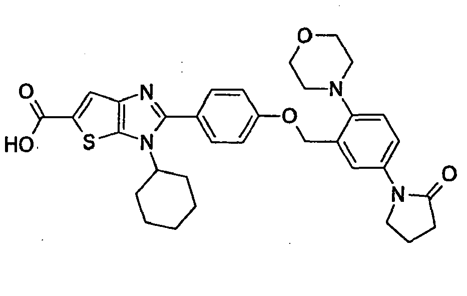 | 400MHz,DMSO-d6 7.82(1 H,d,J=2.8Hz), 7.60(1 H,brs), 7.57(1 H,d,J=2.4Hz), 7.54(2H,d,J=8.8Hz), 7.21 (1 H,d,J=8.8Hz), 7.17(2H,d,J=8.8Hz), 5.23(2H,s), 4.27-4.18(1H,m), 3.81(2H,t,J=6.8Hz), 3.74-3.69(4H,m), 2.91-2.84(4H,m), 2.47(2H,t,J=8.8Hz), 2.09-2.01(2H,m), 2.00-1.79(6H,m), 1.69-1.61(1H,m), 1.37-1.22(3H,m). | 601(M+1) |
| 8 | 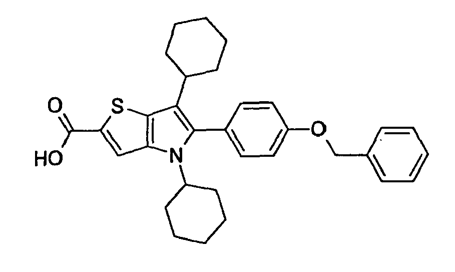 | 400MHz,DMSO-d6 12.66(1H,brs), 7.83(1 H,s), 7.50-7.46(2H,m), 7.43-7.37(2H,m), 7.36-7.32(1H,m), 7.24(2H,d,J=8.8Hz), 7.13(2H,d,J=8.8Hz), 5.14(2H,s), 3.75-3.58(1 H,m), 2.36-2.25(1H,m), 2.00-1.88(2H,m), 1.84-1.45(12H,m), 1.35-1.05(6H,m). | 514(M+1) |

**Table 2**

| Example | Formula | ¹H-NMP, δppm | MS |
|---|---|---|---|
| 12 | 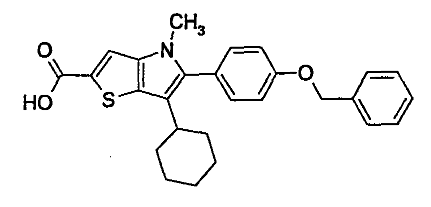 | 400MHz,DMSO-d6 7.48-7.43(2H,m), 7.42-7.36(2H,m), 7.35-7.29(1H,m), 7.24(2H,d,J=8.8Hz), 7.21 (1 H,s), 7.09(2H,d,J=8.8Hz), 5.13(2H,s),3.43(3H,s), 2.45-2.33(1H,m), 1.78-1.53(7H,m), 1.23-1.08(3H,m). | 446(M+1) |
| 14 | 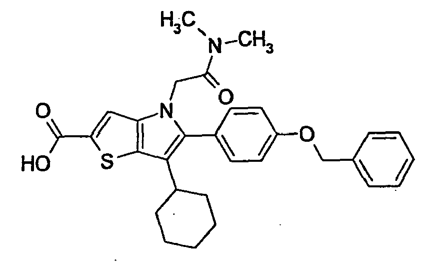 | 400MHz,DMSO-d6 12.57(1 H,brs), 7.68(1 H,s), 7.48-7.44(2H,m), 7.42-7.36(2H,m), 7.35-7.30(1H,m), 7.18(2H,d,J=8.8Hz), 7.09(2H,d,J=8.8Hz), 5.12(2H,s),4.74(2H,s), 2.83(3H,s),2.76(3H,s), 2.44-2.32(1H, m), 1.76-1.50(7H,m), 1.23-1.12(3H,m). | 517(M+1) |
| 15 | 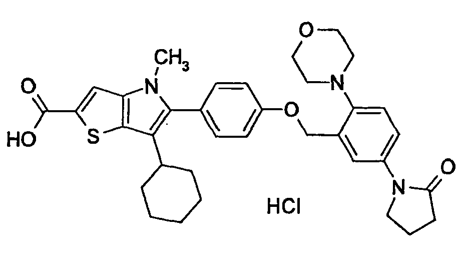 | 400MHz,DMSO-d6 7.80(1 H,d,J=2.8Hz), 7.79(1 H,s), 7.55(1H,dd,J=2.8,8.6Hz), 7.30(2H,d,J=8.8Hz), 7.19(1H,d,J=8.6Hz), 7.13(2H,d,J=8.8Hz), 5.19(2H,s), 3.79(2H,t,J=6.7Hz), 3.74-3.65(4H,m), 3.51 (3H,s), 2.89-2.82(4H,m), 2.54-2.49(1 H,m), 2.45(2H,t,J=8.3Hz), 2.09-1.98(2H,m), 1.76-1.46(7H,m), 1.24-1.12(3H,m). | 614(M+1) |

**Table 3**

| Example | Formula | ¹H-NMR, δppm | MS |
|---|---|---|---|
| 18 | 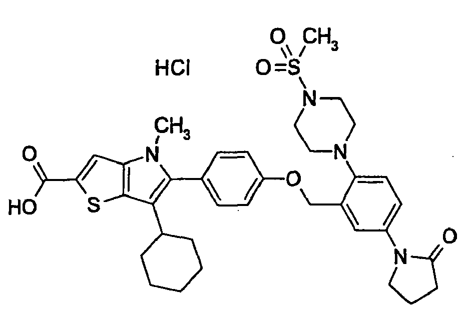 | 400MHz,DMSO-d6 12.56(1 H,brs), 7.87(1 H,d,J=2.2Hz), 7.84(1 H,s), 7.60(1H,dd,J=2.2,8.8Hz), 7.35(2H.d,J=8.6Hz), 7.28(1 H,d,J=8.8Hz), 7.19(2H,d,J=8.6Hz), 5.25(2H,s), 3.85(2H,t,J=7.0Hz), 3.56(3H,s), 3.34-3.27(4H,m), 3.09-2.95(4H,m), 2.95(3H,s), 2.58-2.54(1H,m), 2.51(2H,t,J=8.0Hz) 2.14-2.03(2H,m), 1.82-1.55(7H,m), 1.29-1.18(3H,m). | 691(M+1) |
| 19 | 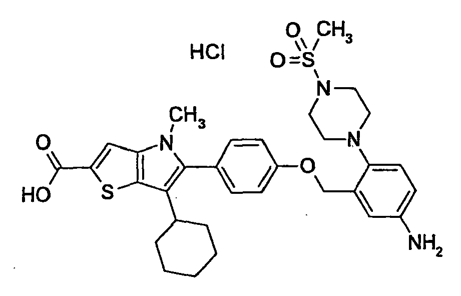 | 400MHz,DMSO-d6 7.82(1H,s), 7.81 (1 H,d,J=2.1 Hz), 7.34(2H,d,J=8.6Hz), 7.29(1 H,d,J=8.8Hz), 7.21 (1 H,dd,J=2.1,8.8Hz), 7.16(2H,d,J=8.6Hz), 5.25(2H,s),3.54(3H,s), 3.33-3.21 (4H,m), 3.05-2.95(4H,m), 2.94(3H,s), 2.47-2.40(1 H,m), 1.81-1.52(7H,m), 1.27-1.13(3H,m). | 622(M+1) |
| 20 | 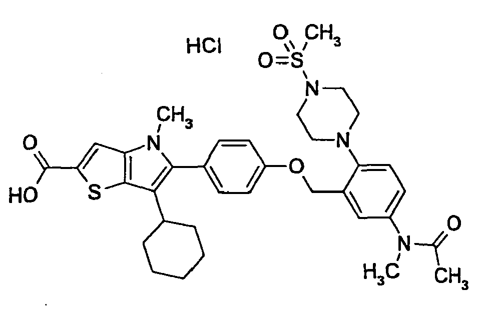 | 400MHz,DMSO-d6 7.79(1 H,s), 7.39-7.37(1 H,m), 7.29(2H,d,J=8.8Hz), 1.28-7.22(2H,m), 7.11(2H,d,J=8.8Hz), 5.24(2H,s),3.49(3H,s), 3.14-3.06(4H,m), 3.05(3H,s), 3.04-2.96(4H,m), 2.92(3H,s), 2.55-2.51(1H,m), 2.14(3H,m), 1.76-1.60(7H,m), 1.26-1.12(3H,m). | 679(M+1) |

**Table 4**

| Example | Formula | ¹H-NMR, δppm | MS |
|---|---|---|---|
| 21 | 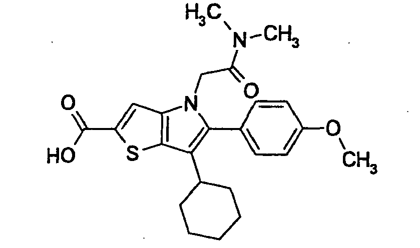 | 400MHz,DMSO-d6 12.46(1 H,brs),7.69(1H,s), 7.18(2H,d,J=8.6Hz), 7.01(2H,d,J=8.6Hz), 4.74(2H,s),3.79(3H,s), 2.85(3H,s),2.77(3H,s), 2.45-2.33(1H,m), 1.75-1.48(6H,m), 1.26-1.10(4H,m). | 441(M+1) |
| 23 | 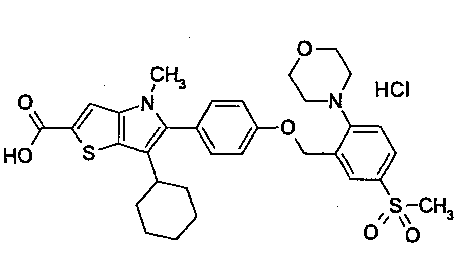 | 400MHz,DMSO-d6 12.59(1 H,brs), 8.02(1 H,d,J=2.3Hz), 7.85(1 H,dd,J=2.3,8.6Hz), 7.78(1H,s), 7.34(1 H,d,J=8.6Hz), 7.32(2H,d,J=8.6Hz), 7.17(2H,d,J=8.6Hz), 5.23(2H,s), 3.78-3.70(4H,m), 3.51(3H,s),3.16(3H,s), 3.02-2.96(4H,m), 2.45-2.37(1H,m), 1.78-1.48(7H,m), 1.25-1.12(3H,m). | 609(M+1) |

**Table 5**

| Example | Formula | ¹H-NMR, δppm | MS |
|---|---|---|---|
| 24 | 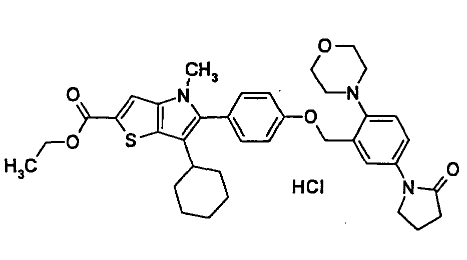 | 400MHz,DMSO-d6 7.87(1 H,s), 7.80(1 H,d,J=2.8Hz), 7.55(1 H,dd,J=2.8,8.8Hz), 7.30(2H,d,J=8.6Hz), 7.19(1H,d,J=8.8Hz), 7.14(2H,d,J=8.6Hz), 5.19(2H,s), 4.25(2H,q,J=6.9Hz), 3.79(2H,t,J=7.2Hz), 3.72-3.65(4H,m), 3.52(3H,s), 2.89-2.82(4H,m), 2.45(2H,t,J=8.3Hz), 2.44-2.35(1H,m), 2.08-1.98(2H,m), 1.75-1.49(7H,m), 1.29(3H,t,J=6.9Hz), 1.24-1.11(3H,m). | 642(M+1) |
| 25 | 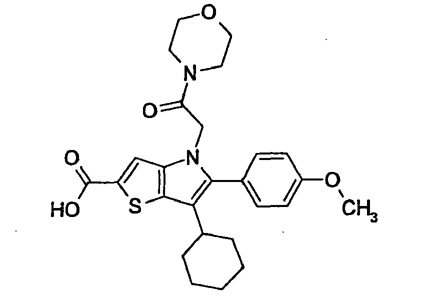 | 400MHz,DMSO-d6 12.60(1 H,brs), 7.73(1 H,s), 7.20(1 H,d,J=8.8Hz), 7.05(1 H,d,J=8.8Hz), 4.81(2H,s),3.81(3H,s), 3.54-3.43(4H,m), 3.41-3.27(4H,m), 2.47-2.37(1 H,m), 1.78-1.68(4H,m), 1.68-1.52(3H,m), 1.27-1.12(3H,m). | 483(M+1) |
| 26 | 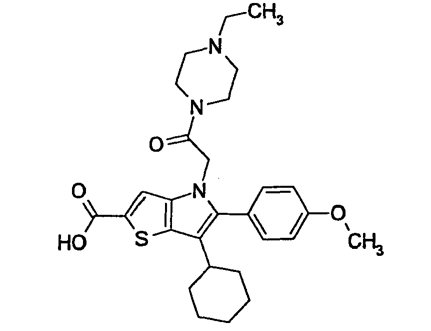 | 400MHz,DMSO-d6 7.71(1H,s), 7.19(1H,d,J=8.6Hz), 7.03(1 H,d,J=8.6Hz), 4.80(2H,s),3.81(3H,s), 3.43-3.26(4H,m), 2.46-2.36(1 H,m), 2.30(2H,q,J=7.2Hz), 2.27-2.16(4H,m), 1.78-1.68(4H,m), 1.67-1.51 (3H,m), 1.28-1.11(3H,m), 0.98(3H,t,J=7.2Hz). | 510(M+1) |

**Table 6**

| Example | Formula | ¹H-NMR, δppm | MS |
|---|---|---|---|
| 27 | 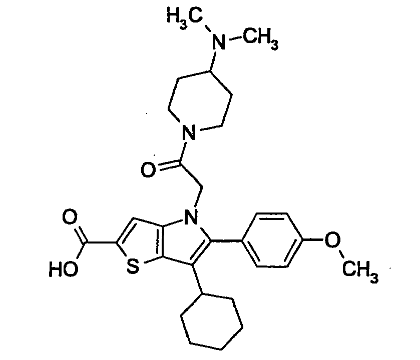 | 400MHz,DMSO-d6 7.69(1 H,s), 7.20(1 H,d,J=8.6Hz), 7.04(1 H,d,J=8.6Hz), 4.84(1H,d,J=17.4Hz), 4.77(1H,d,J=17.4Hz), 4.31-4.22(1H,m), 3.79(3H,s), 3.75-3.67(1H,m), 2.94-2.84(1H,m), 2.63-2.47(2H,m), 2.46-2.36(1H,m), 2.28(6H,s), 1.81-1.51(9H,m), 1.25-0.79(5H,m). | 524(M+1) |

**Table 7**

| Example | Formula | ¹H-NMR, δppm | MS |
|---|---|---|---|
| 29 | 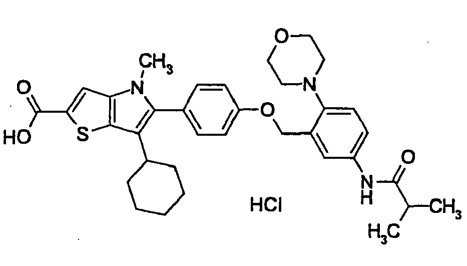 | 400MHz,DMSO-d6 12.60(1 H,brs), 9.80(1 H,s), 7.79(1 H,s), 7.73(1 H,d,J=2.5Hz), 7.58(1 H,dd,J=9.0,2.5Hz), 7.30(2H,d,J=8.6Hz), 7.13(1H,d,J=9.0Hz), 7.13(2H,d,J=8.6Hz), 5.18(2H,s), 3.73-3.66(4H,m), 3.51 (3H,s), 2.86-2.80(4H,m), 2.60-2.51(1 H,m), 2.45-2.37(1H,m), 1.76-1.48(7H,m), 1.24-1.12(3H,m), 1.06(6H,d,J=6.7Hz). | 616.2(M+1) |
| 30 | 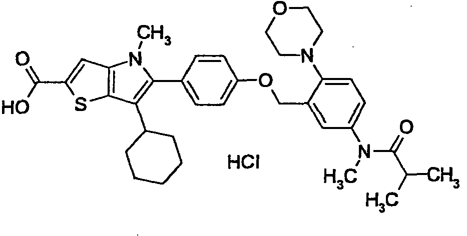 | 400MHz,DMSO-d6 12.67(1 H,brs), 7.78(1 H,s), 7.36-7.33(1H,m), 7.28(2H,d,J=8.8Hz), 7.26-7.21(2H,m), 7.06(2H,d,J=8.8Hz). 5.24(2H,s), 3.75-3.71(4H,m), 3.49(3H,s), 3.08(3H,s), 2.93-2.89(4H,m), 2.44-2.38(1H,m), 2.34-2.29(1H,m), 1.74-1.51(7H,m), 1.22-1.13(3H,m), 0.79(6H,d,J=6.7Hz). | 630.2(M+1) |
| 31 | 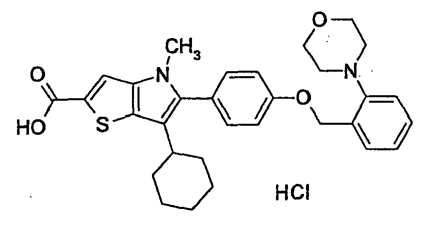 | 400MHz,DMSO-d6 12.68(1 H,brs), 7.79(1 H,s), 7.52-7.47(1H,m), 7.36-7.32(1H,m), 7.30(2H,d,J=8.8Hz), 7.20-7.16(1H,m), 7.15-7.10(1H,m), 7.13(2H,d,J=8.8Hz), 5.22(2H,s), 3.73-3.68(4H,m), 3.51(3H,s), 2.91-2.84(4H,m), 2.45-2.37(1 H,m), 1.75-1.49(7H,m), 1.22-1.13(3H,m). | 531.2(M+1) |

**Table 8**

| Example | Formula | ¹H-NMR, δppm | MS |
|---|---|---|---|
| 32 | 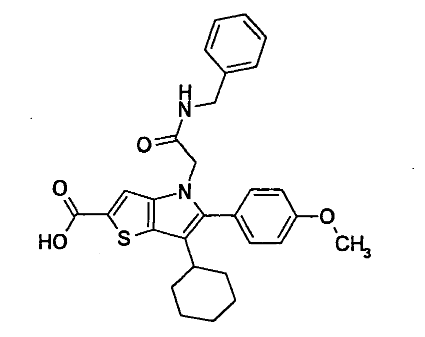 | 400MHz,DMSO-d6 12.61(1H,brs), 8.51(1H,t,J=6.0Hz), 7.75(1 H,s), 7.34-7.28(2H,m), 7.27-7.22(1 H,m), 7.24(2H,d,J=8.0Hz), 7.19-7.15(2H,m), 6.99(2H,d,J=8.0Hz), 4.58(2H,s), 4.25(2H,d,J=4.0Hz), 3.81(3H,s), 2.45-2.35(1H,m), 1.79-1.51(7H,m), 1.25-1.13(3H,m). | 503.1(M+1) |
| 33 | 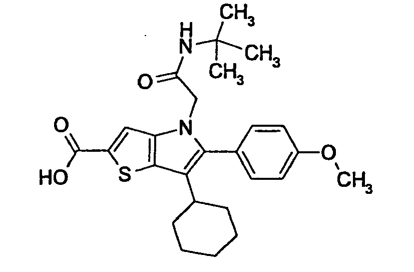 | 400MHz,DMSO-d6 12.58(1 H,brs), 7.69(1H,s), 7.67(1H,s), 7.26(2H,d,J=8.0Hz), 7.04(2H,d,J=8.0Hz), 4.42(2H,s), 3.81(3H,s), 2.45-2.36(1H,m), 1.79-1.51(7H,m), 1.28-1.12(3H,m), 1.22(9H,s). | 468.2(M+1) |
| 34 | 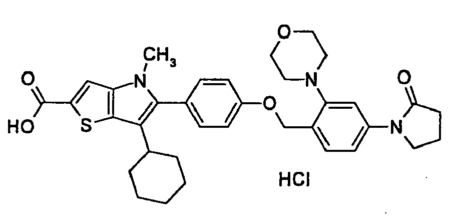 | 400MHz,DMSO-d6 12.57(1H,br), 7.78(1H,s), 7.59(1 H,d,J=2.0Hz), 7.46(1H,d,J=8.4Hz), 7.31-7.26(3H,m), 7.15-7.09(3H,m), 5.15(2H,s), 3.83(2H,t,J=7.0Hz), 3.72-3.68(4H,m), 3.51(3H,s), 2.90-2.85(4H,m), 2.41(1H,m), 2.09-1.99(2H,m), 1.75-1.48(7H,m), 1.24-1.12(3H,m). | 614.2(M+1) 614.2(M+1) |

**Table 9**

| Example | Formula | ¹H-NMR, δppm | MS |
|---|---|---|---|
| 35 | 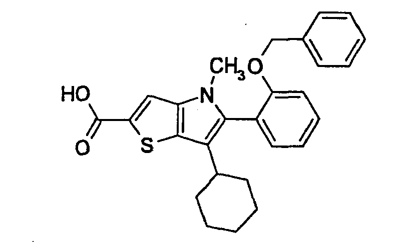 | 400MHz,DMSO-d6 7.78(1H,s), 7.45-7.41(1 H,m), 7.31-7.22(7H,m), 7.07(1H,t,J=7.6Hz), 5.15(2H,s), 3.44(3H,s), 2.35-2.26(1H,m), 1.78-1.44(7H,m), 1.25-1.06(3H,m). | 446.2(M+1) |
| 36 | 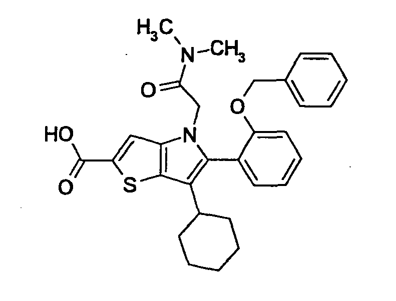 | 400MHz,DMSO-d6 7.49-7.44(1 H,m), 7.39-7.34(1 H,m), 7.30-7.24(5H,m), 7.17(1H,brd,J=8.4Hz), 7.12(1H,dd,J=72,1.6Hz), 7.00(1 H,t,J=7.2Hz), 5.12(2H,s), 4.87(1H,d,J=17.2Hz), 4.40(1H,d,J=16.8Hz), 2.75(3H,s), 2.71 (3H,s), 2.34-2.24(1H,m), 1.75-1.45(7H,m), 1.21-1.09(3H,m). | 517.2(M+1) |
| 37 | 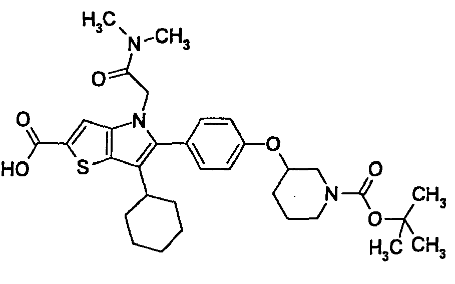 | 400MHz,DMSO-d6 12.60(1H,brs), 7.70(1H,s), 7.20(2H,d,J=12.0Hz), 7.04(2H,d,J=8.0Hz), 4.77(2H,s), 4.52-4.40(1H,m), 3.87-3.74(1 H,m), 3.66-3.53(1 H,m), 3.45-3.02(2H,m), 2.88(3H,s), 2.80(3H,s), 2.48-2.37(1 H,m), 2.01-1.07(23H,m). | 610.3(M+1) |

**Table 10**

| Example | Formula | ¹H-NMR, δppm | MS |
|---|---|---|---|
| 38 | 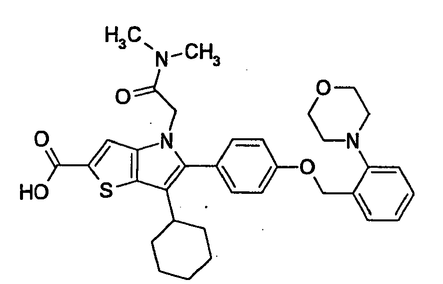 | 400MHz,DMSO-d6 12.63(1 H,brs), 7.69(1H,s), 7.51(1H,dd,J=1.4,8.0Hz), 7.35(1 H,dt,J=1.6,8.0Hz), 7.23-7.18(1H,m), 7.21(2H,d,J=8.0Hz), 7.17-7.10(1H,m), 7.12(2H,d,J=8.0Hz), 5.19(2H,s), 4.76(2H,s), 3.73-3.68(4H,m), 2.92-2.87(4H,m), 2.84(3H,s), 2.78(3H,s), 2.45-2.36(1H,m), 1.78-1.50(7H,m), 1.28-1.12(3H,m). | 602.2(M+1) 602.2(M+1) |
| 39 | 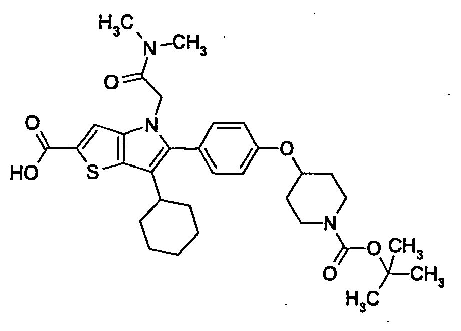 | 400MHz,DMSO-d6 12.59(1H,brs), 7.70(1H,s), 7.19(2H,d,J=8.0Hz), 7.07(2H,d,J=8.0Hz), 4.77(2H,brs), 4.67-4.56(1H,m), 3.76-3.66(2H,m), 3.24-3.11(2H,m), 2.88(3H,s), 2.80(3H,s), 2.47-2.36(1H,m), 2.02-1.90(2H,m), 1.80-1.48(9H,m), 1.41(9H,s), 1.29-1.13(5H,m). | 610.3(M+1) |
| 40 | 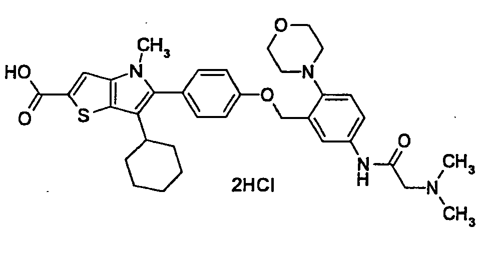 | 400MHz,DMSO-d6 10.81(1H,s), 9.97(1H,brs), 7.82(1H,s), 7.76(1 H,d,J=2.8Hz), 7.62(1 H,dd,J=8.4,2.4Hz), 7.33(2H,d,J=8.4Hz), 7.22(1H,d,J=8.4Hz), 7.14(2H,d,J=8.8Hz), 5.22(2H,s), 4.14(2H,d,J=4.8Hz), 3.74-3.72(4H,m), 3.53(3H,s), 2.90-2.85(4H,m), 2.87(3H,s), 2.86(3H,s), 2.48-2.40(1 H,m), 1.76-1.53(7H,m), 1.25-1.16(3H,m). | 631.2(M+1) |

**Table 11**

| Example | Formula | ¹H-NMR, δppm | MS |
|---|---|---|---|
| 41 | 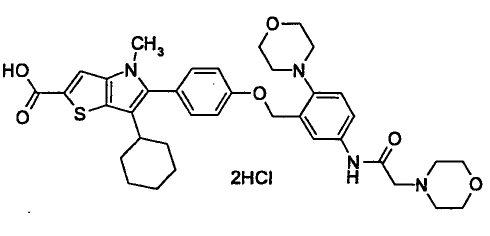 | 400MHz,DMSO-d6 10.78(1H,s), 10.52(1 H,brs), 7.82(1H,s), 7.75(1H,d,J=2.0Hz), 7.61(1H,dd,J=8.4,2.4Hz), 7.33(2H,d,J=8.4Hz), 7.22(1H,d,J=8.8Hz), 7.14(2H,d,J=8.8Hz), 5.22(2H,s), 4.20(2H,s), 3.96-3.91(2H,m), 3.83-3.79(2H,m), 3.76-3.71(4H,m), 3.53(3H,s), 3.50-3.43(2H,m), 3.33-3.23(2H,m), 2.90-2.85(4H,m), 2.48-2.38(1H,m), 1.78-1.52(7H,m), 1.25-1.16(3H,m). | 673.2(M+1) |
| 42 | 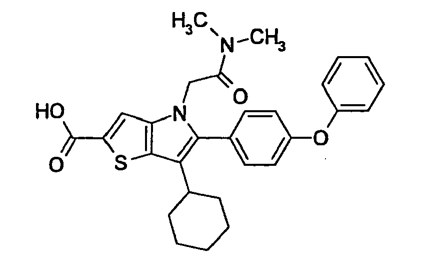 | 400MHz,DMSO-d6 12.64(1H,brs), 7.73(1 H,s), 7.47-7.41(2H,m), 7.31-7.26(2H,m), 7.20(1 H,t,J=7.4Hz), 7.13-7.06(4H,m), 4.81(2H,s), 2.89(3H,s), 2.79(3H,s), 2.48-2.40(1H,m), 1.79-1.53(7H,m), 1.29-1.15(3H,m). | 503.1(M+1) |
| 43 | 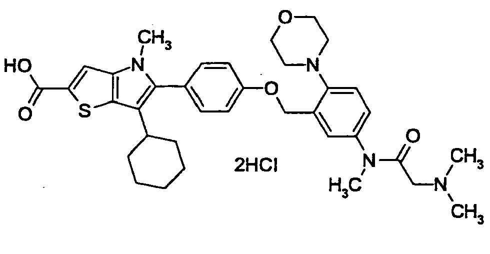 | 400MHz,DMSO-d6 9.52(1H,brs), 7.82(1H,s), 7.56(1H,d,J=2.8Hz), 7.40(1H,dd,J=8.4,2.4Hz), 7.35(2H,d,J=8.4Hz), 7.30(1H,d,J=8.4Hz), 7.20(2H,d,J=8.8Hz), 5.22(2H,s), 3.91-3.89(2H,m), 3.76-3.72(4H,m), 3.54(3H,s), 3.22(3H,s), 2.97-2.92(4H,m), 2.74(3H,brs), 2.73(3H,brs), 2.47-2.39(1 H,m), 1.77-1.51(7H,m), 1.24-1.15(3H,m). | 645.2(M+1) |

**Table 12**

| Example | Formula | ¹H-NMR, δppm | MS |
|---|---|---|---|
| 44 | 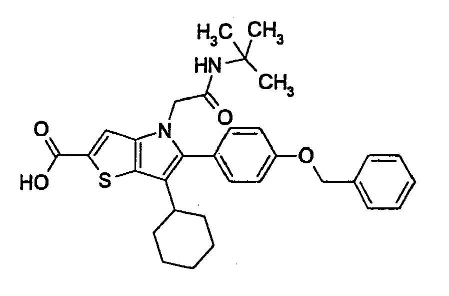 | 400MHz,DMSO-d6 12.62(1 H,brs), 7.67(1 H,s), 7.66(1 H,s), 7.49-7.44(2H,m), 7.42-7.36(2H,m), 7.35-7.30(1H,m), 7.24(2H,d,J=8.8Hz), 7.10(2H,d,J=8.8Hz), 5.13(2H,s), 4.40(2H,s), 2.44-2.33(1H,m), 1.76-1.49(7H,m), 123-1.11(3H,m), 1.19(9H,s). | 545.2(M+1) |
| 45 | 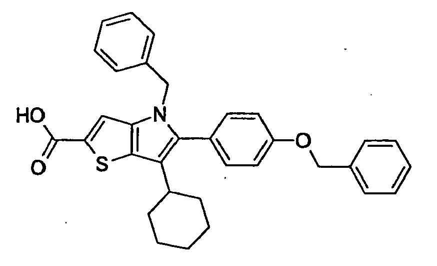 | 400MHz,DMSO-d6 12.67(1 H,brs), 7.57(1 H,s), 7.49-7.46(2H,m), 7.42-7.32(3H,m), 7.28-7.17(5H,m), 7.13-7.08(2H,m), 6.87(2H,d,J=6.8Hz), 5.15(3H,s), 5.13(3H,s), 2.46-2.38(1H,m), 1.77-1.69(4H,m), 1.67-1.51 (3H,m), 1.25-1.14(3H,m). | 522.1(M+1) |
| 46 | 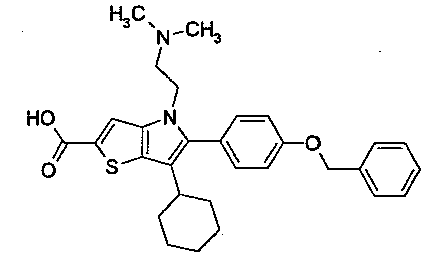 | 400MHz,DMSO-d6 12.59(1 H,brs), 7.74(1 H,s), 7.49(2H,d,J=8.0Hz), 7.41(2H,t,J=7.2Hz), 7.35(1H,d,J=6.8Hz), 7.31(2H,d,J=8.8Hz), 7.15(2H,d,J=8.4Hz), 5.17(2H,s), 3.99-3.94(2H,m), 2.43-2.30(3H,m), 1.75-1.50(7H,m), 1.24-1.11(3H,m). | 503.2(M+1) |

**Table 13**

| Example | Formula | ¹H-NMR, δppm | MS |
|---|---|---|---|
| 47 | 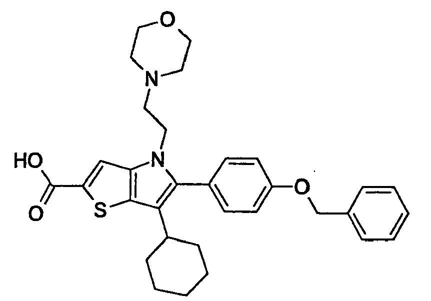 | 400MHz,DMSO-d6 7.52(1 H,s), 7.49(2H,d,J=6.8Hz), 7.41(2H,t,J=7.2Hz), 7.35(1H,d,J=7.2Hz), 7.30(2H,d,J=8.8Hz), 7.13(2H,d,J=8.8Hz), 5.16(1H,s), 3.97-3.92(2H,m), 3.45-3.41(4H,m), 2.44-2.31(3H,m), 2.20-2.15(4H,m), 1.74-1.50(7H,m), 125-1.12(3H,m). | 5452(M+1) |
| 48 | 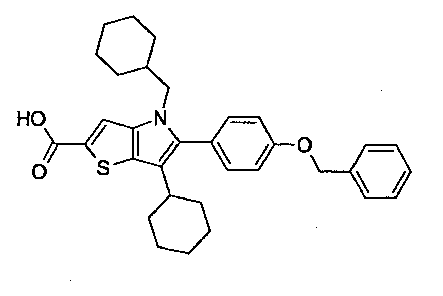 | 400MHz,DMSO-d6 12.65(1H,brs), 7.76(1 H,s), 7.48(2H,brd,J=7.2Hz), 7.40(2H,t,J=7.0Hz), 7.34(1 H,d,J=7.2Hz), 7.26(2H,d,J=8.8Hz), 7.13(2H,d,J=8.8Hz), 5.14(2H,s), 3.77(2H,d,J=7.6Hz), 2.40-2.29(1H,m), 1.72-1.60(5H,m), 1.56-1.39(6H,m), 1.30-1.22(2H,m), 1.19-1.10(3H,m), 1.02-0.92(3H,m), 0.71-0.58(2H,m). | 528.0(M+1) |
| 49 | 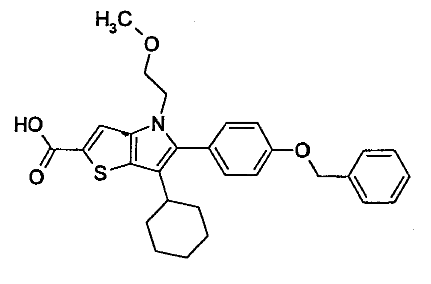 | 400MHz,DMSO-d6 7.51-7.48(3H,m), 7.41(2H,t,J=7.2Hz), 7.36(1 H,d,J=7.2Hz), 7.29(2H,d,J=8.8Hz), 7.13(2H,d,J=8.8Hz), 5.15(2H,s), 4.01-3.96(2H,m), 3.46-3.41(2H,m), 3.09(3H,s), 2.40-2.31 (1 H,m), 1.75-1.51(7H,m), 1.24-1.11(3H,m). | 490.1(M+1) |

**Table 14**

| Example | Formula | ¹H-NMR, δppm | MS |
|---|---|---|---|
| 50 | 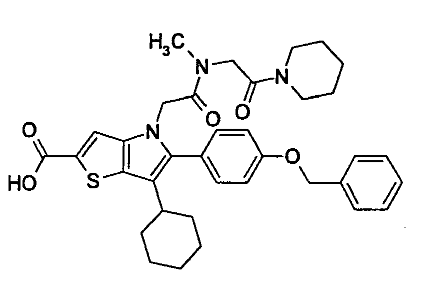 | 400MHz,DMSO-d6 12.59(1 H,brs), 7.61and7.53(1H,s), 7.49-7.44(2H,m), 7.42-7.36(2H,m), 7.35-7.30(1H,m), 7.22and7.20(2H,d,J=8.8Hz), 7.10(2H,d,J=8.8Hz), 5.12and5.11(2H,s), 4.81and4.53(2H,s), 4.25and4.11(2H,s), 3.43-3.35(2H,m), 3.33-3.22(2H,m), 2.88and2.76(3H,s), 2.45-2.37(1H,m), 1.78-1.34(13H,m), 1.25-1.13(3H,m). | 628.2(M+1) |
| 51 | 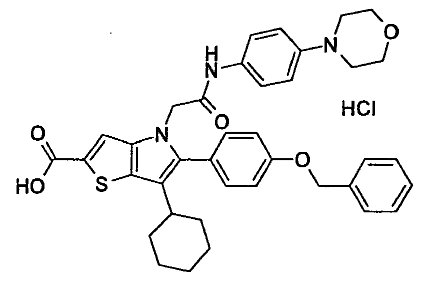 | 400MHz,DMSO-d6 12.66(1 H,brs), 10.11(1 H,brs), 7.74(1 H,s), 7.46-7.40(4H,m), 7.39-7.33(2H,m), 7.33-7.27(1H,m), 7.25(2H,d,J=8.8Hz), 7.18-7.10(2H,m), 7.08(2H,d,J=8.8Hz), 5.08(2H,s),4.68(2H,s), 3.82-3.75(4H,m), 3.20-3.12(4H,m), 2.43-2.33(1H,m), 1.74-1.49(7H,m), 1.22-1.13(3H,m). | 650.3(M+1) |
| 52 | 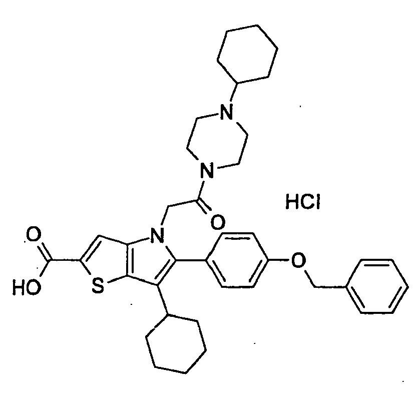 | 400MHz,DMSO-d6 12.57(1 H,brs), 10.86(1H,brs),7.73(1H,s), 7.47-7.43(2H,m), 7.41-7.35(2H,m), 7.35-7.29(1H,m), 7.18(2H,d,J=8.8Hz), 7.11(2H,d,J=8.8Hz), 5.11 (2H,s), 4.91(1H,d,J=18Hz), 4.79(1H,d,J=18Hz), 4.32(1H,d,J=14Hz), 3.90(1H,d,J=14Hz), 3.56-3.44(1H,m), 3.43-3.30(2H,m), 3.17-3.05(2H,m), 2.87-2.73(2H,m), 2.43-2.33(1H,m), 2.18-2.00(2H,m), 1.81-1.48(10H,m), 1.43-0.96(8H,m). | 640.3(M+1) |

**Table 15**

| Example | Formula | ¹H-NMR, δppm | MS |
|---|---|---|---|
| 53 | 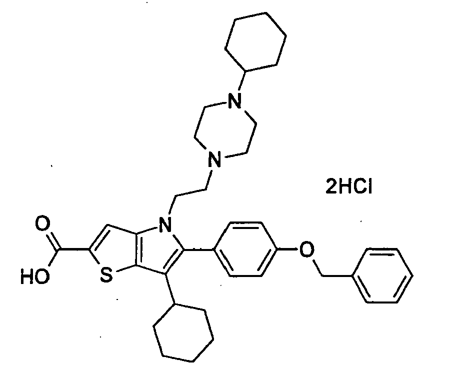 | 400MHz,DMSO-d6 12.76(1H,brs), 7.96(1H,s), 7.49-7.45(2H,m), 7.42-7.37(2H,m), 7.36-7.31(1H,m), 7.33(2H,d,J=8.8Hz), 7.15(2H,d,J=8.8Hz), 5.15(2H,s), 4.37-4.09(4H,m), 3.57-2.92(9H,m), 2.41-2.27(1H,m), 2.08-1.97(2H,m), 1.84-1.74(2H,m), 1.73-1.47(8H,m), 1.42-1.29(2H,m), 1.28-1.00(6H,m). | 626.4(M+1) |
| 54 | 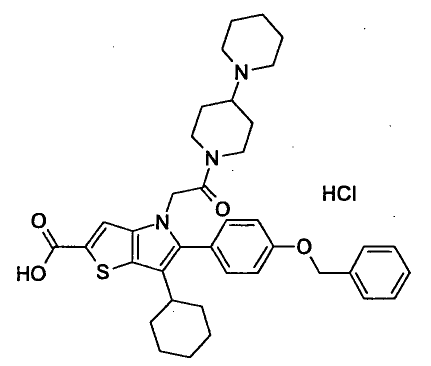 | 400MHz,DMSO-d6 12.51(1H,brs), 10.15(1H,brs), 7.70(1H,s), 7.48-7.44(2H,m), 7.42-7.37(2H,m), 7.36-7.31 (1H,m), 7.19(2H,d,J=8.8Hz). 7.14(2H,d,J=8.8Hz), 5.12(2H,s),4.82(2H,s), 4.37(1H,d,J=12Hz), 3.83(1H,d,J=12Hz), 3.38-3.18(2H,m), 2.97-2.73(4H,m), 2.57-2.47(1H,m), 2.44-2.33(1H,m), 2.08-1.96(2H,m), 1.84-1.48(12H,m), 1.41-1.09(6H,m). | 640.3(M+1) |
| 55 | 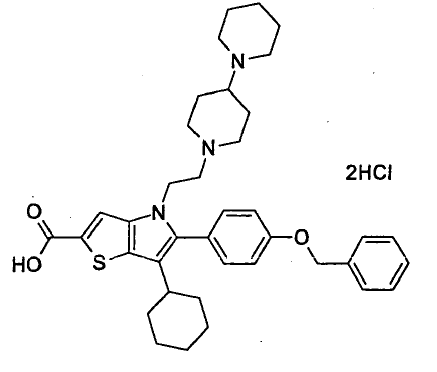 | 400MHz,DMSO-d6 12.76(1 H,brs), 10.92(1 H,brs), 10.75(1H,brs), 8.00(1H,s), 7.49-7.46(2H,m), 7.43-7.38(2H,m), 7.37-7.33(1 H,m), 7.33(2H,d,J=8.6Hz), 7.16(2H,d,J=8.6Hz), 5.14(2H,s), 4.36-4.27(2H,m), 3.54-3.14(6H,m), 2.97-2.79(4H,m), 2.40-2.31(1H,m), 2.30-2.21(1H,m), 2.10-1.96(2H,m), 1.86-1.47(12H,m), 1.43-1.08(6H,m). | 626.3(M+1) |

**Table 16**

| Example | Formula | ¹H-NMR, δppm | MS |
|---|---|---|---|
| 56 | 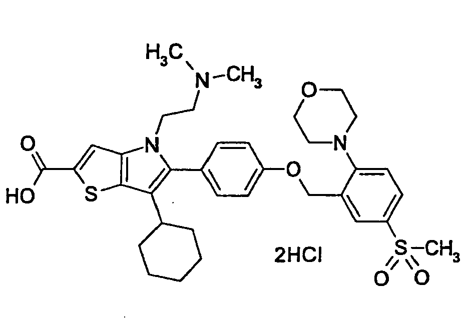 | 300MHz,DMSO-d6 12.76(1 H,brs), 10.36(1H,brs), 8.04(1 H,d,J=2.6Hz), 8.03(1 H,s), 7.90(1 H,dd,J=8.8,2.6Hz), 7.41 (1 H,d,J=8.8Hz), 7.38(2H,d,J=8.8Hz), 7.22(2H,d,J=8.8Hz), 5.28(2H,s), 4.35-4.23(2H,m), 3.81-3.74(4H,m), 3.28-3.17(2H,m), 3.20(3H,s), 3.07-2.99(4H,m), 2.64(6H,s), 2.46-2.33(1H,m), 1.82-1.46(7H,m), 1.29-1.09(3H,m). | 666.2(M+1) |
| 57 | 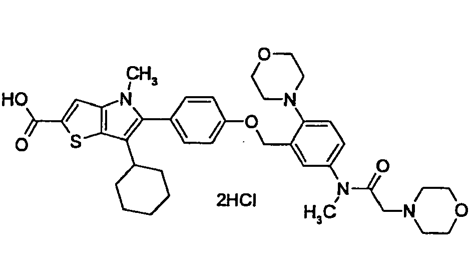 | 400MHz,DMSO-d6 10.11(1H,brs),7.82(1 H,s), 7.57(1 H,d,J=2.4Hz), 7.41 (1 H,dd,J=8.4,2.4Hz), 7.36(2H,d,J=8.8Hz), 7.30(1 H,d,J=8.8Hz), 7.20(2H,d,J=8.4Hz), 5.22(2H,s), 4.00-3.97(2H,m), 3.87-3.85(2H,m), 3.76-3.72(4H,m), 3.55-3.53(5H,m), 3.40-3.31(2H,m), 3.22(3H,s), 3.16-3.08(2H,m), 2.97-2.93(4H,m), 2.45-2.38(1 H,m), 1.77-1.52(7H,m), 1.23-1.15(3H,m). | 687.2(M+1) |
| 58 | 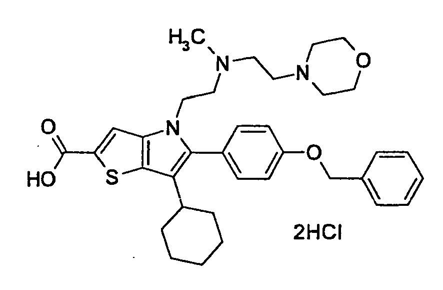 | 400MHz,DMSO-d6 12.73(1H,brs),11.09(1H,brs), 10.65(1H,brs),7.93(1 H,s), 7.49-7.44(2H,m), 7.43-7.37(2H,m), 7.37-7.32(1H, m), 7.16(2H,d,J=8.8Hz), 7.11(2H,d,J=8.8Hz), 5.15(2H,s), 4.37-4.20(2H,m), 3.97-3.29(10H,m), 3.25-3.16(2H,m), 3.09-2.95(2H,m), 2.99(3H,s), 2.44-2.28(1H,m), 1.75-1.45(7H,m), 1.23-1.07(3H,m). | 602.2(M+1) |

**Table 17**

| Example | Formula | ¹H-NMR, δppm | MS |
|---|---|---|---|
| 59 | 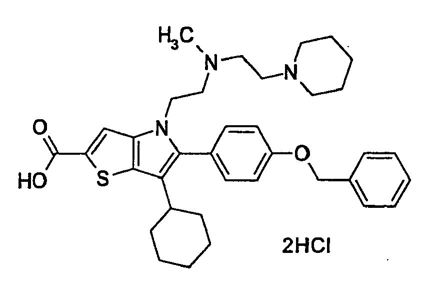 | 400MHz,DMSO-d6 12.72(1 H,brs), 11.21(1H,brs), 10.67(1 H,brs), 8.03(1 H,s), 7.49-7.44(2H,m), 7.43-7.39(2H,m), 7.38-7.32(1H,m), 7.36(2H,d,J=8.8Hz), 7.16(2H,d,J=8.8Hz), 5.15(2H,s), 4.44-4.21(2H,m), 3.67-3.12(6H,m), 2.99(3H,s), 2.91-2.55(4H,m), 2.43-2.31(1 H,m), 1.81-1.47(13H,m), 1.23-1.08(3H,m). | 600.3(M+1) |
| 60 | 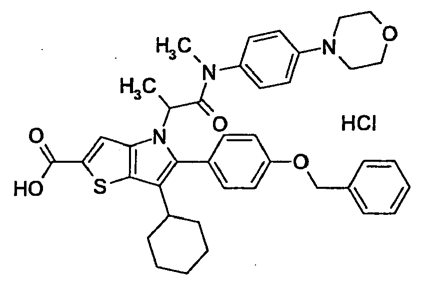 | 400MHz,DMSO-d6 12.60(1 H,brs), 7.77(1H,s), 7.52-7.47(2H,m), 7.44-7.38(2H,m), 7.37-7.31 (1H, m), 7.11(2H,d,J=8.8Hz), 7.03-6.94(2H,m), 6.85(2H,d,J=8.8Hz), 6.71-6.61(2H,m), 5.14(2H,s), 4.74(1 H,q,J=7.0Hz), 3.64-3.58(4H,m), 3.05-3.00(4H,m), 2.98(3H,s), 2.21-2.11(1 H,m), 1.73-1.46(7H,m), 1.42(3H,d,J=7.0Hz), 1.25-1.01(3H,m). | 678.3(M+1) |

The evaluation of the HCV polymerase inhibitory activity of the compound of the present invention is explained in the following. This polymerase is an enzyme coded for by the non-structural protein region called NS5B on the genome RNA of HCV (EMBO J., 15:12-22, 1996).

### Experimental Example [I]

### i) Preparation of enzyme (HCV polymerase)

Using, as a template, a cDNA clone corresponding to the full length genome RNA of HCV BK strain obtained from the blood of a patient with hepatitis C, a region encoding NS5B (J Virol 1991 Mar, 65(3), 1105-13, 544 amino acids after deletion of 47 amino acids on the C-terminal) was amplified by PCR. The objective gene was prepared by adding a 6 His tag {base pair encoding 6 continuous histidine (His)} to the 3' end thereof and transformed to *Escherichia coli.* The *Escherichia coli* capable of producing the objective protein was cultured. The obtained cells were suspended in a buffer solution and crushed in a microfluidizer. The supernatant was obtained by centrifugation and applied to various column chromatographys {mono-S, Sephacryl S-200 (Pharmacia)}, inclusive of metal chelate chromatography, to give a standard enzyme product.

### ii) Synthesis of substrate RNA

Using a synthetic primer designed based on the sequence of HCV genomic 3' untranslated region, a DNA fragment (148 bp) containing polyU and 3'X sequence was entirely synthesized and cloned into plasmid pBluescript SK II(+) (Stratagene). The cDNA encoding full length NS5B, which was prepared in i) above, was digested with restriction enzyme KpnI to give a cDNA fragment containing the nucleotide sequence of from the restriction enzyme cleavage site to the termination codon. This cDNA fragment was inserted into the upstream of 3' untranslated region of the DNA in pBluescript SK II(+) and ligated. The about 450 bp inserted DNA sequence was used as a template in the preparation of substrate RNA. This plasmid was cleaved immediately after the 3'X sequence, linearized and purified by phenol-chloroform treatment and ethanol precipitation to give DNA.

RNA was synthesized (37°C, 3 hr) by run-off method using this purified DNA as a template, a promoter of pBluescript SK II(+), MEGAscript RNA synthesis kit (Ambion) and T7 RNA polymerase. DNase I was added and the mixture was incubated for 1 hr. The template DNA was removed by decomposition to give a crude RNA product. This crude product was treated with phenol-chloroform and purified by ethanol precipitation to give the objective substrate RNA.

This RNA was applied to formaldehyde denaturation agarose gel electrophoresis to confirm the quality thereof and preserved at -80°C.

### iii) Assay of enzyme (HCV polymerase) inhibitory activity

A test substance (compound of the present invention) and a reaction mixture (30 µl) having the following composition were reacted at 25°C for 90 min.

10% Trichloroacetic acid at 4°C and 1% sodium pyrophosphate solution (150 µl) were added to this reaction mixture to stop the reaction. The reaction mixture was left standing in ice for 15 min to insolubilize RNA. This RNA was trapped on a glass filter (Whatman GF/C and the like) upon filtration by suction. This filter was washed with a solution containing 1% trichloroacetic acid and 0.1% sodium pyrophosphate, washed with 90% ethanol and dried. A liquid scintillation cocktail (Packard) was added and the radioactivity of RNA synthesized by the enzyme reaction was measured on a liquid scintillation counter.

The HCV polymerase inhibitory activity (IC₅₀) of the compound of the present invention was calculated from the values of radioactivity of the enzyme reaction with and without the test substance.

The results are shown in Table 18, wherein each symbol means that IC₅₀ falls within the following range.
A: 0.1 µM ≤ IC₅₀ < 1 µM
B: IC₅₀ < 0.1 µM

Reaction mixture: HCV polymerase (0.5 µg/ml) obtained in i), substrate RNA (5 µg/ml) obtained in ii), ATP (50 µM), GTP (50 µM), CTP (50 µM), UTP (2 µM), [5,6-³H]UTP (46 Ci/mmol (Amersham), 1 µCi) 20 mM Tris-HCl (pH 7.5), EDTA (1 mM), MgCl₂ (5 mM), NaCl (50 mM), DTT (1 mM), BSA (0.01%)

**Table 18**

| Example | H544 1a 3X IC₅₀ | H544 1b IC₅₀ | 3X Example | H544 1a 3X IC₅₀ | H544 1b 3X IC₅₀ |
|---|---|---|---|---|---|
| 3 | A | B | 38 | B | B |
| 5 | A | A | 39 | A | B |
| 12 | A | A | 40 | B | B |
| 14 | B | B | 41 | B | B |
| 15 | B | B | 42 | B | B |
| 17 | B | B | 43 | B | B |
| 18 | B | B | 44 | A | A |
| 19 | B | B | 45 | A | A |
| 20 | B | B | 46 | A | A |
| 21 | A | B | 47 | A | A |
| 23 | B | B | 48 | A | A |
| 24 | A | A | 49 | A | A |
| 25 | A | B | 50 | B | B |
| 26 | A | B | 51 | B | B |
| 27 | B | B | 52 | B | B |
| 29 | B | B | 53 | A | A |
| 30 | B | B | 54 | B | B |
| 31 | B | B | 55 | A | A |
| 32 | B | B | 56 | B | B |
| 33 | B | A | 57 | B | B |
| 34 | B | B | 58 | A | A |
| 35 | A | A | 59 | A | B |
| 36 | A | A | 60 | | A |
| 37 | B | B | | | |

### Experimental Example [II]

The test compound was dissolved in DMSO (dimethyl sulfoxide; final concentration 0.5%), and adjusted to a 10-fold concentration of the final concentration with a medium.

Replicon cells (Huh-5-2: manufactured by ReBLikon GmbH) were inoculated on a medium at 5×10³/90 µl/well in a 96-well plate.

The medium was changed to a 4% HSA (human serum albumin)-containing medium (90 µl) the next day, and 10 µl of the above-mentioned adjusted product at each concentration was added.

At 48 hr later, luciferase activity was measured with Steady-Glo (manufactured by PROMEGA). The inhibitory rate relative to the control group (0.5% DMSO addition group) was calculated and EC₅₀ value was determined by proportional calculation, based on the data of two points across 50%, with the concentration of the compound taken as logarithm.

Composition of medium: Dulbecco's modified Eagle's medium (DMEM), 10% fetal bovine serum (FBS), 2 mM L-glutamine, 0.1 mM MEM non-essential amino acid, 100 U/ml penicillin, 0.1 mg/ml streptomycin

As in the test, one showing high replication inhibitory, or HCV polymerase inhibitory activity in the presence of a protein is one of the preferable embodiments.

As is evident from the above-mentioned results, the compound of the present invention shows a high inhibitory activity against HCV polymerase.

Therefore, the compound of the present invention can provide a pharmaceutical agent effective for the prophylaxis or treatment of hepatitis C, based on the anti-HCV effect afforded by the HCV polymerase inhibitory activity. When used concurrently with a different anti-HCV agent, such as interferon, and/or an anti-inflammatory agent and the like, it can provide a pharmaceutical agent more effective for the prophylaxis or treatment of hepatitis C. Its high inhibitory activity specific to HCV polymerase suggests the possibility of the compound being a pharmaceutical agent with slight side effects, which can be used safely for humans.

Formulation Example is given in the following. This example is merely for the purpose of exemplification and does not limit the invention.

### Formulation Example

| | | |
|---|---|---|
| (a) | compound of Example 3 | 10 g |
| (b) | lactose | 50 g |
| (c) | corn starch | 15 g |
| (d) | sodium carboxymethylcellulose | 44 g |
| (e) | magnesium stearate | 1 g |

The entire amounts of (a), (b) and (c) and 30 g of (d) are kneaded with water, dried in vacuo and granulated. The obtained granules are mixed with 14 g of (d) and 1 g of (e) and processed into tablets with a tableting machine to give 1000 tablets each containing 10 mg of (a).

This application is based on a patent application No. 2003-389976 filed in Japan, the contents of which are hereby incorporated by reference.

## Claims

1. A fused ring compound represented by the following formula
[I] or a pharmaceutically acceptable salt thereof: 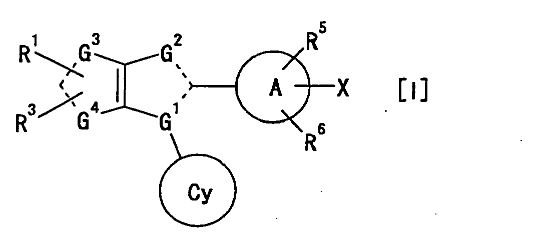
wherein 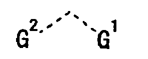 is G²'=C-G¹ or G²-C=G¹', 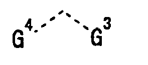 is G⁴'=C-G³ or G⁴-C=G³',
G¹ is a carbon atom or a nitrogen atom,
G¹' is a carbon atom,
G², G³ and G⁴ are each independently an oxygen atom, a sulfur atom, a nitrogen atom or a carbon atom,
G²', G³' and G⁴' are each independently a nitrogen atom or a carbon atom,
provided that when G², G³ and G⁴ are all carbon atoms, G¹ is a nitrogen atom,
when G² is a nitrogen atom or a carbon atom, G² is optionally substituted by the following R²,
R¹ and R³ optionally substitute any atom of 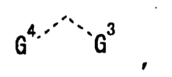 provided that when G³ is an oxygen atom or a sulfur atom, G³ is unsubstituted, and when G⁴ is an oxygen atom or a sulfur atom, G⁴ is unsubstituted,
R¹ is
(1) a carboxyl group,
(2) a carboxylic acid equivalent,
(3) -CONR¹¹R¹²
(wherein R¹¹ and R¹² are each independently
(1') a hydrogen atom,
(2') a C₁₋₆ alkyl group optionally substituted by 1 to 3 substituents selected from the following group E,
(3') a C₂₋₆ alkenyl group optionally substituted by 1 to 3 substituents selected from the following group E,
(4') a C₆₋₁₄ aryl group optionally substituted by 1 to 5 substituents selected from the following group E,
(5') a heterocyclic group optionally substituted by 1 to 5 substituents selected from the following group E or
(6') a C₃₋₈ cycloalkyl group optionally substituted by 1 to 5 substituents selected from the following group E) or
(4) -COOR¹⁰³
(wherein R¹⁰³ is a group selected from the following group C or a glucuronic acid residue),
R² is
(1) a hydrogen atom,
(2) a group selected from the following group E,
(3) a C₁₋₆ alkyl group optionally substituted by 1 to 3 substituents selected from the following group E,
(4) a C₂₋₆ alkenyl group optionally substituted by 1 to 3 substituents selected from the following group E,
(5) 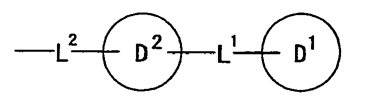
(6) 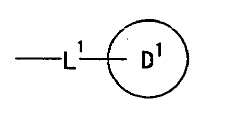
(7) 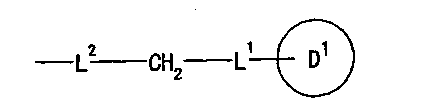 or
(8) 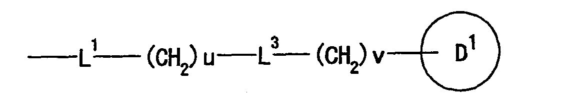 {wherein u and v are each independently 0 or an integer of 1 to 6,
L¹ and L² are each independently
(1') a bond,
(2') C₁₋₆ alkylene,
(3') C₂₋₆ alkenylene,
(4') -(CHR^{L4})ᵤ₁-O-(CHR^{L5})ᵥ₁-,
(5') -(CHR^{L4})ᵤ₁-S-(CHR^{L5})ᵥ₁-,
(6') -(CHR^{L4})ᵤ₁-NR^{L1}-(CHR^{L5})ᵥ₁-,
(7') -(CHR^{L4})ᵤ₁-CO-(CHR^{L5})ᵥ₁-,
(8') -(CHR^{L4})ᵤ₁-CONR^{L2}-(CHR^{L5})ᵥ₁-,
(9') -(CHR^{L4})ᵤ₁-NR^{L2}CO₂-(CHR^{L5})ᵥ₁-,
(10') -(CHR^{L4})ᵤ₁-NR^{L2}CONR^{L3}-(CHR^{L5})ᵥ₁-,
(11') -(CHR^{L4})ᵤ₁-NR^{L2}CO-(CHR^{L5})ᵥ₁-,
(12') -(CHR^{L4})ᵤ₁-NR^{L2}SO₂-(CHR^{L5})ᵥ₁-,
(13') -(CHR^{L4})ᵤ₁-SO₂-(CHR^{L5})ᵥ₁- or
(14') -(CHR^{L4})ᵤ₁-SO₂NR^{L2}-(CHR^{L5})ᵥ₁-
(wherein u1 and v1 are each independently 0 or an integer of 1 to 6,
R^{L1} i s
(1") a hydrogen atom,
(2") a group selected from the following group C,
(3") -COR^{L11},
(4") -CONR^{L11}R^{L12},
(5") -COOR^{L11} or
(6") -SO₂R^{L13}
(wherein R^{L11} and R^{L12} are each independently a hydrogen atom or a group selected from the following group C, and R^{L13} is a group selected from the following group C),
R^{L2} and R^{L3} are each independently
(1") a hydrogen atom,
(2") a group selected from the following group C,
(3") -COR^{L11} or
(4") -SO₂R^{L13}
(wherein R^{L11} and R^{L13} are as defined above) ,
R^{L4} and R^{L5} are each independently a hydrogen atom or a C₁₋₆ alkyl group),
L³ is
(1') -CHR^{L14}- or
(2') -NR^{L14}-
(wherein R^{L14} is a group selected from the following group F), ring D¹ and ring D² are each independently
(1') a C₆₋₁₄ aryl group optionally substituted by 1 to 5 substituents selected from the following group E,
(2') a C₃₋₈ cycloalkyl group optionally substituted by 1 to 5 substituents selected from the following group E or
(3') a heterocyclic group optionally substituted by 1 to 5 substituents selected from the following group E
(wherein said heterocyclic group comprises 1 to 4 heteroatoms selected from oxygen atom, nitrogen atom and sulfur atom)},
R³ is independently a group selected from the following (1) to (20) :
(1) a hydrogen atom,
(2) a halogen atom,
(3) a C₁₋₆ alkanoyl group,
(4) a carboxyl group,
(5) a cyano group,
(6) a nitro group,
(7) a C₁₋₆ alkyl group optionally substituted by 1 to 3 substituents selected from the following group A,
(8) -OR¹⁰¹
(wherein R¹⁰¹ is a hydrogen atom or a group selected from the following group C),
(9) -NR¹⁰²R¹¹⁹
(wherein R¹⁰² and R¹¹⁹ are each independently a hydrogen atom, a C₁₋₆ alkanoyl group or a C₁₋₆ alkylsulfonyl group) ,
(10) -COOR¹⁰³
(wherein R¹⁰³ is a group selected from the following group C or a glucuronic acid residue),
(11) -CONR¹⁰⁴R¹⁰⁵
(wherein R¹⁰⁴ and R¹⁰⁵ are each independently a hydrogen atom, a hydroxyl group, a cyano group, a C₁₋₆ alkoxy group or a C₁₋₆ alkyl group optionally substituted by 1 to 3 substituents selected from the following group A),
(12) -SO₂R¹⁰⁶
(wherein R¹⁰⁶ is a hydroxyl group, an amino group, a C₁₋₆ alkyl group or a C₁₋₆ alkylamino group),
(13) -NHCOR¹⁰⁷
(wherein R¹⁰⁷ is an amino group or a C₁₋₆ alkylamino group) ,
(14) -C (=NR¹⁰⁸) -NH₂
(wherein R¹⁰⁸ is a hydrogen atom, a C₁₋₆ alkyl group optionally substituted by 1 to 3 substituents selected from the following group A, a hydroxyl group or a C₁₋₆ alkoxy group),
(15) -P (=O) (OR¹⁰⁹)₂
(wherein each R¹⁰⁹ is independently a hydrogen atom or a group selected from the following group C),
(16) -P(=O)(OR¹¹⁰)NR¹¹¹R¹¹²
(wherein R¹¹⁰, R¹¹¹ and R¹¹² are each independently a hydrogen atom or a group selected from the following group C),
(17) -CONHCO-R¹¹³
(wherein R¹¹³ is a group selected from the following group C),
(18) -CONHSO₂-R¹¹⁴
(wherein R¹¹⁴ is a group selected from the following group C),
(19) -SO₂NHCO-R¹¹⁵
(wherein R¹¹⁵ is a group selected from the following group C) or (20) a heterocyclic group optionally substituted by 1 to 5 substituents selected from the following group B
(wherein said heterocyclic group comprises 1 to 4 heteroatoms selected from oxygen atom, nitrogen atom and sulfur atom), ring Cy is
(1) a C₃₋₈ cycloalkyl group optionally substituted by 1 to 5 substituents selected from the following group B,
(2) a C₃₋₈ cycloalkenyl group optionally substituted by 1 to 5 substituents selected from the following group B or
(3) a heterocyclic group optionally substituted by 1 to 5 substituents selected from the following group B
(wherein said heterocyclic group comprises 1 to 4 heteroatoms selected from oxygen atom, nitrogen atom and sulfur atom), ring A is
(1) a C₆₋₁₄ aryl group,
(2) a C₃₋₈ cycloalkyl group,
(3) a C₃₋₈ cycloalkenyl group or
(4) a heterocyclic group comprising 1 to 4 heteroatoms selected from oxygen atom, nitrogen atom and sulfur atom,
R⁵ and R⁶ are each independently
(1) a hydrogen atom,
(2) a halogen atom,
(3) a C₁₋₆ alkyl group optionally substituted by 1 to 3 substituents selected from the following group A,
(4) -OR¹¹⁶
(wherein R¹¹⁶ is a hydrogen atom or a group selected from the following group C) or
(5) -NR¹¹⁷R¹¹⁸
(wherein R¹¹⁷ and R¹¹⁸ are each independently a hydrogen atom, a C₁₋₆ alkanoyl group or a group selected from the following group C),
X is
(1) a hydrogen atom,
(2) a halogen atom,
(3) a cyano group,
(4) a nitro group,
(5) an amino group,
(6) a C₁₋₆ alkanoylamino group,
(7) a C₁₋₆ alkylsulfonyl group,
(8) a C₁₋₆ alkyl group optionally substituted by 1 to 3 substituents selected from the following group A,
(9) a C₂₋₆ alkenyl group optionally substituted by 1 to 3 substituents selected from the following group A,
(10) -COOR^{a9}
(wherein R^{a9} is a hydrogen atom or a C₁₋₆ alkyl group),
(11) -CONH- (CH₂)₁-R^{a10}
(wherein R^{a10} is a C₁₋₆ alkyl group optionally substituted by 1 to 3 substituents selected from the following group A, a C₁₋₆ alkoxycarbonyl group or a C₁₋₆ alkanoylamino group, and 1 is 0 or an integer of 1 to 6),
(12) -OR^{a11}
(wherein R^{a11} is a hydrogen atom or a C₁₋₆ alkyl group optionally substituted by 1 to 3 substituents selected from the following group A) or
(13) 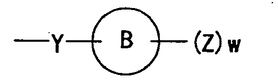
wherein ring B is
(1') a C₆₋₁₄ aryl group,
(2') a C₃₋₈ cycloalkyl group or
(3') a heterocyclic group comprising 1 to 4 heteroatoms selected from oxygen atom, nitrogen atom and sulfur atom,
each Z is independently
(1') a group selected from the following group D,
(2') a C₆₋₁₄ aryl group optionally substituted by 1 to 5 substituents selected from the following group D,
(3') a C₃₋₈ cycloalkyl group optionally substituted by 1 to 5 substituents selected from the following group D,
(4') a C₆₋₁₄ aryl C₁₋₆ alkyl group optionally substituted by 1 to 5 substituents selected from the following group D,
(5') a heterocyclic group optionally substituted by 1 to 5 substituents selected from the following group D
(wherein said heterocyclic group comprises 1 to 4 heteroatoms selected from oxygen atom, nitrogen atom and sulfur atom) or
(6') a heterocycle C₁₋₆ alkyl group optionally substituted by 1 to 5 substituents selected from the following group D
(wherein said heterocycle C₁₋₆ alkyl group is a C₁₋₆ alkyl group substituted by "a heterocyclic group optionally substituted by 1 to 5 substituents selected from group D" as defined above),
w is an integer of 1 to 3,
Y is
(a) C₁₋₆ alkylene,
(b) C₂₋₆ alkenylene or
(c) -Y¹-(CH₂)ₘ-Y²-(CH₂)ₙ-
(wherein m and n are each independently 0 or an integer of 1 to 6,
Y¹ and Y² are each independently
(1') a bond,
(2') -O-,
(3') -NR^{y1}-,
(4') -S-,
(5') -CO-,
(6') -SO-,
(7') -SO₂-,
(8') -CO₂-,
(9') -OCO-,
(10') -CONR^{y2}-,
(11') -NR^{y2}CO-,
(12') -SO₂NR^{y2}-,
(13') -NR^{y2}SO₂-,
(14') -NR^{y2}CO₂-,
(15') -OCONR^{y2}-,
(16') -NR^{y2}CONR^{y3}-,
(17') -CR^{y4}R^{y5}- or
(18') -CH=CH-
(wherein R^{y1} is
(1") a hydrogen atom,
(2") a group selected from the following group C,
(3") -COOR^{y11},
(4") -CONR^{y11}R^{y12},
(5") -COR^{y11} or
(6") -SO₂R^{y13}
(wherein R^{y11} and R^{y12} are each independently a hydrogen atom or a group selected from the following group C, and R^{y13} is a group selected from the following group C),
R^{y2} and R^{y3} are each independently
(1") a hydrogen atom,
(2") a group selected from the following group C,
(3") -COR^{y11} or
(4") -SO₂R^{y13}
(wherein R^{y11} and R^{y13} are as defined above) ,
R^{y4} and R^{y5} are each independently
(1") a hydrogen atom,
(2") a carboxyl group,
(3") a group selected from group F,
(4") -OR^{y14} or
(5") -NHR^{y15}
(wherein R^{y14} is a group selected from the following group C, and R^{y15} is a hydrogen atom, a C₁₋₆ alkyl group, a C₁₋₆ alkanoyl group or a C₆₋₁₄ aryl C₁₋₆ alkyloxycarbonyl group)))
group A;
(1) a halogen atom,
(2) a C₁₋₆ alkoxy C₁₋₆ alkoxy group,
(3) -OR^{a1},
(4) -SR^{a1},
(5) -NR^{a1}R^{a2},
(6) -COOR^{a1},
(7) -CONR^{a1}R^{a2},
(8) -SO₃H,
(9) -SO₂NR^{a1}R^{a2},
(10) -NHCOR^{a1} and
(11) -NHSO₂R^{a3}
(wherein R^{a1} and R^{a2} are each independently a hydrogen atom or a C₁₋₆ alkyl group, and R^{a3} is a C₁₋₆ alkyl group)
group B;
(1) a halogen atom,
(2) a cyano group,
(3) a nitro group,
(4) a C₁₋₆ alkyl group,
(5) a halogenated C₁₋₆ alkyl group,
(6) -(CH₂)ᵣ-OR^{b1},
(7) -(CH₂)ᵣ-SR^{b1},
(8) -(CH₂)ᵣ-NR^{b1}R^{b2},
(9) -(CH₂)ᵣ-COOR^{b1},
(10) -(CH₂)ᵣ-CONR^{b1}R^{b2},
(11) -(CH₂)ᵣ-COR^{b1},
(12) -(CH₂)ᵣ-NR^{b1}-COR^{b2},
(13) -(CH₂)ᵣ-NR^{b1}-SO₂R^{b3},
(14) -(CH₂)ᵣ-SO₂R^{b3},
(15) -(CH₂)ᵣ-SO₂NR^{b1}R^{b2},
(16) -(CH₂)ᵣ-CONR^{b1}-SO₂R^{b3},
(17) - (CH₂)ᵣ-SO₂NR^{b1}-COR^{b2},
(18) -(CH₂)ᵣ-NR^{b1}-COOR^{b3} and
(19) - (CH₂)ᵣ-NR^{b1}-CONR^{b2}R^{b4}
(wherein R^{b1}, R^{b2} and R^{b4} are each independently a hydrogen atom or a C₁₋₆ alkyl group, R^{b3} is a C₁₋₆ alkyl group, and r is 0 or an integer of 1 to 6)
group C:
(1) a C₁₋₆ alkyl group optionally substituted by 1 to 3 substituents selected from the aforementioned group A,
(2) a C₆₋₁₄ aryl group optionally substituted by 1 to 5 substituents selected from the aforementioned group B,
(3) a C₆₋₁₄ aryl C₁₋₆ alkyl group optionally substituted by 1 to 5 substituents selected from the aforementioned group B,
(4) a heterocyclic group optionally substituted by 1 to 5 substituents selected from the aforementioned group B and
(5) a heterocycle C₁₋₆ alkyl group optionally substituted by 1 to 5 substituents selected from the aforementioned group B
group D:
(a) a hydrogen atom,
(b) a halogen atom,
(c) a cyano group,
(d) a nitro group,
(e) a C₁₋₆ alkyl group optionally substituted by 1 to 3 substituents selected from the aforementioned group A,
(f) -(CH₂)ₜ-OR^{d1},
wherein R^{d1} is
(1) a hydrogen atom,
(2) a group selected from the following group F,
(3) a C₂₋₆ alkenyl group optionally substituted by 1 to 3 substituents selected from the aforementioned group A or
(4) a C₂₋₆ alkynyl group optionally substituted by 1 to 3 substituents selected from the aforementioned group A, hereinafter each t is independently 0 or an integer of 1 to 6,
(g) -(CH₂)ₜ-S(O)_{q}-R^{d2},
wherein R^{d2} is
(1) a hydrogen atom or
(2) a group selected from the following group F,
q is 0, 1, 2 or 3,
(h) -(CH₂)ₜ-NR^{d3}R^{d4},
wherein R^{d3} and R^{d4} are each independently,
(1) a hydrogen atom or
(2) a group selected from the following group F,
(i) -(CH₂)ₜ-COOR^{d5},
wherein R^{d5} is
(1) a hydrogen atom or
(2) a group selected from the following group F,
(j) -(CH₂)ₜ-CONR^{d6}R^{d7},
wherein R^{d6} and R^{d7} are each independently
(1) a hydrogen atom,
(2) a hydroxyl group,
(3) a group selected from the following group F or
(4) a C₁₋₆ alkoxy group,
(k) -(CH₂)ₜ-COR^{d8},
wherein R^{d8} is
(1) a hydrogen atom or
(2) a group selected from the following group F,
(1) -(CH₂)ₜ-NR^{d9}CO-R^{d10},
wherein R^{d9} is
(1) a hydrogen atom,
(2) a C₁₋₆ alkyl group optionally substituted by 1 to 3 substituents selected from the aforementioned group A or
(3) a C₁₋₆ alkanoyl group,
R^{d10} is
(1) a hydrogen atom,
(2) an amino group,
(3) a C₁₋₆ alkylamino group or
(4) a group selected from the following group F,
(m) -(CH₂)ₜ-NR^{d11}SO₂-R^{d12},
wherein R^{d11} is
(1) a hydrogen atom,
(2) a C₁₋₆ alkyl group optionally substituted by 1 to 3 substituents selected from the aforementioned group A or
(3) a C₁₋₆ alkanoyl group,
R^{d12} is
(1) a hydrogen atom or
(2) a group selected from the following group F,
(n) -(CH₂)ₜ-SO₂-NR^{d13}R^{d14},
wherein R^{d13} and R^{d14} are each independently
(1) a hydrogen atom or
(2) a group selected from the following group F,
(o) -(CH₂)ₜ-CONR^{d15}-SO₂R^{d16},
wherein R^{d15} and R^{d16} are each independently
(1) a hydrogen atom or
(2) a group selected from the following group F,
(p) -(CH₂)ₜ-SO₂NR^{d17}-COR^{d18},
wherein R^{d17} is
(1) a hydrogen atom or
(2) a group selected from the following group F,
R^{d18} is a group selected from the following group F,
(q) -(CH₂)ₜ-NR^{d19}-COOR^{d20},
wherein R^{d19} and R^{d20} are each independently
(1) a hydrogen atom or
(2) a group selected from the following group F,
(r) -(CH₂)ₜ-NR^{d21}-CONR^{d22}R^{d23},
wherein R^{d21}, R^{d22} and R^{d23} are each independently
(1) a hydrogen atom or
(2) a group selected from the following group F,
(s) -(CH₂)ₜ-C(=NR^{d24})NH₂,
wherein R^{d24} is
(1) a hydrogen atom,
(2) a hydroxyl group,
(3) a C₁₋₆ alkyl group optionally substituted by 1 to 3 substituents selected from the aforementioned group A or
(4) a C₁₋₆ alkoxy group,
(t) -(CH₂)ₜ-O-(CH₂)ₚ-COR^{d25},
wherein R^{d25} is
(1) an amino group,
(2) a C₁₋₆ alkylamino group or
(3) a heterocyclic group optionally substituted by 1 to 5 substituents selected from the aforementioned group B,
p is 0 or an integer of 1 to 6, and
(u) a heterocyclic group optionally substituted by 1 to 5 substituents selected from the aforementioned group B (wherein said heterocyclic group comprises 1 to 4 heteroatoms selected from oxygen atom, nitrogen atom and sulfur atom)
group E:
(a) a halogen atom,
(b) a cyano group,
(c) a nitro group,
(d) -OR^{e1},
wherein R^{e1} is
(1) a hydrogen atom,
(2) a group selected from the following group F,
(3) a C₂₋₆ alkenyl group optionally substituted by 1 to 3 substituents selected from the aforementioned group A or
(4) a C₂₋₆ alkynyl group optionally substituted by 1 to 3 substituents selected from the aforementioned group A,
(e) -S(O)_{q}-R^{e2}
wherein R^{e2} is
(1) a hydrogen atom or
(2) a group selected from the following group F,
q is 0, 1, 2 or 3,
(f) -NR^{e3}R^{e4},
wherein R^{e3} and R^{e4} are each independently
(1) a hydrogen atom or
(2) a group selected from the following group F,
(g) -COOR^{e5},
wherein R^{e5} is
(1) a hydrogen atom or
(2) a group selected from the following group F,
(h) -CONR^{e6}R^{e7},
wherein R^{e6} and R^{e7} are each independently
(1) a hydrogen atom,
(2) a hydroxyl group,
(3) a group selected from the following group F or
(4) a C₁₋₆ alkoxy group,
(i) -COR^{e8},
wherein R^{e8} is
(1) a hydrogen atom or
(2) a group selected from the following group F,
(j) -NR^{e9}CO-R^{e10},
whrein R^{e9} is
(1) a hydrogen atom,
(2) a C₁₋₆ alkyl group or
(3) a C₁₋₆ alkanoyl group,
R^{e10} is
(1) a hydrogen atom,
(2) an amino group,
(3) a C₁₋₆ alkylamino group or
(4) a group selected from the following group F,
(k) -NR^{e11}SO₂-R^{e12},
wherein R^{e11} is
(1) a hydrogen atom,
(2) a C₁₋₆ alkyl group or
(3) a C₁₋₆ alkanoyl group,
R^{e12} is
(1) a hydrogen atom or
(2) a group selected from the following group F,
(1) -SO₂-NR^{e13}R^{e14},
wherein R^{e13} and R^{e14} are each independently
(1) a hydrogen atom or
(2) a group selected from the following group F,
(m) -CONR^{e15}-SO₂R^{e16},
wherein R^{e15} and R^{e16} are each independently
(1) a hydrogen atom or
(2) a group selected from the following group F,
(n) -SO₂NR^{e17}-COR^{e18},
wherein R^{e17} is
(1) a hydrogen atom or
(2) a group selected from the following group F,
R^{e18} is a group selected from the following group F,
(o) -NR^{e19}-COOR^{e20},
whrein R^{e19} and R^{e20} are each independently
(1) a hydrogen atom or
(2) a group selected from the following group F,
(p) -NR^{e21}-CONR^{e22}R^{e23}
wherein R^{e21}, R^{e22} and R^{e23} are each independently
(1) a hydrogen atom or
(2) a group selected from the following group F,
(q) a C₆₋₁₄ aryl group optionally substituted by 1 to 5 substituents selected from the aforementioned group B and
(r) a heterocyclic group optionally substituted by 1 to 5 substituents selected from the aforementioned group B (wherein said heterocyclic group comprises 1 to 4 heteroatoms selected from oxygen atom, nitrogen atom and sulfur atom)
group F:
(1) a C₁₋₆ alkyl group optionally substituted by 1 to 3 substituents selected from the aforementioned group A,
(2) a C₆₋₁₄ aryl group optionally substituted by 1 to. 5 substituents selected from the aforementioned group B,
(3) a heterocyclic group optionally substituted by 1 to 5 substituents selected from the aforementioned group B
(wherein said heterocyclic group comprises 1 to 4 heteroatoms selected from oxygen atom, nitrogen atom and sulfur atom),
(4) a C₃₋₈ cycloalkyl group optionally substituted by 1 to 5 substituents selected from the aforementioned group B,
(5) a C₆₋₁₄ aryl C₁₋₆ alkyl group optionally substituted by 1 to 5 substituents selected from the aforementioned group B,
(6) a heterocycle C₁₋₆ alkyl group optionally substituted by 1 to 5 substituents selected from the aforementioned group B
(wherein said heterocycle C₁₋₆ alkyl group is a C₁₋₆ alkyl group substituted by "a heterocyclic group optionally substituted by 1 to 5 substituents selected from group B" as defined above),
and,
(7) a C₃₋₈ cycloalkyl C₁₋₆ alkyl group optionally substituted by 1 to 5 substituents selected from the aforementioned group B.

2. The fused ring compound of claim 1, wherein 1 to 4 of G¹, G², G³ and G⁴ is a nitrogen atom, or a pharmaceutically acceptable salt thereof.

3. The fused ring compound of claim 2, wherein at least one of G¹ and G² is a nitrogen atom, or a pharmaceutically acceptable salt thereof.

4. The fused ring compound of claim 1, wherein at least one of G¹ and G² is a heteroatom, and at least one of G³ and G⁴ is a heteroatom, or a pharmaceutically acceptable salt thereof.

5. The fused ring compound of claim 1, wherein, in the formula [I], the moiety 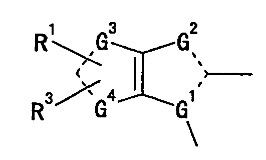 is a fused ring selected from the group consisting of 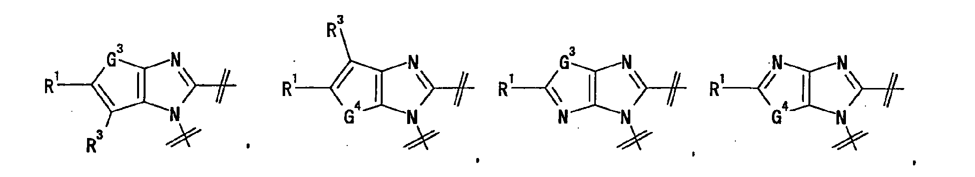 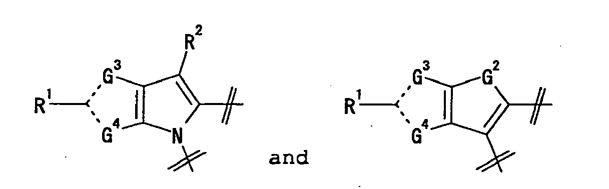 or a pharmaceutically acceptable salt thereof.

6. The fused ring compound of claim 5, wherein, in the formula [I], the moiety 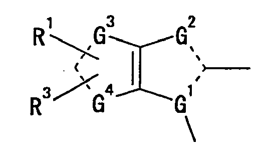 is a fused ring selected from the group consisting of 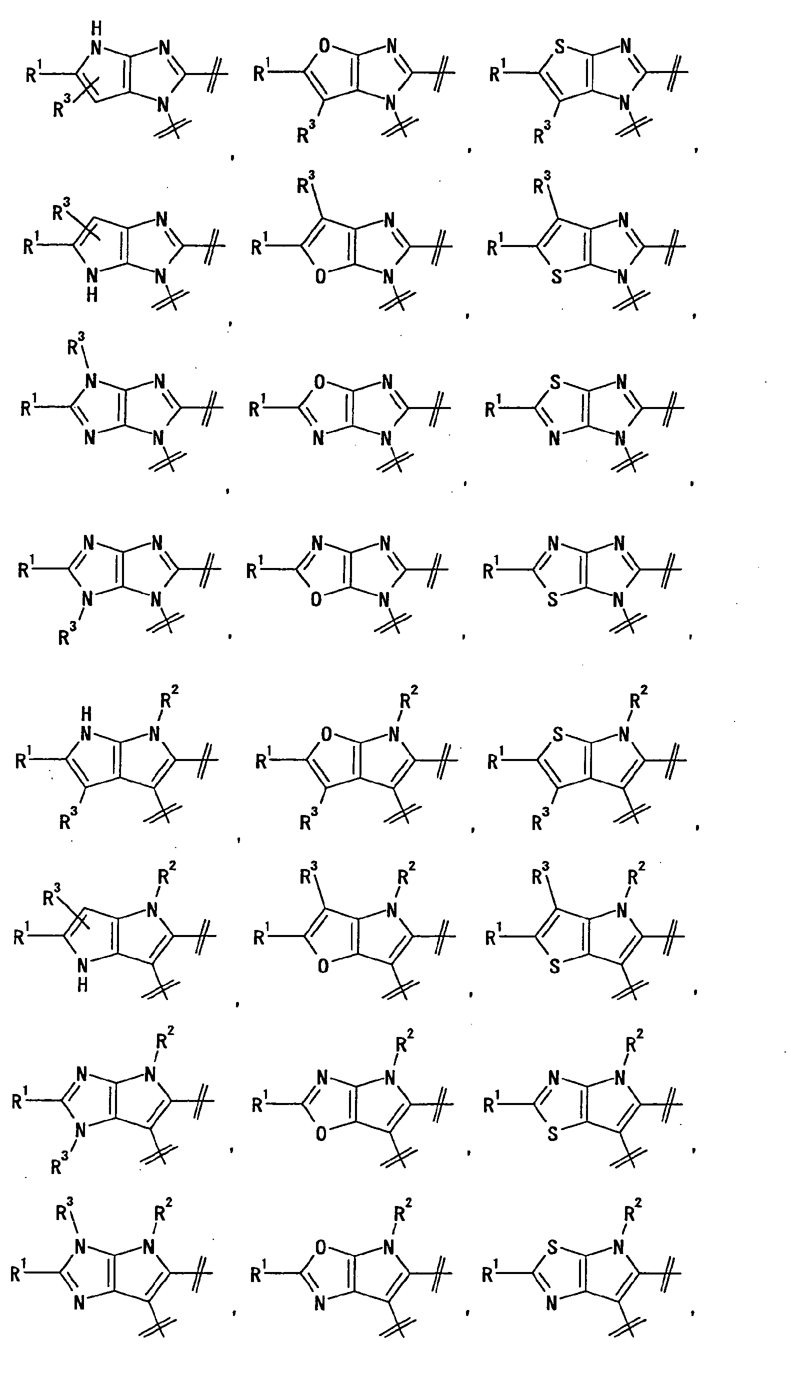 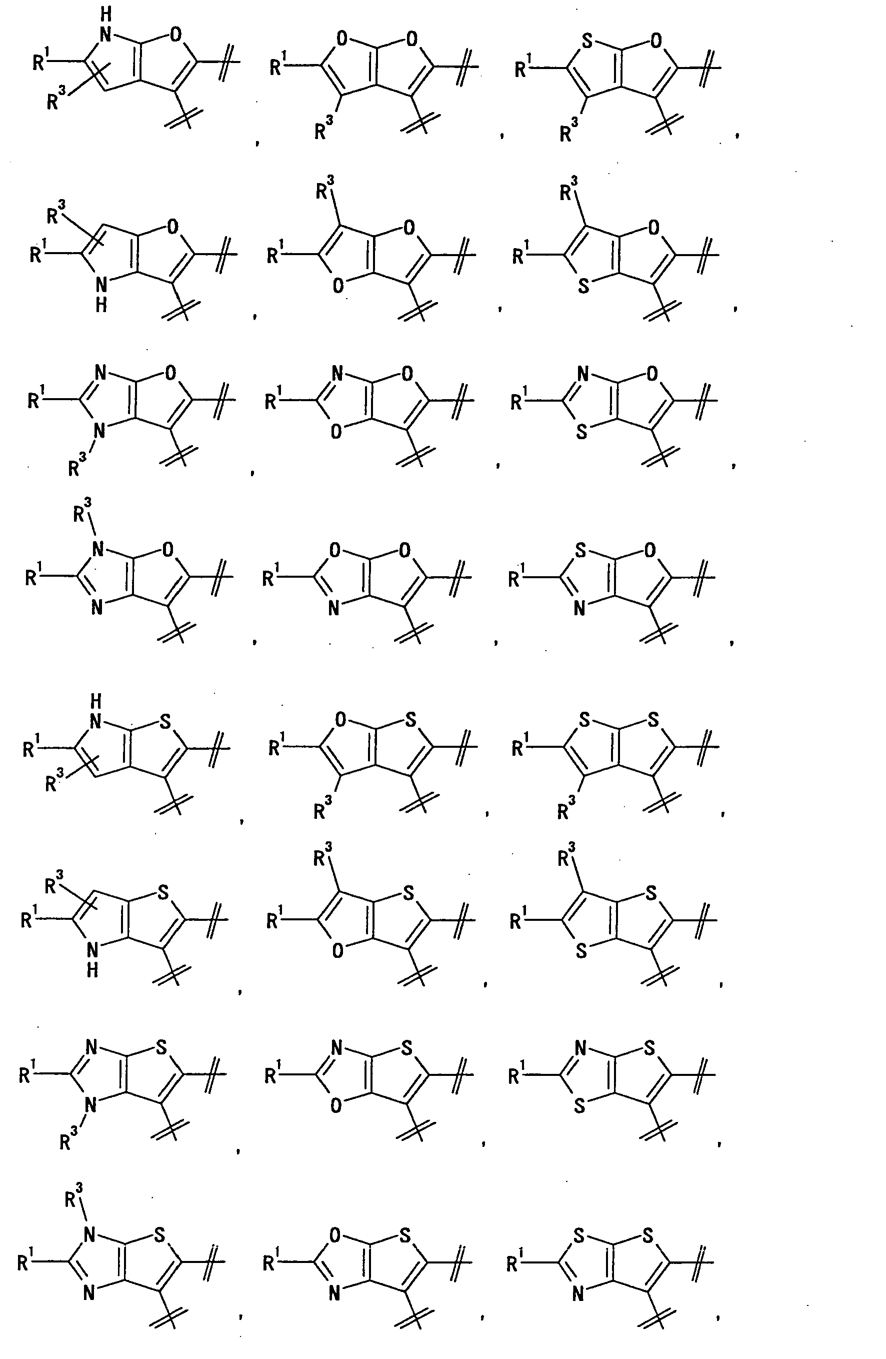 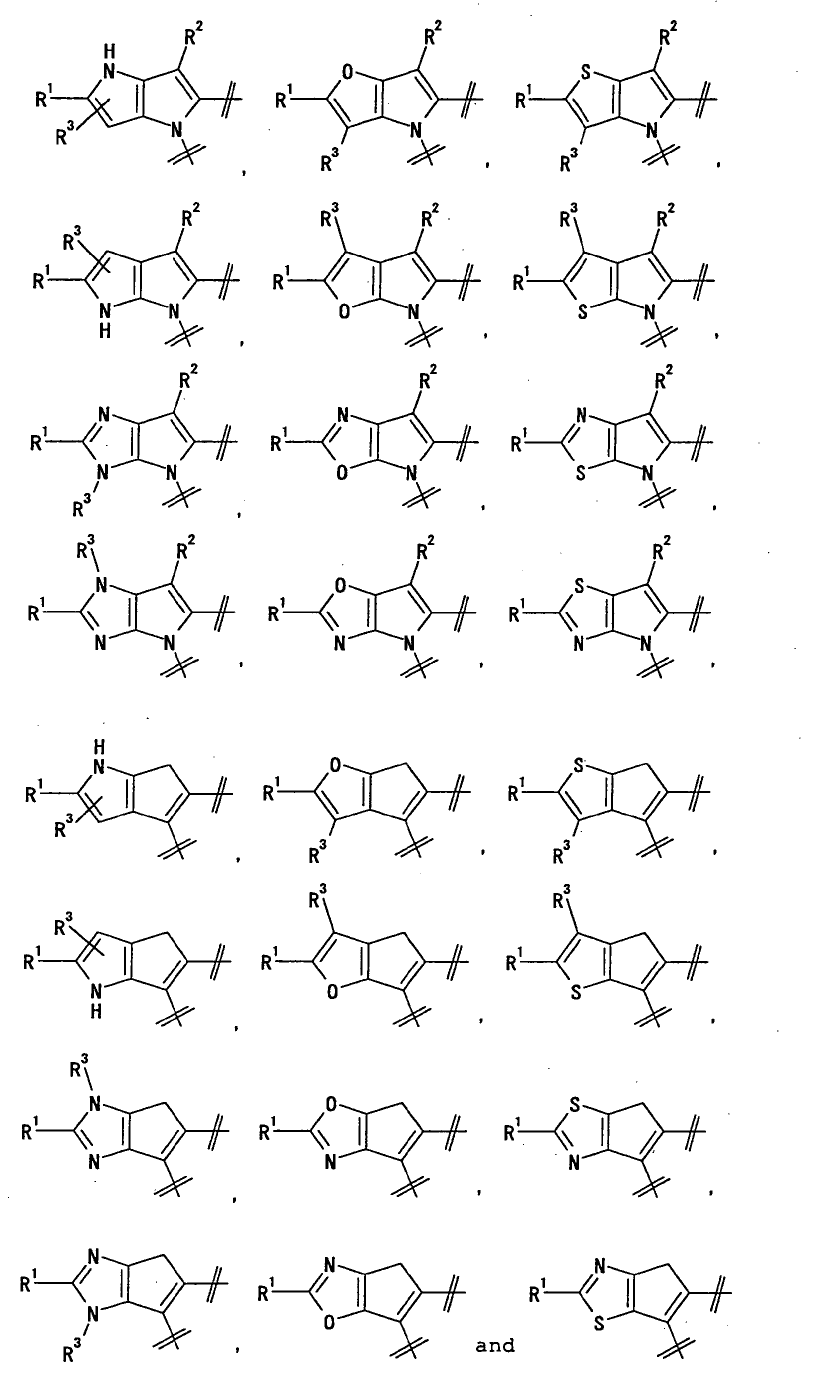 or a pharmaceutically acceptable salt thereof.

7. The fused ring compound of claim 6, wherein, in the formula [I], the moiety 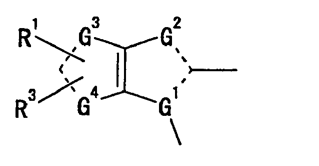 is a fused ring selected from the group consisting of 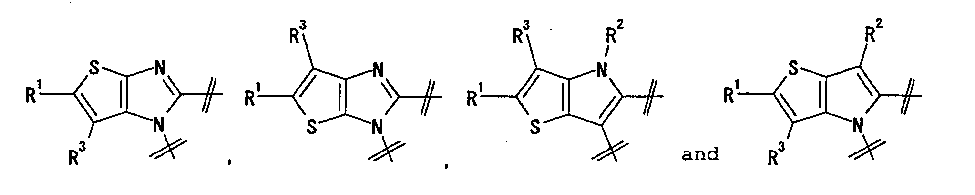 or a pharmaceutically acceptable salt thereof.

8. The fused ring compound of claim 7, which is represented by the following formula [I-1]: 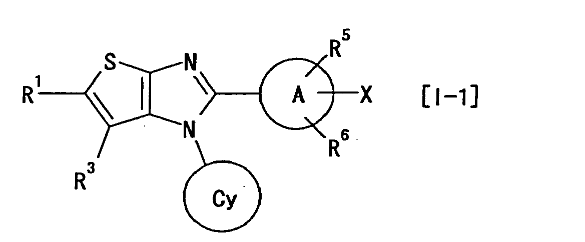 wherein each symbol is as defined in claim 1,
or a pharmaceutically acceptable salt thereof.

9. The fused ring compound of claim 7, which is represented by the following formula [I-2]: 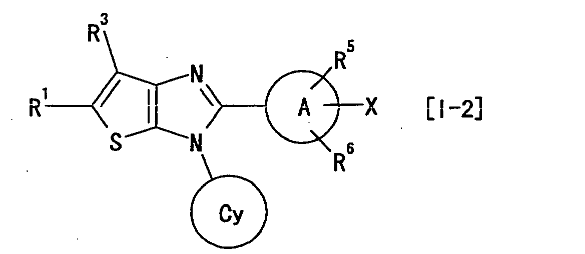 wherein each symbol is as defined in claim 1,
or a pharmaceutically acceptable salt thereof.

10. The fused ring compound of claim 7, which is represented by the following formula [I-3]: 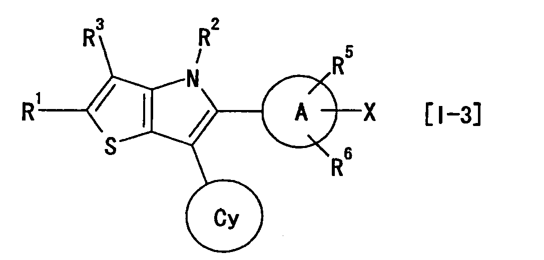 wherein each symbol is as defined in claim 1,
or a pharmaceutically acceptable salt thereof.

11. The fused ring compound of claim 7, which is represented by the following formula [I-4]: 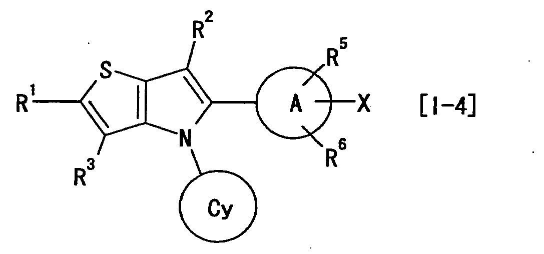 wherein each symbol is as defined in claim 1,
or a pharmaceutically acceptable salt thereof.

12. The fused ring compound of claim 1, wherein R¹ is a carboxyl group, or a pharmaceutically acceptable salt thereof.

13. The fused ring compound of claim 1, wherein R² is
(1) a hydrogen atom,
(2) a C₁₋₆ alkyl group optionally substituted by 1 to 3 substituents selected from group E or
(3) 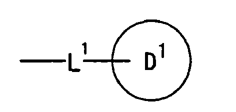
wherein L¹ and ring D¹ are as defined in claim 1, or a pharmaceutically acceptable salt thereof.

14. The fused ring compound of claim 13, wherein R² is a C₁₋₆ alkyl group optionally substituted by 1 to 3 substituents selected from group E, or a pharmaceutically acceptable salt thereof.

15. The fused ring compound of claim 13, wherein R² is 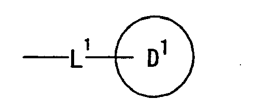 wherein L¹ and ring D¹ are as defined in claim 1, or a pharmaceutically acceptable salt thereof.

16. The fused ring compound of claim 1, wherein R³ is a hydrogen atom, or a pharmaceutically acceptable salt thereof.

17. The fused ring compound of claim 1, wherein ring Cy is a C₃₋₈ cycloalkyl group or a C₃₋₈ cycloalkenyl group, or a pharmaceutically acceptable salt thereof.

18. The fused ring compound of claim 17, wherein ring Cy is a C₃₋₈ cycloalkyl group, or a pharmaceutically acceptable salt thereof.

19. The fused ring compound of claim 1, wherein ring A is a C₆₋₁₄ aryl group, or a pharmaceutically acceptable salt thereof.

20. The fused ring compound of claim 1, wherein R⁵ and R⁶ are each independently a hydrogen atom or a halogen atom, or a pharmaceutically acceptable salt thereof.

21. The fused ring compound of claim 20, wherein R⁵ and R⁶ are each a hydrogen atom, or a pharmaceutically acceptable salt thereof.

22. The fused ring compound of claim 1, wherein X is a hydrogen atom, a halogen atom, a C₁₋₆ alkyl group optionally substituted by 1 to 3 substituents selected from group A or -OR^{a11} (wherein R^{a11} is a hydrogen atom or a C₁₋₆ alkyl group optionally substituted by 1 to 3 substituents selected from group A), or a pharmaceutically acceptable salt thereof.

23. The fused ring compound of claim 22, wherein X is -OR^{a11}, or a pharmaceutically acceptable salt thereof.

24. The fused ring compound of claim 1, wherein X is 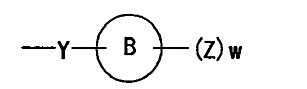 wherein each symbol is as defined in claim 1, or a pharmaceutically acceptable salt thereof.

25. The fused ring compound of claim 24, wherein Y is -(CH₂)ₘ-O-(CH₂)ₙ- wherein each symbol is as defined in claim 1, or a pharmaceutically acceptable salt thereof.

26. The fused ring compound of claim 24, wherein ring B is a C₆₋₁₄ aryl group, or a pharmaceutically acceptable salt thereof.

27. The fused ring compound of claim 24, wherein Z shows 1 to 3 substituents selected from
(1) a hydrogen atom,
(2) a C₆₋₁₄ aryl group optionally substituted by 1 to 5 substituents selected from group D,
(3) a heterocyclic group optionally substituted by 1 to 5 substituents selected from group D,
(4) -(CH₂)ₜ-S(O)_{q}-R^{d2},
(5) -(CH₂)ₜ-NR^{d3}R^{d4} and
(6) -(CH₂)ₜ-NR^{d9}CO-R^{d10}
wherein each symbol is as defined in claim 1, or a pharmaceutically acceptable salt thereof.

28. The fused ring compound of claim 27, wherein at least one of Z is a substituent selected from
a C₆₋₁₄ aryl group optionally substituted by 1 to 5 substituents selected from group D and
a heterocyclic group optionally substituted by 1 to 5 substituents selected from group D, or a pharmaceutically acceptable salt thereof.

29. The fused ring compound of claim 1 or a pharmaceutically acceptable salt thereof, which is selected from the group consisting of
methyl 3-cyclohexyl-2-(4-hydroxyphenyl)-3H-thieno[2,3-d]imidazole-5-carboxylate,
methyl 2-{4-[4'-chloro-4-(2-oxopyrrolidin-1-yl)-biphenyl-2-ylmethoxy]phenyl}-3-cyclohexyl-3H-thieno[2,3-d]imidazole-5-carboxylate,
2-{4-[4'-chloro-4-(2-oxopyrrolidin-1-yl)biphenyl-2-ylmethoxy]phenyl}-3-cyclohexyl-3H-thieno[2,3-d]imidazole-5-carboxylic acid hydrochloride,
2-(4-benzyloxyphenyl)-3-cyclohexyl-3H-thieno[2,3-d]imidazole-5-carboxylic acid hydrochloride,
3-cyclohexyl-2-{4-[2-morpholino-5-(2-oxopyrrolidin-1-yl)benzyloxy]phenyl}-3H-thieno[2,3-d]imidazole-5-carboxylic acid,
methyl 2-(4-benzyloxy-2-fluorophenyl)-1-cyclohex-2-enyl-1H-thieno[2,3-d]imidazole-5-carboxylate,
2-(4-benzyloxy-2-fluorophenyl)-1-cyclohex-2-enyl-1H-thieno[2,3-d]imidazole-5-carboxylic acid hydrochloride,
5-(4-benzyloxyphenyl)-4,6-dicyclohexyl-4H-thieno[3,2-b]pyrrole-2-carboxylic acid,
ethyl 5-(4-benzyloxyphenyl)-6-cyclohex-2-enyl-4H-thieno[3,2-b]pyrrole-2-carboxylate,
ethyl 5-(4-benzyloxyphenyl)-6-cyclohex-2-enyl-4-methyl-4H-thieno[3,2-b]pyrrole-2-carboxylate,
ethyl 5-(4-benzyloxyphenyl)-6-cyclohexyl-4-methyl-4H-thieno[3,2-b]pyrrole-2-carboxylate,
5-(4-benzyloxyphenyl)-6-cyclohexyl-4-methyl-4H-thieno[3,2-b]pyrrole-2-carboxylic acid,
ethyl 6-cyclohexyl-5-(4-hydroxyphenyl)-4-methyl-4H-thieno[3,2-b]pyrrole-2-carboxylate,
5-(4-benzyloxyphenyl)-6-cyclohexyl-4-dimethylcarbamoylmethyl-4H-thieno[3,2-b]pyrrole-2-carboxylic acid,
6-cyclohexyl-4-methyl-5-{4-[2-morpholino-5-(2-oxopyrrolidin-1-yl)benzyloxy]phenyl}-4H-thieno[3,2-b]pyrrole-2-carboxylic acid hydrochloride,
ethyl 5-{4-[5-(N-acetyl-N-methylamino)-2-morpholinobenzyloxy]phenyl}-6-cyclohexyl-4-methyl-4H-thieno[3,2-b]pyrrole-2-carboxylate,
5-{4-[5-(N-acetyl-N-methylamino)-2-morpholinobenzyloxy]phenyl}-6-cyclohexyl-4-methyl-4H-thieno[3,2-b]pyrrole-2-carboxylic acid hydrochloride,
6-cyclohexyl-5-{4-[2-(4-methanesulfonylpiperazin-1-yl)-5-(2-oxopyrrolidin-1-yl)benzyloxy]phenyl}-4-methyl-4H-thieno[3,2-b]pyrrole-2-carboxylic acid hydrochloride,
5-{4-[5-amino-2-(4-methanesulfonylpiperazin-1-yl)benzyloxy]phenyl}-6-cyclohexyl-4-methyl-4H-thieno[3,2-b]pyrrole-2-carboxylic acid hydrochloride,
5-{4-[5-(N-acetyl-N-methylamino)-2-(4-methanesulfonylpiperazin-1-yl)benzyloxy]phenyl}-6-cyclohexyl-4-methyl-4H-thieno[3,2-b]pyrrole-2-carboxylic acid hydrochloride,
6-cyclohexyl-4-dimethylcarbamoylmethyl-5-(4-methoxyphenyl)-4H-thieno[3,2-b]pyrrole-2-carboxylic acid,
6-cyclohexyl-5-[4-(5-methanesulfonyl-2-morpholinobenzyloxy)phenyl]-4-methyl-4H-thieno[3,2-b]pyrrole-2-carboxylic acid hydrochloride,
ethyl 6-cyclohexyl-4-methyl-5-{4-[2-morpholino-5-(2-oxopyrrolidin-1-yl)benzyloxy]phenyl}-4H-thieno[3,2-b]pyrrole-2-carboxylate hydrochloride,
6-cyclohexyl-5-(4-methoxyphenyl)-4-morpholinocarbonylmethyl-4H-thieno[3,2-b]pyrrole-2-carboxylic acid,
6-cyclohexyl-4-(4-ethylpiperazin-1-yl)carbonylmethyl-5-(4-methoxyphenyl)-4H-thieno[3,2-b]pyrrole-2-carboxylic acid,
6-cyclohexyl-4-(4-dimethylaminopiperidino)carbonylmethyl-5-(4-methoxyphenyl)-4H-thieno[3,2-b]pyrrole-2-carboxylic acid,
6-cyclohexyl-5-[4-(5-isobutyrylamino-2-morpholinobenzyloxy)phenyl]-4-methyl-4H-thieno[3,2-b]pyrrole-2-carboxylic acid hydrochloride,
6-cyclohexyl-5-{4-[5-(N-isobutyryl-N-methylamino)-2-morpholinobenzyloxy]phenyl}-4-methyl-4H-thieno[3,2-b]pyrrole-2-carboxylic acid hydrochloride,
6-cyclohexyl-4-methyl-5-[4-(2-morpholinobenzyloxy)phenyl]-4H-thieno[3,2-b]pyrrole-2-carboxylic acid hydrochloride,
4-(benzylcarbamoylmethyl)-6-cyclohexyl-5-(4-methoxyphenyl)-4H-thieno[3,2-b]pyrrole-2-carboxylic acid,
4-(tert-butylcarbamoylmethyl)-6-cyclohexyl-5-(4-methoxyphenyl)-4H-thieno[3,2-b]pyrrole-2-carboxylic acid,
6-cyclohexyl-4-methyl-5-{4-[2-morpholino-4-(2-oxopyrrolidin-1-yl)benzyloxy]phenyl}-4H-thieno[3,2-b]pyrrole-2-carboxylic acid hydrochloride,
5-(2-benzyloxyphenyl)-6-cyclohexyl-4-methyl-4H-thieno[3,2-b]pyrrole-2-carboxylic acid,
5-(2-benzyloxyphenyl)-6-cyclohexyl-4-dimethylcarbamoylmethyl-4H-thieno[3,2-b]pyrrole-2-carboxylic acid,
5-[4-(1-tert-butoxycarbonylpiperidin-3-yloxy)phenyl]-6-cyclohexyl-4-dimethylcarbamoylmethyl-4H-thieno[3,2-b]pyrrole-2-carboxylic acid,
6-cyclohexyl-4-dimethylcarbamoylmethyl-5-[4-(2-morpholinobenzyloxy)phenyl]-4H-thieno[3,2-b]pyrrole-2-carboxylic acid,
5-[4-(1-tert-butoxycarbonylpiperidin-4-yloxy)phenyl]-6-cyclohexyl-4-dimethylcarbamoylmethyl-4H-thieno[3,2-b]pyrrole-2-carboxylic acid,
6-cyclohexyl-5-{4-[5-(2-dimethylaminoacetylamino)-2-morpholinobenzyloxy]phenyl}-4-methyl-4H-thieno[3,2-b]pyrrole-2-carboxylic acid dihydrochloride,
6-cyclohexyl-4-methyl-5-{4-[2-morpholino-5-(2-morpholinoacetylamino) benzyloxy]phenyl}-4H-thieno[3,2-b]pyrrole-2-carboxylic acid dihydrochloride,
6-cyclohexyl-4-dimethylcarbamoylmethyl-5-(4-phenoxyphenyl)-4H-thieno[3,2-b]pyrrole-2-carboxylic acid,
6-cyclohexyl-5-(4-{5-[N-(2-dimethylaminoacetyl)-N-methylamino]-2-morpholinobenzyloxy}phenyl)-4-methyl-4H-thieno[3,2-b]pyrrole-2-carboxylic acid dihydrochloride,
5-(4-benzyloxyphenyl)-4-(tert-butylcarbamoylmethyl)-6-cyclohexyl-4H-thieno[3,2-b]pyrrole-2-carboxylic acid,
4-benzyl-5-(4-benzyloxyphenyl)-6-cyclohexyl-4H-thieno[3,2-b]pyrrole-2-carboxylic acid,
5-(4-benzyloxyphenyl)-6-cyclohexyl-4-(2-dimethylaminoethyl)-4H-thieno[3,2-b]pyrrole-2-carboxylic acid,
5-(4-benzyloxyphenyl)-6-cyclohexyl-4-(2-morpholinoethyl)-4H-thieno[3,2-b]pyrrole-2-carboxylic acid,
5-(4-benzyloxyphenyl)-6-cyclohexyl-4-cyclohexylmethyl-4H-thieno[3,2-b]pyrrole-2-carboxylic acid,
5-(4-benzyloxyphenyl)-6-cyclohexyl-4-(2-methoxyethyl)-4H-thieno[3,2-b]pyrrole-2-carboxylic acid,
5-(4-benzyloxyphenyl)-6-cyclohexyl-4-{[N-methyl-N-(2-oxo-2-piperidinoethyl)carbamoyl]methyl}-4H-thieno[3,2-b]pyrrole-2-carboxylic acid,
5-(4-benzyloxyphenyl)-6-cyclohexyl-4-[(4-morpholinophenylcarbamoyl)methyl]-4H-thieno[3,2-b]pyrrole-2-carboxylic acid hydrochloride,
5-(4-benzyloxyphenyl)-6-cyclohexyl-4-[2-(4-cyclohexylpiperazin-1-yl)-2-oxoethyl]-4H-thieno[3,2-b]pyrrole-2-carboxylic acid hydrochloride,
5-(4-benzyloxyphenyl)-6-cyclohexyl-4-[2-(4-cyclohexylpiperazin-1-yl)ethyl]-4H-thieno[3,2-b]pyrrole-2-carboxylic acid dihydrochloride,
5-(4-benzyloxyphenyl)-4-[2-(1,4'-bipiperidinyl-1'-yl)-2-oxoethyl]-6-cyclohexyl-4H-thieno[3,2-b]pyrrole-2-carboxylic acid hydrochloride,
5-(4-benzyloxyphenyl)-4-[2-(1,4'-bipiperidinyl-1'-yl)ethyl]-6-cyclohexyl-4H-thieno[3,2-b]pyrrole-2-carboxylic acid dihydrochloride,
6-cyclohexyl-4-(2-dimethylaminoethyl)-5-[4-(5-methanesulfonyl-2-morpholinobenzyloxy)phenyl]-4H-thieno[3,2-b]pyrrole-2-carboxylic acid dihydrochloride,
6-cyclohexyl-4-methyl-5-(4-{5-[N-methyl-N-(2-morpholinoacetyl)amino]-2-morpholinobenzyloxy}phenyl)-4H-thieno[3,2-b]pyrrole-2-carboxylic acid dihydrochloride,
5-(4-benzyloxyphenyl)-6-cyclohexyl-4-{2-[N-methyl-N-(2-morpholinoethyl)amino]ethyl}-4H-thieno[3,2-b]pyrrole-2-carboxylic acid dihydrochloride,
5-(4-benzyloxyphenyl)-6-cyclohexyl-4-{2-[N-methyl-N-(2-piperidinoethyl)amino]ethyl}-4H-thieno[3,2-b]pyrrole-2-carboxylic acid dihydrochloride, and
5-(4-benzyloxyphenyl)-6-cyclohexyl-4-{1-[N-methyl-N-(4-morpholinophenyl)carbamoyl]ethyl}-4H-thieno[3,2-b]pyrrole-2-carboxylic acid hydrochloride.

30. A pharmaceutical composition comprising a fused ring compound of any of claims 1 to 29, or a pharmaceutically acceptable salt thereof, and a pharmaceutically acceptable carrier.

31. A hepatitis C virus polymerase inhibitor comprising a fused ring compound of any of claims 1 to 29 or a pharmaceutically acceptable salt thereof as an active ingredient.

32. An anti-hepatitis C virus agent comprising a fused ring compound of any of claims 1 to 29 or a pharmaceutically acceptable salt thereof as an active ingredient.

33. A therapeutic agent for hepatitis C comprising a fused ring compound of any of claims 1 to 29 or a pharmaceutically acceptable salt thereof as an active ingredient.

34. A therapeutic agent for hepatitis C comprising (a) the hepatitis C virus polymerase inhibitor of claim 31, and (b) at least one pharmaceutical agent selected from the group consisting of a different antiviral agent, an antiinflammatory agent and an immunostimulant.

35. A therapeutic agent for hepatitis C comprising (a) the hepatitis C virus polymerase inhibitor of claim 31, and (b) interferon.

36. An anti-hepatitis C virus agent comprising (a) the anti-hepatitis C virus agent of claim 32, and (b) at least one agent selected from the group consisting of a different antiviral agent, an antiinflammatory agent and an immunostimulant.

37. An anti-hepatitis C virus agent comprising (a) the anti-hepatitis C virus agent of claim 32, and (b) interferon.

38. A pharmaceutical composition comprising (a) a fused ring compound of any of claims 1 to 29 or a pharmaceutically acceptable salt thereof, and (b) at least one pharmaceutical agent selected from the group consisting of a different antiviral agent, an antiinflammatory agent and an immunostimulant.

39. A pharmaceutical composition comprising (a) a fused ring compound of any of claims 1 to 29 or a pharmaceutically acceptable salt thereof, and (b) interferon.

40. Use of a fused ring compound of any of claims 1 to 29 or a pharmaceutically acceptable salt thereof for the production of a pharmaceutical agent for treating hepatitis C.

41. Use of a fused ring compound of any of claims 1 to 29 or a pharmaceutically acceptable salt thereof for the production of a hepatitis C virus polymerase inhibitor.

42. A method for treating hepatitis C, which comprises administering an effective amount of a fused ring compound of any of claims 1 to 29 or a pharmaceutically acceptable salt thereof to a mammal.

43. The method of claim 42, further comprising administering an effective amount of at least one pharmaceutical agent selected from the group consisting of a different antiviral agent, an antiinflammatory agent and an immunostimulant to the mammal.

44. The method of claim 42, further comprising administering an effective amount of interferon to the mammal.

45. A method for inhibiting hepatitis C virus polymerase, which comprises administering an effective amount of a fused ring compound of any of claims 1 to 29 or a pharmaceutically acceptable salt thereof to a mammal.

46. The method of claim 45, further comprising administering an effective amount of at least one pharmaceutical agent selected from the group consisting of a different antiviral agent, an antiinflammatory agent and an immunostimulant to the mammal.

47. The method of claim 45, further comprising administering an effective amount of interferon to the mammal.
